# EUROPEAN PATENT APPLICATION

(11) **EP 1 293 213 A1**
(43) Date of publication of application: **19.03.2003**
(21) Application number: 01904402.3
(22) Date of filing: 13.02.2001
(51) Int. Cl.: A61K 45/00, A61K 31/18, A61P 5/42, A61P 25/18

(54) **PREVENTIVES/REMEDIES FOR POSTOPERATIVE STRESS**

(30) Priority: 14.02.2000 JP 2000035963
(71) Applicant: Japan Tobacco Inc., Tokyo 105-8422 (JP)
(72) Inventor: MIYAZAKI, Akira, Japan Tobacco Inc., Takatsuki-shi, Osaka 569-1125 (JP); HAMADA, Atsushi, Jpan Tobacco Inc., Takatsuki-shi, Osaka 569-1125 (JP); SATO, Motohide, Japan Tobacco Inc., Takatsuki-shi, Osaka 569-1125 (JP); HARADA, Kazuhito, Japan Tobacco Inc., Takatsuki-shi, Osaka 569-1125 (JP)
(74) Representative: Best, Michael, Dr.
(86) International application number: JP0100990
(87) International publication number: WO01058489

(57) **Abstract**

The present invention provides pharmaceutical composition for prophylaxis or/and therapy of a postoperative stress, which comprises a CRF antagonist.

## Description

### Technical Field

The present invention relates to a novel use of a compound having corticotropin-releasing factor (abbreviated hereinafter to a CRF) antagonist activating effect. Concretely, it relates to a pharmaceutical composition useful for prophylaxis or therapy of a postoperative stress, which comprises a compound having CRF antagonist activating effect.

### Background Art

In, for example, JP 8259567, JP 7509726, JP 8003041, JP 9132528, JP9507855, JP 10072449, WO 98/05661, WO98/08846, WO 98/08847, JP7509725, JP7509727, JP8500121, JP7509728, JP 9506632, JP918868, etc, it was reported that a compound having CRF antagonist activating effect was useful for therapy of the following diseases; for example, panic, phobias (including acrophobia, social phobia and simple phobia), obsessive-compulsive disorder, post-traumatic stress disorder, single episode depression, recurrent depression, dysthemia, bipolar disorders, cyclothymia, mood disorders, postpartum depression, child abuse induced depression, sleep disorders, stress-induced pain perception (including fibromyalgia), sleeping disorder inducing by fibromyositis, rheumatoid arthritis, osteoarthritis, psoriasis, thyropathy, syndrome of inappropriate antidiarrhetic hormone (ADH), bulimia nervosa, eating disorders, obesity, head trauma, spinal cord trauma, ischemic neuronal damage, excitotoxic neuronal damage, epilepsy, stroke, immune dysfunctions including stress induced immune dysfunctions, phobias, muscle cramp, Parkinson's disease, Huntington's disease, urinary incontinence, senile dementia of the Alzheimer's type, multiinfarct dementia, amyotrophic lateral sclerosis, drug dependence diseases, addictions, hypoglycemia, diseases related to heart or blood vessel of heart (such as hypertension, tachycardia and congestive heart failure), stroke, osteoporosis, premature birth, psychosocial dwarfism, stress-induced fever, ulcer, diarrhea, postoperation ileus, disorder on psychopathology, colon irritation related to stress, inflammatory diseases (such as joint fluid, asthma, allergies, etc), anxiety, depression, fatigue syndrome, headache, ache, cancer, Crohn's disease, convulsivant hematonosis, irritable bowel syndrome (such as irritable colon, immunodeficiency, and human immunodeficiency virus infections), neurodegenerative diseases (such as Alzheimer's disease etc), gastrointestinal diseases, eating disorder (such as neurotic dysorexia etc), hemorrhagic stress, drug and alcohol withdrawal symptoms, drug intoxication stress induced psychotic episodes and difficulty of pregnancy, etc.

And until now, pirenzepine hydrochloride was known as a pharmaceutical agent for therapy of enhancement of gastric juice secretion by a surgical stress, and ranitidine hydrochloride and cimetidine were known as a pharmaceutical agent for therapy of bleeding upper gastrointestinal tract by a surgical stress.

### Disclosure of Invention

At the present days, operations are used in the therapy of many diseases. The operation makes as it did, external injuries of organisms suffered intentionally under medical control, which causes reactions in situ to maintain homeostasis, in other word, reactions by a surgical stress. But in case when these biological reactions will be excessive, those over-reactions have negative effects on the living organisms, and present unfavorable side effect and side symptom on human body.

Such a side effect or side symptom caused by the surgical stress, although it is different more or less depending on patients, includes, for example, psychogenic symptoms such as violent deed, irritation, anxiety and insomnia; physical symptom such as enhancement of gastric juice secretion, bleeding of upper gastrointestinal tract or delay of time to cure injury by an operation; postoperative complications such as infectious disease caused by lowering or fall of physical conditions. In many cases, hypnotic, drug leading to sleep, minor tranquilizer, or remedy mentioned above is used for those symptoms, and most of said therapies are symptomatic treatments. As mentioned above, there has been known no prophylactic or therapeutic drug for the general side effect or the side symptom after an operation so far.

It is generally believed that the reaction of nerve or incretion system plays a central role in the surgical stress, and that the excessive reactions causes the side effect or the side symptom. But actually there are so complicated mutual interactions among various hormones such as adrenocorticotropic hormone (ACTH), cortisol, catecholamines, glucagon, insulin, antidiuretic hormone(ADH), thyroid stimulating hormone and growth hormone during an operation that the mechanism of the surgical stress has not been made clear yet.

And so the inventors of this invention hit upon the possibility of general side effect or side symptom after an operation might result from excessive corticotropin-releasing factor (CRF), and have studied assiduously.

As a result, the inventors have found out that a compound having CRF antagonist activating effect, that is, a compound suppressing excessive production of CRF shows excellent effects on prophylaxis or/and therapy not only for a surgical stress, but also for the side effect or side symptom after an operation. The inventors have completed this invention after additional researches and developments.
Thus, the present invention relates to the followings:
(1) A pharmaceutical composition for prophylaxis or therapy of a postoperative stress, which comprises a compound having CRF antagonist activating effect;
(2) A pharmaceutical composition for prophylaxis or therapy of a postoperative stress according to the above (1), wherein the compound having CRF antagonist activating effect is a 5 to 6 membered nitrogen-containing heterocyclic compound, its fused ring derivative or a salt thereof which has CRF antagonist activating effect;
(3) A pharmaceutical composition for prophylaxis or therapy of a postoperative stress according to the above (1) or (2), wherein the compound having CRF antagonist activating effect is a compound represented by the formula I 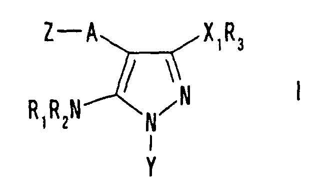 wherein A is CH₂; R₁, R₂ and R₃ are each independently linear C₁-C₆ alkyl, branched C₃-C₈ alkyl, C₃-C₈ alkenyl, wherein the double bond is not adjacent to the N or X₁ when X₁ is oxygen or sulfur, or C₃-C₇ cycloalkyl -(CH₂)ₙ- wherein n is 0, 1, 2, 3, 4; or R₁ and R₂ may be taken together with the nitrogen to form a saturated 4, 5 or 6 membered ring optionally condensed with benzene ring; and R₃ may also be (CH₂)_{q}Q₁R₁₉ wherein q is 0, 1 or 2 and Q₁ is oxygen, sulfur, NH, N(C₁-C₆ alkyl), or a covalent bond when X₁ is not a covalent bond, and R₁₉ may be hydrogen, linear C₁-C₆ alkyl, branched C₃-C₈ alkyl, C₃-C₈ alkenyl, C₃-C₈ cycloalkyl, C₃-C₈ cycloalkyl-(CH₂)ₙ- wherein n is 1 to 4; X₁ is a covalent bond, CH₂, NR, wherein R is hydrogen or linear C₁-C₆ alkyl, oxygen or sulfur; Y is phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, or piperidinyl, each of the above groups may be substituted with one to three of any substitutents of fluoro, chloro, bromo or methyl, or one of trifluoromethyl, with the proviso that Y is not unsubstituted phenyl; and Z is
   (a) 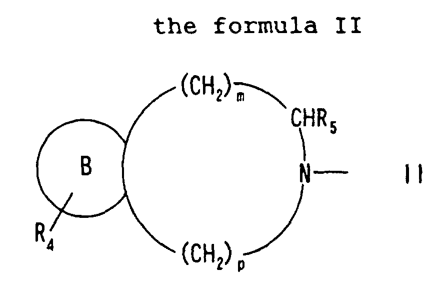 wherein the B ring is phenyl, naphthyl, pyridyl, pyrimidinyl, pyrazinyl, pyridazinyl, triazilyl, pyrrolyl, pyrazolyl, imidazolyl, triazolyl, thienyl, or indolyl, each of the above groups may be substituted with methyl, methoxy, fluoro, chloro, bromo or iodo; or a saturated 5 or 6 membered carbocyclic ring or a partially unsaturated ring having one or two double bonds; R₄ is hydrogen, C₁-C₆ alkyl, C₁-C₆ alkoxy, or hydroxy, fluoro, chloro, bromo, iodo or trifluoromethyl; R₅ is hydrogen, linear C₁-C₆ alkyl, branched C₃-C₈ alkyl, C₃-C₈ alkenyl or (CH₂)ₒ-X₂-(CH₂)ᵣ-Q₂-R₆; R₆ is hydrogen, linear C₁-C₆ alkyl, branched C₃-C₈ alkyl, C₃-C₈ alkenyl; X₂ and Q₂ are each independently oxygen, sulfur, NH, N(C₁-C₆ alkyl), or one of X₂ and Q₂ may be a covalent bond; m is 0 or 1; o is 1 or 2; p is 1 or 2; r is 0, 1, or 2;
   (b) 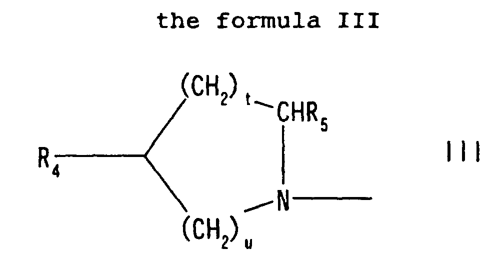 wherein R₄ and R₅ are as defined above, and t and u are each independently 1 or 2;
   (c) -NR₇R₈ wherein R₇ and R₈ are each independently hydrogen, linear C₁-C₆ alkyl, branched C₃-C₈ alkyl, C₃-C₈ alkenyl, (CH₂)ᵥCH₂OH, (CH₂)ᵥNR₉R₁₀ wherein v is 0 to 3, and R₉ and R₁₀ are each independently hydrogen, or linear C₁-C₆ alkyl, C₃-C₁₂ cycloalkyl, C₃-C₁₂ cycloalkyl(CH₂)ₙ, C₆-C₁₀ bicycloalkyl(CH₂)ₙ, wherein n is 0 to 4, C₃-C₆ cycloalkyl group fused with benzene ring, C₁-C₆ hydroxyalkyl, phenyl, C₁-C₃ phenylalkylene, each of the above groups may be substituted with one or two of hydroxy, fluoro, chloro, bromo, C₁-C₅ alkyl or C₁-C₅ alkoxy; or R₇ and R₈ may be taken together with the nitrogen to form a saturated or partially unsaturated 5 to 7 membered ring which may contain one of oxygen, sulfur, NH, or N(C₁₋C₆ alkyl) and which may be substituted with C₁-C₆ alkyl, hydroxyl or phenyl wherein any double bond are not adjacent to any heteroatoms;
   (d) 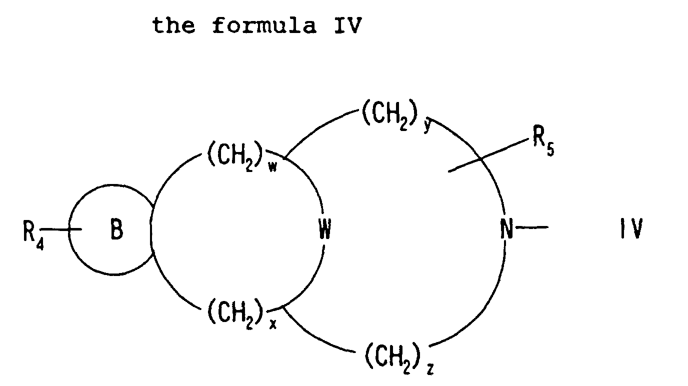 wherein B, R₄ and R₅ are as defined above, w, x, y and z are each independently 1 or 2 and W is (CH₂)_{q} wherein q is as defined above, N(C₁₋C₆ alkyl), or oxygen;
   (e) 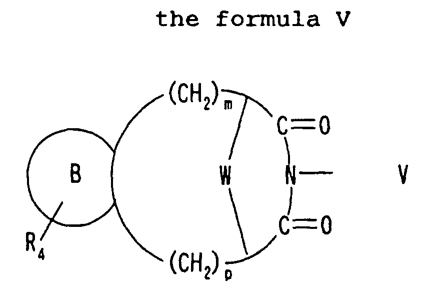 wherein B, R₄, m and p are as defined above;
   (f) 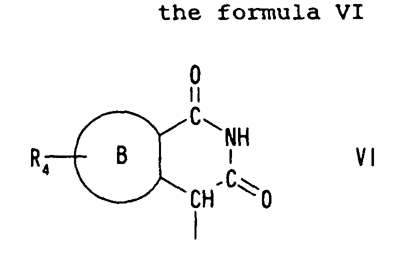 wherein B and R₄ are as defined above; or
   (g) O(CH₂)ᵥR₁₁ₐ wherein v is 0 to 3 and R₁₁ₐ is linear C₁-C₆ alkyl, branched C₃-C₈ alkyl, phenyl, naphthyl, 1 2,3,4-tetrahydronaphthyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, piperidinyl, or thienyl, each of the above groups may be substituted with one or two of any substitutents of fluoro, chloro, bromo, methyl or trifluoromethyl, or its pharmaceutically acceptable acid addition salt;
(4) A pharmaceutical composition for prophylaxis or therapy of a postoperative stress according to the above (1) or (2), wherein the compound having CRF antagonist activating effect is a compound represented by the formula VII 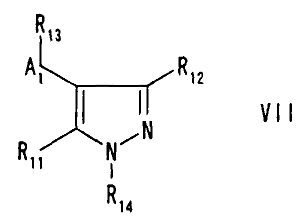 wherein A₁ is C=O or SO₂ or A₁ and R₁₁ taken together with the carbons to which they are attached form 5-pyridyl or pyrimidinyl which may be substituted with R₁₅, wherein R₁₅ is hydrogen, C₁-C₆ alkyl, fluoro, chloro, bromo, hydroxy, amino, O(C₁-C₆ alkyl), NH(C₁-C₆ alkyl), N(C₁-C₆ alkyl)(C₁-C₆ alkyl), SH, S(O)ₙ(C₁-C₆ alkyl) wherein n is 0, 1 or 2, wherein said C₁-C₆ alkyl or C₁-C₆ alkyl moieties may be substituted with from one to three substituents R₁₆ which is hydroxy, amino, C₁-C₃ alkoxy, dimethylamino, diethylamino, methylamino, ethylamino, NH(C=O)CH₃, fluoro, chloro, bromo or C₁-C₃ thioalkyl; R₁₁ is hydrogen, C₁-C₆ alkyl, amino, O(C₁-C₆ alkyl), NH(C₁-C₆ alkyl), N(C₁-C₆ alkyl)(C₁-C₆ alkyl), wherein said C₁₋C₆ alkyl or C₁-C₆ alkyl moieties may be substituted with from one to three substituents R₁₆ as defined above; R₁₂ is hydrogen, C₁-C₆ alkyl, hydroxy, amino, O(C₁-C₆ alkyl), NH(C₁-C₆ alkyl), N(C₁-C₆ alkyl)(C₁-C₆ alkyl), SH, S(O)ₙ(C₁-C₆ alkyl) wherein n is 0,1 or 2, cyano, hydroxy, carboxy or amido, wherein said alkyl may be substituted with one to three of hydroxy, amino, carboxy, amido, NH(C=O) (C₁-C₆ alkyl), N(C₁-C₆ alkyl)(C₁-C₆ alkyl), (C=O)O(C₁-C₆ alkyl), C₁-C₃ alkoxy, C₁-C₃ thioalkyl, fluoro, bromo, chloro, iodo, cyano or nitro; R₁₃ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, azaindolyl, benzoxazolyl, oxazolyl, pyrrolidinyl, thiazolidinyl, morpholinyl, pyridinyl, tetrazolyl, or 9 to 12 membered bicycloalkyl, optionally containing one to three of oxygen, sulfur or N-Z₀ wherein Z₀ is hydrogen, C₁₋C₄ alkyl, C₁₋C₄ alkanoyl, phenyl or phenylmethyl, wherein each one of the above groups may be substituted independently with from one to three of fluoro, chloro, bromo, C₁-C₆ alkyl, C₁-C₆ alkoxy or trifluoromethyl, or one of cyano, nitro, amino, NH(C₁₋₆ alkyl), N(C₁-C₄ alkyl)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NHSO₂(C₁-C₄ alkyl), S(C₁-C₆ alkyl) or SO₂(C₁-C₆ alkyl), wherein said C₁₋C₄ alkyl or C₁₋C₄ alkyl moieties and C₁₋C₆ alkyl or C₁₋C₆ alkyl moieties may be substituted with one or two of fluoro, chloro, hydroxy, amino, methylamino, dimethylamino or acetyl; and R₁₄ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, azaindolyl, benzoxazolyl, oxazolyl, pyrrolidinyl, thiazolidinyl, morpholinyl, pyridinyl, tetrazolyl, or 3 to 8 membered cycloalkyl or 9 to 12 membered bicycloalkyl, optionally containing one to three of oxygen, sulfur or N-Z₀ wherein Z₀ is hydrogen, C₁₋C₄ alkyl, C₁₋C₄ alkanoyl, phenyl or phenylmethyl, wherein each of the above groups may be substituted independently with from one to three of fluoro, chloro, bromo, trifluoromethyl, C₁-C₆ alkyl or C₁-C₆ alkoxy, or one of cyano, nitro, amino, NH(C₁-C₆ alkyl), N(C₁-C₄ alkyl)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NH₂SO₂(C₁-C₄ alkyl), S(C₁-C₆ alkyl) or SO₂(C₁-C₆ alkyl), wherein said C₁-C₄ alkyl or C₁-C₄ alkyl moieties and C₁-C₆ alkyl or C₁-C₆ alkyl moieties may be substituted with one or two of fluoro, chloro, hydroxy, amino, methylamino, dimethylamino or acetyl; with the proviso that (1) R₁₄ is not unsubstituted phenyl; (2) when R₁₁ is amino, R₁₂ is methylthio, R₁₄ is 2,4,6-trichlorophenyl, and A is C=O, then R₁₃ is not 2-chlorophenyl; and (3) R₁₁ and R₁₂ is not both hydrogen, or its pharmaceutically acceptable acid addition salt;
(5) A pharmaceutical composition for prophylaxis or therapy of a postoperative stress according to the above (1) or (2), wherein the compound having CRF antagonist activating effect is a compound represented by the formula VIII 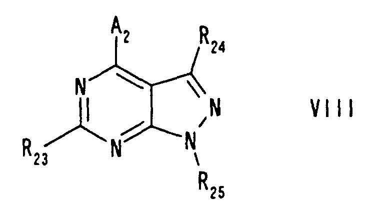 wherein A₂ is NR₂₁R₂₂, CR₂₁R₂₂R₂₁₁, C(=CR₂₁R₂₁₂)R₂₂, NHCR₂₁R₂₂R₂₁₁, OCR₂₁R₂₂R₂₁₁, SCR₂₁R₂₂R₂₁₁, NHNR₂₁R₂₂, CR₂₂R₂₁₁NHR₂₁, CR₂₂R₂₁₁OR₂₁, CR₂₂R₂₁₁SR₂₁ or C(O)R₂₂;
   R₂₁ is hydrogen, or C₁-C₆ alkyl which may be substituted with one or two substituents R₂₆ independently selected from the group consisting of hydroxy, fluoro, chloro, bromo, iodo, C₁-C₆ alkoxy, O-C(=O)-(C₁-C₆ alkyl), O-C(=O)-N(C₁-C₄ alkyl)(C₁-C₂ alkyl), amino, NH(C₁-C₄ alkyl), N(C₁-C₄ alkyl)(C₁-C₂ alkyl), S(C₁-C₆ alkyl), OC(O)NH(C₁-C₄, alkyl), N(C₁-C₂ alkyl)C(O)(C₁-C₄ alkyl), NHC(=O)(C₁-C₄ alkyl), COOH, C(=O)O(C₁-C₄, alkyl), C(=O)NH(C₁-C₄, alkyl), C(=O)N(C₁-C₄ alkyl)(C₁-C₂ alkyl), SH, CN, NO₂, SO(C₁-C₄ alkyl), SO₂(C₁-C₄ alkyl), SO₂NH(C₁-C₄ alkyl) and SO₂N(C₁-C₄ alkyl)(C₁-C₂ alkyl), and wherein said C₁-C₆ alkyl may contain one or two double or triple bonds;
   R₂₂ is C₁-C₁₂ alkyl, aryl or (C₁-C₁₀ alkylene) aryl wherein said aryl is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, azaindolyl, oxazolyl, or benzoxazolyl, or 3 to 8 membered cycloalkyl or (C₁-C₆ alkylene)cycloalkyl, wherein said cycloalkyl may contain one or two of oxygen, sulfur or N-Z₁ wherein Z₁ is hydrogen, C₁-C₄ alkyl, benzyl or C₁-C₄ alkanoyl, wherein R₂₂ may be substituted independently with from one to three of chloro, fluoro or C₁-C₄ alkyl, or one of hydroxy, bromo, iodo, C₁-C₆ alkoxy, O-C(=O)-(C₁-C₆ alkyl), O-C-N(C₁-C₄ alkyl)(C₁-C₂ alkyl), S(C₁-C₆ alkyl), NH₂, NH(C₁-C₂ alkyl), N(C₁-C₄ alkyl)(C₁-C₂ alkyl), N(C₁-C₄ alkyl) -C(=O)(C₁-C₄ alkyl), NHC(=O)(C₁-C₄ alkyl), COOH, C(=O)O(C₁-C₄ alkyl), C(=O)NH(C₁-C₄ alkyl), C(=O)N(C₁-C₄ alkyl)(C₁-C₂ alkyl), SH, CN, NO₂, SO(C₁-C₄ alkyl), SO₂(C₁-C₄ alkyl), SO₂NH(C₁-C₄ alkyl) or SO₂N(C₁-C₄ alkyl) (C₁-C₂ alkyl), and wherein said C₁-C₁₂ alkyl or C₁-C₁₀ alkylene may contain one to three double or triple bonds; or NR₂₁R₂₂ or CR₂₁R₂₂R₂₁₁ may form a 4 to 8 membered ring optionally containing one or two double bonds or one or two of oxygen, sulfur or N-Z₁ wherein Z₁ is hydrogen, C₁-C₄ alkyl, benzyl or C₁-C₄ alkanoyl;
   R₂₃ is hydrogen, C₁-C₆ alkyl, fluoro, chloro, bromo, iodo, hydroxy, amino, O(C₁-C₆ alkyl), NH(C₁-C₆ alkyl), N(C₁-C₄ alkyl)(C₁-C₂ alkyl), SH, S(C₁-C₄ alkyl), SO(C₁-C₄ alkyl), or SO₂(C₁-C₄ alkyl), wherein said C₁-C₄ alkyl or C₁-C₄ alkyl moieties and C₁-C₆ alkyl or C₁-C₆ alkyl moieties may contain one or two double or triple bonds and may be substituted with from one to three substituents R₂₇ independently selected from the group consisting of hydroxy, amino, C₁-C₃ alkoxy, dimethylamino, diethylamino, methylamino, ethylamino, NHC(=O)CH₃, fluoro, chloro and C₁-C₃ thioalkyl;
   R₂₄ is hydrogen, C₁-C₆ alkyl, fluoro, chloro, bromo, iodo, C₁-C₆ alkoxy, amino, NH(C₁-C₆ alkyl), N(C₁-C₆ alkyl) (C₁-C₂ alkyl), SOₙ(C₁-C₆ alkyl) wherein n is 0, 1 or 2, cyano, hydroxy, carboxy or amido, wherein said C₁-C₆ alkyl or C₁-C₆ alkyl moieties may be substituted with one to three of hydroxy, amino, carboxy, amido, NH(C=O)(C₁-C₄ alkyl), NH(C₁-C₄ alkyl), N(C₁-C₄ alkyl)(C₁-C₂ alkyl), (C=O)O(C₁-C₄ alkyl), C₁-C₃ alkoxy, C₁-C₃ thioalkyl, fluoro, bromo, chloro, iodo, cyano or nitro;
   R₂₅ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, pyrrolopyridyl, benzoxazolyl, oxazolyl, pyrrolidinyl, thiazolidinyl, piperazinyl, piperidinyl, tetrazolyl, or 3 to 8 membered cycloalkyl or 9 to 12 membered bicycloalkyl, optionally containing one or two of oxygen, sulfur or N-Z₁ wherein Z₁ is hydrogen, C₁-C₄ alkyl, C₁-C₄ alkanoyl, phenyl or benzyl, wherein each one of the above groups may be substituted independently with from one to three of fluoro, chloro, bromo, formyl, C₁-C₆ alkyl, C₁-C₆ alkoxy or trifluoromethyl, or one of hydroxy, iodo, cyano, nitro, amino, cyclopropyl, NH(C₁-C₄ alkyl), N(C₁-C₄ alkyl)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NHSO₂(C₁-C₄ alkyl), S(C₁-C₆ alkyl), SO₂(C₁-C₆ alkyl), wherein said C₁-C₄ alkyl or C₁-C₄ alkyl moieties and C₁-C₆ alkyl or C₁-C₆ alkyl moieties may have one double or triple bond and may be substituted with one or two of fluoro, chloro, hydroxy, amino, methylamino, dimethylamino or acetyl; with the proviso that R₂₅ is not unsubstituted phenyl; R₂₁₁ is hydrogen, hydroxy, fluoro, chloro, COO(C₁-C₂ alkyl), cyano or CO(C₁-C₂ alkyl); and R₂₁₂ is hydrogen or C₁-C₄ alkyl;
   (a) A₂ is not linear C₁-C₁₂ alkyl;
   (b) R₂₅ is not a sugar group;
   (c) when R₂₃ and R₂₄ are hydrogen and R₂₅ is chlorophenyl, then A₂ is not NH-CH(CH₃)-(CH₂)₃-N(C₂H₅)₂;
   (d) when R₂₃ and R₂₄ are hydrogen and A₂ is NR₂₁R₂₂ wherein R₂₁ is C₃-C₇ cycloalkyl and R₂₂ is C₂-C₆ alkenyl, phenyl-(C₁-C₆ alkylene) or hetero-(C₁-C₆ alkylene) wherein the hetero radical is furyl, thienyl or pyridinyl, and wherein said phenyl may be substituted with fluoro, chloro, bromo or iodo, then R₂₅ is not tetrahydrofuranyl or tetrahydropyranyl;
   (e) when R₂₃ is methoxy, methylthio, or methylsulfonyl, R₂₄ is hydrogen, and R₂₅ is tetrahydrofuranyl or tetrahydropyranyl, then A₂ is not NHC₂H₄C₆H₅, NH(C₁-C₂ alkyl), morpholinyl, hydrazino, which may be substituted with one methyl or two methoxy;
   (f) when R₂₃ is hydrogen, C₁-C₆ alkyl, hydrazino, chloro, bromo, SH, or S(C₁-C₄ alkyl), R₂₄ is hydrogen, and R₂₅ is C₃-C₈ cycloalkyl, then A₂ is not hydrazino, NH(C₁-C₂ alkyl) or N(C₁-C₆ alkyl)(C₁-C₁₂ alkyl);
   (g) when R₂₃ and R₂₄ are hydrogen and A₂ is NH(CH₂)ₘCOOH, wherein m is 1 to 12, then R₂₅ is not phenyl substituted with one of fluoro, chloro, bromo or iodo;
   (h) when R₂₃ is hydrogen, hydroxy, methylthio, chloro or NHbenzyl, R₂₄ is hydrogen, and R₂₅ is chlorophenyl or bromophenyl, than A₂ is not NH(C₁-C₁₂ alkyl), NHallyl, or N(C₁-C₆ alkyl) (C₁-C₁₂ alkyl), wherein said C₁-C₁₂ alkyl may be substituted with NHbenzyl or NC₂H₅ which may be substituted with one or two of bromo, chloro, fluoro, NC₂H₅phenol or morpholinopropyl;
   (i) when R₂₃ and R₂₄ are hydrogen and R₂₅ is nitrophenyl, then A₂ is not NHR₂₂ wherein R₂₂ is C₁-C₁₂ alkyl which may be substituted with two hydroxy, or R₂₂ is phenyl or benzyl;
   (j) when R₂₃ is chloro or O(C₁-C₆ alkyl), R₂₄ is hydrogen, and A₂ is NR₂₁R₂₂, wherein R₂₁ and R₂₂ are independently hydrogen or C₁-C₆ alkyl, then R₂₅ is not chlorophenyl; and
   (k) when R₂₃ is hydrogen, A₂ is benzyl or phenethyl, and R₂₄ is fluoro, chloro, bromo or iodo, then R₂₅ is not 5'-deoxy-ribofuranosyl or 5'-amino-5'-deoxy-ribofuranosyl, or its pharmaceutically acceptable acid addition salt;
(6) A pharmaceutical composition for prophylaxis or therapy of a postoperative stress according to the above (1) or (2), wherein the compound having CRF antagonist activating effect is a compound represented by the formula IX 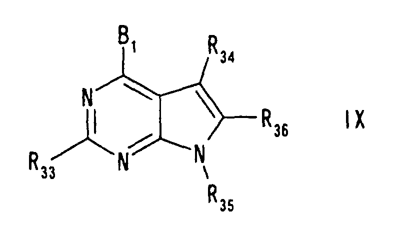 wherein B₁ is NR₃₁R₃₂, CR₃₁R₃₂R₃₁₁, C(=CR₃₂R₃₁₂)R₃₁, NHCR₃₁R₃₂R₃₁₁, OCR₃₁R₃₂R₃₁₁, SCR₃₁R₃₂R₃₁₁, NHNR₃₁R₃₂, CR₃₂R₃₁₁NHR₃₁, CR₃₂R₃₁₁OR₃₁, CR₃₂R₃₁₁SR₃₁, or C(O)R₃₂;
   R₃₁ is hydrogen, or C₁-C₆ alkyl which may be substituted with one or two substituents R₃₇ independently selected from the group consisting of hydroxy, fluoro, chloro, bromo, iodo,C₁-C₈ alkoxy, O-C(=O)-(C₁-C₆ alkyl), O-C(=O)NH(C₁-C₄ alkyl), O-C(=O)-N(C₁-C₄ alkyl) (C₁-C₂ alkyl), amino, NH(C₁-C₄ alkyl), N(C₁-C₄ alkyl)(C₁-C₂ alkyl), S(C₁-C₆ alkyl), N(C₁-C₄ alkyl)C(=O)(C₁-C₄ alkyl), NHC(=O) (C₁-C₄ alkyl), COOH, C(=O)O(C₁-C₄ alkyl), C(=O)NH (C₁-C₄ alkyl), C(=O)N(C₁-C₄ alkyl)(C₁-C₂ alkyl), SH, CN, NO₂, SO (C₁-C₄ alkyl), SO₂(C₁-C₄ alkyl), SO₂NH(C₁-C₄ alkyl) and SO₂N(C₁-C₄ alkyl)(C₁-C₂ alkyl) wherein said C₁-C₆ alkyl may contain one or two double or triple bonds;
   R₃₂ is C₁-C₁₂ alkyl, aryl or (C₁-C₁₀ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, pyrrolopyridyl, oxazolyl or benzoxazolyl, or 3 to 8 membered cycloalkyl or (C₁-C₆ alkylene) cycloalkyl, wherein said cycloalkyl may contain one or two of oxygen, sulfur or N-Z₂ wherein Z₂ is hydrogen, C₁-C₄ alkyl, benzyl or C₁-C₄ alkanoyl, wherein R₃₂ may be substituted independently with from one to three of chloro, fluoro or C₁-C₄ alkyl, or one of hydroxy, bromo, iodo, C₁-C₆ alkoxy, O-C(=O)-(C₁-C₆ alkyl), O-C(=O)-N(C₁-C₄ alkyl)(C₁-C₂ alkyl), S(C₁-C₆ alkyl), NH₂, NH(C₁-C₂ alkyl), N(C₁-C₄ alkyl)(C₁-C₂ alkyl), N(C₁-C₄ alkyl)-C(=O)(C₁-C₄ alkyl), NHC(=O)(C₁-C₄ alkyl), COOH, C(=O)O(C₁-C₄ alkyl), C(=O)NH(C₁-C₄ alkyl), C(=O)N (C₁-C₄ alkyl)(C₁-C₂ alkyl), SH, CN, NO₂, SO(C₁-C₄ alkyl), SO₂(C₁-C₄ alkyl), SO₂NH(C₁-C₄ alkyl), SO₂N(C₁-C₄ alkyl)(C₁-C₂ alkyl), and wherein said C₁-C₁₂ alkyl or C₁-C₁₀ alkylene moieties may contain one to three double or triple bonds; or
   NR₃₁R₃₂ or CR₃₁R₃₂R₃₁₁ may form a saturated 3 to 8 membered ring of which the 5 to 8 membered ring may contain one or two double bonds or one or two of oxygen, sulfur or N-Z₂ wherein Z₂ is hydrogen, C₁-C₄ alkyl, benzyl or C₁-C₄ alkanoyl;
   R₃₃ is hydrogen, C₁-C₆ alkyl, fluoro, chloro, bromo, iodo, hydroxy, amino, O(C₁-C₆ alkyl), NH(C₁-C₆ alkyl), N(C₁-C₄ alkyl)(C₁-C₂ alkyl), SH, S(C₁-C₄ alkyl), SO(C₁-C₄ alkyl), or SO₂(C₁-C₄ alkyl), wherein said C₁-C₄ alkyl moieteis and C₁-C₆ alkyl or C₁-C₆ alkyl moieties may contain one double or triple bond and may be substituted with from one to three substituents R₃₈ independently selected from the group consisting of hydroxy, C₁-C₃ alkoxy, fluoro, chloro and C₁-C₃ thioalkyl;
   R₃₄ is hydrogen, C₁-C₆ alkyl, fluoro, chloro, bromo, iodo, C₁-C₆ alkoxy, amino, NH(C₁-C₆ alkyl), N(C₁-C₆ alkyl)(C₁-C₂ alkyl), SOₙ(C₁-C₆ alkyl) wherein n is 0, 1 or 2, cyano, hydroxy, carboxy or amido, wherein said C₁-C₆ alkyl or C₁-C₆ alkyl moieties may be substituted with one of hydroxy, trifluoromethyl, amino, carboxy, amido, NH(C=O)(C₁-C₄ alkyl), NH(C₁-C₄ alkyl), N(C₁-C₄ alkyl)(C₁-C₂ alkyl), (C=O)O(C₁-C₄ alkyl), C₁-C₃ alkoxy, C₁-C₃ thioalkyl, fluoro, bromo, chloro, iodo, cyano or nitro;
   R₃₅ is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, pyrrolopyridyl, benzoxazolyl, oxazolyl, pyrrolidinyl, thiazolidinyl, morpholinyl, piperizinyl, piperazinyl, tetrazolyl, or 3 to 8 membered cycloalkyl or 9 to 12 membered bicycloalkyl, optionally containing one or two of oxygen, sulfur or N-Z₂ wherein Z₂ is hydrogen, C₁-C₄ alkyl, C₁-C₄ alkanoyl, phenyl or phenylmethyl, wherein each one of the above groups may be substituted independently with from one to three of fluoro, chloro, bromo, formyl, C₁-C₆ alkyl, C₁-C₆ alkoxy or trifluoromethyl, or one of hydroxy, iodo, cyano, nitro, amino, NH(C₁-C₄ alkyl), N(C₁-C₄)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NHSO₂(C₁-C₄ alkyl), S(C₁-C₆ alkyl), SO₂(C₁-C₆ alkyl), wherein said C₁-C₄ alkyl or C₁-C₄ alkyl moieties and C₁-C₆ alkyl or C₁-C₆ alkyl moieties may be substituted with one or two of fluoro, chloro, hydroxy, C₁-C₄ alkoxy, amino, methylamino, dimethylamino or acetyl, wherein said C₁-C₄ alkyl or C₁-C₄ alkyl moieties and C₁-C₆ alkyl or C₁-C₆ alkyl moieties may contain one double or triple bond; with the proviso that R₃₅ is not unsubstituted phenyl;
   R₃₆ is hydrogen, C₁-C₆ alkyl, fluoro, chloro, bromo, iodo, C₁-C₆ alkoxy, formyl, amino, NH(C₁-C₆ alkyl), N(C₁-C₆ alkyl)(C₁-C₂ alkyl), SOₙ(C₁-C₆ alkyl) wherein n is 0, 1 or 2, cyano, carboxy, or amido, wherein said C₁-C₆ alkyl may be substituted with one of hydroxy, trifluoromethyl, amino, carboxy, amido, NH(C=O)(C₁-C₄ alkyl), NH(C₁-C₄ alkyl), N(C₁-C₄ alkyl)(C₁-C₂ alkyl), (C=O)O(C₁-C₄ alkyl), C₁-C₃ alkoxy, C₁-C₃ thioalkyl, fluoro, bromo, chloro, iodo, cyano or nitro;
   R₃₁₁ is hydrogen, hydroxy, fluoro, chloro, COO(C₁-C₂ alkyl), cyano or CO(C₁-C₂ alkyl); and
   R₃₁₂ is hydrogen or C₁-C₄ alkyl; with the proviso that (1) B₁ is not linear C₁-C₁₂ alkyl, (2) when R₃₅ is unsubstituted cycloakyl, R₃₃ and R₃₄ are hydrogen, and R₃₆ is hydrogen or methyl, then B₁ is not NHR₃₂ wherein R₃₂ is benzyl or thienylmethyl, and (3) when R₃₅ is p-bromophenyl and R₃₃, R₃₄, and R₃₆ are methyl, then B₁ is not methylamino or hydroxyethylamino, or its pharmaceutically acceptable acid addition salt;
(7) A pharmaceutical composition for prophylaxis or therapy of a postoperative stress according to the above (1) or (2), wherein the compound having CRF antagonist activating effect is a compound represented by the formula Ia 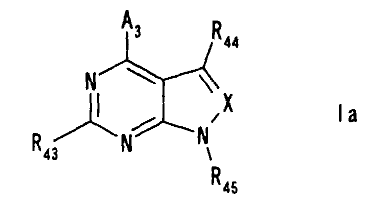 wherein X is nitrogen or -CR₄₆; A₃ is -NR₄₁R₄₂, -CR₄₁R₄₂R₄₁₁, -C(=CR₄₂R₄₁₂)R₄₁, -NHCR₄₁R₄₂R₄₁₁, -OCR₄₁R₄₂R₄₁₁, -SCR₄₁R₄₂R₄₁₁, -NHNR₄₁R₄₂, -CR₄₂R₄₁₁NHR₄₁, -CR₄₂R₄₁₁OR₄₁, -CR₄₂R₄₁₁SR₄₁ or -C(O)R₄₂;
   R₄₁ is hydrogen, or C₁-C₆ alkyl which may optionally be substituted with from one or two substituents independently selected from the group consisting of hydroxy, fluoro, chloro, bromo, iodo, C₁-C₆ alkoxy, -OC(=O)-(C₁-C₆ alkyl), -OC(=O)NH(C₁-C₄ alkyl), -OC(=O)-N(C₁-C₄ alkyl)(C₁-C₂ alkyl), amino, -NH(C₁-C₄ alkyl), -N(C₁-C₂ alkyl)(C₁-C₄ alkyl), -S(C₁-C₆ alkyl), -N(C₁-C₄ alkyl)C(=O)(C₁-C₄ alkyl), -NHC(=O)(C₁-C₄ alkyl), -COOH, -C(=O)O(C₁-C₄ alkyl), -C(=O)NH(C₁-C₄ alkyl), -C(=O)N(C₁-C₄ alkyl)(C₁-C₂ alkyl), -SH, -CN, -NO₂, -SO(C₁-C₄ alkyl), -SO₂(C₁-C₄ alkyl) and -SO₂NH(C₁-C₄ alkyl) -SO₂N(C₁-C₄ alkyl)(C₁-C₂ alkyl), and wherein each of the foregoing C₁-C₆ alkyl moieties in the definition of R₄₁ may contain one or two double or triple bonds;
   R₄₂ is C₁-C₁₂ alkyl, aryl or -(C₁-C₁₀ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, pyrrolopyridyl, oxazolyl or benzoxazolyl, or 3 to 8 membered cycloalkyl or -(C₁-C₆ alkylene)cycloalkyl, wherein one or two of the carbon atoms of any of said cycloalkyl moieties may optionally be replaced independently by oxygen, sulfur or N-Z₃ wherein Z₃ is hydrogen, C₁-C₄ alkyl or C₁-C₄ alkanoyl, and wherein R₄₂ may optionally be substituted with from one to three substituents independently selected from chloro, fluoro and C₁-C₄ alkyl, or one substituent selected from hydroxy, bromo, iodo, C₁-C₆ alkoxy, -OC(=O)-(C₁-C₆ alkyl), -OC(=O)N(C₁-C₄ alkyl)(C₁-C₂ alkyl), -S(C₁-C₆ alkyl), NH₂, -NH(C₁-C₂ alkyl), -N(C₁-C₂ alkyl)(C₁-C₄ alkyl), -N(C₁-C₄ alkyl)-C(=O)-(C₁-C₄ alkyl), -NHC(=O)(C₁-C₄ alkyl), -COOH, -C(=O)O(C₁-C₄ alkyl), -C(=O)NH(C₁-C₄ alkyl), -C(=O)N(C₁-C₄ alkyl)(C₁-C₂ alkyl), -SH, -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl), and wherein each of the foregoing C₁-C₁₂ alkyl and C₁-C₁₀ alkylene moieties in the definition may optionally contain one to three double or triple bonds; or
   R₄₁ and R₄₂ taken together with the atom to which they are attached may form a saturated 3 to 8 membered ring which, if it is a 5 to 8 membered ring, may optionally contain one or two double bonds, and wherein one or two of the carbon atoms of said 5 to 8 membered ring may optionally be replaced independently by oxygen, sulfur or N-Z₃ wherein Z₃ is hydrogen, C₁-C₄ alkyl, C₁-C₄ alkanoyl or benzyl;
   R₄₃ is hydrogen, C₁-C₆ alkyl, fluoro, chloro, bromo, iodo, hydroxy, amino, -O(C₁-C₆ alkyl), -NH(C₁-C₆ alkyl), -N(C₁-C₄ alkyl)(C₁-C₂ alkyl), -SH, -S(C₁-C₄ alkyl), -SO(C₁-C₄ alkyl) or -SO₂(C₁-C₄ alkyl), wherein each of the foregoing C₁-C₄ alkyl and C₁-C₆ alkyl moieties in the definition of R₄₃ may contain one double or triple bond and may optionally be substituted with from one to three substituents independently selected from the group consisting of hydroxy, C₁-C₃ alkoxy, fluoro, chloro and C₁-C₃ thioalkyl;
   R₄₄ is hydrogen, C₁-C₆ alkyl, fluoro, chloro, bromo, iodo, C₁-C₆ alkoxy, formyl, -NH(C₁-C₆ alkyl), -N(C₁-C₆ alkyl)(C₁-C₂ alkyl), -SOₙ(C₁-C₆ alkyl) wherein n is 0, 1 or 2, cyano, hydroxy, carboxy or amido, wherein each of the foregoing C₁-C₆ alkyl moieties in the definition of R₄₄ may optionally be substituted with one substituent selected from hydroxy, trifluoromethyl, amino, carboxy, amido, -NHC(=O)(C₁-C₄ alkyl), -NH(C₁-C₄ alkyl), -N(C₁-C₄ alkyl)(C₁-C₂ alkyl), -C(=O)O(C₁-C₄ alkyl), C₁-C₃ alkoxy, C₁-C₃ thioalkyl, fluoro, bromo, chloro, iodo, cyano and nitro;
   R₄₅ is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, pyrrolopyridyl, benzoxazolyl, oxazolyl, pyrrolidinyl, thiazolidinyl, morpholinyl, piperidinyl, piperazinyl, tetrazolyl, or 3 to 8 membered cycloalkyl or 9 to 12 membered bicycloalkyl ring, wherein one or two of the carbon atoms in said ring may optionally be replaced independently by oxygen, sulfur or N-Z₃ wherein Z₃ is hydrogen, C₁-C₄ alkyl, C₁-C₄ alkanoyl, phenyl or benzyl, and wherein each of the above R₄₅ groups may optionally be substituted with one or more substituents, preferably with two or three substituents, independently selected from fluoro, chloro, bromo, formyl, C₁-C₆ alkyl, C₁-C₆ alkoxy and trifluoromethyl, or one substituent selected from hydroxy, iodo, cyano, nitro, amino, -NH(C₁-C₄ alkyl), -N(C₁-C₄ alkyl)(C₁-C₂ alkyl), -COO(C₁-C₄ alkyl), -CO(C₁-C₄ alkyl), -SO₂NH(C₁-C₄ alkyl), -SO₂N(C₁-C₄ alkyl)(C₁-C₂ alkyl), -SO₂NH₂, -NHSO₂(C₁-C₄ alkyl), -S(C₁-C₆ alkyl) and -SO₂(C₁-C₆ alkyl), and wherein each of the foregoing C₁-C₄ alkyl and C₁-C₆ alkyl moieties in the definition of R₄₅ may optionally be substituted with from one or two substituents independently selected from fluoro, chloro, hydroxy, C₁-C₄ alkoxy, amino, methylamino, dimethylamino and acetyl, and wherein each of the foregoing C₁-C₄ alkyl and C₁-C₆ alkyl moieties in the definition of R₄₅ may optionally contain one double or triple bond; with the proviso that R₄₅ is not unsubstituted phenyl;
   R₄₆ is hydrogen, C₁-C₆ alkyl, fluoro, chloro, bromo, iodo, C₁-C₆ alkoxy, formyl, amino, -NH(C₁-C₆ alkyl), N(C₁-C₆ alkyl)(C₁-C₂ alkyl), -SOₙ(C₁-C₆ alkyl) wherein n is 0, 1 or 2, cyano, carboxy or amido, and wherein each of the foregoing C₁-C₆ alkyl moieties in the definition of R₄₆ may be optionally substituted with one substituent selected from hydroxy, trifluoromethyl, amino, carboxy, amido, -NHC(=O)(C₁-C₄ alkyl), -NH(C₁-C₄ alkyl), -N(C₁-C₄ alkyl)(C₁-C₂ alkyl), -C(=O)O(C₁-C₄ alkyl), C₁-C₃ alkoxy, C₁-C₃ thioalkyl, fluoro, bromo, chloro, iodo, cyano and nitro;
   R₄₁₁ is hydrogen, hydroxy, fluoro, chloro, -COO(C₁-C₂ alkyl), cyano or -C(=O)(C₁-C₂ alkyl; and
   R₄₁₂ is hydrogen or C₁-C₄ alkyl; with the proviso that: (1) when X is -CR₄₆, A₃ is not linear alkyl, (2) when X is -CR₄₆, R₄₅ is unsubstituted cycloalkyl, R₄₃ and R₄₄ are both hydrogen, and R₄₆ is hydrogen or methyl, then A₃ is not -NHR₄₂ wherein R₄₂ is benzyl or thienylmethyl, and (3) when X is -CR₄₆, R₄₅ is p-bromophenyl, and R₄₃, R₄₄ and R₄₆ are methyl, then A₃ is not methylamino or hydroxyethylamino;
   and when X is nitrogen, with the further proviso that:
   (a) A₃ is not linear C₁-C₁₂ alkyl;
   (b) R₄₅ is not a sugar group;
   (c) when R₄₃ and R₄₄ are hydrogen and R₄₅ is chlorophenyl, then A₃ is not -NH-CH(CH₃)-(CH₂)₃-N(C₂H₅)₂;
   (d) when R₄₃ and R₄₄ are hydrogen and A₃ is -NR₄₁R₄₂ wherein R₄₁ is C₃-C₇ cycloalkyl and R₄₂ is C₂-C₆ alkenyl, phenyl-(C₁-C₆ alkylene) or hetero-(C₁-C₆ alkylene) wherein the hetero radical is furyl, thienyl or pyridinyl, and wherein said phenyl may be substituted with fluoro, chloro, bromo or iodo, then R₄₅ is not tetrahydrofuranyl or tetrahydropyranyl;
   (e) when R₄₃ is methoxy, methylthio or methylsulfonyl, R₄₄ is hydrogen, and R₄₅ is tetrahydrofuranyl or tetrahydropyranyl, then A₃ is not -NH(C₁-C₂ alkyl), morpholinyl, hydrazino or -NHC₂H₄C₆H₅, wherein the phenyl may be substituted with one methyl or two methoxy;
   (f) when R₄₃ is hydrogen, C₁-C₆ alkyl, chloro, bromo, -SH or -S-(C₁-C₄ alkyl), R₄₄ is hydrogen, and R₄₅ is C₃-C₈ cycloalkyl, then A₃ is not hydrazino, -NH(C₁-C₂ alkyl) or -N(C₁-C₆ alkyl)(C₁-C₁₂ alkyl);
   (g) when R₄₃ and R₄₄ are hydrogen and A₃ is -NH(CH₂)ₘCOOH wherein m is 1 to 12, then R₄₅ is not phenyl substituted with one of fluoro, chloro, bromo or iodo;
   (h) when R₄₃ is hydrogen, hydroxy, methylthio, chloro or -NHbenzyl, R₄₄ is hydrogen, and R₄₅ is chlorophenyl or bromophenyl, then A₃ is not -NH(C₁-C₁₂ alkyl), -NHallyl, or -N(C₁-C₆ alkyl) (C₁-C₁₂ alkyl), wherein said C₁-C₁₂ alkyl may be substituted with -NC₂H₅, or -NH benzyl which may be substituted with one or two of bromo, chloro, fluoro, -NC₂H₅ phenyl or morpholinopropyl;
   (i) when R₄₃ and R₄₄ are hydrogen and R₄₅ is nitrophenyl, then A₃ is not -NHR₄₂ wherein R₄₂ is phenyl, benzyl or C₁-C₁₂ alkyl which may be substituted by one or two of hydroxy;
   (j) when R₄₃ is chloro or -O(C₁-C₆ alkyl), R₄₄ is hydrogen, and A₃ is -NR₄₁R₄₂ wherein R₄₁ and R₄₂ are independently hydrogen or C₁-C₆ alkyl, then R₄₅ is not chlorophenyl; and
   (k) when R₄₃ is hydrogen, A₃ is benzyl or phenethyl, and R₄₄ is fluoro, chloro, bromo or iodo, then R₄₅ is not 5'-deoxy-ribofuranosyl or 5'-amino-5'-deoxyribofuranosyl, or its pharmaceutically acceptable salt;
(8) A pharmaceutical composition for prophylaxis or therapy of a postoperative stress according to the above (1) or (2), wherein the compound having CRF antagonist activating effect is a compound represented by the formula Ib 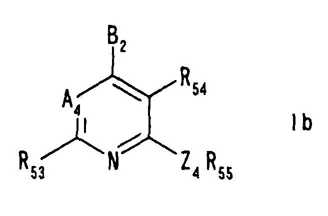 or IIb 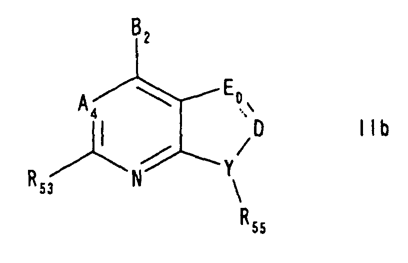 wherein the dashed line represents an optional double bond;
   A₄ is -CR₅₇ or nitrogen;
   B₂ is -NR₅₁R₅₂, -CR₅₁R₅₂R₅₁₁, -C(=CR₅₁R₅₁₂)R₅₂, -NHCR₅₁₁R₅₁R₅₂, -OCR₅₁₁R₅₁R₅₂, -SCR₅₁₁R₅₁R₅₂, -CR₅₁₁R₅₂OR₅₁, -CR₅₁₁R₅₂SR₅₁, -C(S)R₅₂, -NHNR₅₁R₅₂, -CR₅₂R₅₁₁NHR₅₁ or -C(O)R₅₂;
   D is (i) nitrogen or -CR₅₁₀ when a double bond connects E₀ and D and E₀ is -CR₅₄; (ii) -CR₅₁₀ when a double bond connects E₀ and D and E₀ is nitrogen; or (iii) -CR₅₈R₅₉, -CHR₅₁₀, -C=O, -C=S, -C=NH or -C=NCH₃ when a single bond connects E₀ and D;
   E₀ is -CR₅₄ or nitrogen when a double bond connects E₀ and D, and E₀ is -CR₅₄R₅₆ or -NR₅₆ when a single bond connects E₀ and D;
   Y is nitrogen or -CH;
   Z₄ is NH, oxygen, sulfur, -N(C₁-C₂ alkyl) or -CR₅₁₂R₅₁₃, wherein R₅₁₂ and R₅₁₃ are each independently hydrogen, trifluoromethyl or methyl, or one of R₅₁₂ and R₅₁₃ is cyano and the other is hydrogen or methyl;
   R₅₁ is hydrogen or C₁-C₆ alkyl which may optionally be substituted with one or two substituents independently selected from hydroxy, cyano, nitro, fluoro, chloro, bromo, iodo, CF₃, C₁-C₄ alkoxy, -O-CO-(C₁-C₄ alkyl), -O-CO-NH(C₁-C₄ alkyl), -O-CO-N(C₁-C₄ alkyl)(C₁-C₂ alkyl), -NH(C₁-C₄ alkyl), -N(C₁-C₂ alkyl)(C₁-C₄ alkyl), -S(C₁-C₄ alkyl), -N(C₁-C₄ alkyl)CO(C₁-C₄ alkyl), -NHCO(C₁-C₄ alkyl), -CO₂(C₁-C₄ alkyl), -CONH(C₁-C₄ alkyl), -CON(C₁-C₄ alkyl)(C₁-C₂ alkyl), (C₁-C₄ alkyl)sulfinyl, (C₁-C₄ alkyl)sulfonyl and (C₁-C₄ alkyl)sulfanyl, and wherein said C₁-C₆ alkyl, C₁-C₄ alkoxy and C₁-C₄ alkyl moieties in the foregoing R₅₁ groups optionally contain one double or triple bond;
   R₅₂ is C₁-C₆ alkyl, aryl or (aryl)(C₁-C₄ alkyl) wherein said aryl and aryl moieties of said (aryl)(C₁-C₄ alkyl) are selected from the group consisting of phenyl, naphthyl, thienyl, benzothienyl, pyridyl, quinolyl, pyrazinyl, pyrimidyl, imidazolyl, furanyl, benzofuranyl, benzothiazolyl, thiazolyl, isothiazolyl, benzisothiazolyl, benzisoxazolyl, benzimidazolyl, indolyl and benzoxazolyl; or R₅₂ is C₃-C₈ cycloalkyl or (C₃-C₈ cycloalkyl)(C₁-C₆ alkyl), wherein one or two of the ring carbons of said cycloalkyl moieties of said (C₃-C₈ cycloalkyl )(C₁-C₆ alkyl) having at least 4 ring members and cycloalkyl having at least 4 ring members is optionally replaced by oxygen, sulfur or -NR₅₀₄ wherein R₅₁₄ is hydrogen or C₁-C₄ alkyl; and wherein each of the foregoing R₅₂ groups is optionally substituted with from one to three substituents independently selected from chloro, fluoro and C₁-C₄ alkyl, or one substituent selected from bromo, iodo, cyano, nitro, C₁-C₆ alkoxy, -O-CO-(C₁-C₄ alkyl), -O-CO-N(C₁-C₄ alkyl)(C₁-C₂ alkyl), -CO₂(C₁-C₄ alkyl), (C₁-C₄ alkyl)sulfanyl, (C₁-C₄ alkyl)sulfinyl and (C₁-C₄ alkyl)sulfonyl, and wherein said C₁-C₆ alkyl, C₁-C₄ alkyl moieties and C₁-C₆ alkyl moieties of the foregoing R₅₂ groups optionally contain one carbon-carbon double or triple bond; or R₅₁ and R₅₂ of said -NR₅₁R₅₂ and said -CR₅₁R₅₂R₅₁₁ are taken together to form a saturated 5 to 8 member ring, wherein said ring optionally contains one or two carbon-carbon double bonds, and wherein one or two of the ring carbons is optionally replaced by oxygen or sulfur;
   R₅₃ is hydrogen, C₁-C₆ alkyl, fluoro, chloro, bromo, iodo, hydroxy, amino, SH, -NH(C₁-C₄ alkyl),-N(C₁-C₄ alkyl)(C₁-C₂ alkyl), -CH₂OH, -CH₂OCH₃, -O(C₁-C₄ alkyl), (C₁-C₄ alkyl)sulfanyl, (C₁-C₄ alkyl)sulfonyl, or (C₁-C₄ alkyl)sulfinyl, wherein said C₁-C₆ alkyl and C₁-C₄ alky moieties of the foregoing R₅₃ groups optionally contain one double or triple bond and are optionally substituted with from one to three substituents independently selected from hydroxy, amino, C₁-C₃ alkoxy, -NH(C₁-C₂ alkyl), -N(C₁-C₂)₂, -NHCOCH₃, fluoro, chloro and C₁-C₃ thioalkyl;
   R₅₄ is hydrogen, C₁-C₆ alkyl, fluoro, chloro, bromo, iodo, C₁-C₆ alkoxy, formyl, trifluoromethoxy, -CH₂OCH₃, -CH₂OCH₂CH₃, -CH₂CH₂OCH₃, -CH₂CF₃, CF₃, amino, nitro, -NH(C₁-C₄ alkyl), -N(CH₃)₂, -NHCOCH₃, -NHCONHCH₃, (C₁-C₄ alkyl)sulfanyl, (C₁-C₄ alkyl)sulfinyl, (C₁-C₄ alkyl)sulfonyl, cyano, hydroxy, -CO(C₁-C₄ alkyl), -CHO, or -CO₂ (C₁-C₄ alkyl), wherein said C₁-C₆ alkyl, C₁-C₆ alkoxy and the C₁-C₄ alkyl moieties of the foregoing R₅₄ groups optionally contain one double or triple bond and are optionally substituted with one substituent selected from hydroxy, amino, -NHCOCH₃, -NH(C₁-C₂ alkyl), -N(C₁-C₂ alkyl)₂, -CO₂(C₁-C₄ alkyl), -CO(C₁-C₄ alkyl), C₁-C₃ alkoxy, (C₁-C₃ alkyl)sulfany, fluoro, chloro, cyano and nitro;
   R₅₅ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, azaindolyl, benzoxazolyl, oxazolyl, pyrrolidinyl, thiazolidinyl, morpholinyl, pyridinyl, tetrazolyl, or 3 to 8 membered cycloalkyl or 9 to 12 membered bicycloalkyl, wherein said cycloalkyl and bicycloalkyl optionally contain one or two of oxygen, sulfur or -N-G₀ wherein G₀ is hydrogen, C₁-C₄ alkyl, C₁-C₄ alkanoyl, phenyl or benzyl, wherein each of the above R₅₅ groups is optionally substituted with from one to three substituents independently selected from fluoro, chloro, C₁-C₆ alkyl, C₁-C₆ alkoxy and trifluoromethyl, or one substituent selected from bromo, iodo, cyano, nitro, amino, - NH(C₁-C₄ alkyl), -N(C₁-C₄ alkyl)(C₁-C₂ alkyl), -CO₂(C₁-C₄ alkyl), -CO(C₁-C₄ alkyl), -SO₂NH(C₁-C₄ alkyl), -SO₂N(C₁-C₄ alkyl)(C₁-C₂ alkyl), -SO₂NH₂, -NHSO₂(C₁-C₄ alkyl), -S(C₁-C₄ alkyl) and -SO₂(C₁-C₄ alkyl), wherein said C₁-C₄ alkyl moieties and C₁-C₆ alkyl moieties of the foregoing R₅₅ groups optionally contain one double or triple bond and are optionally substituted with one or two substituents independently selected from fluoro, chloro, hydroxy, amino, methylamino, dimethylamino and acetyl;
   R₅₆ is hydrogen or C₁-C₆ alkyl, wherein said C₁-C₆ alkyl is optionally substituted with one of hydroxy, methoxy, ethoxy or fluoro;
   R₅₇ is hydrogen, C₁-C₄ alkyl, fluoro, chloro, bromo, iodo, cyano, hydroxy, C₁-C₄ alkoxy, -CO(C₁-C₄ alkyl), -CO₂(C₁-C₄ alkyl) -OCF₃, CF₃, -CH₂OH, -CH₂OCH₃ or -CH₂OCH₂CH₃;
   R₅₈ and R₅₉ are each independently hydrogen, hydroxy, methyl, ethyl, methoxy or ethoxy; or R₅₈ and R₅₉ taken together form an oxo (=O) group;
   R₅₁₀ is hydrogen, C₁-C₆ alkyl, fluoro, chloro, bromo, iodo, C₁-C₆ alkoxy, formyl, amino, -NH(C₁-C₄ alkyl), -N(C₁-C₄ alkyl)(C₁-C₂ alkyl), -SOₙ(C₁-C₄ alkyl) wherein n is 0, 1 or 2, cyano, carboxy or amido, wherein said C₁-C₆ alkyl and C₁-C₄ alkyl moieties of the foregoing R₅₁₀ groups are optionally substituted with one of hydroxy, trifluoromethyl, amino, carboxy, amido, -NHCO(C₁-C₄ alkyl), -NH(C₁-C₄ alkyl), -N(C₁-C₄ alkyl)(C₁-C₂ alkyl), -CO₂(C₁-C₄ alkyl), C₁-C₃ alkoxy, C₁-C₃ thioalkyl, fluoro, bromo, chloro, iodo, cyano or nitro; and,
   R₅₁₁ is hydrogen, hydroxy, fluoro, or methoxy, or its pharmaceutically acceptable salt;
(9) A pharmaceutical composition for prophylaxis or therapy of a postoperative stress according to the above (1) or (2), wherein the compound having CRF antagonist activating effect is a compound represented by the formula Ic, IIc or IIIc 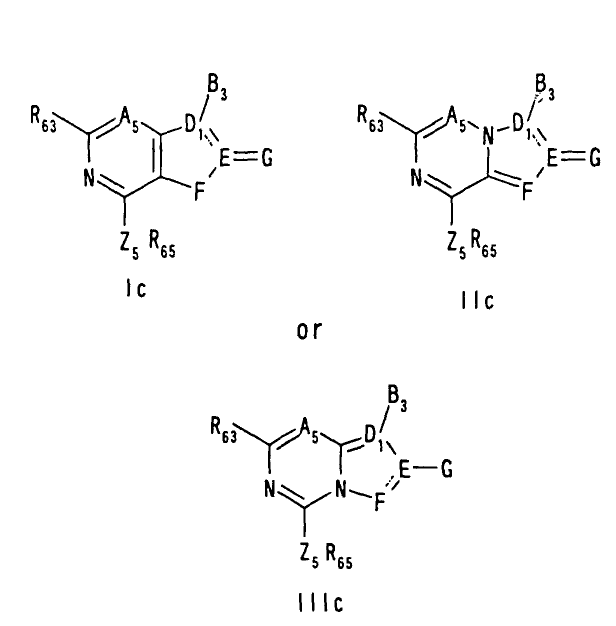 wherein the dashed lines represent optional double bonds;
   A₅ is nitrogen or CR₆₇;
   B₃ is -NR₆₁R₆₂, -CR₆₁R₆₂R₆₁₀, -C(=CR₆₂R₆₁₁)R₆₁, -NHCR₆₁R₆₂R₆₁₀, -OCR₆₁R₆₂R₆₁₀, -SCR₆₁R₆₂R₆₁₀, -CR₆₂R₆₁₀NHR₆₁, -CR₆₂R₆₁₀OR₆₁, -CR₆₂R₆₁₀SR₆₁ or -COR₆₂;
   D₁ is nitrogen and is single bonded to all atoms to which it is attached, or D₁ is carbon and is either double bonded to E in formulas Ic and IIc or double bonded to the adjacent carbon to both fused rings in formulas IIIc, or D₁ is CH and is single bonded to E in formula Ic and IIc;
   E is nitrogen, CH or carbon;
   F is oxygen, sulfur, CHR₆₄ or NR₆₄ when it is single bonded to E, and F is nitrogen or CR₆₄ when it is double bonded to E;
   G, when it is single bonded to E, is hydrogen, C1-C4 alkyl, -S(C₁-C₄ alkyl), -O(C₁-C₄ alkyl), NH₂, -NH(C₁-C₄ alkyl) or -N(C₁-C₂ alkyl)(C₁-C₄ alkyl), wherein each of the C₁-C₄ alkyl groups of G may optionally be substituted with one of hydroxy, -O(C₁-C₂ alkyl) or fluoro; G, when it is double bonded to E, is oxygen, sulfur or NH; and G, when E is nitrogen and double bonded to D₁ or F, is absent;
   R₆₁ is hydrogen, C₁-C₆ alkyl optionally substituted with one or two substituents R₆₈ independently selected from hydroxyl, fluoro, chloro, bromo, iodo, C₁-C₄ alkoxy, CF₃, -C(=O)O-(C₁-C₄) alkyl, -OC(=O)(C₁-C₄ alkyl), -OC(=O)N(C₁-C₄ alkyl)(C₁-C₂ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -S(C₁-C₄ alkyl), -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl), and wherein each of the C₁-C₄ alkyl groups in the foregoing R₆₁ groups may optionally contain one or two double or triple bonds;
   R₆₂ is C₁-C₁₂ alkyl which may optionally contain from one to three double or triple bonds, aryl or (C₁-C₄ alkylene)aryl, wherein said aryl and aryl moiety of said (C₁-C₄ alkylene)aryl is selected from phenyl, naphthyl, thienyl, benzothienyl, pyridyl, quinolyl, pyrazinyl, pyrimidyl, imidazolyl, furanyl, benzofuranyl, benzothiazolyl, isothiazolyl, pyrazolyl, pyrrolyl, indolyl, pyrrolopyridyl, oxazolyl and benzoxazolyl, or C₃-C₈ cycloalkyl or (C₁-C₆ alkylene)(C₃-C₈ cycloalkyl), wherein one or two of the carbon atoms of said cycloalkyl and 5 to 8 membered cycloalkyl moieties of said (C₁-C₆ alkylen)(C₃-C₈ cycloalkyl) may optionally and independently be replaced by oxygen, sulfur or NZ₆₂ wherein Z₆₂ is selected from hydrogen, C₁-C₄ alkyl, benzyl and C₁-C₄ alkanoyl, and wherein each of the foregoing R₆₂ groups may optionally be substituted with from one to three substituents independently selected from chloro, fluoro, hydroxyl and C₁-C₄ alkyl, or one substituent selected from bromo, iodo, C₁-C₆ alkoxy, -OC(=O)(C₁-C₆ alkyl), -OC(=O)N(C₁-C₄ alkyl)(C₁-C₂ alkyl), -S(C₁-C₆ alkyl), amino, -NH(C₁-C₂ alkyl), -N(C₁-C₂ alkyl)(C₁-C₄ alkyl), -N(C₁-C₄ alkyl)-CO-(C₁-C₄ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -SH, -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl); -NR₆₁R₆₂ or CR₆₁R₆₂R₆₁₀ may form a saturated 3 to 8 membered carbocyclic ring which may optionally contain from one to three double bonds and wherein one or two of the ring carbon atoms of such 5 to 8 membered rings may optionally and independently be replaced by oxygen, sulfur or NZ₆₃ wherein Z₆₃ is hydrogen, C₁-C₄ alkyl, benzyl or C₁-C₄ alkanoyl;
   R₆₃ is hydrogen, C₁-C₄ alkyl, -O(C₁-C₄ alkyl), chloro, fluoro, bromo, iodo, -CN, -S(C₁-C₄ alkyl) or -SO₂(C₁-C₄ alkyl) wherein each of the (C₁-C₄ alkyl) moieties in the foregoing R₆₃ groups may optionally be substituted with one substituent R₆₉ selected from hydroxy, fluoro, (C₁-C₂ alkoxy);
   R₆₄ is each independently hydrogen, (C₁-C₆ alkyl), fluoro, chloro, bromo, iodo, hydroxy, cyano, amino, nitro, -O(C₁-C₄ alkyl), -N(C₁-C₄ alkyl)(C₁-C₂ alkyl), -S(C₁-C₄ alkyl), -SO(C₁-C₄ alkyl), -SO₂(C₁-C₄ alkyl), -CO(C₁-C₄ alkyl), -C(=O)H or -C(=O)O(C₁-C₄ alkyl), wherein each of the (C₁-C₆ alkyl) and (C₁-C₄ alkyl) moieties in the foregoing R₆₄ groups may optionally contain one or two double or triple bonds and may optionally be substituted with one or two substituents independently selected from hydroxy, amino, C₁-C₃ alkoxy, dimethylamino, methylamino, ethylamino, -NHC(=O)CH₃, fluoro, chloro, C₁-C₃ thioalkyl, -CN, -COOH, -C(=O)O(C₁-C₄ alkyl), -C(=O)(C₁-C₄ alkyl) and -NO₂;
   R₆₅ is phenyl, naphthyl, thienyl, benzothienyl, pyridyl, quinolyl, pyrazinyl, furanyl, benzofuranyl, benzothiazolyl, benzisothiazolyl, benzisoxazolyl, benzimidazolyl, indolyl, benzoxazolyl or C₃-C₈ cycloalkyl, wherein one or two of the carbon atoms of said cycloalkyl rings that contain at least 5 ring members may optionally and independently be replaced by oxygen, sulfur or NZ₆₄ wherein Z₆₄ is hydrogen, C₁-C₄ alkyl or benzyl; and wherein each of the foregoing R₆₅ groups may optionally be substituted with from one to four substituents R₆₁₂, wherein one to three of said substituents may be selected independently from chloro, C₁-C₆ alkyl and -O(C₁-C₆ alkyl) and one of said substituents may be selected from bromo, iodo, formyl, -CN, -CF₃, -NO₂, -NH₂, -NH(C₁-C₄ alkyl), -N(C₁-C₂ alkyl)(C₁-C₆ alkyl), -C(=O)O(C₁-C₄ alkyl), -C(=O)(C₁-C₄ alkyl), -COOH, -SO₂NH(C₁-C₄ alkyl), -SO₂N(C₁-C₂ alkyl)(C₁-C₄ alkyl), -SO₂NH₂, -NHSO₂(C₁-C₄ alkyl), -S(C₁-C₆ alkyl) and -SO₂(C₁-C₆ alkyl), and wherein each of the C₁-C₄ alkyl and C₁-C₆ alkyl moieties in the foregoing R₆₅ groups may optionally be substituted with one or two substituents independently selected from fluoro, hydroxyl, amino, methylamino, dimethylamino and acetyl;
   R₆₇ is hydrogen, C₁-C₄ alkyl, halo, cyano, hydroxy, -O(C₁-C₄ alkyl), -C(=O)(C₁-C₄ alkyl), -C(=O)O(C₁-C₄ alkyl), -OCF₃, -CF₃, -CH₂OH, -CH₂O(C₁-C₄ alkyl);
   R₆₁₀ is hydrogen, hydroxy, methoxy or fluoro;
   R₆₁₁ is hydrogen or C₁-C₄ alkyl; and
   Z₅ is NH, oxygen, sulfur, -N(C₁-C₄ alkyl), -NC(=O)(C₁-C₂ alkyl) NC(=O)O(C₁-C₂ alkyl) or CR₆₁₃R₆₁₄ wherein R₆₁₃ and R₆₁₄ are independently selected from hydrogen, trifluoromethyl and methyl with the exception that one of R₆₁₃ and R₆₁₄ can be cyano; with the proviso that (a) in the five membered rings of structures Ic, IIc and IIIc, there can not be two double bonds adjacent to each other; and (b) when R₆₄ is attached to nitrogen, it is not halo, cyano or nitro,
   or its pharmaceutically acceptable salt;
(10) A pharmaceutical composition for prophylaxis or therapy of a postoperative stress according to the above (1) or (2), wherein the compound having CRF antagonist activating effect is a compound represented by the formula Id 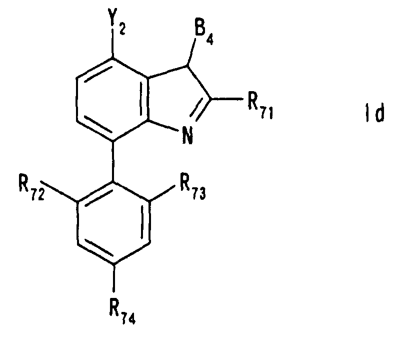 wherein B₄ is hydrogen or (C₁-C₁₀) linear or branched alkyl;
   Y₂ is hydrogen or methyl;
   R₇₁ is hydrogen or (C₁-C₁₀) linear or branched alkyl, wherein said alkyl may optionally be substituted with one or more substituents selected from halo, -S(C₁-C₄)alkyl, amino, -NH(C₁-C₄)alkyl and -N((C₁-C₄)alkyl))₂;
   R₇₂, R₇₃ and R₇₄ are selected independently from hydrogen, fluoro, chloro, (C₁-C₆)alkyl and (C₁-C₆)alkoxy; or two of R₇₂, R₇₃, and R₇₄ are hydrogen and the other is selected from hydroxy, iodo, bromo, formyl, cyano, nitro, trifluoromethyl, amino, (C₁-C₆)alkyl-O-(C₁-C₆)alkyl, -NHCH₃, -N(CH₃)₂, -COCH, -COO(C₁-C₄)alkyl, -CO(C₁-C₄)alkyl, -SO₂-NH(C₁-C₆)alkyl, -SO₂-N((C₁-C₄)alkyl)₂, -SO₂NH₂, -NHSO₂-(C₁-C₄)alkyl, -S(C₁-C₆)alkyl and -SO₂-(C₁-C₆)alkyl, and wherein said C₁-C₄ moieties and C₁-C₆ alkyl moieties in the foregoing R₇₂, R₇₃ and R₇₄ groups may optionally be substituted with one or two of fluoro or one substituent selected from hydroxy, amino, methylamino, dimethylamino and acetyl,
   or its pharmaceutically acceptable salt;
(11) A pharmaceutical composition for prophylaxis or therapy of a postoperative stress according to the above (1) or (2), wherein the compound having CRF antagonist activating effect is a compound represented by the formula Ie 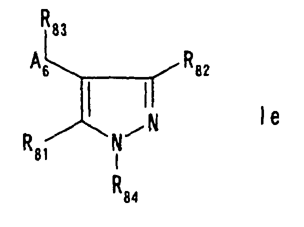 wherein A₆ is C=O or SO₂, or A₆ and R₈₁ together with the carbons to which they are attached form pyrimidinyl or 5-pyridyl which may be substituted with R₈₅ wherein R₈₅ is hydrogen, C₁-C₆ alkyl, fluoro, chloro, bromo, hydroxy, amino, O(C₁-C₆ alkyl), NH(C₁-C₆ alkyl), N(C₁-C₆ alkyl)(C₁-C₆ alkyl), SH or S(O)ₙ(C₁-C₆ alkyl) wherein n is 0, 1 or 2, and said C₁-C₆ alkyl may contain one or two double or triple bonds and may be substituted with from one to three substituents R₈₆, wherein R₈₆ is hydroxy, amino, C₁-C₃ alkoxy, dimethylamino, diethylamino, methylamino, ethylamino, NH(C=O)CH₃, fluoro, chloro, bromo or C₁-C₃ thioalkyl;
   R₈₁ is hydrogen, C₁-C₆ alkyl, amino, O(C₁-C₆ alkyl), NH(C₁-C₆ alkyl), N(C₁-C₆ alkyl)(C₁-C₆ alkyl), wherein said C₁-C₆ alkyl may optionally contain one or two double or triple bonds and may be substituted with from one to three substituents R₈₆ as defined above;
   R₈₂ is hydrogen, C₁-C₆ alkyl, hydroxy, amino, O(C₁-C₆ alkyl), NH(C₁-C₆ alkyl), N(C₁-C₆ alkyl)(C₁-C₆ alkyl), SH, S(O)ₙ(C₁-C₆ alkyl) wherein n is 0, 1, or 2, cyano, hydroxy, carboxy or amido, wherein said alkyl may be substituted with one to three of hydroxy, amino, carboxy, amido, NH(C=O)(C₁-C₆ alkyl), N(C₁-C₆ alkyl)(C₁-C₆ alkyl), (C=O)O(C₁-C₆ alkyl), C₁-C₃ alkoxy, C₁-C₃ thioalkyl, fluoro, bromo, chloro, iodo, cyano or nitro;
   R₈₃ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, azaindolyl, benzoxazolyl, oxazolyl, pyrrolidinyl, thiazolidinyl, morpholinyl, pyridinyl, tetrazolyl, or 3 to 8 membered cycloalkyl or 9 to 12 membered bicycloalkyl, optionally containing one to three of oxygen, sulfur or N-Z₆ wherein Z₆ is hydrogen, C₁-C₄ alkyl, C₁-C₄ alkanoyl, phenyl or phenylmethyl, wherein each one of the above groups may be substituted independently with from one to three of fluoro, chloro, bromo, trifluoromethyl, C₁-C₆ alkyl or C₁-C₆ alkoxy, or one or two of cyano, nitro, amino, NH(C₁-C₆ alkyl), N(C₁-C₄ alkyl)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NHSO₂ (C₁-C₄ alkyl), S(C₁-C₆ alkyl) or SO₂(C₁-C₆ alkyl), wherein said C₁-C₄ alkyl and C₁-C₆ alkyl may be substituted with fluoro, chloro, hydroxy, amino, methylamino, dimethylamino or acetyl; and
   R₈₄ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, azaindolyl, benzoxazolyl, oxazolyl, pyrrolidinyl, thiazolidinyl, morpholinyl, pyridinyl, tetrazolyl, or 9 to 12 membered bicycloalkyl, optionally containing one to three of oxygen, sulfur or N-Z₆ wherein Z₆ is hydrogen, C₁-C₄ alkyl, C₁-C₄ alkanoyl, phenyl or phenylmethyl, wherein each of the above groups may be substituted independently with from one to three of fluoro, chloro, bromo, trifluoromethyl, C₁-C₆ alkyl or C₁-C₆ alkoxy, or one of cyano, nitro, amino, NH(C₁-C₆ alkyl), N(C₁-C₄ alkyl)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NHSO₂(C₁-C₄ alkyl), S(C₁-C₆ alkyl) or SO₂(C₁-C₆ alkyl), wherein said C₁-C₄ alkyl and C₁-C₆ alkyl may be substituted with one or two of fluoro, chloro, hydroxy, amino, methylamino, dimethylamino or acetyl; with the proviso that (1) R₈₄ is not unsubstituted phenyl; (2) when R₈₁ is amino, R₈₂ is methylthio, R₈₄ is 2,4,6-trifluorophenyl, and A₆ is C=O, then R₈₃ is not 2-chloropheny,
   or its pharmaceutically acceptable salt;
(12) A pharmaceutical composition for prophylaxis or therapy of a postoperative stress according to the above (1) or (2), wherein the compound having CRF antagonist activating effect is a compound represented by the formula If 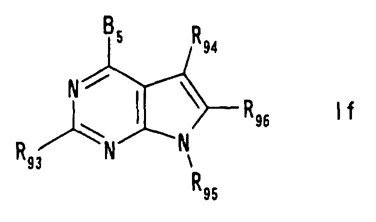 wherein B₅ is NR₉₁R₉₂, CR₉₁R₉₂R₉₁₁, C(=CR₉₂R₉₁₂)R₉₁, NHCR₉₁R₉₂R₉₁₁, OCR₉₁R₉₂R₉₁₁, SCR₉₁R₉₂R₉₁₁, NHNR₉₁R₉₂, CR₉₂R₉₁₁NHR₉₁, CR₉₂R₉₁₁OR₉₁, CR₉₂R₉₁₁SR₉₁ or C(O)R₉₂;
   R₉₁ is hydrogen, or C₁-C₆ alkyl which may be substituted with one or two substituents R₉₇ independently selected from the group consisting of hydroxy, fluoro, chloro, bromo, iodo, C₁-C₈ alkoxy, O-C(=O)-(C₁₋₆ alkyl), O-C(=O)-NH(C₁₋₄ alkyl), O-C(=O)-N(C₁₋₄ alkyl)(C₁₋₂ alkyl), amino, NH(C₁₋₄ alkyl), N(C₁₋₂ alkyl)(C₁₋₄ alkyl), S(C₁₋₆ alkyl), N(C₁₋₄ alkyl)C(=O)(C₁₋₄ alkyl), NHC(=O)(C₁₋₄ alkyl), COOH, C(=O)O(C₁₋₄ alkyl), C(=O)NH(C₁₋₄ alkyl), C(=O)N(C₁₋₄ alkyl)(C₁₋₂ alkyl), SH, CN, NO₂, SO(C₁₋₄ alkyl), SO₂(C₁₋₄ alkyl), SO₂NH(C₁₋₄ alkyl) and SO₂N(C₁₋₄ alkyl)(C₁₋₂ alkyl), and wherein said C₁-C₆ alkyl may contain one or two double or triple bonds;
   R₉₂ is C₁-C₁₂ alkyl, aryl or (C₁-C₁₀ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiolyl, thiazolyl, isoxazolyl, benzisoxazolyl, benzimidazolyl, triazolyl, pyrazolyl, pyrrolyl, indolyl, pyrrolpyridyl, oxazolyl or benzoxazolyl, or 3 to 8 membered cycloalkyl or (C₁-C₆ alkylene) cycloalkyl, wherein said cycloalkyl may contain one or two of oxygen, sulfur or N-Z₇ wherein Z₇ is hydrogen, C₁-C₄ alkyl, benzyl or C₁-C₄ alkanoyl, wherein R₉₂ may be substituted independently by from one to three of chloro, fluoro, or C₁-C₄ alkyl, or one of hydroxy, bromo, iodo, C₁-C₆ alkoxy, O-C(=O)-(C₁₋C₆ alkyl), O-C(=O)-N(C₁₋C₄ alkyl)(C₁₋C₂ alkyl), S(C₁₋C₆ alkyl), NH₂, NH(C₁₋C₂ alkyl), N(C₁₋C₂ alkyl)(C₁₋C₄ alkyl), N(C₁₋C₄ alkyl)C(=O)(C₁₋C₄ alkyl), NHC(=O)(C₁₋C₄ alkyl), COOH, C(=O)O(C₁₋C₄ alkyl), C(=O)NH(C₁₋C₄ alkyl), C(=O)N(C₁₋C₄ alkyl)(C₁₋C₂ alkyl), SH, CN, NO₂, SO(C₁₋C₄ alkyl), SO₂(C₁₋C₄ alkyl), SO₂NH(C₁₋C₄ alkyl), SO₂N(C₁₋C₄ alkyl)(C₁₋C₂ alkyl), and wherein said C₁-C₁₂ alkyl or C₁-C₁₀ alkylene may contain one to three double or triple bonds; or NR₉₁R₉₂ or CR₉₁R₉₂R₉₁₁ may form a saturated 3 to 8 membered carbocyclic ring of which the 5 to 8 membered ring may optionally contain one or two double bonds or one or two of O, S or N-Z₇ wherein Z₇ is hydrogen, C₁-C₄ alkyl, benzyl, or C₁-C₄ alkanoyl;
   R₉₃ is hydrogen, C₁-C₆ alkyl, fluoro, chloro, bromo, iodo, hydroxy, amino, O(C₁₋C₆ alkyl), NH(C₁₋C₆ alkyl), N(C₁₋C₄ alkyl)(C₁₋C₂ alkyl), SH, S(C₁₋C₄ alkyl), SO(C₁₋C₄ alkyl) or SO₂(C₁₋C₄ alkyl), wherein said C₁-C₄ alkyl and C₁-C₆ alkyl may contain one double or triple bond and may be substituted with from one to three substituents R₉₈ independently selected from the group consisting of hydroxy, C₁-C₃ alkoxy, fluoro, chloro or C₁-C₃ thioalkyl;
   R₉₄ is hydrogen, C₁-C₆ alkyl, fluoro, chloro, bromo, iodo, C₁-C₆ alkoxy, amino, NH(C₁-C₆ alkyl), N(C₁-C₆ alkyl)(C₁-C₂ alkyl), SOₙ(C₁-C₆ alkyl) wherein n is 0, 1 or 2, cyano, hydroxy, carboxy, or amido, wherein said C₁-C₆ alkyl may be substituted with one of hydroxy, trifluoromethyl, amino, carboxy, amido, NHC(=O)(C₁₋₄ alkyl), NH(C₁₋₄ alkyl), N(C₁₋₄ alkyl)(C₁₋₂ alkyl), C(=O)O(C₁₋₄ alkyl), C₁-C₃ alkoxy, C₁-C₄ thioalkyl, fluoro, bromo, chloro, iodo, cyano or nitro;
   R₉₅ is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, pyrrolopyridyl, benzoxazolyl, oxazolyl, pyrrolidinyl, thiazolidinyl, morpholinyl, piperidinyl, piperazinyl, tetrazolyl, or 3 to 8 membered cycloalkyl or 9 to 12 membered bicycloalkyl, optionally containing one or two of oxygen, sulfur or N-Z₇ wherein Z₇ is hydrogen, C₁-C₄ alkyl, C₁-C₄ alkanoyl, phenyl or phenylmethyl, wherein each one of the above groups may be substituted independently with from one to three of fluoro, chloro, bromo, formyl, C₁-C₆ alkyl, C₁-C₆ alkoxy or trifluoromethyl, or one of hydroxy, iodo, cyano, nitro, amino, NH(C₁₋₄ alkyl), N(C₁₋₄ alkyl)(C₁₋₂ alkyl), COO(C₁₋₄ alkyl), CO(C₁₋₄ alkyl), SO₂NH(C₁₋₄ alkyl), SO₂N(C₁₋₄ alkyl)(C₁₋₂ alkyl), SO₂NH₂, NHSO₂(C₁₋₄ alkyl), S(C₁₋₆ alkyl), SO₂(C₁₋₆ alkyl), wherein said C₁-C₄ alkyl and C₁-C₆ alkyl may be substituted with one or two of fluoro, chloro, hydroxy, C₁-C₄ alkoxy, amino, methylamino, dimethylamino or acetyl, wherein said C1-C4 alkyl and C1-C6 alkyl may contain one double or triple bond; with the proviso that R₉₅ is not unsubstituted phenyl;
   R₉₆ is hydrogen, C₁-C₆ alkyl, fluoro, chloro, bromo, iodo, C₁-C₆ alkoxy, formyl, amino, NH(C₁-C₆ alkyl), N(C₁-C₆ alkyl)(C₁-C₂ alkyl), SOₙ(C₁-C₆ alkyl) wherein n is 0, 1 or 2, cyano, carboxy or amido, wherein said C₁-C₆ alkyl may be substituted with one of hydroxy, trifluoromethyl, amino, carboxy, amido, NHC(=O)(C₁₋C₄ alkyl), NH(C₁₋C₄ alkyl), N(C₁₋C₄ alkyl)(C₁₋C₂ alkyl), C(=O)O(C₁₋C₄ alkyl), C₁-C₃ alkoxy, C₁-C₃ thioalkyl, fluoro, bromo, chloro, iodo, cyano or nitro;
   R₉₁₁ is hydrogen, hydroxy, fluoro, COO(C₁-C₂ alkyl), cyano or CO(C₁-C₂ alkyl);
   R₉₁₂ is hydrogen or C₁-C₄ alkyl, with the proviso that (1) B₅ is not C₁-C₁₂ linear alkyl; (2) when R₉₅ is unsubstituted cycloalkyl, R₉₃ and R₉₄ are hydrogen, and when R₉₆ is hydrogen or methyl, then B₅ is not NHR₉₂ wherein R₉₂ is benzyl or thienylmethyl; and (3) when R₉₅ is p-bromophenyl, and R₉₃, R₉₄ and R₉₆ are methyl, then B₅ is not methylamino or hydroxyethylamino,
   or its pharmaceutically acceptable acid addition salt;
(13) A pharmaceutical composition for prophylaxis or therapy of a postoperative stress according to the above (1) or (2), wherein the compound having CRF antagonist activating effect is a compound represented by the formula Ig 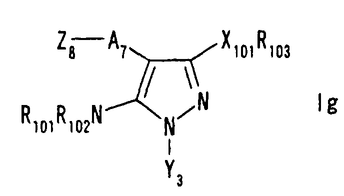 wherein A₇ is CH₂; X₁₀₁ is covalent bond, CH₂, NR₁₀₀ wherein R₁₀₀ is hydrogen, linear C₁-C₆ alkyl or branched C₃-C₈ alkyl, oxygen or sulfur;
   R₁₀₁, R₁₀₂ and R₁₀₃ are each independently linear C₁-C₆ alkyl, branched C₃-C₈ alkyl, C₃-C₈ alkenyl wherein the double bond is not adjacent to nitrogen or X₁₀₁ when X₁₀₁ is oxygen or sulfur, or C₃-C₇ cycloalkyl (CH₂)ₙ wherein n is 0, 1, 2, 3 or 4; or R₁₀₁ and R₁₀₂ may be taken together with the nitrogen to form a saturated 4, 5 or 6 membered ring which is optionally bonded with benzo, R₁₀₃ may also be (CH₂)_{q}Q₁₀₁R₁₀₁₉ wherein q is 0, 1 or 2 and Q₁₀₁ is oxygen, sulfur, NH, N(C₁-C₆ alkyl), or a covalent bond when X₁₀₁ is not a covalent bond, and R₁₀₁₉ is hydrogen, linear C₁-C₆ alkyl, branched C₃-C₈ alkyl, C₃-C₈ alkenyl or C₃-C₆ cycloalkyl(CH₂)ₙ wherein n is 0 to 4;
   Y₃ is phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl or piperidinyl, wherein each of the above groups may be substituted with one to three of any one of fluoro, chloro, bromo, or methyl, or one of trifluoromethyl; with the proviso that Y₃ is not unsubstituted phenyl; and
   Z₈ is IIg
   (a) 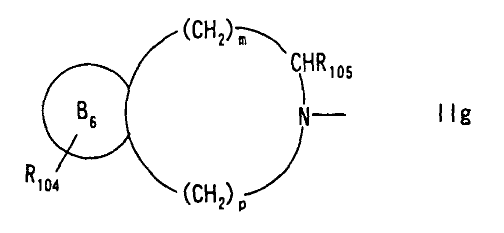 wherein the B₆ ring is phenyl, naphthyl, pyridyl, pyrimidinyl, pyrazinyl, pyridazinyl, triazilyl, pyrrolyl, pyrazolyl, imidazolyl, triazolyl, thienyl, or indolyl, wherein each of the above groups may be substituted with methyl, methoxy, fluoro, chloro, bromo or iodo; or a saturated 5 or 6 membered carbocyclic ring or a partially unsaturated ring having one or two double bonds;
      R₁₀₄ is hydrogen, C₁-C₆ alkyl, C₁-C₆ alkoxy, or hydroxy, fluoro, chloro, bromo, iodo or trifluoromethyl;
      R₁₀₅ is hydrogen, linear C₁-C₆ alkyl, branched C₃-C₈ alkyl, C₃-C₈ alkenyl or (CH₂)ₒ-X₁₀₂-(CH₂)ᵣ-Q₁₀₂-R₁₀₆;
      R₁₀₆ is hydrogen, linear C₁-C₆ alkyl, branched C₃-C₈ alkyl or C₃-C₈ alkenyl;
      X₁₀₂ and Q₁₀₂ are each independently oxygen, sulfur, NH or N(C₁-C₆ alkyl), or one of X₁₀₂ and Q₁₀₂ may be a covalent bond;
      m is 0 or 1;
      o is 1 or 2;
      p is 1 or 2;
      r is 0, 1, or 2;
   (b) 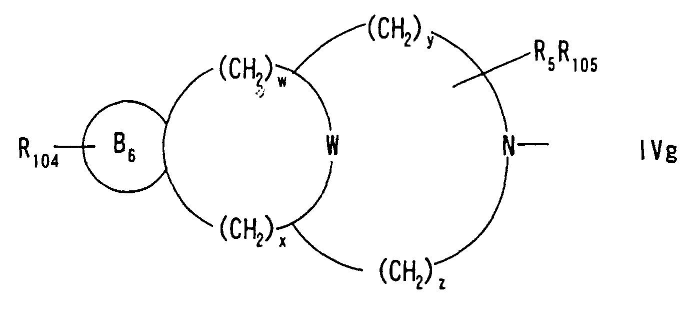 wherein R₁₀₄ and R₁₀₅ are as defined above, and t and u are each independently 1 or 2;
   (c) -NR₁₀₇R₁₀₈ wherein R₁₀₇ and R₁₀₈ are each independently hydrogen or C₁-C₆ linear alkyl, branched C₃-C₈ alkyl, C₃-C₈ alkenyl, (CH₂)ᵥCH₂OH, (CH₂)ᵥNR₁₀₉R₁₀₁₀ wherein v is 0 to 3 and R₁₀₉ and R₁₀₁₀ are each independently hydrogen or linear C₁-C₆ alkyl, C₃-C₁₂ cycloalkyl, (C₃-C₁₂ cycloalkyl)(CH₂)ₙ, (C₆-C₁₀ bicycloalkyl)(CH₂)ₙ, wherein n is 0 to 4, benzofused C₃-C₆ cycloalkyl, C₁-C₆ hydroxyalkyl, phenyl, phenyl (C₁-C₃ alkylene), wherein each of the above groups may be substituted with one or two of hydroxy, fluoro, chloro, bromo, C₁-C₅ alkyl or C₁-C₅ alkoxy; or R₁₀₇ and R₁₀₈ may be taken together with the nitrogen to form a saturated or partially unsaturated 5 to 7 membered ring which may contain one of oxygen, sulfur, NH or N(C₁-C₆ alkyl) and which may be substituted by C₁-C₆ alkyl, hydroxy or phenyl, wherein any double bonds are not adjacent to any heteroatoms; 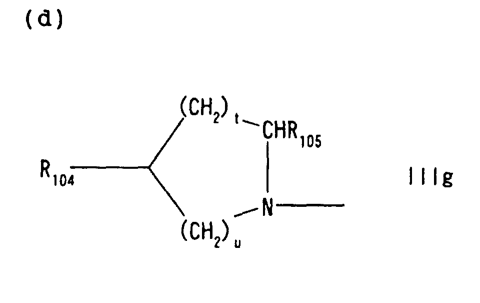 wherein B₆, R₁₀₄ and R₁₀₅ are as defined above, w, x, y and z are each independently 1 or 2, and W₁ is (CH₂)_{q} wherein q is as defined above, N(C₁-C₆ alkyl), or oxygen;
   (e) 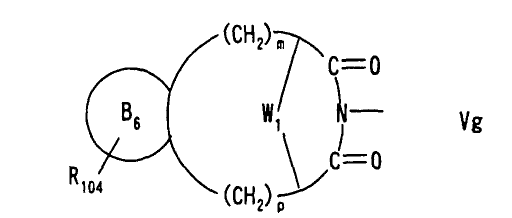 wherein B₆, W₁, R₁₀₄, m and p are as defined above;
   (f) 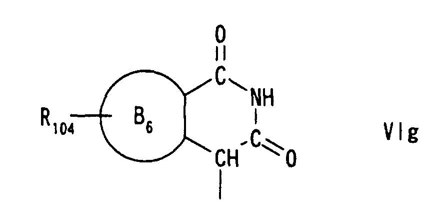 wherein B₆ and R₁₀₄ are as defined
   (g) O(CH₂)ᵥR₁₀₁₁
      wherein v is 0 to 3 and R₁₀₁₁ is linear C₁-C₆ alkyl, branched C₃-C₈ alkyl, phenyl, naphthyl, 1,2,3,4-tetrahydronaphthyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, piperidinyl, or thienyl, wherein each of the above groups may be substituted with one or two of any one of fluoro, chloro, bromo, methyl or trifluoromethyl;
   (h) 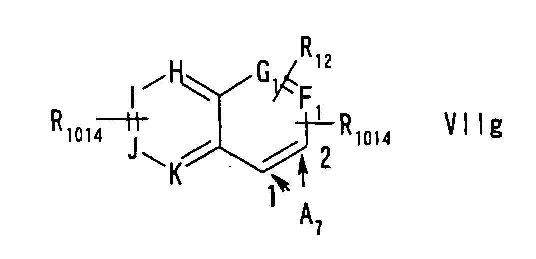 wherein A₇ is as defined above and is attached to position 1 or 2 while R₁₀₁₄ is attached to position 2 or 1 respectively; F₁, G₁, H, I, J and K are independently carbon or nitrogen, with the proviso that not more than three of H, I, J and K are nitrogen with not more than two adjacent nitrogens; R₁₀₁₂ and R₁₀₁₃ each independently are hydrogen, linear C₁-C₆ alkyl, branched C₃-C₈ alkyl, C₃-C₈ alkenyl, fluoro, chloro, bromo, trifluoromethyl, hydroxy, thiol, C₁-C₁₂ alkoxy, C₁-C₁₂ thioalkanyl, or C₃-C₁₂ alkenoxy or C₃-C₁₂ thioalkenyl wherein the double bond is not adjacent to oxygen or sulfur; and R₁₀₁₄ is hydroxy, C₁-C₁₂ alkoxy, C₃-C₁₂ alkenoxy, wherein the double bond is not adjacent to oxygen, or -X₁₀₂-(CH₂)ᵣQ₁₀₂R₁₀₆ wherein X₁₀₂, r, Q₁₀₂ and R₁₀₆ are as defined above in paragraph (a) except that Q₁₀₂ is not sulfur, or R₁₀₁₄ is NR₁₀₁₅R₁₀₁₆ wherein R₁₀₁₅ and R₁₀₁₆ are each independently hydrogen, linear C₁-C₆ alkyl, branched C₃-C₈ alkyl, C₃-C₈ alkenyl, wherein the double bond is not adjacent to nitrogen, or C₃-C₇ cycloalkyl-(CH₂)ₙ wherein n is as defined above, or R₁₀₁₅ and R₁₀₁₆ may be taken together with the nitrogen to form a saturated 5 or 6 membered ring optionally condensed with benzo; or
   (i) 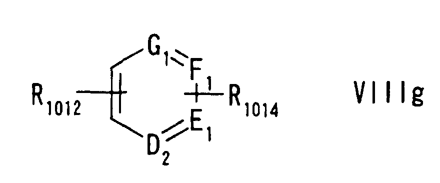 wherein D₂, E₁, F₁ and G₁ are independently carbon or nitrogen, with the proviso that not more than two of D₂, E₁, F₁ and G₁ are nitrogen, R₁₀₁₂ and R₁₀₁₄ are as defined above (h) and A₇ which is defined above is attached to a carbon in formula VIIIg, and R₁₀₁₄ is attached to a carbon located adjacent to the carbon to which A₇ is attached,
      or its pharmaceutically acceptable acid addition salt;
(14) A pharmaceutical composition for prophylaxis or therapy of a postoperative stress as according to the above (1) or (2), wherein the compound having CRF antagonist activating effect is a compound represented by the formula Ih 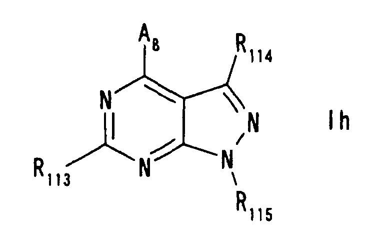 wherein A₈ is NR₁₁₁R₁₁₂, CR₁₁₁R₁₁₂R₁₁₁₁, C(=CR₁₁₁R₁₁₁₂)R₁₁₂, NHCR₁₁₁R₁₁₂R₁₁₁₁, OCR₁₁₁R₁₁₂R₁₁₁₁, SCR₁₁₁R₁₁₂R₁₁₁₁, NHNR₁₁₁R₁₁₂, CR₁₁₂R₁₁₁₁NHR₁₁₁, CR₁₁₂R₁₁₁₁OR₁₁₁, CR₁₁₂R₁₁₁₁SR₁₁₁ or C(O)R₁₁₂;
   R₁₁₁ is hydrogen, or C₁-C₆ alkyl which may be substituted with one or two substituents R₁₁₆ independently selected from the group consisting of hydroxy, fluoro, chloro, bromo, iodo, C₁-C₆ alkoxy, O-C(=O)-(C₁-C₆ alkyl), O-C(=O)-N(C₁-C₄ alkyl)(C₁-C₂ alkyl), amino, NH(C₁-C₄ alkyl), N(C₁-C₂ alkyl)(C₁-C₄ alkyl), S(C₁-C₆ alkyl), OC(O)NH(C₁-C₄ alkyl), N(C₁-C₂ alkyl)C(O)(C₁-C₄ alkyl), NHC(=O)(C₁-C₄ alkyl), COOH, C(=O)O(C₁-C₄ alkyl), C(=O)NH(C₁-C₄ alkyl), C(=O)N(C₁-C₄ alkyl)(C₁-C₂ alkyl), SH, CN, NO₂, SO(C₁-C₄ alkyl), SO₂(C₁-C₄ alkyl), SO₂NH(C₁-C₄ alkyl) and SO₂N(C₁-C₄ alkyl)(C₁-C₂ alkyl), and wherein said C₁-C₆ alkyl may contain one or two double or triple bonds;
   R₁₁₂ is C₁-C₁₂ alkyl, aryl or (C₁-C₁₀ alkylene)aryl wherein said aryl is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, azaindolyl, oxazolyl, or benzoxazolyl, or 3 to 8 membered cycloalkyl or (C₁-C₆ alkylene)cycloalkyl, wherein said cycloalkyl may contain one or two of oxygen, sulfur or N-Z₉ wherein Z₉ is hydrogen, C₁-C₄ alkyl, benzyl or C₁-C₄ alkanoyl, wherein R₁₁₂ may be substituted independently with from one to three of chloro, fluoro, or C₁-C₄ alkyl, or one of hydroxy, bromo, iodo, C₁-C₆ alkoxy, O-C(=O)-(C₁-C₆ alkyl), O-C(=O)-N(C₁-C₄ alkyl)(C₁-C₂ alkyl), S(C₁-C₆ alkyl), NH₂, NH(C₁-C₂ alkyl), N(C₁-C₂ alkyl)(C₁-C₄ alkyl), N(C₁-C₄ alkyl)C(=O)(C₁-C₄ alkyl), NHC(=O)(C₁-C₄ alkyl), COOH, C(=O)O(C₁-C₄ alkyl), C(=O)NH(C₁-C₄ alkyl),C(=O)N(C₁-C₄ alkyl)(C₁-C₂ alkyl), SH, CN, NO₂, SO(C₁-C₄ alkyl), SO₂(C₁-C₄ alkyl), SO₂NH(C₁-C₄ alkyl) or SO₂N(C₁-C₄ alkyl)(C₁-C₂ alkyl), and wherein said C₁-C₁₂ alkyl or C₁-C₁₀ alkylene may contain one to two double or triple bonds; or
   NR₁₁₁R₁₁₂ or CR₁₁₁R₁₁₂R₁₁₁₁ may form a saturated 4 to 8 membered ring optionally containing one or two double bonds or one or two of oxygen, sulfur or N-Z₉ wherein Z₉ is hydrogen, C₁-C₄ alkyl, benzyl, or C₁-C₄ alkanoyl;
   R₁₁₃ is hydrogen, C₁-C₆ alkyl, fluoro, chloro, bromo, iodo, hydroxy, amino, O(C₁-C₆ alkyl), NH(C₁-C₆ alkyl), N(C₁-C₄ alkyl)(C₁-C₂ alkyl), SH, S(C₁-C₄ alkyl), SO(C₁-C₄ alkyl) or SO₂(C₁-C₄ alkyl), wherein said C₁-C₄ alkyl and C₁-C₆ alkyl may contain one or two double or triple bonds and may be substituted with from one to three substituents R₁₁₇ independently selected from the group consisting of hydroxy, amino, C₁-C₃ alkoxy, dimethylamino, diethylamino, methylamino, ethylamino, NHC(=O)CH₃, fluoro, chloro or C₁-C₃ thioalkyl;
   R₁₁₄ is hydrogen, C₁-C₆ alkyl, fluoro, chloro, bromo, iodo, C₁-C₆ alkoxy, amino, NH(C₁-C₆ alkyl), N(C₁-C₆ alkyl) (C₁-C₂ alkyl), SOₙ(C₁-C₆ alkyl) wherein n is 0, 1 or 2, cyano, hydroxy, carboxy, or amido, wherein said C₁-C₆ alkyl may be substituted with one to three of hydroxy, amino, carboxy, amido, NHC(=O)(C₁-C₄ alkyl), NH(C₁-C₄ alkyl), N(C₁-C₄ alkyl)(C₁-C₂ alkyl), C(=O)O(C₁-C₄ alkyl), C₁-C₃ alkoxy, C₁-C₃ thioalkyl, fluoro, bromo, chloro, iodo, cyano or nitro;
   R₁₁₅ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, pyrrolopyridyl, benzoxazolyl, oxazolyl, pyrrolidinyl, thiazolidinyl, piperazinyl, piperidinyl, tetrazolyl, or 3 to 8 membered cycloalkyl or 9 to 12 membered bicycloalkyl, optionally containing one or two of oxygen, sulfur or N-Z₉ wherein Z₉ is hydrogen, C₁-C₄ alkyl, C₁-C₄ alkanoyl, phenyl or benzyl, wherein each one of the above groups may be substituted independently with from one to three of fluoro, chloro, bromo, formyl, C₁-C₄ alkyl, C₁-C₄ alkoxy or trifluoromethyl, or one of hydroxy, iodo, cyano, nitro, amino, cyclopropyl, NH(C₁-C₄ alkyl), N(C₁-C₄ alkyl)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NHSO₂(C₁-C₄ alkyl), S(C₁-C₆ alkyl), SO₂(C₁-C₆ alkyl), wherein said C₁-C₄ alkyl and C₁-C₆ alkyl may contain one or two double or triple bond and may be substituted with one or two of fluoro, chloro, hydroxy, amino, methylamino, dimethylamino or acetyl; with the proviso that R₁₁₅ is not unsubstituted phenyl;
   R₁₁₁₁ is hydrogen, hydroxy, fluoro, chloro, COO(C₁-C₂ alkyl), cyano or CO(C₁-C₂ alkyl); and
   R₁₁₁₂ is hydrogen or C₁-C₄ alkyl; with the proviso that
   (a) A₈ is not linear C₁-C₁₂ alkyl;
   (b) R₁₁₅ is not a sugar group;
   (c) when R₁₁₃ and R₁₁₄ are hydrogen and R₁₁₅ is chlorophenyl, then A₈ is not NHCH(CH₃)-(CH₂)₃-N(C₂H₅)₂;
   (d) when R₁₁₃ and R₁₁₄ are hydrogen and A₈ is NR₁₁₁R₁₁₂, wherein R₁₁₁ is C₃-C₇ cycloalkyl and R₁₁₂ is C₂-C₆ alkenyl, phenyl(C₁-C₆ alkylene) or hetero(C₁-C₆ alkylene) wherein the hetero radical is furyl, thienyl or pyridinyl, and wherein said phenyl may be substituted with fluoro, chloro, bromo or iodo, then R₁₁₅ is not tetrahydrofuranyl or tetrahydropyranyl;
   (e) when R₁₁₃ is methoxy, methylthio or methylsulfonyl, R₁₁₄ is hydrogen, and R₁₁₅ is tetrahydrofuranyl or tetrahydropyranyl, then A₈ is not NH(C₁-C₂ alkyl), morpholinyl or hydrazino, or NHC₂H₄C₆H₅ which may be substituted with one methyl or two methoxy;
   (f) when R₁₁₃ is hydrogen, C₁-C₆ alkyl, hydrazino, chloro, bromo, SH, or S(C₁-C₄ alkyl), R₁₁₄ is hydrogen, and R₁₁₅ is C₃-C₈ cycloalkyl, then A₈ is not hydrazino, NH(C₁-C₂ alkyl) or N(C₁-C₆ alkyl)(C₁-C₁₂ alkyl);
   (g) when R₁₁₃ and R₁₁₄ are hydrogen and A₈ is NH(CH₂)ₘCOOH wherein m is 1 to 12, then R₁₁₅ is not phenyl substituted by one of fluoro, chloro, bromo or iodo;
   (h) when R₁₁₃ is hydrogen, hydroxy, methylthio, chloro or NHbenzyl, R₁₁₄ is hydrogen, and R₁₁₅ is chlorophenyl or bromophenyl, then A₈ is not NH(C₁-C₁₂ alkyl), NHaryl, or N(C₁-C₆ alkyl)(C₁-C₁₂ alkyl), wherein said C₁-C₁₂ alkyl may be substituted with NC₂H₅, or NHbenzyl which may be substituted with one or two of bromo, chloro, fluoro, NC₂H₅ phenyl or morpholinopropyl;
   (i) when R₁₁₃ and R₁₁₄ are hydrogen and R₁₁₅ is nitrophenyl, then A₈ is not NHR₁₁₂, wherein R₁₁₂ is C₁-C₁₂ alkyl which may be substituted with two hydroxy, or R₁₁₂ is phenyl or benzyl;
   (j) when R₁₁₃ is chloro or O(C₁-C₆ alkyl), R₁₁₄ is hydrogen, and A₈ is NR₁₁₁R₁₁₂, wherein R₁₁₁ and R₁₁₂ are independently hydrogen or C₁-C₆ alkyl, then R₁₁₅ is not chlorophenyl; and
   (k) when R₁₁₃ is hydrogen, A₈ is benzyl or phenethyl, and R₁₁₄ is fluoro, chloro, bromo or iodo, then R₁₁₅ is not 5'-deoxy-ribofuranosyl or 5'-amino-5'-deoxy-ribofuranosyl, or its pharmaceutically acceptable acid addition salt;
(15) A pharmaceutical composition for prophylaxis or therapy of a postoperative stress according to the above (1) or (2), wherein the compound having CRF antagonist activating effect is a compound represented by the formula Ii 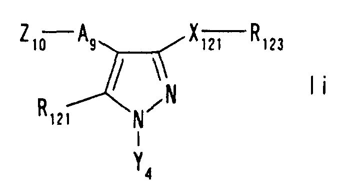 wherein A₉ is CH₂,
   R₁₂₁ is hydrogen, linear or branched C₁-C₆ alkyl, C₃-C₈ alkyl containing one or not adjacent two double bonds, hydroxy, O(C₁-C₆ alkyl), SH, S(C₁-C₆ alkyl), C₃-C₆ cycloalkyl, morpholinyl, piperidinyl or aryl, wherein said aryl may be substituted with one to three of fuloro, chloro, bromo, hydroxy, O(C₁-C₆ alkyl), SH, S(C₁-C₆ alkyl), amino, NH(C₁-C₆ alkyl) or N(C₁-C₆ alkyl), or one of iodo, nitro or cyano and is selected from the group consisting of phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl and thiazolidinyl;
   R₁₂₃ is linear C₁-C₆ alkyl, branched C₃-C₈ alkyl, C₃-C₈ alkenyl wherein the double bond is not adjacent to X₁₂₁ when X₁₂₁ is heteroatom, C₃-C₇ cycloalkyl(CH₂)ₙ wherein n is 0 to 4, or (CH₂)_{q}Q₁₂₁R₁₂₁₉ wherein q is 0, 1 or 2 and Q₁₂₁ is oxygen, sulfur, NH, N(C₁-C₆ alkyl), or covalent bond when X₁₂₁ is not covalent bond, R₁₂₁₉ is hydrogen, linear C₁-C₆ alkyl, branched C₃-C₈ alkyl, C₃-C₈ alkenyl, C₃-C₆ cycloalkyl or C₃-C₆ cycloalkyl-(CH₂), with the proviso that X₁₂₁ and Q₁₂₁ are not both heteroatom when q is 1;
   X₁₂₁ is covalent bond, CH₂, oxygen, sulfur or NR₁₂₀ wherein R₁₂₀ is hydrogen, linear C₁-C₆ alkyl or branched C₃-C₈ alkyl;
   Y₄ is phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl or piperidinyl, wherein each of the above groups may be substituted with one to three of any one of fluoro, chloro, bromo, or methyl, or one of trifluoromethyl; with the proviso that Y₄ is not unsubstituted phenyl; and
   Z₁₀ is
   (a) 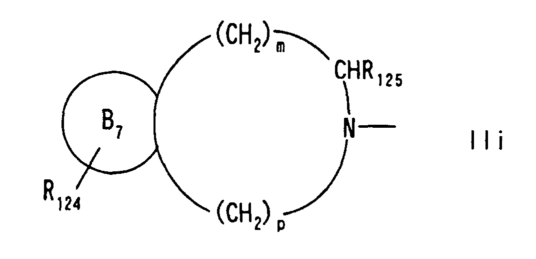 wherein the B₇ ring is phenyl, naphthyl, pyridyl, pyrimidinyl, pyrazinyl, pyridazinyl, triazolyl, pyrrolyl, pyrazolyl, imidazolyl, thienyl or indolyl, wherein each of the above groups may be substituted with methyl, methoxy, trifluoromethoxy, fluoro, chloro, bromo or iodo, or a saturated 5 or 6 membered carbocyclic ring or a partially unsaturated ring having one or two double bonds;
      R₁₂₄ is hydrogen, C₁-C₆ alkyl, C₁-C₆ alkoxy, hydroxy, fluoro, chloro, bromo, iodo or trifluoromethyl;
      R₁₂₅ is hydrogen, linear C₁-C₆ alkyl, branched C₃-C₈ alkyl, C₃-C₈ alkeny, or (CH₂)ₒ-X₁₂₂- (CH₂)ᵣ-Q₁₂₂-R₁₂₆;
      X₁₂₂ and Q₁₂₂ are each independently oxygen, sulfur, NH, N(C₁-C₆ alkyl), or one of X₁₂₂ and Q₁₂₂ may be a covalent bond;
      R₁₂₆ is hydrogen, linear C₁-C₆ alkyl, branched C₃-C₈ alkyl, or C₃-C₈ alkenyl;
      m is 0 or 1;
      o is 1 or 2;
      p is 1 or 2;
      r is 0, 1, or 2;
   (b) 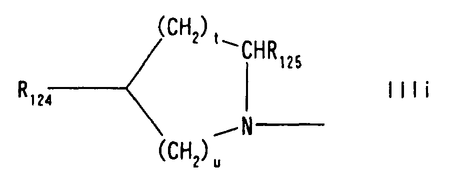 wherein R₁₂₄ and R₁₂₅ are as defined above, and t and u are each independently 1 or 2;
   (c) -NR₁₂₇R₁₂₈ wherein R₁₂₇ and R₁₂₈ are each independently hydrogen or linear C₁-C₆ alkyl, branched C₃-C₈ alkyl, C₃-C₈ alkenyl, (CH₂)ᵥCH₂OH, (CH₂)ᵥNR₁₂₉R₁₂₁₀ wherein v is 0 to 3, and R₁₂₉ and R₁₂₁₀ are each independently hydrogen or linear C₁-C₆ alkyl, (C₃-C₁₂ cycloalkyl)(CH₂)ₙ, (C₆-C₁₀ bicycloalkyl)(CH₂)ₙ, benzofused C₃-C₆ cycloalkyl, C₁-C₆ hydroxyalkyl, phenyl(CH₂)ₙ wherein n is 0 to 4, wherein each of the above groups may be substituted with one or two of hydroxy, fluoro, chloro, bromo, C₁-C₅ alkyl or C₁-C₅ alkoxy, or R₁₂₇ and R₁₂₈ may be taken together with nitrogen to form a saturated or partially unsaturated 5 to 7 membered ring which may contain one of oxygen, sulfur, NH or N(C₁-C₆ alkyl) and which may be substituted with C₁-C₆ alkyl, hydroxy or phenyl, wherein any double bonds are not adjacent to any heteroatoms;
   (d) 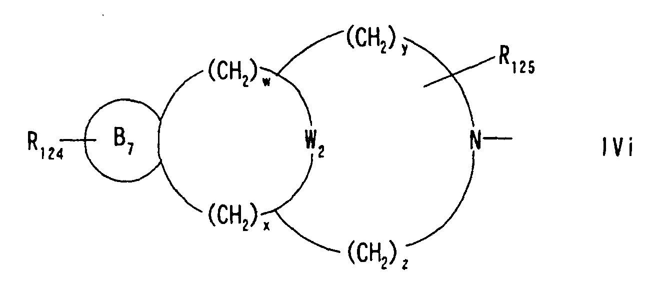 wherein B₇, W₂, R₁₂₄ and R₁₂₅ are as defined above, w, x, y and z are each independently 1 or 2, and W₂ is (CH₂)_{q} wherein q is as defined above, N(C₁-C₆ alkyl) or oxygen;
   (e) 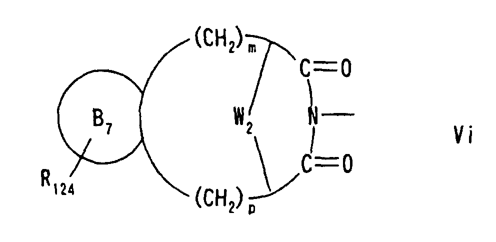 wherein B₇, R₁₂₄, m and p are as defined above;
   (f) 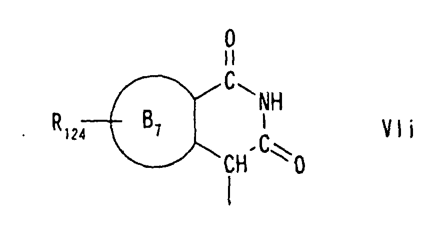 wherein B₇ and R₁₂₄ are as defined above;
   (g) O(CH₂)ᵥR₁₂₁₁
      wherein v is 0 to 3 and R₁₂₁₁ is linear C₁-C₆ alkyl, branched C₃-C₈ alkyl, phenyl, naphthyl, 1,2,3,4-tetrahydronaphthyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, piperidinyl, or thienyl, wherein each of the above groups may be substituted with one or two of any one of fluoro, chloro, bromo, methyl or trifluoromethyl;
   (h) 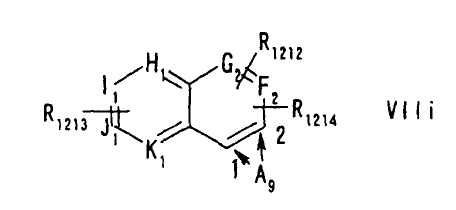 wherein A₉ is as defined above and is attached to position 1 or 2, while R₁₂₁₄ is attached to position 2 or 1 respectively; F₂, G₂, H₁, I₁, J₁ and K₁ are independently carbon or nitrogen, with the proviso that not more than three of H₁, I₁, J₁ and K₁ are nitrogen with not more than two adjacent nitrogens; R₁₂₁₂ and R₁₂₁₃ each independently are hydrogen, linear C₁-C₆ alkyl, branched C₃-C₈ alkyl, C₃-C₈ alkenyl, fluoro, chloro, bromo, trifluoromethyl, hydroxy, thiol, C₁-C₁₂ alkoxy, C₁-C₁₂ thioalkanyl, or C₃-C₁₂ alkenoxy or C₃-C₁₂ thioalkenyl, wherein the double bond is not adjacent to oxygen; and R₁₂₁₄ is hydroxy, C₁-C₁₂ alkoxy, C₃-C₁₂ alkenoxy, wherein the double bond is not adjacent to oxygen, or -X₁₂₂-(CH₂)ᵣQ₁₂₂R₁₂₆ wherein X₁₂₂, r, Q₁₂₂ and R₁₂₆ are as defined above in paragraph (a) except that Q₁₂₂ is not sulfur, or NR₁₂₁₅R₁₂₁₆ wherein R₁₂₁₅ and R₁₂₁₆ are each independently hydrogen, linear C₁-C₆ alkyl, branched C₃-C₈ alkyl, C₃-C₈ alkenyl, wherein the double bond is not adjacent to nitrogen, or C₃-C₇ cycloalkyl-(CH₂)ₙ wherein n is as defined above, or R₁₂₁₅ and R₁₂₁₆ may be taken together with the nitrogen to form a saturated 5 or 6 membered ring optionally condensed with benzo; or
   (i) 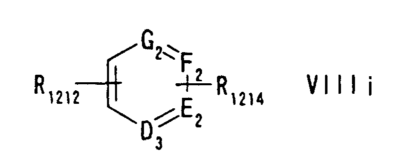 wherein D₃, E₂, F₂ and G₂ are independently carbon or nitrogen, with the proviso that not more than two of D₃, E₂, F₂ and G₂ are nitrogen, R₁₂₁₂ and R₁₂₁₄ are as defined above (h), the above-defined A₉ is attached to the formula VIIIi, and R₁₂₁₄ is attached to a carbon located adjacent to the carbon to which A₉ is attached,
      or its pharmaceutically acceptable acid addition salt;
(16) A pharmaceutical composition for prophylaxis or therapy of a postoperative stress according to the above (1) or (2), wherein the compound having CRF antagonist activating effect is a compound represented by the formula Ij 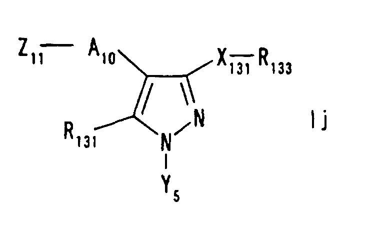 wherein A₁₀ is CH₂, R₁₃₁ is linear or branched C₁-C₆ alkyl, C₃-C₆ alkyl containing one or not adjacent two double bonds, hydroxy, O(C₁-C₆ alkyl), SH, S(C₁-C₆ alkyl), SO₂(C₁-C₆ alkyl), C₃-C₆ cycloalkyl, morpholinyl, piperidinyl or aryl, wherein said aryl may be substituted with one to three of fuloro, chloro, bromo, hydroxy, O(C₁-C₆ alkyl), SH, S(C₁-C₆ alkyl), amino, NH(C₁-C₆ alkyl), N(C₁-C₆ alkyl)₂, or one of iodo, nitro, cyano and is selected from the group consisting of phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl and thiazolidinyl;
   R₁₃₃ is linear C₁-C₆ alkyl, branched C₃-C₈ alkyl, C₃-C₈ alkenyl, wherein the double bond is not adjacent to X₁₃₁ when X₁₃₁ is heteroatom, C₃-C₇ cycloalkyl(CH₂)ₙ wherein n is 0 to 4, or (CH₂)_{q}Q₁₃₁R₁₃₁₉ wherein q is 0, 1 or 2 and Q₁₃₁ is oxygen, sulfur, NH or N(C₁-C₆ alkyl), or covalent bond when X₁₃₁ is not covalent bond, R₁₃₁₉ is hydrogen, linear C₁-C₆ alkyl, branched C₃-C₈ alkyl, C₃-C₈ alkenyl, C₃-C₆ cycloalkyl or C₃-C₆ cycloalkyl(CH₂), with the proviso that X₁₃₁ and Q₁₃₁ is not both heteroatom when q is 1;
   X₁₃₁ is covalent bond, CH₂, oxygen, sulfur or NR₁₃₀ wherein R₁₃₀ is hydrogen, linear C₁-C₆ alkyl or branched C₃-C₈ alkyl;
   Y₅ is phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, or piperidinyl, wherein each of the above groups may be substituted with one to three of any one of fluoro, chloro, bromo, formyl. C₁-C₆ alkyl, branched C₃-C₈ alkyl, C₁-C₆ alkoxy or trifuloromethyl, or one of hydroxyl, iodo, cyano, nitro, amino, NH(C₁-C₄ alkyl), N(C₁-C₄ alkyl)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NHSO₂(C₁-C₄ alkyl), S(C₁-C₆ alkyl) or SO₂(C₁-C₆ alkyl), wherein said C₁-C₄ alkyl and C₁-C₆ alkyl may be substituted with one or two of fluoro, chloro, hydroxy, C₁-C₄ alkoxy, amino, methylamino, dimethylamino or acetyl and may contain one double bond or triple bond, and wherein each of above Y₅ groups may be substituted with one to three of any one of fluoro, chloro, bromo or methyl, or one of trifluoromethyl; and
   Z₁₁ is
   (a) 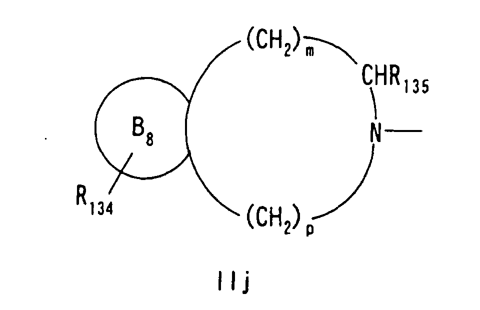 wherein the B₈ ring is phenyl, naphthyl, pyridyl, pyrimidinyl, pyrazinyl, pyridazinyl, triazolyl, pyrrolyl, pyrazolyl, imidazolyl, thienyl or indolyl, wherein each of the above groups may be substituted with methyl, methoxy, trifluoromethyl, fluoro, chloro, bromo or iodo, or a saturated 5 or 6 membered carbocyclic ring or a partially unsaturated ring having one or two double bonds;
      R₁₃₄ is hydrogen, C₁-C₆ alkyl, C₁-C₆ alkoxy, hydroxy, fluoro, chloro, bromo, iodo or trifluoromethyl;
      R₁₃₅ is hydrogen, linear C₁-C₆ alkyl, branched C₃-C₈ alkyl, C₃-C₈ alkenyl or (CH₂)ₒ-X₁₃₂-(CH₂)ᵣ-Q₁₃₂-R₁₃₆;
      X₁₃₂ and Q₁₃₂ are each independently oxygen, sulfur, NH or N(C₁-C₆ alkyl), or one of X₁₃₂ and Q₁₃₂ may be a covalent bond;
      R₁₃₆ is hydrogen, linear C₁-C₆ alkyl, branched C₃-C₈ alkyl or C₃-C₈ alkenyl;
      m is 0 or 1;
      o is 1 or 2;
      p is 1 or 2;
      r is 0, 1, or 2;
   (b) 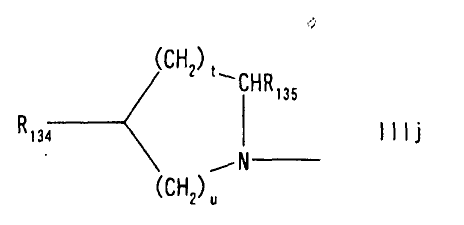 wherein R₁₃₄ and R₁₃₅ are as defined above, and t and u are each independently 1 or 2;
   (c) -NR₁₃₇R₁₃₈ wherein R₁₃₇ and R₁₃₈ are each independently hydrogen, linear C₁-C₆ alkyl, branched C₃-C₈ alkyl, C₃-C₈ alkenyl, (CH₂)ᵥCH₂OH, (CH₂)ᵥNR₁₃₉R₁₃₁₀ wherein v is 0 to 3 and R₁₃₉ and R₁₃₁₀ are each independently hydrogen or linear C₁-C₆ alkyl, (C₃-C₁₂ cycloalkyl)(CH₂)ₙ, (C₆-C₁₀ bicycloalkyl)(CH₂)ₙ, benzofused C₃-C₈ cycloalkyl, C₁-C₆ hydroxyalkyl, phenyl(CH₂)ₙ, wherein each of the above groups may be substituted with one or two of hydroxy, fluoro, chloro, bromo, C₁-C₅ alkyl or C₁-C₅ alkoxy, or R₁₃₇ and R₁₃₈ may be taken together with the nitrogen to form a saturated or partially unsaturated 5 to 7 membered ring which may contain one of oxygen, sulfur, NH or N(C₁-C₆ alkyl) and may be substituted with C₁-C₆ alkyl, hydroxy or phenyl, and n is 0 to 4;
   (d) 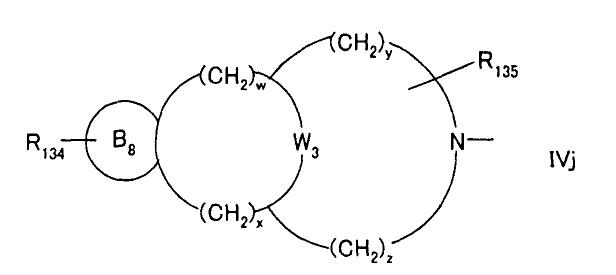 wherein B₈, W₃, R₁₃₄ and R₁₃₅ are as defined above, w, x, y and z are each independently 1 or 2, and W₃ is (CH2)_{q} wherein q is as defined above, N(C₁-C₆ alkyl) or oxygen;
   (e) 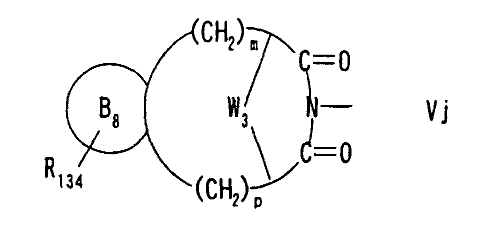 wherein B₈, R₁₃₄, m and p are as defined above;
   (f) 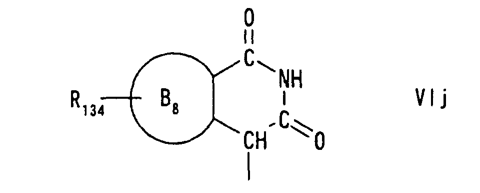 wherein B₈ and R₁₃₄ are as defined above;
   (g) O(CH₂)ᵥR₁₃₁₁
      wherein v is 0 to 3 and R₁₃₁₁ is linear C₁-C₆ alkyl, branched C₃-C₈ alkyl, phenyl, naphthyl, 1,2,3,4-tetrahydronaphthyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, piperidinyl, or thienyl, wherein each of the above groups may be substituted with one or two of any one of fluoro, chloro, bromo, methyl or trifluoromethyl;
   (h) 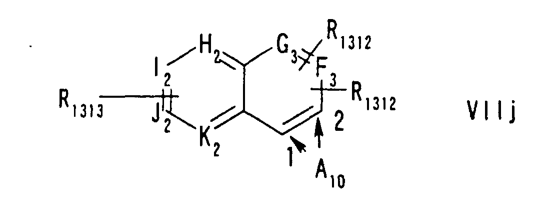 wherein A₁₀ is as defined above and is attached to position 1 or 2 while R₁₃₁₄ is attached to position 2 or 1 respectively; F₃, G₃, H₂, I₂, J₂ and K₂ are independently carbon or nitrogen, with the proviso that not more than three of H₂, I₂, J₂ and K₂ are nitrogen with not more than two adjacent nitrogens; R₁₃₁₂ and R₁₃₁₃ each independently are hydrogen, linear C₁-C₆ alkyl, branched C₃-C₈ alkyl, C₃-C₈ alkenyl, fluoro, chloro, bromo, trifluoromethyl, hydroxy, thiol, C₁-C₁₂ alkoxy, C₁-C₁₂ thioalkyl, or C₃-C₁₂ alkenoxy or C₃-C₁₂ thioalkenyl, wherein the double bond is not adjacent to oxygen; and R₁₃₁₄ is hydroxy, C₁-C₁₂ alkoxy, C₃-C₁₂ alkenoxy, wherein the double bond is not adjacent to oxygen, or -X₁₃₂-(CH₂)ᵣQ₁₃₂R₁₃₆ wherein X₁₃₂, r, Q₁₃₂ and R₁₃₆ are as defined above in paragraph (a) except that Q₁₃₂ is not sulfur, or R₁₃₁₄ is NR₁₃₁₅R₁₃₁₆, wherein R₁₃₁₅ and R₁₃₁₆ are each independently hydrogen, linear C₁-C₆ alkyl, branched C₃-C₈ alkyl, C₃-C₈ alkenyl, wherein the double bond is not adjacent to nitrogen or C₃-C₇ cycloalkyl(CH₂)ₙ wherein n is as defined above, or R₁₃₁₅ and R₁₃₁₆ may be taken together with the nitrogen to form a saturated 5 or 6 membered ring which may be condensed with benzo; or
   (i) 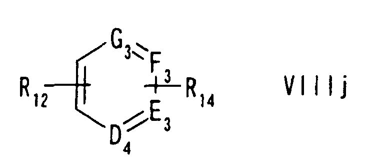 wherein D₄, E₃, F₃ and G₃ are independently carbon or nitrogen, with the proviso that not more than two of D₄, E₃, F₃ and G₃ are nitrogen, R₁₃₁₂ and R₁₃₁₄ are as defined above (h), A₁₀ which is defined above is attached to a carbon in formula VIIIj, and R₁₃₁₄ is attached to a carbon located adjacent to the carbon to which A₁₀ is attached,
      or its pharmaceutically acceptable acid addition salt;
(17) A pharmaceutical composition for prophylaxis or therapy of a postoperative stress according to the above (1) or (2), wherein the compound having CRF antagonist activating effect is a compound represented by the formula Ik, IIk or IIIk 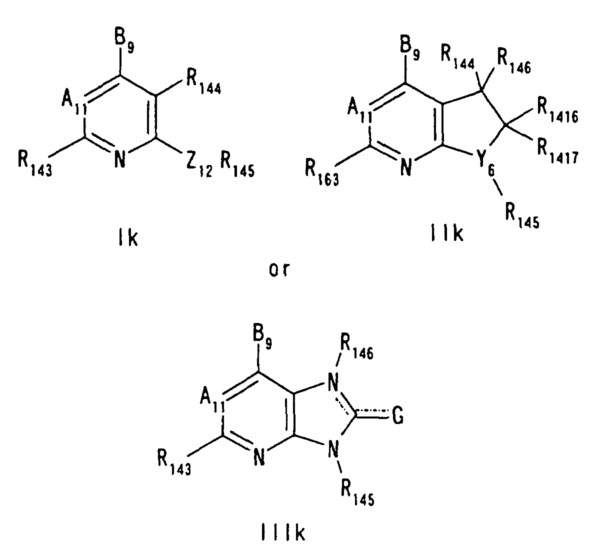 wherein the dashed lines represent optional double bonds;
   A₁₁ is -CR₁₄₇ or nitrogen;
   B₉ is -NR₁₄₁R₁₄₂, -CR₁₄₁R₁₄₂R₁₄₁₁, -C(=CR₁₄₂R₁₄₁₂)R₁₄₁, -NHCHR₁₄₁R₁₄₂, -OCHR₁₄₁R₁₄₂, -SCHR₁₄₁R₁₄₂, -CHR₁₄₂OR₁₄₁₂, -CHR₁₄₂SR₁₄₁₂, -C(S)R₁₄₂ or -C(O)R₁₄₂;
   G₄ is oxygen, sulfur, NH, NH₃, hydrogen, methoxy, ethoxy, trifluoromethoxy, methyl, ethyl, thiomethoxy, NH₂, NHCH₃, N(CH₃)₂ or trifluoromethyl;
   Y₆ is -CH or nitrogen;
   Z₁₂ is NH, oxygen, sulfur, -N(C₁-C₂ alkyl) or -C(R₁₄₁₃R₁₄₁₄), wherein R₁₄₁₃ and R₁₄₁₄ are each independently hydrogen, trifluoromethyl or methyl, or one of R₁₄₁₃ and R₁₄₁₄ is cyano and the other is hydrogen or methyl;
   R₁₄₁ is C₁-C₆ alkyl which may optionally be substituted with one or two substituents R₁₄₈ independently selected from the group consisting of hydroxy, fluoro, chloro, bromo, iodo, CF₃, C₁-C₄ alkoxy, -O-CO-(C₁-C₄ alkyl), -O-CO-NH-(C₁-C₄ alkyl), -O-CO-N(C₁-C₄ alkyl)(C₁-C₂ alkyl), -NH(C₁-C₄ alkyl), -N(C₁-C₂ alkyl)(C₁-C₄ alkyl), -S(C₁-C₄ alkyl), -N(C₁-C₄ alkyl)CO(C₁-C₄ alkyl), -NHCO(C₁-C₄ alkyl), -COO(C₁-C₄ alkyl), -CONH(C₁-C₄ alkyl), -CON(C₁-C₄ alkyl)(C₁-C₂ alkyl), CN, NO₂, -SO(C₁-C₄ alkyl) and -SO₂(C₁-C₄ alkyl), and wherein said C₁-C₆ alkyl and (C₁-C₄)alkyl moieties in the foregoing R₁₄₁ groups may optionally contain one carbon-carbon double or triple bond;
   R₁₄₂ is C₁-C₁₂ alkyl, aryl or -(C₁-C₄ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, benzisoxazolyl, benzimidazolyl, indolyl or benzoxazolyl, or 3 to 8 membered cycloalkyl or -(C₁-C₆ alkylene)cycloalkyl, wherein one or two of the ring carbons of said cycloalkyl having at least 4 ring members and cycloalkyl moieties of said -(C₁-C₆ alkylene)cycloalkyl having at least 4 ring members may optionally be replaced by oxygen, sulfur or N-R₁₄₉ wherein R₁₄₉ is hydrogen or C₁-C₄ alkyl; and wherein each of the foregoing R₁₄₂ groups may optionally be substituted with from one to three substituents independently selected from chloro, fluoro and C₁-C₄ alkyl, or one substituent selected from bromo, iodo, C₁-C₆ alkoxy, -O-CO-(C₁-C₆ alkyl), -O-CO-N(C₁-C₄ alkyl)(C₁-C₂ alkyl), -S(C₁-C₆ alkyl), CN, NO₂, -SO(C₁-C₄ alkyl) and -SO₂(C₁-C₄ alkyl), and wherein said C₁-C₁₂ alkyl and C₁-C₄ alkylene moieties of said (C₁-C₄ alkylene)aryl may optionally contain one carbon-carbon double or triple bond;
   or -NR₁₄₁R₁₄₂ or -CR₁₄₁R₁₄₂R₁₄₁₁ may form a saturated 5 to 8 membered carbocyclic ring which may optionally contain one or two carbon-carbon double bonds and in which one or two of the ring carbons may optionally be replaced by oxygen or sulfur;
   R₁₄₃ is methyl, ethyl, fluoro, chloro, bromo, iodo, cyano, methoxy, OCF₃, methylthio, methylsulfonyl, CH₂OH or CH₂OCH₃;
   R₁₄₄ is hydrogen, C₁-C₄ alkyl, fluoro, chloro, bromo, iodo, C₁-C₄ alkoxy, trifluoromethoxy, -CH₂OCH₃, -CH₂OCH₂CH₃, -CH₂CH₂OCH₃, -CH₂OF₃, CF₃, amino, nitro, -NH(C₁-C₄ alkyl), -N(CH₃)₂, -NHCOCH₃, -NHCONHCH₃, -SOₙ(C₁-C₄ alkyl) wherein n is 0, 1 or 2, cyano, hydroxy, -CO(C₁-C₄ alkyl), -CHO, cyano or -COO(C₁-C₄ alkyl), wherein said C₁-C₄ alkyl may optionally contain one double or triple bond and may optionally be substituted with one substituent selected from hydroxy, amino, -NHCOCH₃, -NH(C₁-C₂ alkyl), -N(C₁-C₂ alkyl)₂, -COO(C₁-C₄ alkyl), -CO(C₁-C₄ alkyl), C₁-C₃ alkoxy, C₁-C₃ thioalkyl, fluoro, chloro, cyano and nitro;
   R₁₄₅ is phenyl, naphthyl, thienyl, benzothienyl, pyridyl, quinolyl, pyrazinyl, pyrimidyl, furanyl, benzofuranyl, benzothiazolyl or indolyl, wherein each of the above groups R₁₄₅ is substituted with from one to three substituents independently selected from fluoro, chloro, C₁-C₆ alkyl and C₁-C₆ alkoxy, or one substituent selected from hydroxy, iodo, bromo, formyl, cyano, nitro, trifluoromethyl, amino, -(C₁-C₆ alkyl)O(C₁-C₆)alkyl, -NHCH₃, -N(CH₃)₂, -COOH, -COO(C₁-C₄ alkyl), -CO(C₁-C₄ alkyl), -SO₂NH(C₁-C₄ alkyl), -SO₂N(C₁-C₄ alkyl)(C₁-C₂ alkyl), -SO₂NH₂, -NHSO₂(C₁-C₄ alkyl), -S(C₁-C₆ alkyl) and -SO₂(C₁-C₆ alkyl), and wherein C₁-C₄ alkyl and C₁-C₆ alkyl moieties of the foregoing R₁₄₅ groups may optionally be substituted with one or two fluoro or one substituent selected from hydroxy, amino, methylamino, dimethylamino and acetyl;
   R₁₄₆ is hydrogen or C₁-C₆ alkyl, wherein said C₁-C₆ alkyl may optionally be substituted with one of hydroxy, methoxy, ethoxy or fluoro;
   R₁₄₇ is hydrogen, methyl, fluoro, chloro, bromo, iodo, cyano, hydroxy, -O(C₁-C₄ alkyl), -C(O)(C₁-C₄ alkyl), -C(O)O(C₁-C₄ alkyl), -OCF₃, CF₃, -CH₂OH, -CH₂OCH₃ or -CH₂OCH₂CH₃;
   R₁₄₁₁ is hydrogen, hydroxy, fluoro, or methoxy;
   R₁₄₁₂ is hydrogen or C₁-C₄ alkyl; and
   R₁₄₁₆ and R₁₄₁₇ are each independently hydrogen, hydroxy, methyl, ethyl, methoxy or ethoxy, with the proviso that R₁₄₁₆ and R₁₄₁₇ are not both methoxy or ethoxy;
   or R₁₄₁₆ and R₁₄₁₇ may be take together to form an oxo (=O) group; with the proviso that when G₄ is oxygen, sulfur, NH or NCH₃, G₄ is double bonded to 5 membered ring of structure IIIk, and with the further proviso that R₁₄₆ is absent when nitrogen to which it is attached is double bonded to an adjacent ring carbon atom,
   or its pharmaceutically acceptable salt;
(18) A pharmaceutical composition for prophylaxis or therapy of a postoperative stress according to the above (1) or (2), wherein the compound having CRF antagonist activating effect is a compound represented by the formula Im 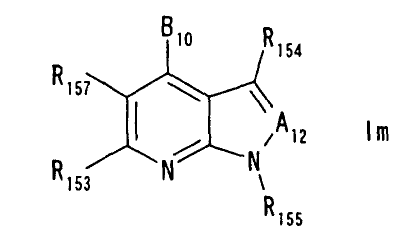 wherein A₁₂ is nitrogen or -CR₁₅₆;
   B₁₀ is -NR₁₅₁R₁₅₂, -CR₁₅₁R₁₅₂R₁₅₁₁, -C(=CR₁₅₂R₁₅₁₂)R₁₅₁, -NHCHR₁₅₁R₁₅₂, -OCHR₁₅₁R₁₅₂, -SCHR₁₅₁R₁₅₂, -CHR₁₅₂OR₁₅₁₂, -CHR₁₅₂SR₁₅₁₂, -C(S)R₁₅₁ or -C(O)R₁₅₁;
   R₁₅₁ is C₁-C₆ alkyl which may optionally be substituted with one or two substituents independently selected from the group consisting of hydroxy, fluoro, chloro, bromo, iodo, C₁-C₄ alkoxy, -O-CO-(C₁-C₄ alkyl), -O-CO-NH(C₁-C₄ alkyl), -O-CO-N(C₁-C₄ alkyl)(C₁-C₂ alkyl), -NR(C₁-C₄ alkyl), -N(C₁-C₂ alkyl)(C₁-C₄ alkyl), -S(C₁-C₄ alkyl), -N(C₁-C₄ alkyl)CO(C₁-C₄ alkyl), -NHCO(C₁-C₄ alkyl), -COO(C₁-C₄ alkyl), -CONH(C₁-C₄ alkyl), -CON(C₁-C₄ alkyl)(C₁-C₂ alkyl), CN, NO₂, -SO(C₁-C₄ alkyl) and -SO₂(C₁-C₄ alkyl), and wherein any of the foregoing C₁-C₄ alkyl and C₁-C₆ alkyl groups may optionally contain one carbon-carbon double or triple bond;
   R₁₅₂ is C₁-C₁₂ alkyl, aryl, -(C₁-C₄ alkylene)aryl, wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzoisothiazolyl, thiazolyl, isoxazolyl, benzoisoxazolyl, benzoimidazolyl, triazolyl, pyrazolyl, pyrrolyl, indolyl, oxazolyl or benzoxazolyl, or 3 to 8 membered cycloalkyl or -(C₁-C₆ alkylene)cycloalkyl, wherein one or two of the ring carbons of said cycloalkyl having at least 4 ring members and cycloalkyl moieties of said -(C₁-C₆ alkylene)cycloalkyl having at least 4 ring members may optionally be replaced by oxygen, sulfur or N-Z₁₃ wherein Z₁₃ is hydrogen or C₁-C₄ alkyl, and wherein each of foregoing R₁₅₂ groups may optionally be substituted with from one to three substituents independently selected from chloro, fluoro and C₁-C₄ alkyl, or one substituent selected from bromo, iodo, C₁-C₆ alkoxy, -O-CO-(C₁-C₆ alkyl), -S(C₁-C₆ alkyl), -COO(C₁-C₄ alkyl), CN, NO₂, -SO(C₁-C₄ alkyl) and -SO₂(C₁-C₄ alkyl), and wherein said C₁-C₁₂ alkyl and C₁-C₄ alkylene moieties of said -(C₁-C₄ alkylene)aryl may optionally contain one carbon-carbon double or triple bond;
   or -NR₁₅₁R₁₅₂ may form a saturated 5 to 8 membered heterocyclic ring, or -CHR₁₅₁R₁₅₂ may form a saturated 5 to 8 membered carbocyclic ring, wherein each of these rings may optionally contain one or two carbon-carbon double bonds and wherein one or two of the carbon atoms of each of these rings may optionally be replaced by sulfur or oxygen;
   R₁₅₃ is C₁-C₄ alkyl, fluoro, chloro, bromo, iodo, -CH₂OH, -CH₂OCH₃, -O(C₁-C₃ alkyl), -S(C₁-C₃ alkyl) or -SO₂(C₁-C₃ alkyl), wherein said C₁-C₃ alkyl may optionally contain one carbon-carbon double or triple bond;
   R₁₅₄ is hydrogen, C₁-C₆ alkyl, fluoro, chloro, bromo, iodo, C₁-C₄ alkoxy, amino,-NHCH₃, -N(CH₃)₂, -CH₂OH, -CH₂OCH₃ or -SOₙ(C₁-C₄ alkyl) wherein n is 0, 1 or 2, cyano, hydroxy, -CO(C₁-C₄ alkyl), -CHO or -COO(C₁-C₄ alkyl), wherein said C₁-C₄ alkyl moieties in the foregoing R₁₅₄ groups may optionally contain one carbon-carbon double or triple bond;
   R₁₅₅ is phenyl, naphthyl, thienyl, benzothienyl, pyridyl, pyrimidyl, benzofuranyl, pyrazinyl or benzothiazolyl, wherein each one of foregoing R₁₅₅ groups may optionally be substituted with from one to three substituents independently selected from fluoro, chloro, C₁-C₆ alkyl and C₁-C₆ alkoxy, or by one substituent selected from iodo, hydroxy, bromo, formyl, cyano, nitro, amino, trifluoromethyl, -NH(C₁-C₄ alkyl), -N(C₁-C₆)(C₁-C₂ alkyl), -COO(C₁-C₄ alkyl), -CO(C₁-C₄ alkyl), -COOH, -SO₂NH(C₁-C₄ alkyl), -SO₂N(C₁-C₄ alkyl)(C₁-C₂ alkyl), -SO₂NH₂, -NHSO₂(C₁-C₄ alkyl), -S(C₁-C₆ alkyl) and -SO₂(C₁-C₆ alkyl), wherein each of said C₁-C₄ alkyl moieties and C₁-C₆ alkyl moieties in the foregoing R₁₅₅ groups may optionally be substituted with one to three fluorine;
   R₁₅₆ is hydrogen, C₁-C₄ alkyl, fluoro, chloro, bromo, iodo, -CH₂OH, -CH₂OCH₃ or C₁-C₄ alkoxy;
   R₁₅₇ is hydrogen, C₁-C₄ alkyl, fluoro, chloro, bromo, iodo, -O(C₁-C₄ alkyl), cyano, -CH₂OH, -CH₂O(C₁-C₂ alkyl), -CO(C₁-C₂ alkyl) or -COO(C₁-C₂ alkyl);
   R₁₅₁₁ is hydrogen, hydroxy, fluoro or methoxy; and
   R₁₅₁₂ is hydrogen or C₁-C₄ alkyl; with the proviso that when A₁₂ is nitrogen, then: (a) B₁₀ is not unsubstituted alkyl; (b) R₁₅₅ is not unsubstituted phenyl or monosubstituted phenyl; and (c) R₁₅₃ is not unsubstituted alkyl,
   or its pharmaceutically acceptable salt;
(19) A pharmaceutical composition for prophylaxis or therapy of a postoperative stress according to the above (1) or (2), wherein the compound having CRF antagonist activating effect is a compound represented by the formula In 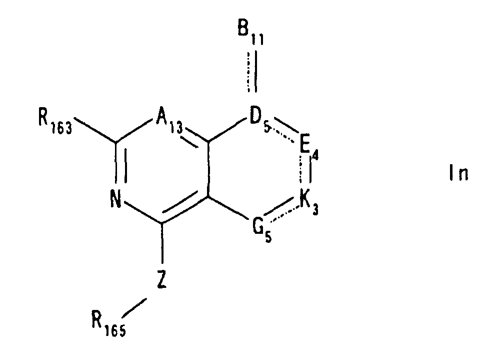 wherein the dashed lines represent optional double bonds;
   A₁₃ is nitrogen or CR₁₆₇;
   B₁₁ is -NR₁₆₁R₁₆₂, -CR₁₆₁R₁₆₂R₁₆₁₀, -C(=CR₁₆₂R₁₆₁₁)R₁₆₁, -NHCR₁₆₁R₁₆₂R₁₆₁₀. -OCR₁₆₁R₁₆₂R₁₆₁₀, SCR₁₆₁R₁₆₂R₁₆₁₀, -CR₁₆₂R₁₆₁₀NHR₁₆₁ -CR₁₆₂R₁₆₁₀OR₁₆₁, -CR₁₆₂R₁₆₁₀SR₁₆₁ or -COR₁₆₂, and is single bonded to D₅, or B₁₁ is -CR₁₆₁R₁₆₂, and is double bonded to D₅ and D₅ is carbon;
   D₅ is nitrogen or CR₁₆₄ and is single bonded to all atoms to which it is attached, or D₅ is carbon and is double bonded to E₄ or double bonded to B₁₁;
   E₄ is oxygen, nitrogen, sulfur, C=O, C=S, CR₁₆₆R₁₆₁₂, NR₁₆₆ or CR₁₆₆, or E₄ is a two atom spacer, wherein one of the atoms is oxygen, nitrogen, sulfur, C=O, C=S, CR₁₆₆R₁₆₁₂, NR₁₆₆ or CR₁₆₆, and the other is CR₁₆₆R₁₆₁₂ or CR₁₆₉;
   K₃ and G₅ are each independently C=O, C=S, sulfur, oxygen, CHR₁₆₈ or NR₁₆₈ when single bonded to both adjacent ring atoms, or nitrogen or CR₁₆₈ when it is double bonded to one adjacent ring atom; 6 or 7 membered ring that contains D₅, E₄, K₃ and G₅ may contain from one to three double bonds, from zero to two heteroatoms selected from oxygen, nitrogen and sulfur, and from zero to two C=O or C=S groups, wherein the carbon atoms of such groups are part of the ring and the oxygen and sulfur atoms are substituents on the ring;
   R₁₆₁ is C₁-C₆ alkyl which may optionally be substituted with from one or two substituents independently selected from hydroxy, fluoro, chloro, bromo, iodo, C₁₋₄ alkoxy, CF_{3,} -C(=O)(C₁₋C₄ alkyl), -C(=O)-O-(C₁₋C₄ alkyl), -OC(=O)(C₁₋C₄ alkyl), -OC(=O)N(C₁₋C₄ alkyl)(C₁₋C₂ alkyl), -NHCO(C₁₋C₄ alkyl), -COOH, -COO(C₁₋C₄ alkyl), -CONH(C₁₋C₄ alkyl), -CON(C₁₋C₄ alkyl)(C₁₋C₂ alkyl), -S(C₁₋C₄ alkyl), -CN, -NO₂, -SO(C₁₋C₄ alkyl), -SO₂NH(C₁₋C₄ alkyl) and -SO₂N(C₁-C₄ alkyl)(C₁₋C₂ alkyl), wherein each of the C₁-C₄ alkyl groups in the foregoing R₁₆₁ groups may optionally contain one or two double or triple bonds;
   R₁₆₂ is C₁-C₁₂ alkyl which may optionally contain from one to three double or triple bonds, aryl or (C₁-C₄ alkylene)aryl, wherein said aryl and aryl moieties of said (C₁-C₄ alkylene)aryl is selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl, or C₃-C₈ cycloalkyl or (C₁-C₆ alkylene)(C₃-C₈ cycloalkyl), wherein one or two of the carbon atoms of said cycloalkyl and one or two of the carbon atoms of 5 to 8 membered cycloalkyl moieties of said (C₁-C₆ alkylene)(C₃-C₈ cycloalkyl) may optionally and independently be replaced by oxygen or sulfur, and wherein each of the foregoing R₁₆₂ groups may optionally be substituted with from one to three substituents selected from chloro, fluoro and C₁-C₄ alkyl, or one substituent selected from C₁-C₆ alkoxy, -OC(=O)(C₁-C₆ alkyl), -OC(=O)N(C₁-C₄ alkyl)(C₁-C₂ alkyl), -S(C₁-C₆ alkyl), amino, -NH(C₁-C₂ alkyl), -N(C₁-C₄ alkyl)(C₁-C₂ alkyl), -N(C₁-C₄ alkyl)-CO-(C₁-C₄ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -SH, -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl);
   -NR₁₆₁R₁₆₂ or CR₁₆₁R₁₆₂R₁₆₁₀ may form a ring selected from saturated 3 to 8 membered rings, and 5 to 8 membered rings of which may optionally contain one or two double bonds, and wherein one or two of the ring carbon atoms of such 5 to 8 membered rings may optionally and independently be replaced by oxygen, sulfur or NZ₁₆₃ wherein Z₁₆₃ is hydrogen or C₁-C₄ alkyl;
   R₁₆₃ is hydrogen, C₁-C₄ alkyl, -O(C₁-C₄ alkyl), fluoro, chloro, bromo, iodo, -S(C₁-C₄ alkyl) or -SO₂(C₁-C₄ alkyl);
   R₁₆₄ is hydrogen, C₁-C₂ alkyl, hydroxy or fluoro; each R₁₆₆, R₁₆₈ and R₁₆₉ that is attached to a carbon atom is selected independently from hydrogen, C₁-C₂ alkyl, fluoro, chloro, bromo, iodo, hydroxy, hydroxymethyl, formyl, trifluoromethyl, cyano, amino, nitro, -O(C₁-C₂ alkyl), -N(C₁-C₂ alkyl)(C₁-C₂ alkyl), -S(C₁-C₂ alkyl), -CO(C₁-C₂ alkyl), -C(=O)H and -C(=O)O(C₁-C₂alkyl), wherein each of the C₁-C₂ alkyl moieties in the foregoing R₁₆₆, R₁₆₈, and R₁₆₉ groups may optionally contain one double or triple bond; and each R₁₆₆, R₁₆₈, and R₁₆₉ that is attached to a nitrogen atom is selected independently from hydrogen and C₁-C₄ alkyl;
   R₁₆₅ is substituted phenyl, substituted naphthyl, substituted pyridyl or substituted pyrimidyl, wherein each of the foregoing R₁₆₅ groups is substituted with from two to four substituents R₁₆₁₅, wherein from one to three of said substituents may be selected independently from chloro, C₁-C₆ alkyl, -O(C₁-C₆ alkyl) and -(C₁-C₆ alkylene)O(C₁-C₆ alkyl), and one of said substituents may be selected, independently, from bromo, iodo, formyl, cyano, trifluoromethyl, nitro, amino, -NH(C₁-C₄ alkyl), -N(C₁-C₂ alkyl)(C₁-C₆ alkyl), -C(=O)O(C₁-C₄ alkyl), -C(=O)(C₁-C₄ alkyl), -COOH, -SO₂NH(C₁-C₄ alkyl), -SO₂N(C₁-C₂ alkyl)(C₁-C₄ alkyl), -SO₂NH₂, -NHSO₂(C₁-C₄ alkyl), -S(C₁-C₆ alkyl) and -SO₂(C₁-C₆ alkyl), and wherein each of the C₁-C₄ alkyl moieties and C₁-C₆ alkyl moieties in the foregoing R₁₆₅ groups may optionally be substituted with one or two substituents independently selected from fluoro, hydroxy, amino, methylamino, dimethylamino and acetyl;
   R₁₆₇ is hydrogen, methyl, halo (e.g. chloro, fluoro, iodo or bromo), hydroxy, methoxy, -C(=O)(C₁-C₂ alkyl), -C(=O)O(C₁-C₂ alkyl), trifluoromethoxy, hydroxymethyl, trifluoromethyl or formyl;
   R₁₆₁₀ is hydrogen, hydroxy, methoxy or fluoro;
   R₁₆₁₁ is hydrogen or C₁-C₄ alkyl;
   R₁₆₁₂ is, hydrogen or methyl; and
   Z₁₄ is NH, oxygen, sulfur, -N(C₁-C₄ alkyl) or -CR₁₆₁₃R₁₆₁₄,
   wherein R₁₆₁₃ and R₁₆₁₄ are independently selected from hydrogen and methyl with the exception that one of R₁₆₁₃ and R₁₆₁₄ may optionally be cyano, with the proviso that: (a) in 6 or 7 membered rings of structures in formula In, there cannot exist two double bonds adjacent to each other and (b) when D₅ is carbon and is double bonded to B₁₁, then B₁₁ is CR₁₆₁R₁₆₂,
   or its pharmaceutically acceptable salt;
(20) A pharmaceutical composition for prophylaxis or therapy of a postoperative stress according to the above (1) or (2), wherein the compound having CRF antagonist activating effect is a compound represented by the formula Io 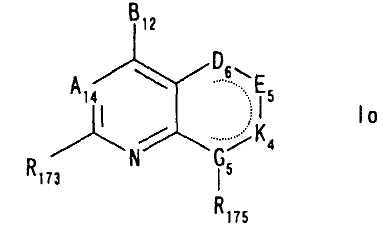 wherein the dashed lines represent optional double bonds;
   A₁₄ is nitrogen or CR₁₇₇;
   B₁₂ is -NR₁₇₁R₁₇₂, -CR₁₇₁R₁₇₂R₁₇₁₀, -C(=CR₁₇₂R₁₇₁₁)R₁₇₁, -NHCR₁₇₁R₁₇₂R₁₇₁₀, -OCR₁₇₁R₁₇₂R₁₇₁₀, -SCR₁₇₁R₁₇₂R₁₇₁₀, -CR₁₇₂R₁₇₁₀NHR₁₇₁, -CR₁₇₂R₁₇₁₀OR₁₇₁, -CR₁₇₂R₁₇₁₀SR₁₇₁ or -COR₁₇₂;
   G₅ is nitrogen or CR₁₇₄ and is single bonded to all atoms to which it is attached, or G₅ is carbon and is double bonded to K₄;
   K₄ is nitrogen or CR₁₇₆ when double bonded to G₅ or E₅, or K₄ is oxygen, sulfur, C=O, C=S, CR₁₇₆R₁₇₁₂ or NR₁₇₈ when single bonded to both adjacent ring atoms, or K₄ is a two atom spacer, wherein one of the atoms of the spacer is oxygen, nitrogen, sulfur, C=O, C=S, CR₁₇₆R₁₇₁₂, NR₁₇₆ or CR₁₇₆, and the other is CR₁₇₆R₁₇₁₂ or CR₁₇₉ ;
   D₆ and E₅ are each independently C=O, C=S, sulfur, oxygen, CR₁₇₄R₁₇₆ or NR₁₇₈ when single bonded to both adjacent ring atoms, or nitrogen or CR₁₇₄ when it is double bonded to one adjacent ring atom;
   6 or 7 membered ring that contains D₆, E₅, K₄ and G₅ may contain from one to three double bonds, from zero to two heteroatoms selected from oxygen, nitrogen and sulfur, and from zero to two C=O or C=S groups, wherein the carbon atoms of such groups are part of the ring and the oxygen and sulfur atoms are substituents on the ring;
   R₁₇₁ is C₁-C₆ alkyl which may optionally be substituted with from one or two substituents independently selected from hydroxy, fluoro, chloro, bromo, iodo, C₁-C₄ alkoxy, CF₃, -C(=O)(C₁-C₄ alkyl), -C(=O)-O-(C₁-C₄ alkyl), -OC(=O)(C₁-C₄ alkyl), -OC(=O)N(C₁-C₄ alkyl)(C₁-C₂ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -S(C₁-C₄ alkyl), -CN, -NO₂, -SO(C₁-C₄ alkyl), SO₂NH(C₁-C₄ alkyl) and -SO₂N(C₁-C₄ alkyl)(C₁-C₂ alkyl), wherein each of the C₁-C₄ alkyl groups in the foregoing R₁₇₁ groups may optionally contain one or two double or triple bonds;
   R₁₇₂ is C₁-C₁₂ alkyl which may optionally contain from one to three double or triple bonds, aryl or (C₁-C₄ alkylene)aryl, wherein said aryl and aryl moieties of said (C₁-C₄ alkylene)aryl is selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl, or C₃-C₈ cycloalkyl or (C₁-C₆ alkylene)(C₃-C₈ cycloalkyl), wherein one or two of the carbon atoms of said cycloalkyl and one or two of the carbon atoms of 5 to 8 membered cycloalkyl moieties of said (C₁-C₆ alkylene)(C₃-C₈ cycloalkyl) may optionally and independently be replaced by oxygen, sulfur or NZ₁₅ wherein Z₁₅ is hydrogen, C₁-C₄ alkyl or benzyl, and wherein each of the foregoing R₁₇₂ groups may optionally be substituted with from one to three substituents independently selected from chloro, fluoro and C₁-C₄ alkyl, or one substituent selected from C₁-C₆ alkoxy, -OC(=O)(C₁-C₆ alkyl), -OC(=O)N(C₁-C₄ alkyl)(C₁-C₂ alkyl), -S(C₁-C₆ alkyl), amino, -NH(C₁-C₂ alkyl), -N(C₁-C₂ alkyl)(C₁-C₄ alkyl), -N(C₁-C₄ alkyl)-CO-(C₁-C₄ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -SH, -CN, -NO₂, SO(C₁-C₄ alkyl), -SO₂(C₁-C₄ alkyl), SO₂NH(C₁-C₄ alkyl) and SO₂N(C₁-C₄ alkyl)(C₁-C₂ alkyl);
   -NR₁₇₁R₁₇₂ or CR₁₇₁R₁₇₂R₁₇₁₀ may form a ring selected from saturated 3 to 8 membered rings, and 5 to 8 membered rings of which may optionally contain one or two double bonds, wherein one or two of the ring carbon atoms of such 5 to 8 membered rings may optionally and independently be replaced by oxygen, sulfur or NZ₁₇₃ wherein Z₁₇₃ is hydrogen, or C₁-C₄ alkyl;
   R₁₇₃ is hydrogen, C₁-C₄ alkyl, -O(C₁-C₄ alkyl), fluoro, chloro, bromo, iodo, S(C₁-C₄ alkyl) or SO₂(C₁-C₄ alkyl);
   each R₁₇₈, R₁₇₉ and R₁₇₁₂ is selected independently from hydrogen and C₁-C₂ alkyl;
   R₁₇₄ and R₁₇₆ that is attached to a carbon atom is hydrogen, C₁-C₂ alkyl, hydroxy or fluoro;
   each R₁₇₆, R₁₇₈ and R₁₇₉ that is attached to a carbon atom is selected independently from hydrogen, C₁-C₆ alkyl, fluoro, chloro, bromo, iodo, hydroxy, hydroxy(C₁-C₂ alkyl), trifluoromethyl, cyano, amino, nitro, -O(C₁-C₄ alkyl), -N(C₁-C₄ alkyl)(C₁-C₂ alkyl), CH₂SCH₃, -S(C₁-C₄ alkyl), CO(C₁-C₄ alkyl), -C(=O)H and C(=O)O(C₁-C₄ alkyl), wherein each of the C₁-C₂ alkyl moieties in the foregoing R₁₇₄ and R₁₇₆ groups may optionally contain one double or triple bond, and R₁₇₆ that is attached to a nitrogen atom is selected independently from hydrogen and C₁-C₄ alkyl;
   R₁₇₅ is substituted phenyl, substituted naphthyl, substituted pyridyl or substituted pyrimidyl, wherein each of the foregoing R₁₇₅ groups is substituted with from two to four substituents R₁₇₁₃, wherein up to three of said substituents may be selected from chloro, C₁-C₆ alkyl, -O(C₁-C₆ alkyl) and -(C₁-C₆ alkylene)O(C₁-C₆ alkyl), and one of said substituents may be selected from bromo, iodo, formyl, cyano, trifluoromethyl, nitro, amino, -NH(C₁-C₄ alkyl), -N(C₁-C₂ alkyl)(C₁-C₆ alkyl), -C(=O)O(C₁-C₄ alkyl), -C(=O)(C₁-C₄ alkyl), -COOH, SO₂NH(C₁-C₆ alkyl), SO₂N(C₁-C₂ alkyl)(C₁-C₄ alkyl), SO₂NH₂, -NHSO₂(C₁-C₄ alkyl), -(C₀-C₁ alkylene)-S-(C₁-C₂ alkyl), -(C₀-C₁ alkylene)-SO-(C₁-C₂ alkyl), -(C₀-C₁ alkylene)-SO₂-(C₁-C₂ alkyl) and -(C₁-C₄ alkylene)-OH, and wherein each of the C₁-C₄ alkyl moieties and C₁-C₆ alkyl moieties in the foregoing R₁₇₅ groups may optionally be substituted with one or two substituents independently selected from fluoro, hydroxy, amino, methylamino, dimethylamino and acetyl;
   R₁₇₇ is hydrogen, methyl, halo (e.g., chloro, fluoro, iodo or bromo), hydroxy, methoxy, -C(=O)(C₁-C₂ alkyl), -C(=O)O(C₁-C₂ alkyl), hydroxymethyl, trifluoromethyl or formyl;
   R₁₇₁₀ is hydrogen, hydroxy, methoxy or fluoro;
   R₁₇₁₁ is hydrogen or C₁-C₄ alkyl; and
   R₁₇₁₂ is hydrogen or methyl; with the proviso that in the ring containing D₆, E₅, K₄ and G₅ of formula Io, there cannot exist two double bonds adjacent to each other,
   or its pharmaceutically acceptable salt;
(21) A pharmaceutical composition for prophylaxis or therapy of a postoperative stress according to the above (1) or (2), wherein the compound having CRF antagonist activating effect is a compound represented by the formula Ip 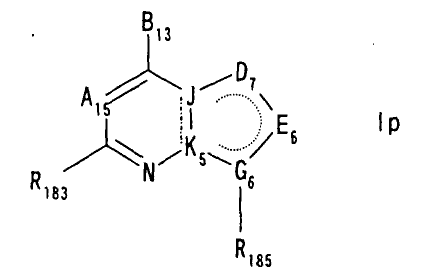 wherein the dashed lines represent optional double bonds;
   A₁₅ is nitrogen or CR₁₈₇;
   B₁₃ is -NR₁₈₁R₁₈₂, -CR₁₈₁R₁₈₂R₁₈₁₀, -C(=CR₁₈₂R₁₈₁₁)R₁₈₁, -NHCR₁₈₁R₁₈₂R₁₈₁₀, -OCR₁₈₁R₁₈₂R₁₈₁₀, -SCR₁₈₁R₁₈₂R₁₈₁₀, -CR₁₈₂R₁₈₁₀NHR₁₈₁, -CR₁₈₂R₁₈₁₀OR₁₈₁, -CR₁₈₂R₁₈₁₀SR₁₈₁ or -COR₁₈₂;
   J and K₅ are each independently nitrogen or carbon and both J and K₅ are not nitrogens;
   D₇ and E₆ are each selected independently from nitrogen, CR₁₈₄, C=O, C=S, sulfur, oxygen, CR₁₈₄R₁₈₆ and NR₁₈₈;
   G₆ is nitrogen or carbon;
   the ring containing D₇, E₆, G₆, K₅ and J in formula Ip may be a saturated or unsaturated 5 membered ring, and may optionally contain one or two double bonds and may optionally contain from one to three heteroatoms in the ring and may optionally have one or two C=O or C=S groups;
   R₁₈₁ is C₁-C₆ alkyl which may optionally be substituted with one or two substituents independently selected from hydroxy, fluoro, chloro, bromo, iodo, C₁-C₄ alkyl, CF₃, -C(=O)-O-(C₁-C₄ alkyl), -OC(=O)(C₁-C₄ alkyl), -OC(=O)N(C₁-C₄ alkyl)(C₁-C₂ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -S(C₁-C₄ alkyl), -CN, -NO₂, -SO(C₁-C₄ alkyl), SO₂NH(C₁-C₄ alkyl) and SO₂N(C₁-C₄ alkyl)(C₁-C₂ alkyl), wherein each of the C₁-C₄ alkyl groups in the foregoing R₁₈₁ groups may optionally contain one or two double or triple bonds;
   R₁₈₂ is C₁-C₁₂ alkyl which may optionally contain from one to three double or triple bonds, aryl or (C₁-C₄ alkylene)aryl, wherein said aryl and aryl moieties of said (C₁-C₄ alkylene)aryl is selected from the group consisting of phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl,, pyrrolyl, indolyl, pyrrolopyridyl, oxazolyl and benzoxazolyl, or C₃-C₈ cycloalkyl or (C₁-C₆ alkylene)(C₃-C₆ cycloalkyl), wherein one or two of the carbon atoms of said cycloalkyl and one or two of the carbon atoms of 5 to 8 membered cycloalkyl moieties of said (C₁-C₆ alkylene)(C₃-C₈ cycloalkyl) may optionally and independently be replaced by oxygen, sulfur or NZ₁₈₂ wherein Z₁₈₂ is selected from hydrogen, C₁-C₄ alkyl, benzyl and C₁-C₄ alkanoyl, and wherein each of the foregoing R₁₈₂ groups may optionally be substituted with from one to three substituents independently selected from chloro, fluoro and C₁-C₄ alkyl, or one substituent selected from bromo, iodo, C₁-C₆ alkoxy, -OC(=O)(C₁-C₆ alkyl), -OC(=O)N(C₁-C₄ alkyl)(C₁-C₂ alkyl), -S(C₁-C₆ alkyl), amino, -NH(C₁-C₂ alkyl), -N(C₁-C₂ alkyl)(C₁-C₄ alkyl), -N(C₁-C₄ alkyl)-CO-(C₁-C₄ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -SH, -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl);
   -NR₁₈₁R₁₈₂ or CR₁₈₁R₁₈₂R₁₈₁₀ may form a saturated 3 to 8 membered carbocyclic ring which may optionally contain from one to three double bonds and wherein one or two of the ring carbon atoms of such 5 to 8 membered rings may optionally and independently be replaced by oxygen, sulfur atom or NZ₁₈₃ wherein Z₁₈₃ is hydrogen, C₁-C₄ alkyl, benzyl or C₁-C₄ alkanoyl;
   R₁₈₃ is hydrogen, C₁-C₄ alkyl, -O(C₁₋₄ alkyl), chloro, fluoro, bromo, iodo, (C₁-C₂ alkylene)-O-(C₁-C₂ alkyl), (C₁-C₂ alkylene)-OH, or -S(C₁-C₄ alkyl);
   each R₁₈₄ is independently hydrogen, (C₁-C₆ alkyl), fluoro, chloro, bromo, iodo, hydroxy, cyano, amino, (C₁-C₂ alkylene)-OH, CF₃, CH₂SCH₃, nitro, -O(C₁-C₄ alkyl), -N(C₁-C₄ alkyl)(C₁-C₂ alkyl), -S(C₁-C₄ alkyl), -CO(C₁-C₄ alkyl), -C(=O)H or -C(=O)O(C₁-C₄ alkyl);
   R₁₈₆ is hydrogen, methyl or ethyl;
   R₁₈₈ is hydrogen or C₁-C₄ alkyl;
   R₁₈₅ is phenyl, pyridyl, pyrazinyl, pyrimidyl, pyridazinyl, and wherein each of the foregoing R₁₈₅ groups is substituted with from one to four substituents R₁₈₁₃ wherein one to three of said substituents may be selected independently from fluoro, chloro, C₁-C₆ alkyl and -O(C₁-C₆ alkyl) and one of said substituents may be selected from bromo, iodo, formyl, OH, (C₁-C₄ alkylene)-OH, (C₁-C₄ alkylene)-O-(C₁-C₂ alkyl), cyano, trifluoromethyl, nitro, amino, -NH(C₁₋C₄ alkyl), -N(C₁₋C₂ alkyl)(C₁-C₆ alkyl), -OCO(C₁-C₄ alkyl), -(C₁-C₄ alkylene)-O-(C₁-C₄ alkyl), -S-(C₁-C₆ alkyl), -(C₁-C₄ alkylene)-S-(C₁-C₄ alkyl), -C(=O)O(C₁-C₄ alkyl), -C(=O)(C₁-C₄ alkyl), -COOH, -SO₂NH(C₁-C₆ alkyl), -SO₂N(C₁-C₂ alkyl)(C₁-C₄ alkyl), -SO₂NH₂, -NHSO₂(C₁-C₄ alkyl), -S(C₁-C₆ alkyl) and -SO₂(C₁-C₆ alkyl), and wherein each of the C₁-C₄ alkyl moieties and C₁-C₆ alkyl moieties in the foregoing R₁₈₅ groups may optionally have one or two double bonds;
   R₁₈₇ is hydrogen, C₁-C₄ alkyl, halo, (e.g. chloro, fluoro, iodo or bromo), hydroxy, -O(C₁-C₄ alkyl), -C(=O)(C₁-C₄ alkyl), -C(=O)O(C₁-C₄ alkyl), -OCF₃, trifluoromethyl, hydroxymethyl or -CH₂O(C₁-C₂ alkyl);
   R₁₈₁₀ is hydrogen, hydroxy, methoxy or fluoro;
   R₁₈₁₁ is hydrogen or C₁-C₄ alkyl, with the proviso that in the formula IP (a) when both J and K₅ are carbons and D₇ is CR₁₈₄ and E₆ is nitrogen, then G₆ can not be nitrogen (b) when both J and K₅ are carbons and D₇ and G₆ are nitrogens, then E₆ can not be CR₁₈₄, C=O or C=S (c) when both J and K₅ are carbons and D₇ and E₆ are carbons, then G₆ can not be nitrogen (d) when G₆ is carbon, it must be double bonded to E₆ and (e) in the ring containing J, K₅, D₇, E₆ and G₆, there can not be two double bonds adjacent to each other,
   or its pharmaceutically acceptable salt;
(22) A pharmaceutical composition for prophylaxis or therapy of a postoperative stress according to the above (1), wherein the compound having CRF antagonist activating effect is a compound represented by the formula (A) 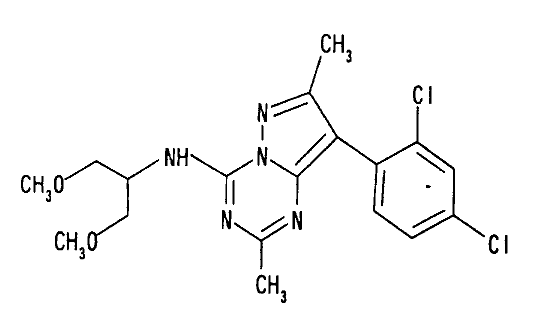 , formula (B) 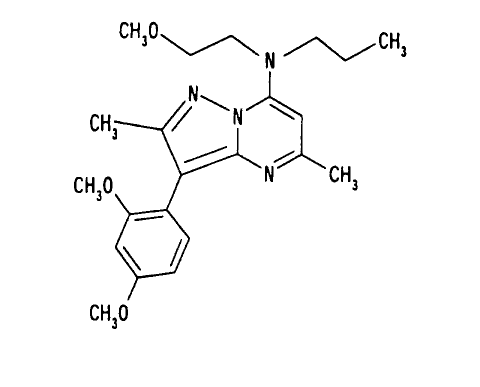 , formula (C) 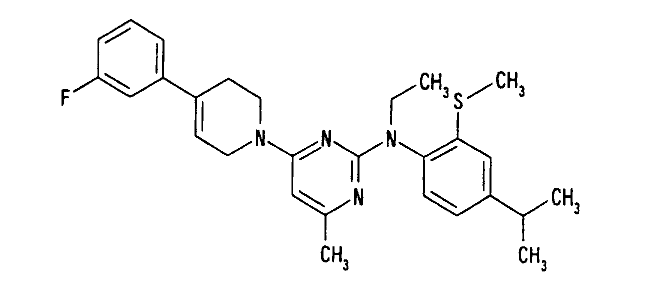 , formula (D) 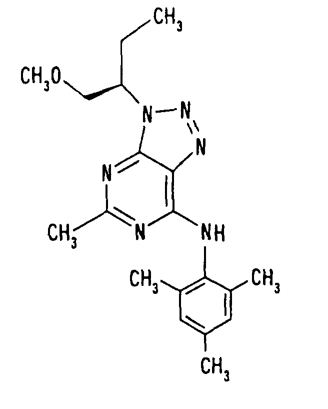 , formula (E) 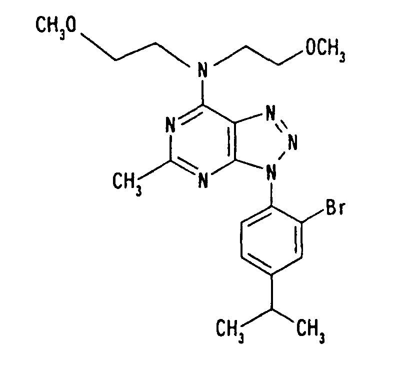 or the formula (F) 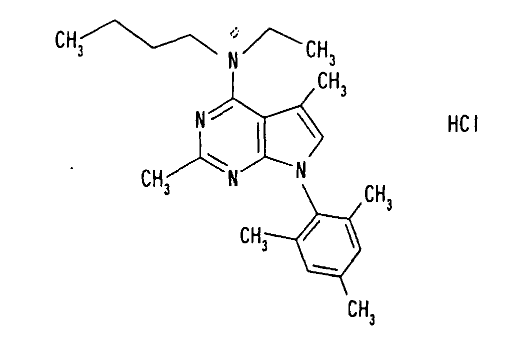
(23) A pharmaceutical composition for prophylaxis or therapy of a postoperative stress according to the above (1),
   wherein the compound having CRF antagonist activating effect is N-butyl-N-(2,5-dimethyl-7-(2,4,6-trimethylphenyl)-7H-pyrrolo(2,3-d)pyrimidine-4-yl)-N-ethylamine acid salt;
(24) A compound having CRF antagonist activating effect is a compound represented by the formula Iq 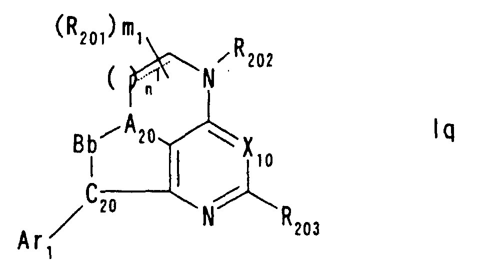 wherein n₁ is 1 or 2;
   A₂₀ and C₂₀ are each independently nitrogen, carbon or CH;
   Bb is nitrogen or CR₂₀₄;
   with the proviso that at least one of A₂₀, Bb and C₂₀ is nitrogen, A₂₀ Bb and C₂₀ are not all nitrogen, and either A₂₀-Bb or Bb-C₂₀ is a double bond;
   X₁₀ is nitrogen or CH;
   Ar₁ is aryl, substituted aryl, heteroaryl or substituted heteroaryl;
   R₂₀₁ is an optional substituent, which at each occurrence independently selected from alkyl, alkyliden, arylalkyl and heteroarylalkyl, and m₁ is 0, 1, 2 or 3 and represents the number of R₂₀₁ substituents;
   R₂₀₂ is -C(H)_{0,1}(R₂₀₅)(R₂₀₆) or -SO₂R₂₀₆;
   R₂₀₃ is hydrogen or alkyl;
   R₂₀₄ is hydrogen, alkyl or haloalkyl;
   R₂₀₅ is hydrogen, keto, alkyl, alkyliden or halo; and
   R₂₀₆ is a radical of the formula -Y₂₀-Z₂₀-R₂₀₇, with the proviso that Y₂₀ is an alkanediyl, substituted alkanediyl or a bond, Z₂₀ is NH, -N(R₂₀₈), oxygen, sulfur, SO₂, C(=O), C(=O)O, OC(=O), NHC(=O), C(=O)NH, NH(SO₂), (SO₂)NH, NR₂₀₉C(=O)O or a bond;
   R₂₀₇ is hydrogen, alkyl, substituted alkyl, aryl, substituted aryl, aralkyl, substituted aralkyl, heterocycle, substituted heterocycle, heterocyclealkyl, or substituted heterocyclealkyl; or
   R₂₀₈ and R₂₀₉ are the same or different and are independently alkyl, substituted alkyl, aryl, substituted aryl, aralkyl, substituted aralkyl, heterocycle, substituted heterocycle, heterocyclealkyl or substituted heterocyclealkyl; or
   R₂₀₇ and R₂₀₈ taken together with the nitrogen atom to which they are attached form a heterocycle ring or substituted heterocycle ring; or
   R₂₀₅ and R₂₀₆ taken together form cycloalkyl, substituted cycloalkyl, cycloalkylcycloalkyl, substituted cycloalkylcycloalkyl, cycloalkylaryl, substituted cycloalkylaryl, cycloalkylheterocycle or substituted cycloalkylheterocycle, and the symbol ------ represents single bonds or double bonds,
   its stereoisomer, pro-drugs or its pharmaceutically acceptable salt;
(25) A pharmaceutical composition for prophylaxis or therapy of a postoperative stress according to the above (1),
   wherein the compound having CRF antagonist activating effect is a compound represented by the formula Ir 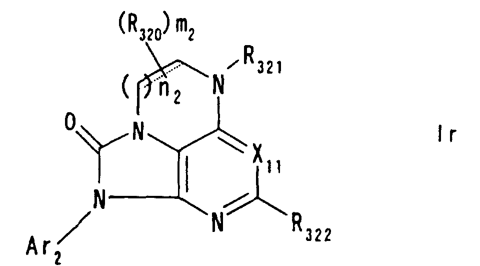 wherein n₂ is 1 or 2;
   m₂ is 0, 1, 2 or 3;
   X₁₁ is N or CR' ;
   R₃₂₀ is an optional substituent, which at each occurrence may be independently selected from C₁-C₆ alkyl, C₃-C₆ alkenyl, C₁-C₆ alkylidenyl or C₁-C₆ alkylAr₂;
   R₃₂₁ represents -C(H)_{0,1}(R₃₂₃)(R₃₂₄);
   R₃₂₂ is hydrogen or C₁-C₆ alkyl;
   R₃₂₃ is selected from hydrogen, keto, C₁-C₆ alkyl, mono or di(C₃-C₆ cycloalkyl)methyl, C₃-C₆ cycloalkyl, C₃-C₆ alkenyl, hydroxy C₁-C₆ alkyl, C₁-C₆ alkylcarbonyloxy C₁-C₆ alkyl or C₁-C₆ alkyloxy C₁-C₆ alkyl; and
   R₃₂₄ is selected from hydrogen, Ar₂₁, C₁-C₆ alkyl Ar₂₁, OAr₂₁, C₁-C₈ alkyl, C₁-C₆ alkyloxy, C₃-C₆ cycloalkyl, mono or di(C₃-C₆ cycloalkyl)methyl, C₃-C₆ alkenyl, C₃-C₆ alkynyl, C₁-C₆ alkyloxy C₁-C₆ alkyl, C₁-C₆ alkoxyAr_{21,} hydroxyl C₁-C₆ alkyl, thienyl C₁-C₆ alkyl, furanyl C₁-C₆ alkyl, C₁-C₆ alkylthio C₁-C₆ alkyl, morpholinyl, mono or di(C₁-C₆ alkyl)amino C₁-C₆ alkyl, amino, (C₁-C₆ alkyl)amino, di(C₁-C₆ alkyl)amino, (C₁-C₆ alkylAr₂₁)amino, (C₁-C₆ alkyl)(Ar₂₁)amino, C₁-C₆ alkylcarbonyl C₁-C₆ alkyl, C₁-C₆ alkylcarbonyloxy C₁-C₆ alkyl, sulfonyl(C₁-C₈ alkyl), C(=O) C₁-C₆ alkyl, C₁-C₈ alkyl substituted with phthalimido, Ar₂₁, OAr₂₁, NHAr₂₁, C(=O)Ar₂₁, C(=O)NHAr₂₁ or -C(=O)NH₂, or a radical represented by the formula -(C₁₋₆alkanediyl)-Y₂₁-(CO)_{0.1}-Ar₂₁ wherein Y₂₁ is O, NH or chemical bond, or
   R₃₂₃ and R₃₂₄ taken together with carbon atom to which they are attached form C₅-C₈ cycloalkyl, C₅-C₈ cycloalkenyl, C₃-C₁₂ heterocycle ring, phenyl, naphthyl or C₅-C₈ cycloalkyl condensed to Ar₂₁, which may optionally be substituted with not less than one substituents independently selected from C₁-C₆ alkyl;
   Ar₂ is phenyl, naphthyl or aromatic C₃-C₁₂ heterocycle, each of which may optionally be substituted with one, two or three substituents independently selected from halo, C₁-C₆ alkyl, triflouromethyl, O(triflouromethyl), hydroxy, cyano, C₁-C₆ alkyloxy, phenyloxy, benzoxy, C₁-C₆ alkylthio, nitro, amino, mono or di(C₁-C₆ alkyl)amino, (C₁-C₆ alkyl)(C₁-C₆ alkanoyl)amino or piperidinyl, two of the above substituents optionally combining to form C₁-C₆ alkylidinyl or C₁-C₆ alkylidenyl, whose one, two or three carbons is/are replaced by hetero atom or hetero atoms selected independently from oxygen, nitrogen or sulfur, and
   Ar₂₁ is phenyl, naphthyl or aromatic C₃-C₁₂ heterocycle ring optionally substituted with one, two or three substituents which is/are independently selected from a group consisting of halo, C₁-C₆ alkyl, C₁-C₆ alkyloxy, di(C₁-C₆ alkyl)amino, di(C₁-C₆ alkyl)amino C₁-C₆ alkyl, triflouromethyl, suifonyl(C₁-C₆ alkyl) and C₁-C₆ alkyl substituted with morpholinyl, and the symbol ------ represents single bond or double bond,
   its stereoisomer, pro-drugs or its pharmaceutically acceptable salt;
(26) A pharmaceutical composition for prophylaxis or therapy of a postoperative stress according to the above (1), wherein the compound having CRF antagonist activating effect is a compound represented by the formula Is 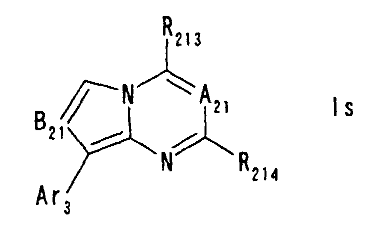 wherein A₂₁ and B₂₁ are selected from CR₂₁₅ wherein R₂₁₅ shows the group selected from hydrogen and C₁-C₆ alkyl, and nitrogen;
   R₂₁₃ is NR₂₁₆R₂₁₇;
   R₂₁₄ is C₁-C₆ alkyl;
   R₂₁₆ is selected from the group consisting of hydrogen, C₁-C₆ alkyl, mono- or di(C₃-C₆ cycloalkyl)methyl, C₃-C₆ cycloalkyl, C₃-C₆ alkenyl, hydroxy C₁-C₄ alkyl, C₁-C₆ alkylcarbonyloxy C₁-C₆ alkyl and C₁-C₆ alkyloxy C₁-C₆ alkyl;
   R₂₁₇ is selected from C₁-C₈ alkyl, mono or di(C₃-C₆ cycloalkyl)methyl, Ar₂₂CH₂, C₃-C₆ alkenyl, C₁-C₆ alkyloxy C₁-C₆ alkyl, hydroxyl C₁-C₆ alkyl, thienylmethyl, furanylmethyl, C₁-C₆ alkylthio C₁-C₆ alkyl, morpholinyl, mono or di(C₁-C₆alkyl)amino C₁-C₆ alkyl, di(C₁-C₆ alkyl)amino, C₁-C₆ alkylcarbonyl C₁-C₆ alkyl, C₁-C₆ alkyl substituted with imidazolyl, or a radical of the formula (C₁-C₆ alkanediyl)-O-CO-Ar₂₂;
   or R₂₁₆ and R₂₁₇ taken together with nitrogen to which they are attached form a pyrrolidinyl, piperidinyl, homopiperidinyl or morpholinyl, which is optionally substituted with C₁-C₆ alkyl or C₁-C₆ alkyloxy;
   Ar₃ is selected from phenyl substituted with one, two or three substituents independently selected from halo, C₁-C₆ alkyl, triflouromethyl, cyano, C₁-C₆ alkyloxy, benzyloxy, C₁-C₆ alkylthio, nitro, amino and mono or di(C₁-C₆ alkyl)amino, and pyridinyl substituted with one, two or three substituents independently selected from halo, C₁-C₆ alkyl, triflouromethyl, hydroxy, cyano, C₁-C₆ alkyloxy, benzyloxy, C₁-C₆ alkylthio, nitro, amino, mono or di(C₁-C₆ alkyl)amino and piperidinyl, and
   Ar₂₂ is selected from phenyl, pyridinyl and phenyl substituted with one, two or three substituents independently selected from halo, C₁-C₆ alkyl, C₁-C₆ alkyloxy, di(C₁-C₆ alkyl)amino C₁-C₆ alkyl, triflouromethyl and C₁-C₆ alkyl substituted with morpholinyl,
   its stereoisomer, pro-drugs or its pharmaceutically acceptable salt;
(27) A pharmaceutical composition for prophylaxis or therapy of a postoperative stress according to the above (1), wherein the compound having CRF antagonist activating effect is a compound represented by the formula It 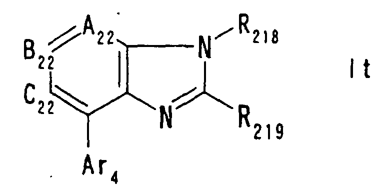 wherein A₂₂, B₂₂ and C₂₂ are selected from CR₂₂₀ and nitrogen; with the proviso that when B₂₂ is nitrogen, then A₂₂ and C₂₂ are CR₂₂₀;
   R₂₂₀ is selected from hydrogen and C₁-C₆ alkyl;
   R₂₁₈ is selected from NR₂₂₁R₂₂₂ and R₂₂₃;
   R₂₁₉ is C₁-C₆ alkyl;
   R₂₂₁ is selected from hydrogen, C₁-C₆ alkyl, mono- or di(C₃-C₆ cycloalkyl)methyl, C₃-C₆ cycloalkyl, C₃-C₆ alkenyl, hydroxy C₁-C₆ alkyl, C₁-C₆ alkylcarbonyloxy C₁-C₆ alkyl and C₁-C₆ alkyloxy C₁-C₆ alkyl;
   R₂₂₂ and R₂₂₃ are independently selected from C₁-C₈ alkyl, mono or di(C₃-C₆ cycloalkyl)methyl, Ar₂₃CH₂, C₃-C₆ alkenyl, C₁-C₆ alkyloxy C₁-C₆ alkyl, hydroxy C₁-C₆ alkyl, thienylmethyl, furanylmethyl, C₁-C₆ alkylthio C₁-C₆ alkyl, morpholinyl, mono or di(C₁-C₆alkyl)amino C₁-C₆ alkyl, di(C₁-C₆ alkyl)amino, C₁-C₆ alkylcarbonyl C₁-C₆ alkyl, C₁-C₆ alkyl substituted with imidazolyl, or a radical of the formula (C₁-C₆ alkanediyl)-O-CO-Ar₂₃;
   or R₂₂₁ and R₂₂₂ taken together with nitrogen to which they are attached form a pyrrolidinyl, piperidinyl, homopiperidinyl or morpholinyl, wherein optionally substituted with C₁-C₆ alkyl or C₁-C₆ alkyloxy;
   Ar₄ is selected from phenyl substituted with one, two or three substituents independently selected from halo, C₁-C₆ alkyl, triflouromethyl, cyano, C₁-C₆ alkyloxy, benzyloxy, C₁-C₆ alkylthio, nitro, amino and mono or di(C₁-C₆ alkyl)amino, and pyridinyl substituted with one, two or three substituents independently selected from halo, C₁-C₆ alkyl, triflouromethyl, hydroxy, cyano, C₁-C₆ alkyloxy, benzyloxy, C₁-C₆ alkylthio, nitro, amino, mono or di(C₁-C₆ alkyl)amino and piperidinyl, and
   Ar₂₃ is selected from phenyl, pyridinyl and phenyl substituted with one, two or three substituents independently selected from halo, C₁-C₆ alkyl, C₁-C₆ alkyloxy, di(C₁-C₆alkyl)amino C₁-C₆ alkyl, triflouromethyl and C₁-C₆ alkyl substituted with morpholinyl,
   its stereoisomer, pro-drugs or its pharmaceutically acceptable salt;
(28) A pharmaceutical composition for prophylaxis or therapy of a postoperative stress according to the above (1),
   wherein the compound having CRF antagonist activating effect is a compound represented by the formula Iu 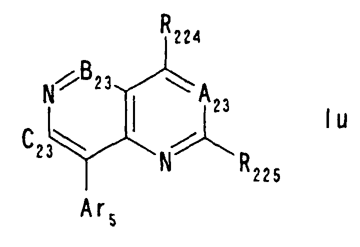 wherein A₂₃, B₂₃ and C₂₃ are selected from CR₂₂₆ and nitrogen, with the proviso that B₂₃ and C₂₃ are not both nitrogen;
   R₂₂₆ is selected from hydrogen and C₁-C₆ alkyl;
   R₂₂₄ is NR₂₂₇R₂₂₈;
   R₂₂₅ is C₁-C₆ alkyl;
   R₂₂₇ is selected from hydrogen, C₁-C₆ alkyl, mono or di(C₃-C₆ cycloalkyl)methyl, C₃-C₆ cycloalkyl, C₃-C₆ alkenyl, hydroxy C₁-C₆ alkyl, C₁-C₆ alkylcarbonyloxy C₁-C₆ alkyl and C₁-C₆ alkyloxy C₁-C₆ alkyl;
   R₂₂₈ is independently selected from C₁-C₈ alkyl, mono or di(C₃-C₆ cycloalkyl)methyl, Ar₂₄CH₂, C₃-C₆ alkenyl, C₁-C₆ alkyloxy C₁-C₆ alkyl, hydroxy C₁-C₆ alkyl, thienylmethyl, furanylmethyl, C₁-C₆ alkylthio C₁-C₆ alkyl, morpholinyl, mono or di(C₁-C₆ alkyl)amino C₁-C₆ alkyl, di(C₁-C₆ alkyl)amino, C₁-C₆ alkylcarbonyl C₁-C₆ alkyl, C₁-C₆ alkyl substituted with imidazolyl, and a radical of the formula (C₁-C₆ alkanediyl)-O-CO-Ar₂₄;
   or R₂₂₇ and R₂₂₈ taken together with nitrogen to which they are attached form a pyrrolidinyl, piperidinyl, homopiperidinyl or morpholinyl, which is optionally substituted with C₁-C₆ alkyl or C₁-C₆ alkyloxy;
   Ar₅ is selected from phenyl substituted with one, two or three substituents independently selected from halo, C₁-C₆ alkyl, triflouromethyl, cyano, C₁-C₆ alkyloxy, benzyloxy, C₁-C₆ alkylthio, nitro, amino and mono or di(C₁-C₆ alkyl)amino, and pyridinyl substituted with one, two or three substituents independently selected from halo, C₁-C₆ alkyl, triflouromethyl, hydroxy, cyano, C₁-C₆ alkyloxy, benzyloxy, C₁-C₆ alkylthio, nitro, amino, mono or di(C₁₋₆ alkyl)amino and piperidinyl; and
   Ar₂₄ is selected from phenyl, pyridinyl, and phenyl substituted with one, two or three substituents independently selected from halo, C₁-C₆ alkyl, C₁-C₆ alkyloxy, di(C₁-C₆ alkyl)amino C₁-C₆ alkyl, triflouromethyl and C₁-C₆ alkyl substituted with morpholinyl,
   its stereoisomer, pro-drugs or its pharmaceutically acceptable salt;
(29) A pharmaceutical composition for prophylaxis or therapy of a postoperative stress according to the above (1), wherein the compound having CRF antagonist activating effect is a compound represented by the formula Iv 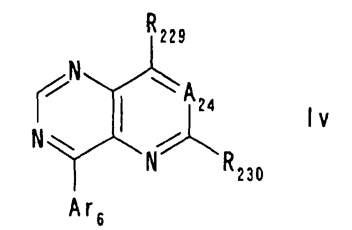 wherein A₂₄ is selected from CR₂₃₁ and nitrogen;
   R₂₃₁ is selected from hydrogen and C₁-C₆ alkyl;
   R₂₂₉ is NR₂₃₂R₂₃₃;
   R₂₃₀ is C₁-C₆ alkyl;
   R₂₃₂ is selected from hydrogen, C₁-C₆ alkyl, mono or di(C₃-C₆ cycloalkyl)methyl, C₃-C₆ cycloalkyl, C₃-C₆ alkenyl, hydroxy C₁-C₆ alkyl, C₁-C₆ alkylcarbonyloxy C₁-C₆ alkyl and C₁-C₆ alkyloxy C₁-C₆ alkyl;
   R₂₃₃ is independently selected from C₁-C₈ alkyl, mono or di(C₃-C₆ cycloalkyl)methyl, Ar₂₅CH₂, C₃-C₆ alkenyl, C₁-C₆ alkyloxy C₁-C₆ alkyl, hydroxy C₁-C₆ alkyl, thienylmethyl, furanylmethyl, C₁-C₆ alkylthio C₁-C₆ alkyl, morpholinyl, mono or di(C₁-C₆ alkyl)amino C₁-C₆ alkyl, di(C₁-C₆ alkyl)amino, C₁-C₆ alkylcarbonyl C₁-C₆ alkyl, C₁-C₆ alkyl substituted with imidazolyl, and a radical of the formula-(C₁-C₆ alkanediyl)-O-CO-Ar₂₅;
   or R₂₃₂ and R₂₃₃ taken together with nitrogen to which they are attached form a pyrrolidinyl, piperidinyl, homopiperidinyl or morpholinyl, which is optionally substituted with C₁-C₆ alkyl or C₁-C₆ alkyloxy;
   Ar₆ is selected from phenyl substituted with one, two or three substituents independently selected from halo, C₁-C₆ alkyl, triflouromethyl, cyano, C₁-C₆ alkyloxy, benzyloxy, C₁-C₆ alkylthio, nitro, amino and mono or di(C₁-C₆ alkyl)amino, and pyridinyl substituted with one, two or three substituents independently selected from halo, C₁-C₆ alkyl, triflouromethyl, hydroxy, cyano, C₁-C₆ alkyloxy, benzyloxy, C₁-C₆ alkylthio, nitro, amino, mono or di(C₁-C₆ alkyl)amino and piperidinyl; and
   Ar₂₅ is selected from phenyl, pyridinyl, and phenyl substituted with one, two or three substituents independently selected from halo, C₁-C₆ alkyl, C₁-C₆ alkyloxy, di(C₁-C₆ alkyl)amino C₁-C₆ alkyl, triflouromethyl and C₁-C₆ alkyl substituted with morpholinyl,
   its stereoisomer, pro-drugs or its pharmaceutically acceptable salt;
(30) A pharmaceutical composition for prophylaxis or therapy of a postoperative stress according to the above (1), wherein the compound having CRF antagonist activating effect is a compound represented by the formula Iw 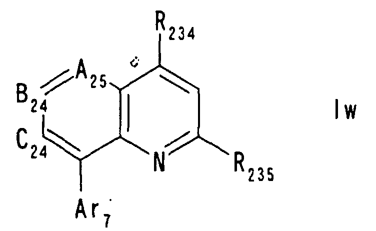 wherein A₂₅, B₂₄ and C₂₄ are selected from CR₂₃₆ and nitrogen, with the proviso that B₂₄ and C₂₄ are not both nitrogen;
   R₂₃₆ is selected from hydrogen and C₁-C₆ alkyl;
   R₂₃₄ is NR₂₃₇R₂₃₈;
   R₂₃₅ is C₁-C₆ alkyl;
   R₂₃₇ is selected from hydrogen, C₁-C₆ alkyl, mono or di(C₃-C₆ cycloalkyl)methyl, C₃-C₆ cycloalkyl, C₃-C₆ alkenyl, hydroxy C₁-C₆ alkyl, C₁-C₆ alkylcarbonyloxy C₁-C₆ alkyl and C₁-C₆ alkyloxy C₁-C₆ alkyl;
   R₂₃₈ is independently selected from C₁-C₈ alkyl, mono or di(C₃-C₆ cycloalkyl)methyl, Ar₂₆CH₂, C₃-C₆ alkenyl, C₁-C₆ alkyloxy C₁-C₆ alkyl, hydroxy C₁-C₆ alkyl, thienylmethyl, furanylmethyl, C₁-C₆ alkylthio C₁-C₆ alkyl, morpholinyl, mono or di(C₁-C₆ alkyl)amino C₁-C₆ alkyl, di(C₁-C₆ alkyl)amino, C₁-C₆ alkylcarbonyl C₁-C₆ alkyl, C₁-C₆ alkyl substituted with imidazolyl, and a radical of the formula -( C₁-C₆ alkanediyl)-O-CO-Ar₂₆;
   or R₂₃₇ and R₂₃₈ taken together with nitrogen to which they are attached form a pyrrolidinyl, piperidinyl, homopiperidinyl or morpholinyl, which is optionally substituted with C₁-C₆ alkyl or C₁-C₆ alkyloxy;
   Ar₇ is selected from phenyl substituted with one, two or three substituents independently selected from halo, C₁-C₆ alkyl, triflouromethyl, cyano, C₁-C₆ alkyloxy, benzyloxy, C₁-C₆ alkylthio, nitro, amino and mono or di(C₁-C₆ alkyl)amino, and pyridinyl substituted with 1, 2 or 3 substituents independently selected from halo, C₁-C₆ alkyl, triflouromethyl, hydroxy, cyano, C₁-C₆ alkyloxy, benzyloxy, C₁-C₆ alkylthio, nitro, amino, mono or di(C₁-C₆ alkyl)amino and piperidinyl; and
   Ar₂₆ is selected from phenyl, pyridinyl, and phenyl substituted with one, two or three substituents independently selected from halo, C₁-C₆ alkyl, C₁-C₆ alkyloxy, di(C₁-C₆ alkyl)amino C₁-C₆ alkyl, triflouromethyl and C₁-C₆ alkyl substituted with morpholinyl,
   its stereoisomer, pro-drugs or its pharmaceutically acceptable salt;
(31) A pharmaceutical composition for prophylaxis or therapy of a postoperative stress according to the above (1), wherein the compound having CRF antagonist activating effect is a compound represented by the formula Ix 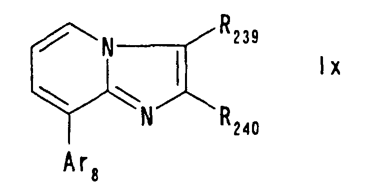 wherein R₂₃₉ is selected from NR₂₄₁R₂₄₂ and R₂₄₃;
   R₂₄₀ is C₁-C₆ alkyl;
   R₂₄₁ is selected from hydrogen, C₁-C₆ alkyl, mono or di(C₃-C₆ cycloalkyl)methyl, C₃-C₆ cycloalkyl, C₃-C₆ alkenyl, hydroxy C₁-C₆ alkyl, C₁-C₆ alkylcarbonyloxy C₁-C₆ alkyl and C₁-C₆ alkyloxy C₁-C₆ alkyl;
   R₂₄₂ and R₂₄₃ are independently selected from C₁-C₈ alkyl, mono or di(C₃-C₆ cycloalkyl)methyl, Ar₂₇CH₂, C₃-C₆ alkenyl, C₁-C₆ alkyloxy C₁-C₆ alkyl, hydroxy C₁-C₆ alkyl, thienylmethyl, furanylmethyl, C₁-C₆ alkylthio C₁-C₆ alkyl, morpholinyl, mono or di(C₁-C₆ alkyl)amino C₁-C₆ alkyl, di(C₁-C₆ alkyl)amino, C₁-C₆ alkylcarbonyl C₁-C₆ alkyl, C₁-C₆ alkyl substituted with imidazolyl, and a radical of the formula-(C₁-C₆ alkanediyl)-O-CO-Ar₂₇;
   or R₂₄₁ and R₂₄₂ taken together with nitrogen to which they are attached form a pyrrolidinyl, piperidinyl, homopiperidinyl or morpholinyl, which is optionally substituted with C₁-C₆ alkyl or C₁-C₆ alkyloxy;
   Ar₈ is selected from phenyl substituted with one, two or three substituents independently selected from halo, C₁-C₆ alkyl, triflouromethyl, cyano, C₁-C₆ alkyloxy, benzyloxy, C₁-C₆ alkylthio, nitro, amino and mono or di(C₁-C₆ alkyl)amino, and pyridinyl substituted with one, two or three substituents independently selected from halo, C₁-C₆ alkyl, triflouromethyl, hydroxy, cyano, C₁-C₆ alkyloxy, benzyloxy, C₁-C₆ alkylthio, nitro, amino, mono or di(C₁-C₆ alkyl) amino and piperidinyl; and
   Ar₂₇ is selected from phenyl, pyridinyl, and phenyl substituted with one, two or three substituents independently selected from halo, C₁-C₆ alkyl, C₁-C₆ alkyloxy, di(C₁-C₆ alkyl)amino C₁-C₆ alkyl, triflouromethyl and C₁-C₆ alkyl substituted with morpholinyl,
   its stereoisomer, pro-drugs or its pharmaceutically acceptable salt;
(32) A pharmaceutical composition for prophylaxis or therapy of a postoperative stress according to the above (1), wherein the compound having CRF antagonist activating effect is a compound represented by the formula Iy and Iz 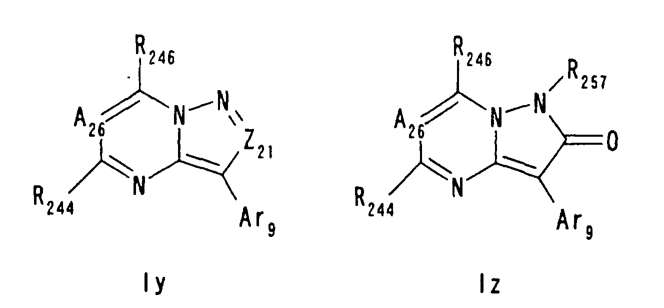 wherein A₂₆ is nitrogen or CR₂₄₇;
   Z₂₁ is nitrogen or CR₂₄₅;
   Ar₉ is selected from phenyl, naphthyl, pyridyl, pyrimidyl, triazinyl, furanyl, thienyl, benzothienyl, benzofuranyl, 2,3-dihydrobenzofuranyl, 2,3-dihydrobenzothienyl, indanyl, 1,2-benzopyranyl, 3,4-dihydro-1,2-benzopyranyl, tetralinyl, each Ar₉ is optionally substituted with 1 to 5 R₂₄₈ groups and each Ar₉ is attached to an unsaturated carbon atom;
   R₂₄₇ is, at each occurrence, independently selected from hydrogen, C₁-C₄ alkyl, C₂-C₄ alkenyl, C₂-C₄ alkynyl, C₃-C₆ cycloalkyl, C₄-C₇ cycloalkylalkyl, halo, cyano, C₁-C₄ haloalkyl;
   R₂₄₄ is, at each occurrence, independently selected from hydrogen, C₁-C₄ alkyl, C₂-C₄ alkenyl, C₂-C₄ alkynyl, halo, cyano, C₁-C₄ haloalkyl, C₁-C₁₂ hydroxyalkyl, C₂-C₁₂ alkoxyalkyl, C₂-C₁₀ cyanoalkyl, C₃-C₆ cycloalkyl, C₄-C₁₀ cycloalkylalkyl, NR₂₅₂R₂₅₃, C₁-C₄ alkyl-NR₂₅₂R₂₅₃, NR₂₅₂COR₂₅₃, OR₂₅₄, SH and S(O)n₃R₂₅₅;
   R₂₄₅ is selected from hydrogen, C₁-C₄ alkyl, C₂-C₄ alkenyl, C₂-C₄ alkynyl, C₃-C₆ cycloalkyl, C₄-C₁₀ cycloalkylalkyl, C₁-C₄ hydroxyalkyl, halo, cyano, -NR₂₄₉R₂₅₀, NR₂₅₂COR₂₅₃, -NR₂₄₉S(O)n₃R₂₅₀, S(O)n₃R₂₄₉R₂₅₀, C₁-C₄ haloalkyl, -OR₂₅₀, SH and -S(O)n₃R₂₅₅;
   R₂₄₆ is selected from hydrogen, OR₂₅₀, SH, S(O)n₃R₂₅₆, COR₂₅₀, CO₂R₂₅₀, OC(O)R₂₅₆, NR₂₅₁COR₂₅₀, N(COR₂₅₀)₂, NR₂₅₁CONR₂₄₉R₂₅₀, NR₂₅₁CO₂R₂₅₆, NR₂₄₉R₂₅₀, NR₂₄₉ₐR₂₅₀ₐ, N(OR₂₅₀)R₂₄₉, CONR₂₄₉R₂₅₀, aryl, heteroaryl and heterocyclyl, or is selected from C₁-C₁₀ alkyl, C₂-C₁₀ alkenyl, C₂-C₁₀ alkynyl, C₃-C₈ cycloalkyl, C₅-C₈ cycloalkenyl, C₄-C₁₂ cycloalkylalkyl or C₆-C₁₀ cycloalkenylalkyl, each of which may be optionally substituted with one to three substituents independently selected from the group consisting of C₁-C₆ alkyl, C₃-C₆ cycloalkyl, halo, C₁-C₄ haloalkyl, cyano, OR₂₅₈, SH, S(O)n₃R₂₅₆, COR₂₅₈, CO₂R₂₅₈, OC(O)R₂₅₆, NR₂₅₁COR₂₅₈, N(COR₂₅₈)₂, NR₂₅₁CONR₂₅₉R₂₅₈, NR₂₅₁CO₂R₂₅₆, NR₂₅₉R₂₅₈, CONR₂₅₉R₂₅₈, aryl, heteroaryl and heterocyclyl;
   R₂₄₈ is, at each occurrence, independently selected at each occurrence from C₁-C₁₀ alkyl, C₂-C₁₀ alkenyl, C₂-C₁₀ alkynyl, C₃-C₆ cycloalkyl, C₄-C₁₂ cycloalkylalkyl, NO₂, halo, cyano, C₁-C₄ haloalkyl, NR₂₄₉R₂₅₀, NR₂₅₁COR₂₅₀, NR₂₅₁CO₂R₂₅₀, COR₂₅₀, OR₂₅₀, CONR₂₄₉R₂₅₀, CO(NOR₂₅₂)R₂₅₀, CO₂R₂₅₀ and S(O)n₃R₂₅₀, wherein each of such C₁-C₁₀ alkyl, C₂-C₁₀ alkenyl, C₂-C₁₀ alkynyl, C₃-C₆ cycloalkyl and C₄-C₁₂ cycloalkylalkyl of the above groups are, at each occurrence, optionally substituted with one to three substituents independently selected from C₁-C₄ alkyl, NO₂, halo, cyano, NR₂₄₉R₂₅₀, NR₂₅₁COR₂₅₀, NR₂₅₁CO₂R₂₅₀, COR₂₅₀, OR₂₅₀, CONR₂₄₉R₂₅₀, CO₂R₂₅₀, CO(NOR₂₅₂)R₂₅₀ and S(O)n₃R₂₅₀;
   R₂₄₉ and R₂₅₀, R₂₄₉ₐ and R₂₅₀ₐ are, at each occurrence, independently selected from hydrogen, C₁-C₁₀ alkyl, C₃-C₁₀ alkenyl, C₃-C₁₀ alkynyl, C₁-C₁₀ haloalkyl with 1 to 10 halogens, C₂-C₈ alkoxyalkyl, C₃-C₆ cycloalkyl, C₄-C₁₂ cycloalkylalkyl, C₅-C₁₀ cycloalkenyl, or C₆-C₁₄ cycloalkenylalkyl, wherein each of the above groups, at each occurrence, may optionally be substituted with one to three substituents independently selected from C₁-C₆ alkyl, C₃-C₆ cycloalkyl, halo, C₁-C₄ haloalkyl, cyano, OR₂₅₈, SH, S(O)n₃R₂₅₆, COR₂₅₈, CO₂R₂₅₈, OC(O)R₂₅₆, NR₂₅₁COR₂₅₈, N(COR₂₅₈)₂, NR₂₅₁CONR₂₅₉R₂₅₈, NR₂₅₁CO₂R₂₅₆, NR₂₅₉R₂₅₈, CONR₂₅₉R₂₅₈, aryl, heteroaryl and heterocyclyl, or R₂₄₉ and R₂₅₀, R₂₄₉ₐ and R₂₅₀ₐ are, at each occurrence, independently selected from aryl, aryl(C₁-C₄ alkyl), heteroaryl, heteroaryl(C₁-C₄ alkyl), heterocyclyl and heterocyclyl(C₁-C₄ alkyl);
   alternatively, NR₂₄₉R₂₅₀ and NR₂₄₉ₐR₂₅₀ₐ are independently piperidine, pyrrolidine, piperazine, N-methylpiperidine, morpholine or thiomorpholine, each optionally substituted with one to three of C₁-C₄ alkyl;
   R₂₅₁ is, at each occurrence, independently selected from hydrogen or C₁-C₄ alkyl;
   R₂₅₂ and R₂₅₃ are, at each occurrence, each independently selected from hydrogen, C₁-C₄ alkyl and C₃-C₆ cycloalkyl;
   R₂₅₄ is selected from hydrogen, C₁-C₄ alkyl, C₁-C₄ haloalkyl and C₃-C₆ cycloalkyl;
   R₂₅₅ is C₁-C₄ alkyl or C₁-C₄ haloalkyl;
   R₂₅₆ is selected from C₁-C₄ alkyl, C₁-C₄ haloalkyl, C₂-C₈ alkoxyalkyl, C₃-C₆ cycloalkyl, C₄-C₁₂ cycloalkylalkyl, aryl, aryl(C₁-C₄ alkyl)-, heteroaryl or heteroaryl(C₁-C₄ alkyl)-;
   R₂₅₇ is, at each occurrence, selected from C₁-C₁₀ alkyl, C₃-C₁₀ alkenyl, C₃-C₁₀ alkynyl, C₃-C₈ cycloalkyl, or C₄-C₁₂ cycloalkylalkyl, each of which is optionally substituted with one to three substituents independently selected from C₁-C₆ alkyl, C₃-C₆ cycloalkyl, halo, C₁-C₄ haloalkyl, cyano, OR₂₅₈, SH, S(O)n₃R₂₅₈, COR₂₅₈, CO₂R₂₅₈, OC(O)R₂₅₈, NR₂₅₁COR₂₅₈, N(COR₂₅₈)₂, NR₂₅₁CONR₂₅₉R₂₅₈, NR₂₅₁CO₂R₂₅₈, NR₂₅₉R₂₅₈, CONR₂₅₉R₂₅₈, and C₁-C₆ alkylthio, C₁-C₆ alkylsulfinyl and C₁-C₆ alkylsulfonyl;
   R₂₅₈ and R₂₅₉ are, at each occurrence, independently selected from hydrogen, C₁-C₆ alkyl, C₃-C₁₀ cycloalkyl and C₄-C₁₆ cycloalkylalkyl, with the proviso that R₂₅₈ of S(O)ₙ₃R₂₅₈ cannot be hydrogen;
   aryl is, at each occurrence, phenyl or naphthyl, each optionally substituted with one to five substituents independently selected from C₁-C₆ alkyl, C₃-C₆ cycloalkyl, halo, C₁-C₄ haloalkyl, cyano, OR₂₅₈, SH, S(O)n₃R₂₅₈, COR₂₅₈, CO₂R₂₅₈, OC(O)R₂₅₈, NR₂₅₁COR₂₅₈, N(COR₂₅₈)₂, NR₂₅₁CONR₂₅₉R₂₅₈, NR₂₅₁CO₂R₂₅₈, NR₂₅₉R₂₅₈ and CONR₂₅₉R₂₅₈;
   heteroaryl is, at each occurrence, pyridyl, pyrimidyl, triazinyl, furanyl, pyranyl, quinolinyl, isoquinolinyl, thienyl, imidazolyl, thiazolyl, indolyl, pyrrolyl, oxazolyl, benzofuranyl, benzothienyl, benzothiazolyl, isoxazolyl, pyrazolyl, 2,3-dihydrobenzothienyl or 2,3-dihydrobenzofuranyl, each being optionally substituted with one to five substituents independently selected from C₁-C₆ alkyl, C₃-C₆ cycloalkyl, halo, C₁-C₄ haloalkyl, cyano, OR₂₅₈, SH, S(O)n₃R₂₅₈, -COR₂₅₈, CO₂R₂₅₈, OC(O)R₂₅₈, NR₂₅₁COR₂₅₈, N(COR₂₅₈)₂, NR₂₅₁CONR₂₅₉R₂₅₈, NR₂₅₁CO₂R₂₅₈, NR₂₅₉R₂₅₈ and CONR₂₅₉R₂₅₈ ;
   heterocyclyl is, at each occurrence, a saturated or partially saturated heteroaryl optionally substituted with one to five substituents independently selected from C₁-C₆ alkyl, C₃-C₆ cycloalkyl, halo, C₁-C₄ haloalkyl, cyano, OR₂₅₈, SH, S(O)n₃R₂₅₈, -COR₂₅₈, CO₂R₂₅₈, OC(O)R₂₅₈, NR₂₅₁COR₂₅₈, N(COR₂₅₈)₂, NR₂₅₁CONR₂₅₉R₂₅₈, NR₂₅₁CO₂R₂₅₈, NR₂₅₉R₂₅₈ and CONR₂₅₉R₂₅₈;
   n3 is, at each occurrence, independently 0, 1 or 2; its isomer, its stereoisomer forms or mixture of its stereoisomer, or its pharmaceutically acceptable salt or its pro-drugs form compound.
(33) A pharmaceutical composition for prophylaxis or therapy of a postoperative stress according to the above (1),
   wherein the compound having CRF antagonist activating effect is a compound represented by the formula Iaa 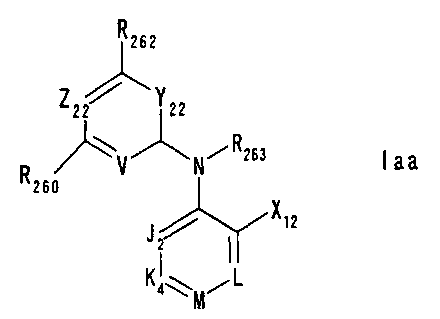 wherein
   Y₂₂ is CR₂₆₂ₐ, N, or CR₂₈₈;
   when Y₂₂ is CR₂₆₂ₐ or N:
   R₂₆₀ is independently, at each occurrence, selected from the group consisting of C₁-C₄ alkyl, C₂-C₄ alkenyl, C₂-C₄ alkynyl, halogen, C₁-C₂ haloalkyl, NR₂₆₅R₂₆₆, OR₂₆₇, and S(O)ₙₙR₂₆₇;
   R₂₆₂ is C₁-C₄ alkyl, aryl, C₃-C₆ cycloalkyl, C₁-C₂ haloalkyl, halogen, nitro, NR₂₆₅R₂₆₆, OR₂₆₇, S(O)ₙₙR₂₆₇, C(=O)R₂₆₈, C (=O)NR₂₆₅R₂₆₆, C(=S)NR₂₆₅R₂₆₆, -(CHR₂₇₅)ₖₙNR₂₆₅R₂₆₆, (CH₂)ₖₙOR₂₆₇, C(=O)NR₂₆₉CH(R₂₇₀)CO₂R₂₇₁, -C(OH)(R₂₈₄)(R₂₈₄ₐ), - (CH₂)ₚₙS(O)ₙₙ-alkyl, -(CHR₂₇₅)R₂₈₄, -C(CN)(R₂₈₄)(R₂₇₅) when R₂₈₄ is not -NH- containing ring groups, -C(=O)R₂₈₄,-CH(CO₂R₂₇₅)₂, NR₂₆₉C(=O)CH( R₂₇₀)NR₂₆₉R₂₇₁, NR₂₆₉CH( R₂₇₀)CO₂R₂₇₁; substituted C₁-C₄ alkyl, substituted C₂-C₄ alkenyl, substituted C₂-C₄ alkynyl, substituted C₁-C₄ alkoxy, aryl-(substituted C₁-C₄) alkyl, aryl-(substituted C₁-C₄)alkoxy, substituted C₃-C₆ cycloalkyl, amino-(substituted C₁-C₄)alkyl, substituted C₁-C₄ alkylamino, wherein any substituent containing carbon can be substituted with R₂₈₆;
   2-pyridinyl, imidazolyl, 3-pyridinyl, 4-pyridinyl, 2-methyl-3-pyridinyl, 4-methyl-3-pyridinyl, furanyl, 5-methyl-2-furanyl, 2,5-dimethyl-3-furanyl, 2-thienyl, 3-thienyl, 5-methyl-2-thienyl, 2-pheno-thiazinyl, 4-pyrazinyl, azetidinyl, phenyl, 1H-indazolyl, 2-pyrrolidonyl, 2H,6H-1,5,2-dithiazinyl, 2H-pyrrolyl, 3H-indolyl, 4-piperidonyl, 4aH-carbazolyl, 4H-quinolizinyl, 6H-1,2,5-thiadiazinyl, acridinyl, azocinyl, azepinyl, benzofuranyl, benzothiophenyl, carbazolyl, chromanyl, chromenyl, cinnolinyl, decahydroquinolinyl, furazanyl, imidazolidinyl, indolinyl, indolizinyl, indolyl, isobenzofuranyl, isochromanyl, isoindolinyl, isoindolyl, isoquinolinyl, benzimidazolyl, isothiazolyl, isoxazolyl, morpholinyl, naphthyridinyl, octahydroisoquinolinyl, oxazolidinyl, oxazolyl, phenanthridinyl, phenanthrolinyl, phenazinyl, phenoxathiinyl, phenoxazinyl, phthalazinyl, piperazinyl, piperidinyl, pteridinyl, purinyl, pyranyl, pyrazolidinyl, pyrazolinyl, pyrazolyl, pyridazinyl, pyrimidinyl, pyrrolidinyl, pyrrolinyl, pyrrolyl, quinazolinyl, quinolinyl, quinoxalinyl, quinuclidinyl, β-carbolinyl, tetrahydrofuranyl, tetrahydroisoquinolinyl, tetrahydroquinolinyl, tetrazolyl, thianthrenyl, thiazolyl, thiophenyl, triazinyl, xanthenyl; or 1-tetrahydroquinolinyl or 2-tetrahydroisoquinolinyl either of which can be substituted with 0 to 3 groups chosen from keto and C₁-C₄ alkyl;
   J₂, K₄ and L are, at each occurrence, independently selected from the group consisting of N, CH, and CX₁₂₁;
   M is CR₂₆₄ or N;
   V is CR₂₆₀ₐ or N;
   Z₂₂ is CR₂₆₁ or N;
   R₂₆₀, R₂₆₁, and R₂₆₂ₐ are, at each occurrence, independently selected from the group consisting of hydrogen, halo, halomethyl, C₁-C₃ alkyl, and cyano;
   R₂₆₃ is (CH₂)ₘₙOR₂₇₅, C₁-C₄ alkyl, allyl, propargyl, (CH₂)ₘₙR₂₇₂, or -(CH₂)ₙₘOC(O)R₂₇₅;
   X₁₂ is halogen, aryl, heteroaryl, S(O)_{ZN}R₂₆₇, SR₂₆₇, halomethyl, -(CH₂)ₚₙOR₂₆₇, cyano, -(CHR₂₇₅)ₚₙNR₂₇₃R₂₇₄, -C(=O)R₂₆₇, C₁-C₆ alkyl, C₄-C₁₀ cycloalkylalkyl, C₁-C₁₀ alkenyl, C₂-C₁₀ alkynyl, C₁-C₁₀ alkoxy, aryl-(C₂-C₁₀)-alkyl, C₃-C₆ cycloalkyl, aryl-(C₁-C₁₀)-alkoxy, nitro, thio-(C₁-C₁₀)-alkyl, -C(=NOR₂₇₅)- C₁-C₄-alkyl, -C(=NOR₂₇₅)H, or -C(=O)NR₂₇₃R₂₇₄, wherein any carbon containing substituents can be substituted with R₂₇₇;
   X₁₂₁ is, at each occurrence, independently selected from the group consisting of hydrogen, halogen, aryl, heteroaryl, S(O)ₙₙR₂₆₇, halomethyl, - (CHR₂₇₅)ₚₙOR₂₆₇, cyano, - (CHR₂₇₅)ₚₙNR₂₇₃R₂₇₄, C(=O)R₂₆₇, C₁-C₆ alkyl, C₂-C₁₀ alkenyl, C₂-C₁₀ alkynyl, C₁-C₁₀ alkoxy, aryl-(C₁-C₁₀)-alkyl, C₃-C₆ cycloalkyl, aryl-(C₁-C₁₀)-alkoxy, nitro, thio-(C₁-C₁₀)-alkyl, -C(=NOR₂₇₅)-C₁-C₄-alkyl, -C(=NOR ₂₇₅)H, and -C(=O)NR₂₇₃R₂₇₄, wherein any carbon containing substituents can be substituted by R₂₇₇;
   R₂₆₄ is halo, -C(=NOR₂₇₅)-C₁-C₄-alkyl, C₁-C₆ alkyl, C₁-C₃ haloalkyl, -(CHR₂₇₅)ₚₙOR₂₆₇, - (CHR₂₇₅)ₚₙS(O)ₙₙR₂₆₇, -(CHR₂₇₅)ₚₙNR₂₇₃R₂₇₄, C₃-C₆ cycloalkyl, C₂-C₁₀ alkenyl, C₂-C₁₀ alkynyl, aryl-(C₂-C₁₀)-alkyl, aryl-(C₁-C₁₀)-alkoxy, cyano, C₃-C₆ cycloalkoxy, nitro, amino-(C₂-C₁₀)-alkyl, thio-(C₂-C₁₀)-alkyl, SOₙₙ(R₂₆₇), C(=O)R₂₆₇, -C(=NOR₂₇₅)H, or C(=O)NR₂₇₃R₂₇₄, wherein any carbon containing substituents can be substituted by R₂₇₇;
   R₂₆₅ and R₂₆₆ are, at each occurrence, independently selected from the group consisting of hydrogen, C₁-C₆ alkyl, C₃-C₁₀ cycloalkyl, C₁-C₆ alkoxy, (C₄-C₁₂)-cycloalkylalkyl, - (CH₂)ₖₙR₂₇₂, (CHR₂₇₅)ₚₙOR₂₆₇, -(C₁-C₆ alkyl)-aryl, heteroaryl, -S(O)_{zn}-aryl or -(C₁-C₆ alkyl)-heteroaryl and aryl, wherein said aryl or heteroaryl groups are optionally substituted with 1-3 groups selected from the group consisting of hydrogen, halogen, C₁-C₆ alkyl, C₁-C₆ alkoxy, amino, NHC(=O)(C₁-C₆ alkyl), NH(C₁-C₆ alkyl), N(C₁-C₆ alkyl)₂, nitro, carboxy, CO₂(C₁-C₆ alkyl), cyano, and S(O)_{zn}-(C₁-C₆-alkyl); or can be taken together to form -(CH₂)_{qn}A₂₇(CH₂)ᵣₙ- which is optionally substituted with 0 to 3 R₂₇₆; or, when considered with the commonly attached nitrogen, can be taken together to form a heterocycle, said heterocycle being substituted on carbon with 1 to 3 groups consisting of hydrogen, C₁-C₆ alkyl, hydroxy, or C₁-C₆ alkoxy;
   A₂₇ is CH₂, O, NR₂₈₄, C(=O),S(O)ₙₙ, N(C(=O)R₂₇₆), N(R₂₇₈),C(H)(NR₂₇₃R₂₇₄), C(H)(OR₂₇₉), C(H)(C(=O)R₂₈₀), or N(S(O)ₙₙR₂₈₀);
   R₂₆₇ is, at each occurrence, independently selected from the group consisting of hydrogen; C₁-C₆ alkyl, -(C₄-C₁₂) cycloalkylalkyl, (CH₂)ₜₙR₂₈₁, C₃-C₁₀ cycloalkyl, -NR₂₆₅R₂₆₆, aryl, heteroaryl, -NR₂₇₅(CH₂)ₙₙNR₂₆₅R₂₆₆, -(CH₂)ₖₙR₂₈₄, and (CH₂)ₜₙheteroaryl or (CH₂)ₜₙaryl, either of which can optionally be substituted with 1 to 3 groups selected from the group consisting of hydrogen, halogen, C₁-C₆ alkyl, C₁-C₆ alkoxy, amino, NHC(=O)(C₁-C₆ alkyl), NH(C₁-C₆ alkyl), N(C₁-C₆alkyl)₂, nitro, carboxy, CO₂(C₁-C₆ alkyl), cyano, or S(O)_{zn}(C₁-C₆ -alkyl;
   R₂₆₈ is, at each occurrence, independently selected from R₂₆₉, hydroxy, C₁-C₄ alkoxy, C₃-C₆ cycloalkyl, C₂-C₄ alkenyl, aryl substituted with 0 to 3 R₂₇₇ and -(C₁-C₆ alkyl)-aryl substituted with 0 to 3 R₂₇₇;
   R₂₆₉, R₂₇₅, R₂₈₂ and R₂₈₃ are, at each occurrence, independently selected from hydrogen or C₁-C₄ alkyl;
   R₂₇₀ is C₁-C₄ alkyl substituted with 0 to 3 groups chosen from the group consisting of keto, amino, sulfhydryl, hydroxy, guanidinyl, p-hydroxyphenyl, imidazolyl, phenyl, indolyl, and indolinyl, or (CH₂)ₜₙ when taken together with an adjacent R₂₆₉;
   R₂₇₁ is hydrogen or an appropriate amine protecting group for nitrogen or an appropriate carboxylic acid protecting group for carboxyl;
   R₂₇₂ is, at each occurrence, independently selected from the group consisting of CN, OR₂₇₈, SR₂₇₈, and C₃-C₆ cycloalkyl;
   R₂₇₃ and R₂₇₄ are, at each occurrence, independently selected from the group consisting of hydrogen, C₄-C₁₀ cycloalkyl-alkyl, and R₂₇₈;
   R₂₇₆ is, at each occurrence, independently selected from the group consisting of R₂₆₉, C₁-C₄ alkoxy, halo, OR₂₈₂, SR₂₈₂, NR₂₈₂R₂₈₃, and (C₁-C₆) alkyl (C₁-C₄)alkoxy,
   R₂₇₇ is, at each occurrence, independently selected from the group consisting of R₂₆₉, hydroxy, halogen, C₁-C₂ haloalkyl, C₁-C₄ alkoxy, C(=O)R₂₈₃, and cyano;
   R₂₇₈ is, at each occurrence, independently selected from the group consisting of C₁-C₆ alkyl, C₃-C₆ cycloalkyl, (CH₂)_{wn}R₂₈₁, and aryl substituted with 0 to 3 R₂₇₇;
   R₂₇₉ is, at each occurrence, independently selected from the group consisting of R₂₆₉, C(=O)R₂₉₀, and C₂-C₄ alkenyl;
   R₂₈₀ is, at each occurrence, independently selected from the group consisting of R₂₆₉, C₁-C₄ alkoxy, NR₂₈₂R₂₈₃, and hydroxyl;
   R₂₈₁ is, at each occurrence, independently selected from the group consisting of cyano, OR₂₈₃, SR₂₈₃, SR₂₈₂R₂₈₃, C₁-C₆ alkyl, C₃-C₆ cycloalkyl, -S(O)ₙₙR₂₉₀, and -C(=O)R₂₈₄;
   R₂₈₄, which can be optionally substituted with 0 to 3 R₂₇₆, is independently selected, at each occurrence, from the group consisting of phenyl, pyrazolyl, imidazolyl, 2-methyl-3-pyridinyl, 4-methyl-3-pyridinyl, furanyl, 5-methyl-2-furanyl, 2,5-dimethyl-3-furanyl, 2-thienyl, 3-thienyl, 5-methyl-2-thienyl, 2-pheno-thiazinyl, 4-pyrazinyl, azetidinyl, 1H-indazolyl, 2-pyrrolidonyl, 2H,6H-1,5,2-dithiazinyl, 2H-pyrrolyl, 3H-indolyl, 4-piperidonyl, 4aH-carbazolyl, 4H-quinolizinyl, 6H-1,2,5-thiadiazinyl, acridinyl, azocinyl, azepinyl, benzofuranyl, benzothiophenyl, carbazolyl, chromanyl, chromenyl, cinnolinyl, decahydroquinolinyl, furazanyl, indolinyl, indolizinyl, indolyl, isobenzofuranyl, isochromanyl, isoindolinyl, isoindolyl, isoquinolinyl, benzimidazolyl, isothiazolyl, isoxazolyl, morpholinyl, naphthyridinyl, octahydroisoquinolinyl, oxazolidinyl, oxazolyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazolidinyl, pyridazinyl, pyridyl, pyrimidinyl, pyrrolidinyl, pyrrolinyl, pyrrolyl, quinazolinyl, quinolinyl, quinoxalinyl, quinuclidinyl, β-carbolinyl, tetrahydrofuranyl, tetrazolyl, thianthrenyl, thiazolyl, thiophenyl, triazinyl, xanthenyl; and 1-tetrahydroquinolinyl or 2-tetrahydroisoquinolinyl either of which can be substituted with 0 to 3 groups chosen from keto and C₁-C₄ alkyl;
   R₂₈₄ₐ, which can be optionally substituted with 0 to 3 R₂₇₆, is independently selected, at each occurrence, from the group consisting of H and R₂₈₄,
   R₂₈₆ is, at each occurrence, independently selected from the group consisting of C₁-C₃ alkyl, C₂-C₄ alkenyl, C₂-C₄ alkynyl, C₂-C₄ alkoxy, aryl, nitro, cyano, halogen, aryloxy, and heterocycle optionally linked through oxygen;
   R₂₉₀ is, at each occurrence, independently selected from the group consisting of C₁-C₄ alkyl, C₃-C₇ cycloalkyl, C₄-C₁₀ cycloalkylalkyl, and aryl-(C₁-C₄)alkyl;
   kn, mn, and rn are, at each occurrence, independently selected from 1 to 4;
   nn is, at each occurrence, independently selected from 0 to 2;
   pn, qn, and zn are, at each occurrence, independently selected from 0 to 3;
   tn and wn are, at each occurrence, independently selected from 1 to 6, with the proviso that when J₂ is CX₁₂₁ and K₄ and L are both CH, and M is CR₂₆₄, then
      (A) when V and Y₂₂ are N, Z₂₂ is CH and R₂₆₀ and R₂₆₂ are methyl,
         (1) and when R₂₆₃ is methyl, then
            (a) R₂₆₄ can not be methyl when X₁₂ is OH and X₁₂₁ is H;
            (b) R₂₆₄ can not be -NHCH₃-, or -N(CH₃)₂ when X₁₂ and X₁₂₁ are -OCH₃; and
            (c) R₂₆₄ can not be -N(CH₃)₂, when X₁₂ and X₁₂₁ are -OCH₂CH₃;
         (2) and when R₂₆₃ is ethyl, then
            (a) then R₂₆₄ can not be methylamine when X₁₂ and X₁₂₁ are -OCH₃;
            (b) R₂₆₄ can not be OH when X₁₂ is Br and X₁₂₁ is OH; and
            (c) R₂₆₄ can not be -CH₂OH or -CH₂N(CH₃)₂, when X₁₂ is -SCH₃ and X₁₂₁ is H;
      (B) when V and Y₂₂ are N, Z₂₂ is CH, R₂₆₃ is ethyl, R₂₆₄ is isopropyl, X₁₂ is Br, X₁₂₁ is H, and
         (1) when R₂₆₀ is CH₃, then
            (a) R₂₆₂ can not be OH, piperazin-1-yl, -CH₂-piperidin-1-yl, -CH₂-(N-4-methylpiperazin-1-yl), -C(O)NH-phenyl, -CO₂H, -CH₂O-(4-pyridyl), -C(O)NH₂, 2-indolyl, -CH₂O-(4-carboxyphenyl), -N(CH₂CH₃)(2-bromo-4-isopropylphenyl);
         (2) when R₂₆₀ is -CH₂CH₂CH₃, then R₂₆₂ can not be -CH₂CH₂CH₃;
      (C) when V, Y₂₂ and Z₂₂ are N, R₂₆₃ is ethyl, and
         (1) R₂₆₄ is isopropyl, X₁₂ is bromo, and X₁₂₁ is H, and
            (a) R₂₆₂ can not be OH or -OCH₂CN when R₂₆₀ is CH₃; and
            (b) R₂₆₂ can not be -N(CH₃)₂ when R₂₆₀ is -N(CH₃)₂;
         (2) R₂₆₄ is -OCH₃, X₁₂ is -OCH₃, and X₁₂₁ is H, then R₂₆₂ and R₂₆₀ can not both be chloro;
            further provided that when J₂, K₄, and L are all CH and M is CR₂₆₄, then
      (D) at least one of V, Y₂₂, and Z₂₂ must be N;
      (E) when V is CR₂₆₀ₐ, Z₂₂ and Y₂₂ can not both be N;
      (F) when Y₂₂ is CR₂₆₂ₐ, Z₂₂ and V can not both be N;
      (G) when Z₂₂ is CR₂₆₁, V and Y₂₂ must both be N;
      (H) Z₂₂ can be N only when both V and Y₂₂ are N or when V is CR₂₆₀ₐ and Y₂₂ is CR₂₆₂ₐ,
      (I) when V and Y₂₂ are N, Z₂₂ is CR₂₆₁, and R₂₆₁ is H or C₁-C₃ alkyl, and R₂₆₃ is C₁-C₃ alkyl, then R₂₆₂ can not be 2-pyridinyl, indolyl, indolinyl, imidazolyl, 3-pyridinyl, 4-pyridinyl, 2-methyl-3-pyridinyl, 4-methyl-3-pyridinyl, furanyl, 5-methyl-2-furanyl, 2,5-dimethyl-3-furanyl, 2-thienyl, 3-thienyl, 5-methl-2-thienyl, 2-phenothiazinyl, or 4-pyrazinyl;
      (J) when V and Y₂₂ are N, Z₂₂ is CR₂₆₁, R₂₆₁ is H or C₁-C₃ alkyl, R₂₆₃ is C₁-C₄ alkyl, R₂₆₄, X₁₂ and/or X₁₂₁ are OH, halo, CF₃, C₁-C₄ alkyl, C₁-C₄ alkoxy, C₁-C₄ alkylthio, cyano, amino, carbarmoyl, or C₁-C₄ alkanoyl, and when R₂₆₀ is C₁-C₄ alkyl, then R₂₆₂ can not be -NH(substituted phenyl) or -N(C₁-C₄ alkyl)(substituted phenyl);
   and in the formula Iaa, when Y₂₂ is CR₂₈₈:
   J₂, K₄, L, M, Z₂₂, A₂₇, kn, mn, nn, pn, qn, rn, tn, wn, R₂₆₂, R₂₆₉, R₂₇₀, R₂₇₁, R₂₇₂, R₂₇₅, R₂₇₇, R₂₇₈, R₂₈₀, R₂₈₂, R₂₈₃, R₂₈₄, and R₂₈₆ are as defined above, then R₂₈₄ₐ, in addition to being as defined above, can also be C₁-C₄ alkyl, with the proviso that V is N;
   R₂₆₀ is C₁-C₂ alkyl, C₂-C₄ alkenyl, C₂-C₄ alkynyl, C₂-C₄ alkoxy, halogen, amino, methylamino, dimethylamino, aminomethyl, or N-methylaminomethyl;
   R₂₆₁ is, at each occurrence, independently selected from the group consisting of hydrogen, halo, C₁-C₃ alkyl, nitro, amino, and -CO₂R₂₆₉;
   R₂₆₃ is taken together with R₂₈₈ to form a 5-membered ring and is -C(R₂₈₇) = or -N= when R₂₈₈ is -C(R₂₈₉) or -N=, or -CH(R₂₈₇) when R₂₈₈ is -CH(R₂₈₉);
   X₁₂ is Cl, Br, I, S(O)ₙₙR₂₆₇, OR₂₆₇, halomethyl, - (CHR₂₇₅)ₚₙOR₂₆₇, cyano, -(CHR₂₇₅)ₚₙNR₂₇₃R₂₇₄, C(=O)R₂₆₇, C₁-C₆ alkyl, C₂-C₁₀ alkenyl, C₂-C₁₀ alkynyl, C₁-C₁₀ alkoxy, aryl-(C₁-C₁₀)-alkyl, C₃-C₆ cycloalkyl, aryl-(C₁-C₁₀)-alkoxy, nitro, thio-(C₁-C₁₀)-alkyl, -C(=NOR₂₇₅)-C₁-C₄-alkyl, -(=NOR₂₇₅)H, or C(=O)NR₂₇₃R₂₇₄, where substitution by R₂₇₇ can occur on any carbon containing substituents;
   X₁₂₁ is hydrogen, Cl, Br, I, S(O)ₙₙR₂₆₇, -(CHR₂₇₅)ₚₙOR₂₆₇, halomethyl, cyano, -(CHR₂₇₅)ₚₙNR₂₇₃R₂₇₄, C(=O)R₂₆₇, C₁-C₆ alkyl, C₂-C₁₀ alkenyl, C₂-C₁₀ alkynyl, C₁-C₁₀ alkoxy, aryl-(C₁-C₁₀)-alkyl, C₃-C₆ cycloalkyl, aryl-(C₂-C₁₀)-alkoxy, nitro, thio-(C₂-C₁₀)-alkyl, -C(=NOR ₂₇₅)-C₁-C₄-alkyl, -C(=NOR₂₇₅)H, or C(=O)NR₂₆₇R₂₇₄, where substitution by R₂₇₇ can occur on any carbon containing substituents;
   R₂₆₄ is halo, -C(=NOR₂₇₅)-C₁-C₄ alkyl, C₁-C₆ alkyl, C₁-C₃ haloalkyl, C₁-C₆ alkoxy, (CHR₂₇₅)ₚₙOR₂₆₇, (CHR₂₇₅)ₚₙS(O)ₙₙR₂₆₇, (CHR₂₇₅)ₚₙNR₂₇₃R₂₇₄, C₃-C₆ cycloalkyl, C₂-C₁₀ alkenyl, C₂-C₁₀ alkynyl, aryl-(C₂-C₁₀)-alkyl, aryl-(C₁-C₁₀)-alkoxy, cyano, C₃-C₆ cycloalkoxy, nitro, amino-(C₁-C₁₀)-alkyl, thio-(C₁-C₁₀)-alkyl, SOₙₙ(R₂₆₇), C(=O)R₂₆₇, -C(=NOR₂₇₅)H, or C(=O)NR₂₆₇R₂₇₄, wherein substitution by R₂₇₇ can occur on any carbon containing substituents;
   R₂₆₅ and R₂₆₆ are, at each occurrence, independently selected from the group consisting of hydrogen, C₁-C₆ alkyl, C₃-C₁₀ cycloalkyl, -(CH₂)ₖₙR₂₇₂, (C₄-C₁₂)-cycloalkylalkyl, C₁-C₆ alkoxy, -(C₁-C₆ alkyl)-aryl, heteroaryl, aryl, -S(O)_{zn}-aryl or -(C₁-C₆ alkyl)-heteroaryl and aryl, wherein the aryl or heteroaryl groups are optionally substituted with 1 to 3 substituents selected from hydrogen, halogen, C₁-C₆ alkyl, C₁-C₆ alkoxy, amino, NHC(=O)(C₁-C₆ alkyl), NH(C₁-C₆ alkyl), N(C₁-C₆ alkyl)₂, nitro, carboxy, CO₂(C₁-C₆ alkyl), and cyano; or can be taken together to form -(CH₂)_{qn}A₂₇(CH₂)ᵣₙ-, optionally substituted with 0 to 3 R₂₇₆; or, when considered with the commonly attached nitrogen, can be taken together to form a heterocycle, said heterocycle being substituted on carbon with 1 to 3 groups consisting of hydrogen, C₁-C₆ alkyl, hydroxy, or C₁-C₆ alkoxy;
   R₂₆₇ is, at each occurrence, independently selected from the group consisting of hydrogen, C₁-C₆ alkyl, -(C₄-C₁₂) cycloalkylalkyl, (CH₂)ₜₙR₂₈₁, C₃-C₁₀ cycloalkyl, -(C₁-C₆ alkyl)-aryl, heteroaryl, -NR₂₇₅, -N(CH₂)ₙₙNR₂₆₅R₂₆₆, -(CH₂)ₖₙR₂₈₄, (C₁-C₆ alkyl)-heteroaryl or aryl optionally substituted with 1-3 groups selected from hydrogen, halogen, C₁-C₆ alkyl, C₁-C₆ alkoxy, amino, NHC(=O)(C₁-C₆ alkyl), NH(C₁-C₆ alkyl), N(C₁-C₆ alkyl)₂, nitro, carboxy, CO₂(C₁-C₆ alkyl), and cyano;
   R₂₆₈ is, at each occurrence, independently selected from R₂₆₉, hydroxy, C₁-C₄ alkoxy, C₃-C₆ cycloalkyl, C₂-C₄ alkenyl, and aryl substituted with 0 to 3 R₂₇₇;
   R₂₇₃ and R₂₇₄ are, at each occurrence, independently selected from the group consisting of hydrogen, C₁-C₆ alkyl, C₃-C₆ cycloalkyl, (CH₂)ₜₙR₂₈₁, and aryl substituted with 0 to 3 R₂₇₇;
   R₂₇₆ is, at each occurrence, independently selected at each occurrence from the group consisting of R₂₆₉, C₁-C₄ alkoxy, halo, OR₂₈₂, SR ₂₈₂, and NR₂₈₂R₂₈₃;
   R₂₇₉ is, at each occurrence, independently selected from the group consisting of R₂₆₉ and C(=O)R₂₉₀;
   R₂₈₁ is, at each occurrence, independently selected from the group consisting of OR₂₈₃, SR₂₈₃, NR₂₈₂R₂₈₃, C₃-C₆ cycloalkyl, -S(O)ₙₙR₂₉₀, and -C(=O)R₂₈₄;
   R₂₈₅ is hydrogen or halogen;
   R₂₈₇ is C₁-C₂ alkyl, C₂-C₄ alkenyl, C₂-C₄ alkynyl, hydrogen, C₁-C₂ alkoxy, halogen, or C₂-C₄ alkylamino;
   R₂₈₈ is taken together with R₂₆₃ to form a five membered ring and is:
      - CH(R₂₈₉)- when R₂₆₃ is -CH(R₂₈₇)-,
      - C(R₂₈₉)- when R₂₆₃ is -C(R₂₈₇)= or -N=;
   R₂₆₉ is hydrogen, cyano, C₁-C₂ alkyl, C₁-C₂ alkoxy, halogen, C₁-C₂ alkenyl, nitro, amido, carboxy, or amino;
   R₂₉₀ is C₁-C₄ alkyl, C₃-C₇ cycloalkyl, or aryl-(C₁-C₄) alkyl, with the proviso that when J₂, K₄, and L are all CH, M is CR₂₆₄, Z₂₂ is CH, R₂₆₂ is CH₃,
   R₂₈₇ is H, R₂₆₄ is isopropyl, X₁₂ is Br, X₁₂₁ is H, and R₂₆₀ is CH₃ then R₂₈₉ can not be H, -CO₂H, or -CH₂NH₂;
      and further provided that when J₂, K₄ and L are all CH; M is CR₂₆₄; Z₂₂ is N; and
      (A) R₂₈₈ is -C(R₂₈₉), then one of R₂₈₇ or R₂₈₉ is hydrogen;
      (B) R₂₈₈ is N, then R₂₆₂ is not halo, NH₂, NO₂, CF₃, CO₂H, CO₂-alkyl, alkyl, acyl, alkoxy, OH, or -(CH₂)ₘₙO alkyl;
      (C) R₂₈₈ is N, X₁₂ or X₁₂₁ are bromo or methyl, and R₂₆₄ is nitro, then R₂₈₇ is not methyl; or
      (D) R₂₈₈ is N, R₂₆₀ is CH₃ and R₂₆₂ is amino, then R₂₆₄ is not halogen or methyl,
         its pharmaceutically acceptable salt or pro-drugs;
(34) A pharmaceutical composition for prophylaxis or therapy of a postoperative stress according to the above (1), wherein the compound having CRF antagonist activating effect is a compound represented by the formula Iab or Iac 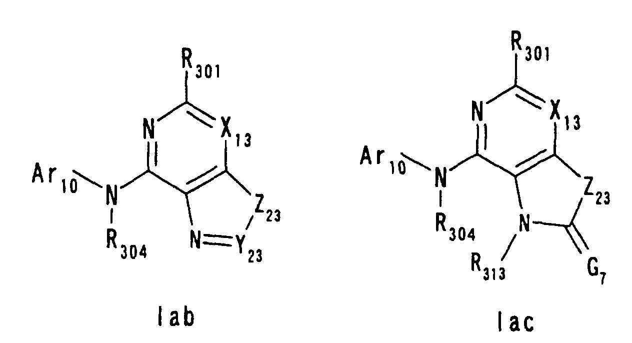 wherein
   X₁₃ is N or CR₃₀₁;
   Y₂₃ is N or CR₃₀₂;
   Z₂₃ is NR₃₀₃, O, or S(O)n₄;
   G₇ is O or S;
   Ar₁₀ is phenyl, naphthyl, pyridyl, pyrimidinyl, triazinyl, furanyl, quinolinyl, isoquinolinyl, thienyl, imidazolyl, thiazolyl, indolyl, pyrrolyl, oxazolyl, benzofuranyl, benzothienyl, benzthiazolyl, isoxazolyl or pyrazolyl, each optionally substituted with 1 to 5 R₃₀₅ groups;
   R₃₀₁ is, at each occurrence, independently H, C₁-C₄ alkyl, C₂-C₄ alkenyl, C₂-C₄ alkynyl, halo, CN, C₁-C₄ haloalkyl, -NR₃₀₉R₃₁₀, NR₃₀₉COR₃₁₀, -OR₃₁₁, SH or -S(O)n₄R₃₁₂;
   R₃₀₂ is H, C₁-C₄ alkyl, C₁-C₆ cycloalkyl, halo, CN, -NR₃₀₆R₃₀₇, NR₃₀₉COR₃₁₀, C₁-C₄ haloalkyl, -OR₃₀₇, SH or -S(O) n₄R₃₁₂;
   R₃₀₃ is H, C₁-C₁₀ alkyl, C₂-C₁₀ alkenyl, C₂-C₁₀ alkynyl, C₃-C₈ cycloalkyl or C₄-C₁₂ cycloalkylalkyl, each optionally substituted with 1 to 3 substituents independently selected at each occurrence from C₁-C₆ alkyl, C₃-C₆ cycloalkyl, halo, C₁-C₄ haloalkyl, cyano, -OR₃₀₇, SH, -S(O)ₙ₄R₃₁₃, -COR₃₀₇, -CO₂R₃₀₇, -OC(O)R₃₁₃, -NR₃₀₈COR₃₀₇, -N(COR₃₀₇)₂, -NR₃₀₈CONR₃₀₆R₃₀₇, -NR₃₀₈CO₂R₃₁₃, -NR₃₀₆R₃₀₇, -CONR₃₀₆R₃₀₇, aryl², heteroaryl² and heterocyclyl², where said aryl², heteroaryl² or heterocyclyl² is optionally substituted with 1 to 3 substituents independently selected at each occurrence from a group consisting of C₁-C₆ alkyl, C₃-C₆ cycloalkyl, halo, C₁-C₄ haloalkyl, cyano, -OR₃₀₇, SH, -S(O)ₙ₄R₃₁₃, -COR₃₀₇, -CO₂R₃₀₇, -OC(O)R₃₁₃, -NR₃₀₈COR₃₀₇, -N(COR₃₀₇)₂, -NR₃₀₈CONR₃₀₆R₃₀₇, -NR₃₀₈CO₂R₃₁₃, -NR₃₀₆R₃₀₇ and -CONR₃₀₆R₃₀₇;
   R₃₀₄ is H, C₁-C₄ alkyl, allyl, or propargyl, wherein C₁-C₄ alkyl, allyl, or propargyl is optionally substituted with C₃-C₆ cycloalkyl and, wherein C₁-C₄ alkyl is optionally substituted with -OR₃₀₇, -S(O)ₙ₄R₃₁₂ or -CO₂R₃₀₇;
   R₃₀₅ is, at each occurrence, independently C₁-C₁₀ alkyl, C₂-C₁₀ alkenyl, C₂-C₁₀ alkynyl, C₃-C₈ cycloalkyl, C₄-C₁₂ cycloalkylalkyl, -NO₂, halo, -CN, C₁-C₄ haloalkyl, -NR₃₀₆R₃₀₇, -NR₃₀₈COR₃₀₇, -NR₃₀₈CO₂R₃₀₇, -COR₃₀₇, -OR₃₀₇, -CONR₃₀₆R₃₀₇, -CO(NOR₃₀₉)R₃₀₇, CO₂R₃₀₇, or -S(O)ₙ₄R₃₀₇, where C₁-C₁₀ alkyl, C₂-C₁₀ alkenyl, C₂-C₁₀ alkynyl, C₃-C₆ cycloalkyl and C₄-C₁₂ cycloalkylalkyl are optionally substituted with 1 to 3 substituents optionally and independently selected from C₁-C₄ alkyl, nitro, halo, -CN, -NR₃₀₆R₃₀₇, NR₃₀₈COR₃₀₇, NR₃₀₈CO₂R₃₀₇, -COR₃₀₇, -OR₃₀₇, -CONR₃₀₆R₃₀₇, CO₂R₃₀₇, -CO(NOR₃₀₉)R₃₀₇, or -S(O)ₙ₄R₃₀₇;
   R₃₀₆ and R₃₀₇ are, at each occurrence, independently H, C₁-C₄ alkyl, C₁-C₄ haloalkyl, C₂-C₈ alkoxyalkyl, C₃-C₆ cycloalkyl, C₄-C₁₂ cycloalkylalkyl, aryl², aryl²(C₁-C₄ alkyl)-, heteroaryl² or heteroaryl²(C₁-C₄ alkyl)-; or NR₃₀₆R₃₀₇ is piperidine, pyrrolidine, piperazine, N-methylpiperazine, morpholine or thiomorpholine;
   R₃₀₈ is, at each occurrence, independently H or C₁-C₄ alkyl;
   R₃₀₉ and R₃₁₀ are, at each occurrence, independently selected from H, C₁-C₄ alkyl, or C₃-C₆ cycloalkyl;
   R₃₁₁ is H, C₁-C₄ alkyl, C₁-C₄ haloalkyl, or C₃-C₆ cycloalkyl;
   R₃₁₂ is C₁-C₄ alkyl or C₁-C₄ haloalkyl;
   R₃₁₃ is C₁-C₄ alkyl, C₁-C₄ haloalkyl, C₂-C₈ alkoxyalkyl, C₃-C₆ cycloalkyl, C₄-C₁₂ cycloalkylalkyl, aryl², aryl²(C₁-C₄ alkyl)-, heteroaryl² or heteroaryl²(C₁-C₄ alkyl)-;
   Aryl² is phenyl or naphthyl, each optionally substituted with 1 to 3 substituents optionally and independently selected from C₁-C₆ alkyl, C₃-C₆ cycloalkyl, halo, C₁-C₄ haloalkyl, cyano, -OR₃₀₇, SH, -S(O)ₙ₄R₃₁₃, -COR₃₀₇, -CO₂R₃₀₇, -OC(O)R₃₁₃, -NR₃₀₈COR₃₀₇, -N(COR₃₀₇)₂, -NR₃₀₈CONR₃₀₆R₃₀₇, -NR₃₀₈CO₂R₃₁₃, -NR₃₀₆R₃₀₇, and -CONR₃₀₆R₃₀₇;
   heteroaryl² is pyridyl, pyrimidinyl, triazinyl, furanyl, quinolinyl, isoquinolinyl, thienyl, imidazolyl, thiazolyl, indolyl, pyrrolyl, oxazolyl, benzofuranyl, benzothienyl, benzthiazolyl, isoxazolyl, pyrazolyl, triazolyl, tetrazolyl, or indazolyl, each optionally substituted with 1 to 3 substituents optionally and independently selected from C₁-C₆ alkyl, C₃-C₆ cycloalkyl, halo, C₁-C₄ haloalkyl, cyano, -OR₃₀₇, SH, -S(O)ₙ₄R₃₁₃, -COR₃₀₇, -CO₂R₃₀₇, -OC(O)R₃₁₃, -NR₃₀₈COR₃₀₇, -N(COR₃₀₇)₂, -NR₃₀₈CONR₃₀₆R₃₀₇, -NR₃₀₈CO₂R₃₁₃, -NR₃₀₆R₃₀₇, and -CONR₃₀₆R₃₀₇;
   heterocyclyl² is saturated or partially saturated heteroaryl, optionally substituted with 1 to 3 substituents optionally and independently selected from C₁-C₆ alkyl, C₃-C₆ cycloalkyl, halo, C₁-C₄ haloalkyl, cyano, -OR₃₀₇, SH, -S(O)ₙ₄R₃₁₃, -COR₃₀₇, -CO₂R₃₀₇, -OC(O)R₃₁₃, -NR₃₀₈COR₃₀₇, -N(COR₃₀₇)₂, -NR₃₀₈CONR₃₀₆R₃₀₇, -NR₃₀₈CO₂R₃₁₃, -NR₃₀₆R₃₀₇, and -CONR₃₀₆R₃₀₇;
   n4 is optionally and independently 0, 1 or 2;
   provided that R₃₀₄ in formula Iab is not H;
   (a) when X₁₃ is N, Y₂₃ is N, and Z₂₃ is NR₃₀₃, then R₃₀₁ is H, R₃₀₃ is H or benzyl, and Ar₁₀ is p-methylphenyl;
   (b) when X₁₃ is N, Y₂₃ is N, and Z₂₃ is NR₃₀₃, then R₃₀₁ is butyl, R₃₀₃ is benzyl, and Ar₁₀ is phenyl;
   (c) when X₁₃ is N, Y₂₃ is CH, and Z₂₃ is NR₃₀₃, then R₃₀₃ is methyl, R₃₀₁ is H, and Ar₁₀ is phenyl or 2-fluorophenyl;
   (d) when X₁₃ is N, Y₂₃ is CH, and Z₂₃ is NR₃₀₃, then R₃₀₃ is methyl, R₃₀₁ is Cl and Ar₁₀ is phenyl;
   (e) when X₁₃ is N, Y₂₃ is CH, and Z₂₃ is NR₃₀₃, then R₃₀₁ is Cl, R₃₀₃ is benzyl, and Ar₁₀ is phenyl or substituted phenyl;
   (f) when X₁₃ is N, Y₂₃ is CH, and Z₂₃ is NR₃₀₃, then R₃₀₃ is p-methylbenzyl, and Ar₁₀ is phenyl;
   (g) when X₁₃ is N, Y₂₃ is CR₃₀₂, and Z₂₃ is NR₃₀₃, then R₃₀₂ is CH₃, R₃₀₃ is H, and Ar₁₀ is phenyl or phenyl substituted with methyl, ethyl, isopropyl, fluoro or chloro;
   (h) when X₁₃ is N, Y₂₃ is N, and Z₂₃ is NR₃₀₃, then R₃₀₃ is cyclopropylmethyl, R₃₀₁ is H, and Ar₁₀ is 2-bromo-4-isopropylphenyl, or
   (i) when X₁₃ is N, Y₂₃ is N, and Z₂₃ is S, then R₃₀₁ is H, and Ar₁₀ is 2-bromo-4-isopropylphenyl, its pharmaceutically acceptable salt or pro-drugs;
(35) A pharmaceutical composition for prophylaxis or therapy of a postoperative stress according to the above (1), wherein the compound having CRF antagonist activating effect is a compound represented by the formula Iad 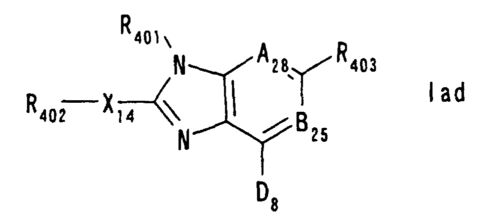 wherein
   A₂₈ is N or C-R₄₀₇;
   B₂₅ is N or C-R₄₀₈;
   with the proviso that at least one of the groups A₂₈ and B₂₅ is N;
   D₈ is an aryl³ or heteroaryl³ group attached to an unsaturated carbon atom;
   X₁₄ is selected from the group consisting of CH-R₄₀₉, N-R₄₁₀, O, S(O)n₅ and a bond;
   N₅ is 0, 1 or 2;
   R₄₀₁ is selected from the group consisting of C₁-C₁₀ alkyl, C₂-C₁₀ alkenyl, C₂-C₁₀ alkynyl, C₃-C₈ cycloalkyl, C₃-C₆ cycloalkyl- C₁-C₆ alkyl, C₁-C₄ alkoxy- C₁-C₄ alkyl, -SO₂- C₁-C₁₀ alkyl, -SO₂-R₄₀₁ₐ, and -SO₂-R_{401b};
   R₄₀₁ is substituted with 0 to 1 substituent selected from the group consisting of -CN, -S(O)n₅R_{414b}, -COR₄₁₃ₐ, -CO₂R₄₁₃ₐ, -NR₄₁₅ₐCOR₄₁₃ₐ, -N(COR₄₁₃ₐ)₂, -NR₄₁₅ₐCONR₄₁₃ₐR₄₁₆ₐ, -NR₄₁₅ₐCO₂R_{414b}, -CONR₄₁₃ₐR₄₁₆ₐ, 1-morpholinyl, 1-piperidinyl, 1-piperazinyl, and C₃-C₈ cycloalkyl, wherein 0 to 1 carbon atom in said C₄-C₈ cycloalkyl is replaced by 0 to 1 group selected from the group consisting of -O, -S(O)n₅, -NR₄₁₃ₐ, -NCO₂R_{414b}, -NCOR_{414b} and -NSO₂R_{414b}, and wherein N₄ in said 1-piperazinyl is substituted with 0 to 1 substituent selected from the group consisting of R₄₁₃ₐ, CO₂R_{414b}, COR_{414b} and SO₂R_{414b};
   Also R₄₀₁ is optionally substituted with 0 to 3 substituents independently selected from the group consisting of R₄₀₁ₐ, R_{401b}, R_{401c}, C₁-C₆ alkyl, C₂-C₈ alkenyl, C₂-C₈ alkynyl, Br, Cl, F, I, C₁-C₄ haloalkyl, -OR₄₁₃ₐ, -NR₄₁₃ₐR₄₁₆ₐ, C₁-C₄ alkoxy- C₁-C₄ alkyl, and C₃-C₈ cycloalkyl which is substituted with 0 to 1 R₄₀₉ and in which 0 to 1 carbons of C₄-C₈ cycloalkyl is replaced by -O-;
   with the proviso that R₄₀₁ is other than
   (a) cyclohexyl-(CH₂)₂- group;
   (b) 3-cyclopropyl-3-methoxypropyl group;
   (c) unsubstituted-(alkoxy)methyl group; and,
   (d) 1-hydroxyalkyl group;
   also with the proviso that when R₄₀₁ is alkyl substituted with OH, then the carbon adjacent to N in the ring is other than CH₂;
   R₄₀₁ₐ is aryl and is selected from the group consisting of phenyl, naphthyl, indanyl and indenyl, each R₄₀₁ₐ being substituted with 0 to 1 -OR₄₁₇ and 0 to 5 substituents optionally and independently selected from the group consisting of C₁-C₆ alkyl, C₃-C₆ cycloalkyl, Br, Cl, F, I, C₁-C₄ haloalkyl, -CN, nitro, SH, -S(O)n₅R₄₁₈, -COR₄₁₇, -OC(O)R₄₁₈, -NR₄₁₅ₐCOR₄₁₇, -N(COR₄₁₇)₂, -NR₄₁₅ₐCONR₄₁₇ₐR₄₁₉ₐ, -NR₄₁₅ₐCO₂R₄₁₈, -NR₄₁₇ₐR₄₁₉ₐ, and -CONR₄₁₇ₐR₄₁₉ₐ;
   R_{401b} is heteroaryl and is selected from the group consisting of pyridyl, pyrimidinyl, triazinyl, furanyl, quinolinyl, isoquinolinyl, thienyl, imidazolyl, thiazolyl, indolyl, pyrrolyl, oxazolyl, benzofuranyl, benzothienyl, benzothiazolyl, benzoxazolyl, isoxazolyl, pyrazolyl, triazolyl, tetrazolyl, indazolyl, 2,3-dihydrobenzofuranyl, 2,3-dihydrobenzothienyl, 2,3-dihydrobenzothienyl-S-oxide, 2,3-dihydrobenzothienyl-S-dioxide, indolinyl, benzoxazolin-2-onyl, benzodioxolanyl and benzodioxane, each heteroaryl being substituted on 0 to 4 carbon atoms with a substituent optionally and independently selected from the group consisting of C₁-C₆ alkyl, C₃-C₆ cycloalkyl, Br, Cl, F, I, C₁-C₄ haloalkyl, -CN, nitro, -OR₄₁₇, SH, -S(O)ₘ₃R₄₁₈, -COR₄₁₇, -OC(O)R₄₁₈, -NR₄₁₅ₐCOR₄₁₇, -N(COR₄₁₇)₂, -NR₄₁₅ₐCONR₄₁₇ₐR₄₁₉ₐ, -NR₄₁₅ₐCO₂R₄₁₈, -NR₄₁₇ₐR₄₁₉ₐ, and -CONR₄₁₇ₐR₄₁₉ₐ and each heteroaryl being substituted on any nitrogen atom with 0 to 1 substituent selected from the group consisting of R₄₁₅ₐ, CO₂R_{414b}, COR_{414b}, and SO₂R_{414b};
   R_{401c} is heterocyclyl and is a saturated or partially saturated heteroaryl³, each heterocyclyl being substituted on 0 to 4 carbon atoms with a substituent optionally and independently selected from the group consisting of C₁-C₆ alkyl, C₃-C₆ cycloalkyl, Br, Cl, F, I, C₁-C₄ haloalkyl, -CN, nitro, -OR₄₁₃ₐ, SH, -S(O)n₅R_{414b}, -COR₄₁₃ₐ, -OC(O)R_{414b}, -NR₄₁₅ₐCOR₄₁₃ₐ, -N(COR₄₁₃ₐ)₂, -NR₄₁₅ₐCONR₄₁₃ₐR₄₁₆ₐ, -NR₄₁₅ₐCO₂R_{414b}, -NR₄₁₃ₐR₄₁₆ₐ, and -CONR₄₁₃ₐR₄₁₆ₐ and each heterocyclyl being substituted on any nitrogen atom with 0 to 1 substituent selected from the group consisting of R₄₁₃ₐ, CO₂R_{414b}, COR_{414b} and SO₂R_{414b} and wherein any sulfur atom is optionally monooxidized or dioxidized;
   with the proviso that R₄₀₁ is other than a -(CH₂)₁₋₄-aryl³, -(CH₂)₁₋₄-heteroaryl³, or -(CH₂)₁₋₄ heterocyclyl, wherein the aryl³, heteroaryl³, or heterocyclyl group is substituted or unsubstituted;
   R₄₀₂ is selected from the group consisting of C₁-C₄ alkyl, C₃-C₈ cycloalkyl, C₂-C₄ alkenyl, and C₂-C₄ alkynyl and is substituted with 0 to 3 substituents selected from the group -CN, hydroxy, halo and C₁-C₄ alkoxy;
   alternatively, when X₁₄ is a bond, then R₄₀₂ is selected from the group consisting of -CN, CF₃ and C₂F₅;
   R₄₀₃, R₄₀₇ and R₄₀₈ are, at each occurrence, independently selected from the group H, Br, Cl, F, I, -CN, C₁-C₄ alkyl, C₃-C₈ cycloalkyl, C₁-C₄ alkoxy, C₁-C₄ alkylthio, C₁-C₄ alkylsulfinyl, C₁-C₄ alkylsulfonyl, amino, C₁-C₄ alkylamino, (C₁-C₄ alkyl)₂amino and phenyl, wherein each phenyl is substituted with 0 to 3 groups selected from the group consisting of C₁-C₇ alkyl, C₃-C₈ cycloalkyl, Br, Cl, F, I, C₁-C₄ haloalkyl, nitro, C₁-C₄ alkoxy, C₁-C₄ haloalkoxy, C₁-C₄ alkylthio, C₁-C₄ alkyl sulfinyl, C₁-C₄ alkylsulfonyl, C₁-C₆ alkylamino and (C₁-C₄ alkyl)₂amino;
   with the proviso that when R₄₀₁ is unsubstituted C₁-C₁₀ alkyl, then R₄₀₃ is other than substituted or unsubstituted phenyl;
   R₄₀₉ and R₄₁₀ are, at each occurrence, independently selected from the group consisting of H, C₁-C₄ alkyl, C₃-C₆ cycloalkyl- C₁-C₄ alkyl and C₃-C₈ cycloalkyl;
   R₄₁₃ is selected from the group consisting of H, C₁-C₄ alkyl, C₁-C₄ haloalkyl, C₁-C₄ alkoxy- C₁-C₄ alkyl, C₃-C₆ cycloalkyl, C₃-C₆ cycloalkyl- C₁-C₆ alkyl, aryl³, aryl³(C₁-C₄ alkyl)-, heteroaryl³ and heteroaryl³(C₁-C₄ alkyl)-;
   R₄₁₃ₐ and R₄₁₆ₐ are, at each occurrence, independently selected from the group consisting of H, C₁-C₄ alkyl, C₁-C₄ haloalkyl, C₁-C₄ alkoxy- C₁-C₄ alkyl, C₃-C₆ cycloalkyl, and C₃-C₆ cycloalkyl C₁-C₆ alkyl;
   R₄₁₄ is selected from the group consisting of C₁-C₄ alkyl, C₁-C₄ haloalkyl, C₁-C₄ alkoxy- C₁-C₄ alkyl, C₃-C₆ cycloalkyl, C₃-C₆ cycloalkyl- C₁-C₆ alkyl, aryl³, aryl³(C₁-C₄ alkyl)-, heteroaryl³ and heteroaryl³(C₁-C₄ alkyl)- and benzyl, each benzyl being substituted on the aryl moiety with 0 to 1 substituent selected from the group consisting of C₁-C₄ alkyl, Br, Cl, F, I, C₁-C₄ haloalkyl, nitro, C₁-C₄ alkoxy C₁-C₄ haloalkoxy, and dimethylamino;
   R₄₁₄ₐ is selected from the group consisting of C₁-C₄ alkyl, C₁-C₄ haloalkyl, C₁-C₄ alkoxy- C₁-C₄ alkyl, C₃-C₆ cycloalkyl, C₃-C₆ cycloalkyl- C₁-C₆ alkyl and benzyl, each benzyl being substituted on the aryl moiety with 0 to 1 substituents selected from the group consisting of C₁-C₄ alkyl, Br, Cl, F, I, C₁-C₄ haloalkyl, nitro, C₁-C₄ alkoxy, C₁-C₄ haloalkoxy, and dimethylamino;
   R_{414b} is selected from the group consisting of C₁-C₄ alkyl, C₁-C₄ haloalkyl, C₁-C₄ alkoxy- C₁-C₄ alkyl, C₃-C₆ cycloalkyl, and C₃-C₆ cycloalkyl- C₁-C₆ alkyl;
   R₄₁₅ is, at each occurrence, independently selected from the group consisting of H, C₁-C₄ alkyl, C₃-C₇ cycloalkyl, C₃-C₆ cycloalkyl- C₁-C₆ alkyl, phenyl and benzyl, each phenyl or benzyl being substituted on the aryl moiety with 0-3 groups chosen from the group consisting of C₁-C₄ alkyl, Br, Cl, F, I, C₁-C₄ haloalkyl, nitro, C₁-C₄ alkoxy, C₁-C₄ haloalkoxy, and dimethylamino;
   R₄₁₅ₐ is, at each occurrence, independently selected from the group consisting of H, C₁-C₄ alkyl, C₃-C₇ cycloalkyl, and C₃-C₆ cycloalkyl- C₁-C₆ alkyl;
   R₄₁₇ is, at each occurrence, selected from the group consisting of H, C₁-C₆ alkyl, C₃-C₁₀ cycloalkyl, C₃-C₆ cycloalkyl-C₁-C₆ alkyl, C₁-C₂ alkoxy -C₁-C₂ alkyl, C₁-C₄ haloalkyl, R₄₁₄S(O)n₅- C₁-C₄ alkyl, and R_{417b}R_{419b}N- C₂-C₄ alkyl;
   R₄₁₈ and R₄₁₉ are, at each occurrence, independently selected from the group consisting of H, C₁-C₆ alkyl, C₃-C₁₀ cycloalkyl, C₃-C₆ cycloalkyl- C₁-C₆ alkyl, C₁-C₂ alkoxy- C₁-C₂ alkyl, and C₁-C₄ haloalkyl;
   alternatively, in NR₄₁₇R₄₁₉ moiety, R₄₁₇ and R₄₁₉ taken together form 1-pyrrolidinyl, 1-morpholinyl, 1-piperidinyl or 1-piperazinyl, wherein N₄ in 1-piperazinyl is substituted with 0 to 1 substituent selected from the group consisting of R₄₁₃, CO₂R₄₁₄, COR₄₁₄ and SO₂R₄₁₄;
   alternatively, in NR_{417b}R_{419b} moiety, R_{417b} and R_{419b} taken together form 1-pyrrolidinyl, 1-morpholinyl, 1-piperidinyl or 1-piperazinyl, wherein N₄ in 1-piperazinyl is substituted with 0 to 1 substituent selected from the group consisting of R₄₁₃, CO₂R₄₁₄, COR₄₁₄ and SO₂R₄₁₄;
   R₄₁₇ₐ and R₄₁₉ₐ are, at each occurrence, independently selected from the group consisting of H, C₁-C₆ alkyl, C₃-C₁₀ cycloalkyl, C₃-C₆ cycloalkyl- C₁-C₆ alkyl and C₁-C₄ haloalkyl;
   aryl³ is, at each occurrence, independently selected from the group consisting of phenyl, naphthyl, indanyl and indenyl, each aryl³ being substituted with 0 to 5 substituents optionally and independently selected from the group consisting of C₁-C₆ alkyl, C₃-C₆ cycloalkyl, methylenedioxy, C₁-C₄ alkoxy- C₁-C₄ alkoxy, -OR₄₁₇, Br, Cl, F, I, C₁-C₄ haloalkyl, -CN, -NO₂, SH, -S(O)n₅R₄₁₈, -COR₄₁₇, -CO₂R₄₁₇, -OC(O)R₄₁₈, -NR₄₁₅COR₄₁₇, -N (COR₄₁₇)₂, -NR₄₁₅CONR₄₁₇R₄₁₉, -NR₄₁₅CO₂R₄₁₈, -NR₄₁₇R₄₁₉, and -CONR₄₁₇R₄₁₉ and up to 1 phenyl, each phenyl substituent being substituted with 0 to 4 substituents selected from the group consisting of C₁-C₃ alkyl, C₁-C₃ alkoxy, Br, Cl, F, I, -CN, dimethylamino, CF₃, C₂F₅, OCF₃, SO₂Me and acetyl;
   heteroaryl³ is, at each occurrence, independently selected from the group consisting of pyridyl, pyrimidinyl, triazinyl, furanyl, quinolinyl, isoquinolinyl, thienyl, imidazolyl, thiazolyl, indolyl, pyrrolyl, oxazolyl, benzofuranyl, benzothienyl, benzothiazolyl, benzoxazolyl, isoxazolyl, triazolyl, tetrazolyl, indazolyl, 2,3-dihydrobenzofuranyl, 2,3-dihydrobenzothienyl, 2,3-dihydrobenzothienyl-S-oxide, 2,3-dihydrobenzothienyl-S-dioxide, indolinyl, benzoxazolin-2-on-yl, benzodioxolanyl and benzodioxane, each heteroaryl³ being substituted on 0 to 4 carbon atoms with a substituent optionally and independently selected from the group consisting of C₁-C₆ alkyl, C₃-C₆ cycloalkyl, Br, Cl, F, I, C₁-C₄ haloalkyl, -CN, nitro, -OR₄₁₇, SH, -S(O)ₘ₃R₄₁₈, -COR₄₁₇, -CO₂R₄₁₇, -OC(O)R₄₁₈, -NR₄₁₅COR₄₁₇, -N(COR₄₁₇)₂, -NR₄₁₅CONR₄₁₇R₄₁₉, -NR₄₁₅CO₂R₄₁₈, -NR₄₁₇R₄₁₉, and -CONR₄₁₇R₄₁₉ and each heteroaryl being substituted on any nitrogen atom with 0 to 1 substituent selected from the group consisting of R₄₁₅, CO₂R₄₁₄ₐ, COR₄₁₄ₐ and SO₂R₄₁₄ₐ;
   with the proviso that when D₈ is imidazole or triazole, then R₄₀₁ is other than unsubstituted linear or branched C₁-C₆ alkyl or C₃-C₆ cycloalkyl,
   its stereoisomer or its pharmaceutically acceptable salt;

(36) A pharmaceutical composition for prophylaxis or therapy of a postoperative stress according to the above (1), wherein the compound having CRF antagonist activating effect is a compound represented by the formula Iae 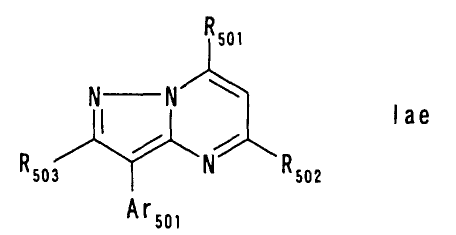 wherein
   R₅₀₁ is NR₅₀₄R₅₀₅ or OR₅₀₅;
   R₅₀₂ is C₁-C₆ alkyl, C₁-C₆ alkoxy or C₁-C₆ alkylthio;
   R₅₀₃ is hydrogen, C₁-C₆ alkyl, C₁-C₆ alkylsulfonyl, C₁-C₆ alkylsulfoxy or C₁-C₆ alkylthio;
   R₅₀₄ is hydrogen, C₁-C₆ alkyl, mono or di(C₃-C₆ cycloalkyl)methyl, C₃-C₆ cycloalkyl, C₃-C₆ alkenyl, hydroxy C₁-C₆ alkyl, C₁-C₆ alkylcarbonyloxy C₁-C₆ alkyl or C₁-C₆ alkyloxy C₁-C₆ alkyl;
   R₅₀₅ is C₁-C₈ alkyl, mono or di(C₃-C₆ cycloalkyl)methyl, Ar₅₀₂CH₂, C₃-C₆ alkenyl, C₁-C₆ alkyloxy C₁-C₆ alkyl, hydroxy C₁-C₆ alkyl, thienylmethyl, furanylmethyl, C₁-C₆ alkylthio C₁-C₆ alkyl, morpholinyl, mono or di(C₁-C₆ alkyl)amino C₁-C₆ alkyl, di(C₁-C₆ alkyl)amino, C₁-C₆ alkylcarbonyl C₁-C₆ alkyl, C₁-C₆ alkyl substituted with imidazolyl; or a radical represented by the formula -Alk-O-CO-Ar₅₀₂;
   or R₅₀₄ and R₅₀₅ taken together with the nitrogen to which they are attached may form a pyrrolidinyl, piperidinyl, homopiperidinyl or morpholinyl, optionally substituted with C₁-C₆ alkyl or C₁-C₆ alkyloxy C₁-C₆ alkyl; and
   Ar₅₀₁ is phenyl, phenyl substituted with one, two or three substituents independently selected from halo, C₁-C₆ alkyl, trifluoromethyl, hydroxy, cyano, C₁-C₆ alkyloxy, benzyloxy, C₁-C₆ alkylthio, nitro, amino and mono or di(C₁-C₆ alkyl)amino, pyridinyl, pyridinyl substituted with one, two or three substituents independently selected from halo, C₁-C₆ alkyl, trifluoromethyl, hydroxy, cyano, C₁-C₆ alkyloxy, benzyloxy, C₁-C₆ alkylthio, nitro, amino and mono or di(C₁-C₆ alkyl)amino and piperidinyl, and additionally wherein said substituted phenyl may optionally be substituted with one or more halogens;
   Ar₅₀₂ is phenyl, phenyl substituted with one, two or three substituents each independently selected from halo, C₁-C₆ alkyl, C₁-C₆ alkyloxy, di(C₁-C₆ alkyl)amino C₁-C₆ alkyl, trifluoromethyl and C₁-C₆ alkyl substituted with morpholinyl, or pyridinyl; and
   Alk is C₁-C₆ alkanediyl; with the proviso that 5-methyl-3-phenyl-7-(phenylmethoxy)-pyrazolo(1,5-a)-pyrimidine and 2,5-dimethyl-7-(methylamino)-3-phenyl-pyrazolo(1,5-a)-pyrim idine are not included,
   its stereoisomer or its pharmaceutically acceptable acid addition salt;
(37) A pharmaceutical composition for prophylaxis or therapy of a postoperative stress according to the above (1),
   wherein the compound having CRF antagonist activating effect is a compound represented by the formula Iaf 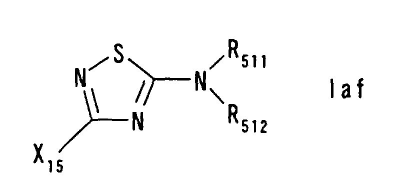 wherein R₅₁₁ and R₅₁₂ are substituents independently selected from a group consisting of C₁-C₆ alkyl, C₃-C₆ alkenyl, C₂-C₆ alkylether, C₂-C₆ alkylthioether, C₄-C₉ cycloalkylalkyl, C₇-C₂₀ dicycloalkylalkyl, C₇-C₂₀ cycloalkylarylalkyl, C₇-C₂₀ arylalkyl, C₇-C₂₀ diarylalkyl, C₃-C₁₄ heteroaryl and C₃-C₁₄ heteroarylalkyl, and substituted derivatives thereof; and
   X₁₅ is a substituent selected from substituted C₃-C₁₄ monocyclic or fused homoaryl or heteroaryl groups,
   its stereoisomer or pharmaceutically acceptable salt;
(38) A pharmaceutical composition for prophylaxis or therapy of a postoperative stress according to the above (1), wherein the compound having CRF antagonist activating effect is a compound represented by the formula Iag or Iah 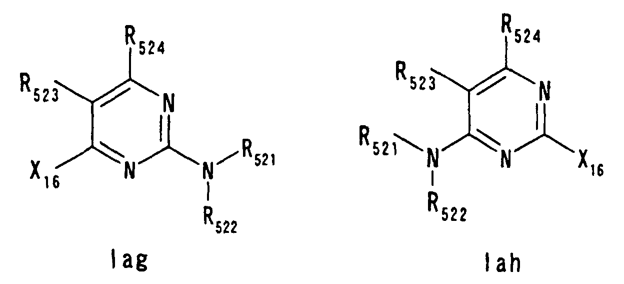 wherein R₅₂₁ and R₅₂₂ are independently selected from a group consisting of C₁-C₆ alkyl, C₃-C₆ alkenyl, C₂-C₆ alkylether, C₂-C₆ alkylthioether, C₄-C₉ cycloalkylalkyl, C₇-C₂₀ dicycloalkylalkyl, C₇-C₂₀ cycloalkylarylalkyl, C₇-C₂₀ arylalkyl, C₇-C₂₀ diarylalkyl, C₃-C₁₄ heteroaryl and C₃-C₁₄ heteroarylalkyl, and substituted derivatives thereof;
   R₅₂₃ and R₅₂₄ are independently selected from a group consisting of hydrogen, amino, C₁-C₆ substituted amino, halogen, C₁-C₂ alkyl, C₁-C₆ carbonyl-containing groups and C₁-C₆ sulfur containing groups; and
   X₁₆ is selected from substituted C₃-C₁₄ monocyclic or fused homoaryl or heteroaryl,
   its stereoisomer or its pharmaceutically acceptable salt;
(39) A pharmaceutical composition for prophylaxis or therapy of a postoperative stress according to the above (1), wherein the compound having CRF antagonist activating effect is a compound represented by the formula Iai 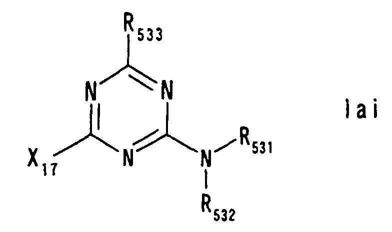 wherein R₅₃₁ and R₅₃₂ are independently selected from C₁-C₆ alkyl, C₃-C₆ alkenyl, C₂-C₆ alkylether, C₂-C₆ alkylthioether, C₄-C₉ cycloalkylalkyl, C₇-C₂₀ dicycloalkylalkyl, C₇-C₂₀ cycloalkylarylalkyl, C₇-C₂₀ arylalkyl, C₇-C₂₀ diarylalkyl, C₃-C₁₄ heteroaryl and C₃-C₁₄ heteroarylalkyl, and substituted derivatives thereof;
   R₅₃₃ is selected from hydrogen, amino, C₁-C₆ alkyl and C₁-C₆ alkoxy; and
   X₁₇ is selected from substituted C₃-C₁₄ monocyclic or fused homoaryl or heteroaryl,
   its stereoisomer or its pharmaceutically acceptable salt;
(40) A pharmaceutical composition for prophylaxis or therapy of a postoperative stress according to the above (1), wherein the compound having CRF antagonist activating effect is a compound represented by the formula Iaj or Iak 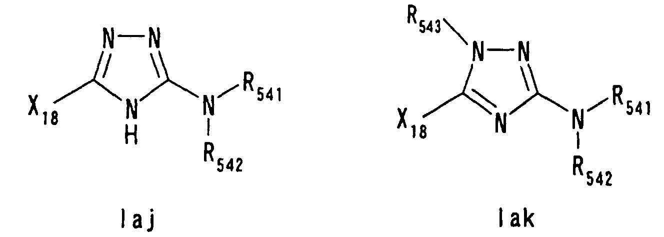 wherein R₅₄₁ and R₅₄₂ are independently selected from C₁-C₆ alkyl, C₃-C₆ alkenyl, C₂-C₆ alkylether, C₂-C₆ alkylthioether, C₄-C₉ cycloalkylalkyl, C₇-C₂₀ dicycloalkylalkyl, C₇-C₂₀ cycloalkylarylalkyl, C₇-C₂₀ arylalkyl, C₇-C₂₀ diarylalkyl, C₃-C₁₄ heteroaryl and C₃-C₁₄ heteroarylalkyl, and substituted derivatives thereof;
   R₅₄₃ is a radical selected from hydrogen or methyl; and
   X₁₈ is selected from substituted C₃-C₁₄ monocyclic or fused homoaryl or heteroaryl,
   its stereoisomer or its pharmaceutically acceptable salt;
(41) A pharmaceutical composition for the prophylaxis or therapy of a postoperative stress, wherein the compound having CRF antagonist activating effect is a compound represented by any one of the formula Ial 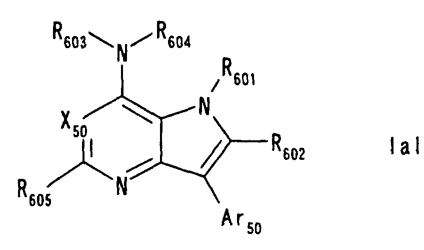 wherein Ar₅₀ is phenyl, 2-, 3- or 4- pyridyl, 2- or 3-thienyl, 4- or 5-pyrimidyl, each of which is mono-, di- or trisubstituted with halogen, hydroxy and lower alkyl, or lower alkoxy, with the proviso that at least one of the ortho positions of the Ar₅₀ substituent is substituted;
   X₅₀ is CH or nitrogen;
   R₆₀₁ is lower alkyl;
   R₆₀₂ is hydrogen, halogen, lower alkyl, lower alkoxy or thioalkoxy; or
   R₆₀₁ and R₆₀₂ taken together represent-(CH₂)n₅-A10-(CH₂)m₅-, wherein n5 is 2, 3 or 4, A10 is methylene, oxygen, sulfur or NR₆₀₆, wherein R₆₀₆ is lower alkyl, and m5 is 0, 1 or 2;
   R₆₀₃ and R₆₀₄ are the same or different and represent hydrogen or lower alkyl;
   phenyl, 2-, 3- or 4-pyridyl, 2- or 3-thienyl or 2-, 4-or 5-pyrimidyl, each of which is mono- or disubstituted with halogen, hydroxy, lower alkyl or lower alkoxy; phenyl lower alkyl, 2-, 3- or 4-pyridyl lower alkyl, 2-or 3-thienyl lower alkyl or 2-, 4- or 5-pyrimidyl lower alkyl;
   cycloalkyl having 3-8 carbon atoms or cycloalkyl lower alkyl where the cycloalkyl portion has 3-8 carbon atoms; 2-hydroxyethyl or 3-hydroxypropyl optionally mono or di substituted with lower alkyl with the proviso that not both R₆₀₃ and R₆₀₄ are hydrogen; or
   R₆₀₃ and R₆₀₄ taken together represent -(CH₂)ₙ₆-A11-(CH₂)m₆-
   wherein n6 is 2 or 3,
   A11 is methylene, 1, 2-phenylene, oxygen, sulfur or NR₆₀₆
   wherein R₆₀₇ is lower alkyl, phenyl, 2-, 3- or 4-pyridyl, 2- or 3-thienyl or 2-, 4- or 5-pyrimidyl, phenyl lower alkyl, 2-, 3- or 4-pyridyl lower alkyl, 2- or 3-thienyl lower alkyl, or 2-, 4- or 5-pyrimidyl lower alkyl, and m6 is 1, 2 or 3 and
   R₆₀₅ is hydrogen, halogen, lower alkyl, lower alkoxy or thioalkoxy,
   the formula Iam 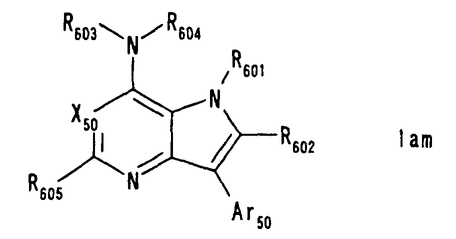 wherein Ar₅₀ is phenyl, 2-, 3- or 4-pyridyl, 2- or 3-thienyl, 4- or 5-pyrimidyl, each of which is mono-, di-, or trisubstituted with halogen, hydroxy, lower alkyl, or lower alkoxy, with the proviso that at least one of the ortho positions of the Ar₅₀ substituent is substituted;
   X₅₀ is CH or nitrogen;
   R₆₀₁ is lower alkyl;
   R₆₀₂ is hydrogen, halogen, lower alkyl, lower alkoxy, or thioalkoxy; or
   R₆₀₃ and R₆₀₄ are the same or different represent hydrogen or lower alkyl;
   phenyl, 2-, 3- or 4-pyridyl, 2- or 3-thienyl or 2-, 4-or 5-pyrimidyl, each of which is mono- or disubstituted with halogen, hydroxy, lower alkyl or lower alkoxy; phenyl lower alkyl, 2-, 3- or 4-pyridyl lower alkyl, 2-or 3-thienyl lower alkyl or 2-, 4- or 5-pyrimidyl lower alkyl;
   cycloalkyl having 3 to 8 carbon atoms, or cycloalkyl lower alkyl where the cycloalkyl portion has 3 to 8 carbon atoms;
   2-hydroxyethyl or 3-hydroxypropyl optionally mono or disubstituted with lower alkyl with the proviso that not both R₆₀₃ and R₆₀₄ are hydrogen; or
   R₆₀₃ and R₆₀₄ taken together represent -(CH₂)ₙ₆-A12-(CH₂)ₘ₆- where n6 is 2 or 3, A12 is methylene, 1,2-phenylene, oxygen, sulfur or NR₆₀₆,
   wherein R₆₀₆ is lower alkyl, and m6 is 1, 2 or 3, and
   R₆₀₅ is hydrogen, halogen, lower alkyl, lower alkoxy or thioalkoxy,
   the formula Ian 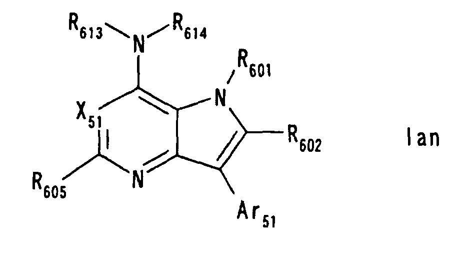 wherein Ar₅₁ is a phenyl mono-, di-, or trisubstituted with halogen, hydroxy, lower alkyl or lower alkoxy, with the proviso that at least one of the ortho positions of the Ar₅₁ substituent is substituted;
   X₅₁ is nitrogen;
   R₆₀₁ is lower alkyl;
   R₆₀₂ is hydrogen, halogen, lower alkyl, lower alkoxy or thioalkoxy; or
   R₆₁₃ and R₆₁₄ are the same or different and represent hydrogen or lower alkyl;
   cycloalkyl having 3 to 8 carbon atoms, or cycloalkyl lower alkyl where the cycloalkyl portion has 3 to 8 carbon atoms; 2-hydroxyethyl or 3-hydroxypropyl optionally mono or disubstituted with lower alkyl with the proviso that not both R₆₁₃ and R₆₁₄ are hydrogen; or
   R₆₁₃ and R₆₁₄ taken together represent -(CH₂)ₙ₆-A12-(CH₂)ₘ₆-
   where n5 is 2 or 3,
   A12 is methylene, 1,2phenylene, oxygen, sulfur or NR₆₀₆,
   wherein R₆₀₆ is lower alkyl, and
   m6 is 1, 2 or 3;
   R₆₀₅ is hydrogen, halogen, lower alkyl, lower alkoxy or thioalkoxy,
   and the formula Iao 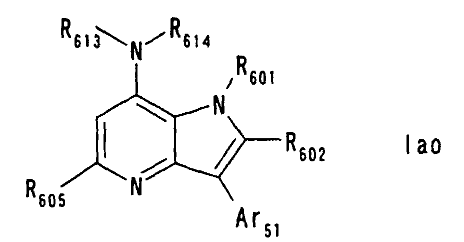 wherein Ar₅₁ is phenyl mono-, di-, or trisbstituted with halogen, hydroxy, lower alkyl or lower alkoxy, with the proviso that at least one of the ortho positions of the Ar₅₁ substituent is substituted;
   R₆₀₁ is lower alkyl;
   R₆₀₂ is hydrogen, halogen, lower alkyl, lower alkoxy or thioalkoxy; or
   R₆₁₃ and R₆₁₄ are the same or different and represent hydrogen or lower alkyl;
   cycloalkyl having 3 to 8 carbon atoms or cycloalkyl lower alkyl where cycloalkyl portion has 3 to 8 carbon atoms;
   2-hydroxyethyl or 3-hydroxypropyl optionally mono or disubstituted with lower alkyl with the proviso that not both R₆₁₃ and R₆₁₄ are hydrogen; or
   R₆₁₃ and R₆₁₄ taken together represent -(CH₂)ₙ₆-A12-(CH₂)ₘ₆-
   wherein n6 is 2 or 3,
   A12 is methylene, 1, 2-phenylene, oxygen, sulfur or NR₆₀₆
   wherein R₆₀₆ is lower alkyl, and
   m6 is 1, 2 or 3, and
   R₆₀₅ is hydrogen, halogen, lower alkyl, lower alkoxy or thioalkoxy,
   or its pharmaceutically acceptable salts;
(42) A pharmaceutical composition for prophylaxis or therapy of a postoperative stress according to the above (1), wherein the compound having CRF antagonist activating effect is a compound represented by the formula Iap 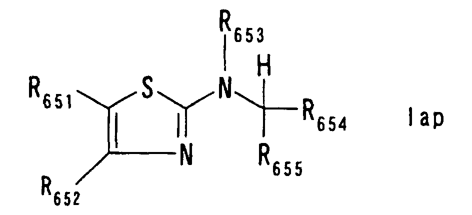 wherein R₆₅₁ is hydrogen or C₁-C₅ alkyl;
   R₆₅₂ is a radical represented by the formula (A20): 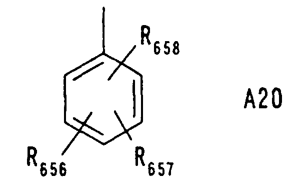 wherein R₆₅₆ is C₁-C₅ hydroxyalkyl, and R₆₅₇ and R₆₅₈, which may be the same or different, each represents hydrogen, halogen or C₁-C₅ hydroxyalkyl, trifluoromethyl, C₁-C₅ alkoxy or C₁-C₅ alkyl, a radical represented by the formula (B20): 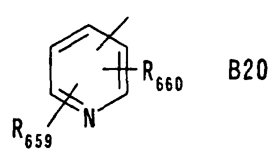 wherein R₆₅₉ and R₆₆₀, which may be the same or different, each represents hydrogen, halogen, C₁-C₅ alkyl or C₁-C₅ alkoxy, or a radical represented by the formula (C20): 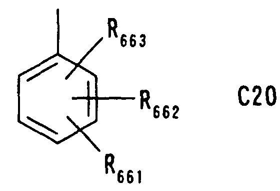 wherein R₆₆₁, R₆₆₂ and R₆₆₃, which may be the same or different, each represents hydrogen, halogen, trifluoromethyl, C₁-C₅ alkoxy or a C₁-C₅ alkyl,
   R₆₅₃ is C₁-C₅ alkyl, C₁-C₅ hydroxyalkyl, 2-tetrahydropyranyloxyalkyl wherein the alkyl moiety comprises one to five carbon atoms, C₂-C₁₀ alkoxyalkyl or C₃-C₁₁ acyloxyalkyl;
   R₆₅₄ is C₃-C₆ cycloalkyl, C₁-C₅ hydroxyalkyl, C₂-C₁₀ alkoxyalkyl, C₄-C₁₁ cycloalkyloxyalkyl, C₂-C₁₀ hydroxyalkyloxyalkyl, C₃-C₁₂ alkoxyalkyloxyalkyl, C₃-C₁₁ acyloxyalkyl or C₂-C₁₀ alkylthioalkyl; and
   R₆₅₅ is C₃-C₆ cycloalkyl, phenyl, thienyl or pyridyl, which may be optionally substituted with one or more halogens, C₁-C₅ alkoxy, C₁-C₅ alkyl or trifluoromethyl, or a radical represented by the formula (D20): 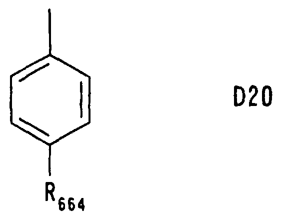 wherein R₆₆₄ is carboxyl, C₂-C₆ carboxyalkyl, C₂-C₆ alkoxycarbonyl, C₃-C₁₁ acyloxyalkyl, C₂-C₁₀ alkoxyalkyl, C₈-C₁₆ aralkoxyalkyl, wherein optionally substituted on the aromatic ring with one or more halogens, C₁-C₃ alkoxy or trifluoromethyl, monohalogenated C₁-C₅ alkyl, linear or branched C₁-C₅ hydroxyalkyl, a radical represented by the formula (E20): 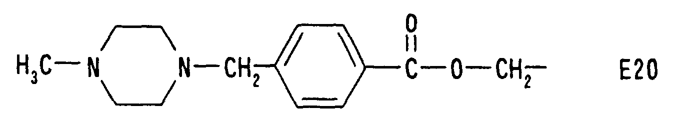 or C₁-C₅ sulfoxyalkyl,
   3-hydroxyalkyl-6-pyridyl or 2-hydroxyalkyl-5-pyridyl, wherein the alkyl contain one to five carbon atoms, with the proviso that when R₆₅₃ is C₁-C₅ alkyl, R₆₅₄ is cycloalkyl and R₆₅₅ is either cycloalkyl or phenyl, thienyl or pyridyl, which may be optionally substituted with one or more halogens, C₁-C₅ alkoxy, C₁-C₅ alkyl or trifluoromethyl, then R₆₅₂ is not a radical represented by the formula (C20),
   its stereoisomer or its additional salt;
(43) A pharmaceutical composition for prophylaxis or therapy of a postoperative stress according to the above (1), wherein the compound having CRF antagonist activating effect is a compound represented by Iaq 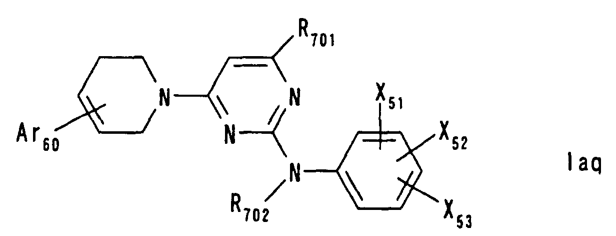 wherein Ar₆₀ represents phenyl substituted with one to three substituents selected from halogen, C₁-C₅ alkyl, C₁-C₅ alkoxy and trifluoromethyl, phenyl, thienyl or furanyl; R₇₀₁ represents hydrogen, C₁-C₅ alkyl, amino or amino substituted with one or two C₁-C₅ alkyl groups; R₇₀₂ represents C₁-C₅ alkyl, C₄-C₇ cycloalkylalkyl, C₂-C₅ alkenyl or C₂-C₅ alkynyl; and X₅₁, X₅₂ and X₅₃, which may be the same or different, each represent hydrogen, halogen, C₁-C₅ alkyl, C₁-C₅ alkoxy, C₁-C₅ alkylthio, amino or amino substituted with one or two C₁-C₅ alkyl groups, or its pharmaceutically acceptable salt;
(44) A pharmaceutical composition for prophylaxis or therapy of a postoperative stress according to the above (1), wherein the compound having CRF antagonist activating effect is a compound represented by the formula Iar, 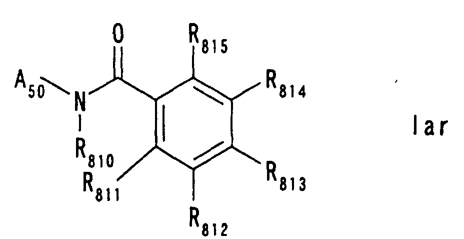 wherein A₅₀ is a group represented by the formula Ias; 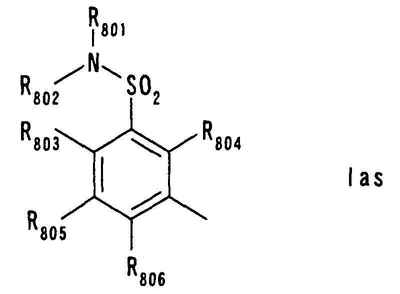 (wherein R₈₀₁ and R₈₀₂ may be the same or different and represent hydrogen;
   C₁-C₄ lower alkyl which may be substituted with substituent selected from hydroxy, C₁-C₄ lower alkoxy,
   C₃-C₇ cycloalkyl and aryl;
   halogenated C₁-C₄ lower alkyl;
   C₃-C₇ cycloalkyl or
   aryl, or
   R₈₀₁ and R₈₀₂ taken together may form a saturated 5 to 6 membered heterocycle ring, which may be substituted by one to four C₁-C₄ lower alkyls and which optionally may contain nitrogen, oxygen or sulfur in addition to nitrogen adjacent to R₈₀₁ and R₈₀₂,
   R₈₀₃ and R ₈₀₄ may be the same or different and represent
   hydrogen;
   halogen;
   C₁-C₄ lower alkyl;
   C₁-C₄ lower alkoxy or
   amino which may be substituted with substituent selected from C₁-C₄ lower alkyl and C₁-C₄ lower alkylsulfonyl,
   R₈₀₅ is hydrogen;
   halogen;
   C₁-C₄ lower alkyl which may be substituted with substituent selected from hydroxy and C₁-C₄ lower alkoxy;
   halogenated C₁-C₄ lower alkyl;
   C₁-C₄ lower alkoxy which may be substituted with substituent selected from halogen and C₃-C₇ cycloalkyl;
   C₂-C₅ lower alkanoyl;
   nitro;
   cyano;
   carbamoyl, which may be substituted with C₁-C₄ lower alkyl;
   C₁-C₄ lower alkylsulfonyl;
   carboxy;
   C₂-C₅ lower alkoxycarbonyl;
   amino, which may be substituted with substituent selected from a group consisting of C₁-C₄ lower alkyl which may be substituted with substituent selected from halogen atom and C₃-C₇ cycloalkyl, C₂-C₅ lower alkanoyl, and C₃-C₇ cycloalkyl;
   a saturated 5 to 6 membered heterocycle ring having nitrogen;
   C₃-C₇ cycloalkyl or
   C₃-C₇ cycloalkoxy,
   R₈₀₆ is hydrogen;
   halogen atom;
   C₁-C₄ lower alkyl;
   amino, which may be substituted with C₁-C₄ lower alkyl;
   cyano;
   carboxy;
   C₂-C₅ lower alkoxycarbonyl;
   C₂-C₅ lower alkanoyl;
   carbamoyl;
   C₁-C₄ lower alkylthio;
   C₁-C₄ lower alkyl sulfinyl;
   C₁-C₄ lower alkylsulfonyl;
   C₃-C₇ cycloalkyl;
   a saturated 5 to 6 membered heterocycle ring having one to two heteroatoms selected from nitrogen, oxygen and sulfur or
   -OR₈₁₆, wherein R₈₁₆ is hydrogen;
   C₁-C₆ alkyl, which may be substituted with substituent selected from hydroxy, C₁-C₄ lower alkoxy, triflouromethyl, cyano, amino which may be substituted with C₁-C₄ lower alkyl, carboxy and C₂-C₅ lower alkoxycarbonyl;
   C₂-C₄ lower alkenyl;
   C₂-C₄ lower alkynyl;
   carbamoyl which may be substituted with C₁-C₄ lower alkenyl;
   C₁-C₄ lower alkylsulfonyl; phosphono;
   C₃-C₇ cycloalkyl;
   aryl or
   benzyl, or
   R₈₀₅ and R₈₁₆ may be taken together to form a saturated 5 to 6 membered heterocyclic ring which may be substituted with C₁-C₄ lower alkyl or oxo),
   or the formula Iat 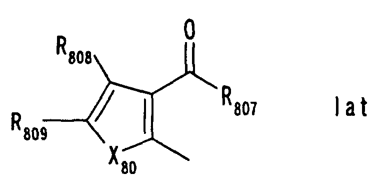 (wherein R₈₀₇ is
   hydroxy;
   C₁-C₄ lower alkoxy or
   -NR₈₁₇R₈₁₈ wherein
   R₈₁₇ and R₈₁₈ may be the same or different and represent hydrogen;
   C₁-C₄ lower alkyl;
   C₁-C₄ lower alkoxy or
   benzyl, or
   R₈₁₇ and R₈₁₈ may be taken together to form a saturated 5 to 6 membered heterocyclic ring, which at each occurrence may have nitrogen, oxygen and sulfur in addition to nitrogen adjacent to R₈₁₇ and R₈₁₈,
   R₈₀₈ is C₁-C₄ lower alkyl, which may be substituted with a substituent selected from C₁-C₄ lower alkoxy and amino which may be substituted with C₁-C₄ lower alkyl,
   R₈₀₉ is aminosulfonyl, which may be substituted with C₁-C₄ lower alkyl or
   -COR₈₁₉ wherein R₈₁₉ is hydroxy;
   C₁-C₄ lower alkyl;
   C₁-C₄ lower alkoxy;
   C₃-C₇ cycloalkyl;
   aryl or - NR₈₂₀R₈₂₁
   wherein R₈₂₀ and R₈₂₁ may be the same or different and represent hydrogen;
   C₁-C₄ lower alkyl;
   C₃-C₇ cycloalkyl;
   aryl or benzyl, or
   R₈₂₀ and R₈₂₁ may be taken together to form a saturated 5 to 6 membered heterocyclic ring, which may have nitrogen, oxygen or sulfur in addition to nitrogen adjacent to R₈₂₀ and R₈₂₁,
   X₈₀ is -S-;
   -O- or
   -NH-),
   R₈₁₀ is hydrogen;
   C₁-C₄ lower alkyl, which may be substituted with substituent selected from carboxy and C₂-C₅ lower alkoxycarbonyl,
   R₈₁₁ is hydrogen;
   halogen atom or
   hydroxy,
   R₈₁₂ is hydrogen;
   halogen atom or
   nitro,
   R₈₁₃ represents
   -OR₈₂₂ wherein R₈₂₂ is
   hydrogen;
   alkali metal or
   phosphono,
   -YR₈₂₃ wherein Y represents -O- or -NH-, and R₈₂₃ is C₁-C₄ lower alkyl which may be substituted with substituent selected from aryl, carboxy and C₂-C₅ lower alkoxycarbonyl;
   aryl which may be substituted with carboxy or carboxy,
   R₈₁₄ is hydrogen;
   halogen atom;
   nitro;
   triflouromethyl;
   amino which may be substituted with C₁-C₄ lower alkyl or C₁-C₄ lower alkylsulfonyl;
   aminosulfonyl;
   C₁-C₄ lower alkylsulfonyl;
   C₂-C₅ lower alkanoyl;
   carboxy;
   C₂-C₅ lower alkoxycarbonyl;
   carbamoyl which may be substituted with hydroxy or cyano,
   R₈₁₅ is hydrogen;
   halogen atom or
   hydroxy,
   its pharmaceutical acceptable salt, hydrate or solvate;
(45) A pharmaceutical composition for prophylaxis or therapy of a postoperative stress according to the above (1), wherein the compound having CRF antagonist activating effect is a compound represented by each of the following formulas 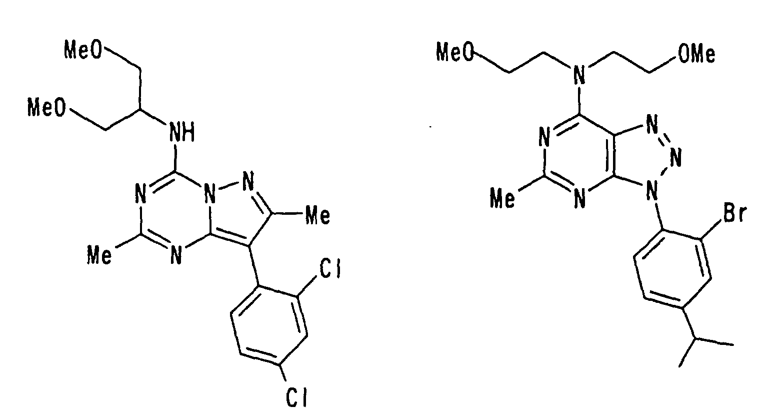 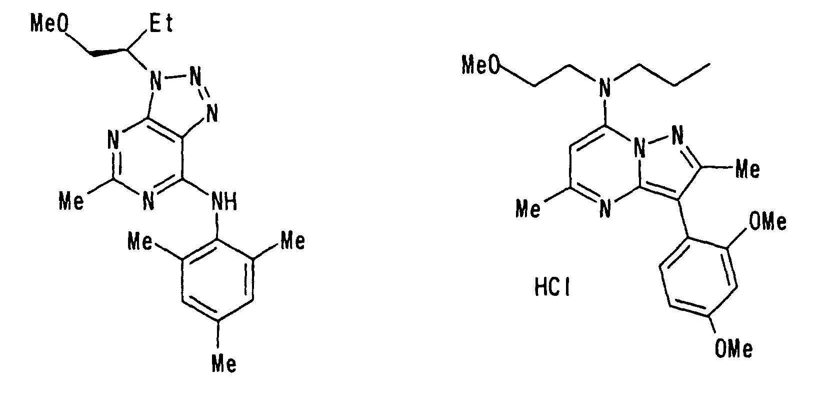 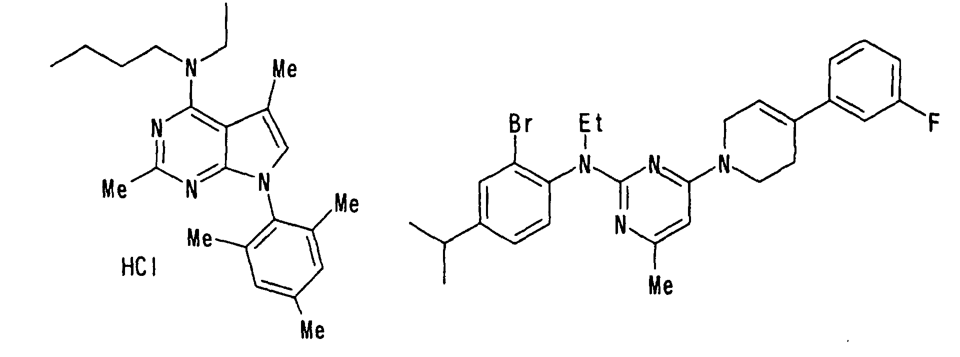 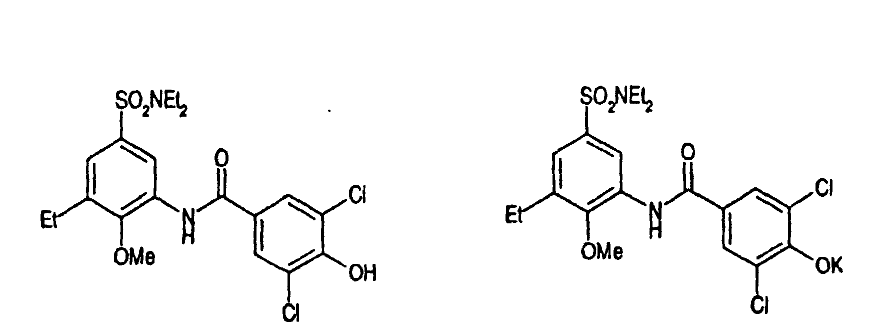 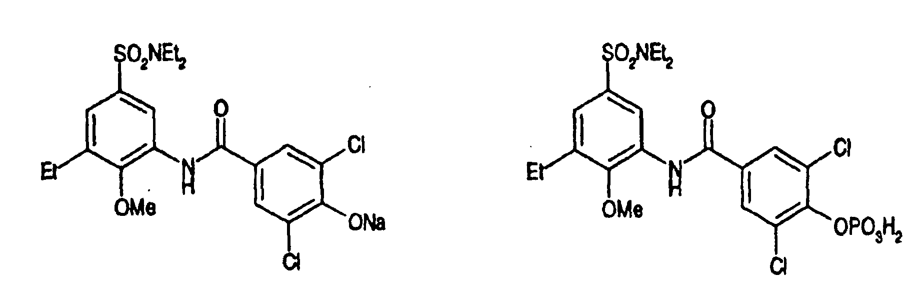 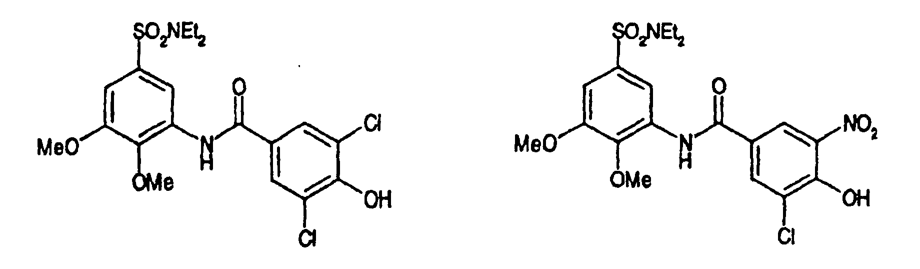 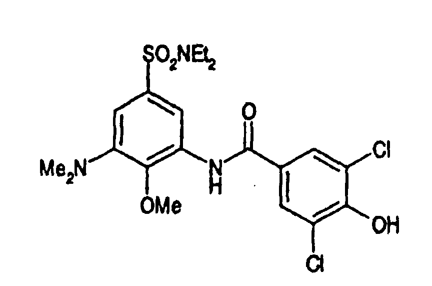 wherein Me and Et are each methyl and ethyl, or its pharmaceutically acceptable salt; and
(46) A pharmaceutical composition for prophylaxis or therapy of a postoperative stress according to the above (1), wherein the compound having CRF antagonist activating effect is a compound represented by one of the following development numbers: DMP-695; DPC-696; SG-058; SK-390; SP904; XQ-041; IL-488; NBI-27728; NBI-29356; NBI-30525; NBI-30775; NBI-34041; NBI-35965; NBI-37582; NGD-98-1 and CRA-0165, or its pharmaceutically acceptable salt.

A compound having CRF antagonist activating effect (abbreviated hereinafter as a CRF antagonist) used in the present invention is preferably a nonpeptidic CRF antagonist, more preferably a 5 to 6 membered nitrogen-containing heterocyclic compound or its fused ring compound having a CRF antagonist activity. The said nitrogen-containing heterocyclic compound may be either a monocycle compound or fused ring compound. As the monocycle compound, for example, a 5 to 6 membered heterocyclic compound containing 1 to 3 nitrogens is preferable. And as the fused ring compound, a fused ring compound, formed by condensing a 6 membered heterocyclic compound containing 1 to 3 nitrogens and a 5 membered heterocyclic compound containing 1 to 2 nitrogens is preferable.

As said nitrogen-containing heterocyclic compound, there may be exemplified an azole compound, a pyridine compound, a pyrimidine compound, or a fused ring derivative consisting of the above specifically exemplified heterocyclic ring and a 5 to 6 membered heterocyclic ring. Concretely, there may be exemplified an azole derivative such as pyrazole, a pyridine derivative, a pyrimidine derivative and a fused ring derivative formed by condensing a 5 to 6 membered heterocyclic compound and azole, pyridine or pyrimidine. As the fused ring derivative including there may be exemplified by a pyrazolopyrimidine derivative, a pyrrolopyrimidine derivative, a benzimidazole derivative and a pyrido- (or pyrimido)- 6,6- (or 6,7-) bicyclo derivative, etc.

To give preferable actual examples, there are compounds shown by the formula (A), (B), (C), (D), (E), (F) or (G).

In the present specification, the meanings of the symbols in the chemical formulae are defined as follows.

As an alkyl, there may be exemplified a straight- or branched-chain alkyl group of C1 to C12, for example, methyl, ethyl, n-propyl, isopropyl, butyl, isobutyl, sec-butyl, tert-butyl, pentyl, isopentyl, hexyl, isohexyl and the like.

As a cycloalkyl, there may be exemplified a cycloalkyl group of C3 to C10 such as, for example, cyclopropyl, cyclobutyl, cyclopentyl, cyclohexyl, cycloheptyl, cyclooctyl, cyclononyl, cyclodecyl and the like.

As an alkoxy, there may be exemplified a straight- or branched-chain alkoxy group of C1 to C12, for example, methoxy, ethoxy, n-propoxy, isopropoxy, n-butoxy, isobutoxy, sec-butoxy, tert-butoxy, n-pentyloxy, isopentyloxy, neopentyloxy, hexyloxy, heptyloxy, octyloxy, nonyloxy, decyloxy, undecyloxy, dodecyloxy and the like.

As an alkenyl, there may be exemplified a straight- or branched-chain alkenyl group of C2 to C10, for example, vinyl, 1-propenyl, 2-butenyl, 2-pentenyl, 1-hexenyl, 1-heptenyl, 1-octenyl, 3-decenyl and the like.

As an alkenyloxy, there may be exemplified a straight-or branched-chain alkenyloxy group of C2 to C10, for example, vinyloxy, 1-propenyloxy, 2-butenyloxy, 2-pentenyloxy, 1-hexenyloxy, 1-heptenyloxy, 1-octenyloxy, 3-decenyloxy and the like.

As an alkylene, there may be exemplified a straight- or branched-chain alkylene group of C1 to C6, for example, methylene, ethylene, propylene, 1-methylpropylene, 2-methyl butylenes, 2-ethylbutylene and the like.

As an alkanoyl, there may be exemplified a straight- or branched-chain alkanoyl group of C1 to C10, for example, formyl, acetyl, propionyl, butyryl, hexanoyl, octanoyl and the like.

When the CRF antagonist (for example, the compound shown by above-mentioned formulae) to be used in the present invention forms a salt and when the said salt is used as a medicine, the said salt is preferably pharmaceutically acceptable. As a pharmaceutically acceptable salt, there may be exemplified by a base addition salt or an acid addition salt, for example, a salt with an inorganic bases, a salt with an organic bases, a salt with an inorganic acids, a salt with an organic acids, a salt with a basic amino acids or an acidic amino acid.

As the salt with an inorganic base, there may be exemplified a salt with an alkali metal such as sodium salt and potassium salt, a salt with an alkaline earth metal such as calcium salt and magnesium salt, aluminum salt and ammonium salt. As the salt with an organic base, there may be exemplified salt with trimethylamine, triethylamine, pyridine, picoline, ethanolamine, diethanolamine, triethanolamine, dicyclohexylamine, N,N-dibenzylethylenediamine or the like. As the salt with an inorganic acid, there may be exemplified a salt with hydrochloric acid, sulfuric acid, phosphoric acid, hydrobromic acid, nitric acid or the like. As the salt with an organic acid, there may be exemplified by a salt with formic acid, acetic acid, malic acid, maleic acid, fumaric acid, oxalic acid, tartaric acid, succinic acid, citric acid, lactic acid, methansulfonic acid, p-toluenesulfonic acid, palmitic acid, salicylic acid, stearic acid or the like. As the salt with a basic amino acid, there may be exemplified salts with arginine, lysine, ornithine and the like. As the salts with acidic amino acid, there may be exemplified a salt with aspartic acid, glutaminic acid or the like.

The CRF antagonist, which is contained in the preparation of the present invention, include the stereoisomers such as cis and trans isomer, racemic mixture and optical isomers such as R(rectus) isomer and S(sinister) isomer. According to the compound, isomers are formed depending upon the conformation, and the said isomers are also included in the present invention. The CRF antagonist may be hydrate thereof or anhydrate thereof.

The CRF antagonist used in the present invention, for example, the compound shown by the above formulae and the salt thereof, etc. can be produced by a known producing method *per se* or a producing method similar to that. For example, the CRF antagonist can be produced according to the production method described in the Japanese Patent Laid-Open No. 13041/1996, Japanese Patent Laid-Open No. 259567/1996, Japanese Patent Laid-Open No. 132528/1997, Japanese Patent Laid-Open No. 188682/1997, Japanese Patent Laid-Open No. 72449/1998, Japanese Patent Laid-Open No. 509725/1995, Japanese Patent Laid-Open No. 509726/1995, Japanese Patent Laid-Open No. 509727/1995, Japanese Patent Laid-Open No. 509728/1995, Japanese Patent Laid-Open No. 500121/1996, Japanese Patent Laid-Open No. 506632/1997, Japanese Patent Laid-Open No. 507249/1997, Japanese Patent Laid-Open No. 507855/1997, Japanese Patent Laid-Open No., especially a method described in the examples thereof or a method similar to that.

The CRF antagonist is useful as an excellent agent for the prophylaxis or/and therapy of a postoperative stress.

The preparation containing the CRF antagonist of the present invention is low toxic, and they can be administered orally or parenterally to a mammal (for example, mouse, rat, hamster, rabbit, cat, dog, bovine, sheep, monkey, human being, etc.) in safety.

The preparation containing the CRF antagonists of the present invention may be any one of solid preparations such as powder, fine grain agents, granules, tablets, capsules, etc.; liquid preparations such as syrups, emulsions, injections, etc.; ointment or suppository. The preparation of the present invention can be produced by usual methods, for example, blending, kneading, granulating, tablet compression, coating, sterilizing treatment or emulsification. Besides, as regards production of the preparation, for example, each clause of the Japanese Pharmacopoeia preparation general rule can be referred.

The content of the CRF antagonist in the preparation of the present invention varies depending upon the form of the preparation. But the said content is usually in a range of 0.01 to 100 wt %, preferably 0.1 to 50 wt %, more preferably 0.5 to 20 wt % per the whole preparation.

The CRF antagonist is used for pharmaceutical formulation as it is or mixed with appropriate pharmaceutically acceptable carrier. The pharmaceutically acceptable carrier includes a wide variety of organic or inorganic carriers which is customarily used, and the carrier is compounded in the pharmaceutical composition as excipient, binder, excipient, lubricant, disintegrant in the solid pharmaceutical preparations, or as solvent, adjuvant, suspending agent, isotonicity-conferring agent and soothing agent in the liquid pharmaceutical preparation. Where necessary, pharmaceutical additive such as a preservative, an antioxidant, a coloring agent, a sweetener, etc. can also be used.

Preferable example of the excipient includes e.g. starch, α-starch, lactose, white sugar, calcium carbonate, calcium phosphate, dextrin, carboxymethyl cellulose sodium, Arabian gum, light anhydrous silicic acid, lower-substituted hydroxypropylmethyl cellulose, etc. Preferable example of the binder includes α-starch, Arabian gum, carboxymethyl cellulose, hydroxypropylmethyl cellulose, crystalline cellulose, impalpable powder cellulose, alginic acid, gelatine, polyvinyl pyrrolidone, etc. Preferable example of the lubricant includes stearic acid, magnesium stearate, calcium stearate, talc, etc. Preferable example of the disintegrant includes lactose, white sugar, carboxymethyl cellulose calcium, etc.

Preferable example of the solvent includes water for injection, physiological saline, ringer solution, alcohol, propylene glycol, polyethylene glycol, etc. Preferable example of the solubilizers includes polyethylene glycol, propylene glycol, D-mannitol, trehalose, etc. Preferable example of the emulsifier includes surfactants such as sodium lauryl sulfate, etc. Preferable example of the isotonicity-conferring agent includes sodium chloride, glycerine, D-mannitol, etc. Preferable example of the soothing agents includes benzyl alcohol, etc.

The dose of the CRF antagonist may be varied depending upon the objects of administration, the route of administration, symptom and age of the patient. For example, when the CRF antagonist is parenterally administered to an adult, a daily dose is 0.005 to 50mg, preferably 0.05 to 10mg, more preferably 0.2 to 4mg per body weight 1kg, in a single dose or in divided doses 2 or 3 times a day.

The preparation of the present invention may contain one kind of CRF antagonist or mixture of not less than two kinds of CRF antagonists. And the preparation containing different kinds of CRF antagonists may be administered at the same time or at regular intervals. And in the preparations of the present invention, other pharmaceutically active ingredients may be contained to an appropriate amount. As the appropriate other pharmaceutically active ingredient, there may be exemplified central nervous system agent (for example imipramine), etc.

Moreover, to explain embodiments of the present invention, Detailed Description of following specifications are incorporated herein by reference in their entireties; Japanese Patent Laid-Open No. 3041/1996(Japanese Patent Application No. 170453/1995), Japanese Patent Laid-Open No. 509728/1995(Japanese Patent Application No. 514191/1994), Japanese Patent Laid-Open No. 507855/1997(Japanese Patent Application No. 501868/1996), Japanese Patent Laid-Open No. 132528/1997(Japanese Patent Application No. 311499/1996), Japanese Patent Laid-Open No. 188682/1997(Japanese Patent Application No. 317763/1996), Japanese Patent Laid-Open No./1998 72449(Japanese Patent Application No. 147738/1997), Japanese Patent Laid-Open No. 509725/1995(Japanese Patent Application No. 514147/1994), Japanese Patent Laid-Open No. 509726/1995(Japanese Patent Application No. 514157/1994), Japanese Patent Laid-Open No. 509727/1995(Japanese Patent Application No. 514158/1994), Japanese Patent Laid-Open No. 506632/1997(Japanese Patent Application No. 506632/1997), Japanese Patent Laid-Open No. 500121/1996(Japanese Patent Application No. 506653/1994), PCT/IB95/00439(W095/33750), PCT/IB97/00918(W098/05661), PCT/IB97/00904(W098/08846), PCT/IB97/00922(W098/08847), Japanese Patent Laid-Open No. 259567/1996(Japanese Patent Application No. 37839/1996), PCT/US99/27054(WO 00/27846), PCT/US99/26984(WO 00/27850), PCT/US98/02932(WO 98/35967), PCT/US97/13072(WO 98/03510), PCT/US94/11050(WO 95/10506), PCT/US97/04852(WO 97/35539), PCT/US98/13913(WO 99/01454), PCT/EP97/00459(WO 97/29109), PCT/US96/09633(WO 96/39400), PCT/JP98/01330(WO 98/42699), Japanese Patent Laid-Open No. 224051/1995(Japanese Patent Application No. 316765/1994), Japanese Patent Application No. 337579/2000.

### Brief Description of Drawings

Fig.1 shows effect of CP-154526 against rise of ACTH concentration by the postoperation stress in mouse.
Fig.2 shows effect of CP-154,526 against rise of corticosterone concentration in plasma by the postoperative stress (visualization of viscera for 30 minutes) .

### Best Mode for Carrying Out the Invention

Hereinafter, the present invention is described in more detail by reference to the Examples, Production Examples and Experiments. But they should not be construed as limiting the invention in any way. And the description can be changed without deviation from the present invention.

### Example 1 Production of injection

N-butyl-N-[2,5-dimethyl-7-(2,4,6-trimethylphenyl)-7H -pyrrolo[2,3-d]pyrimidine-4-yl]-N-ethylamine chloride (abbreviated hereinafter to CP154,526 HCl) 1000mg is dissolved in water to make a 50 ml solution in the whole quantity. The solution is disinfected and filtered by the usual manner. The obtained filtrate is charged in 12cm³ vials, each of the vials containing 1mL the solution, followed by freeze-drying by the usual manner. The freeze-dried product in vials is dissolved by adding the distilled water for injection just before use.

### Example 2 Production of injections

An aqueous solution, which contains CP154,526 HCl 15mg and mannitol 15mg per 1ml of the solution, is prepared. The aqueous solution is disinfected and filtered by the usual manner. The obtained filtrate is charged in 18cm³ vials, each of the vials containing 2mL of the solution. The vials are freeze-dried by the usual manner to obtain freeze-dried vials. The freeze-dried product in vials is dissolved in water for injection just before use.

### Example 3 Production of capsule

| | | |
|---|---|---|
| (1) | CP154,526 HCl | 25mg |
| (2) | lactose | 19mg |
| (3) | impalpable powder cellulose | 10mg |
| (4) | magnesium stearate | 1mg |

(1), (2), (3) and (4) are mixed, and the mixture is charged in the gelatin capsules.

### Example 4 Production of coated tablet

| | | |
|---|---|---|
| (1) | CP154,526 HCl | 10.0mg |
| (2) | lactose | 50.0mg |
| (3) | cornstarch | 35.0mg |
| (4) | hydroxypropylmethyl cellulose | 3.0mg |
| (5) | magnesium stearate | 2.0mg |

Mixture of CP154,526 HCl 10mg, lactose 50.0mg and cornstarch 35.0mg is granulated by using 0.03ml of 10 wt % hydroxypropylmethyl cellulose solution (3.0mg as hydroxypropylmethyl cellulose), followed by drying at 40 °C and sifting. The obtained granules are mixed with magnesium stearate 2.0mg, and the mixture is compressed. The obtained naked tablets are coated with sugar by using aqueous suspension containing sucrose, titanium dioxide, talc and Arabian gum to obtain coated tablets.

### Example 4 Production of tablet

| | | |
|---|---|---|
| (1) | CP154,526 HCl | 10.0mg |
| (2) | lactose | 60.0mg |
| (3) | cornstarch | 40.0mg |
| (4) | soluble starch | 7.0mg |
| (5) | magnesium stearate | 3.0mg |

Mixture of CP154,526 HCl 10mg and magnesium stearate 3.0mg is granulated by using 0.07ml of soluble starch solution (7.0mg as soluble starch), followed by drying at 40 °C and mixing with lactose 60.0mg and cornstarch 40.0mg. The mixture is compressed to obtain tablets.

### Production Example 1

### Synthesis of N,N-diethyl-3-ethyl-4-hydroxy-5-nitrobenzenesulfonamide

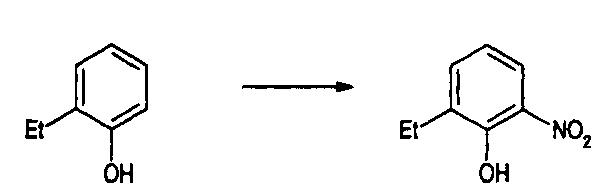

(1) Sodium nitrate (20.9g), concentrated hydrochloric acid (80ml) and fuming nitric acid (0.2ml) were added under cooling with ice to a solution of 2-ethylphenol (30.0g) in the mixed solvent of diethylether (400ml) and water (400ml), and the mixture was stirred at room temperature overnight before the organic layer was extracted. And the aqueous layer was extracted with ethyl acetate. The combined organic extracts were washed with water and saturated sodium chloride aqueous solution and dried over sodium sulfate. After drying, the combined extracts were concentrated in vacuo. The residue was purified by silica gel column chromatography (developing solvent; ethyl acetate: hexane = 1: 50) to give 2-ethyl-6-nitrophenol as a yellowish oily compound (17.9g, in 44% yield). 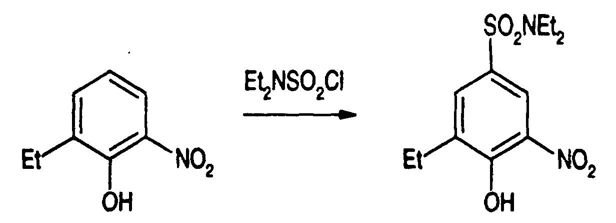
(2) Mixture of diethyl amine (41.0ml) and triethyl amine (31.0ml) was dropwise added under cooling with ice to solution of sulfuryl chloride (49.0ml) in chloroform (300ml). After adding dropwise, the mixture was stirred at room temperature for 3hrs, and the reaction mixture was added to ice water (600ml). And the organic layer was extracted, washed with 10% of hydrochloric acid, water and saturated sodium chloride aqueous solution, and dried over sodium sulfate. After drying, the organic layer was concentrated in vacuo to give diethylsulfamoyl chloride (41.7g, in 61% yield) as a yellowish oily compound.

Next, diethylsulfamoiyl chloride (5.1g) obtained in above-mentioned (1) and aluminum chloride (8.8g) were added under cooling with ice to a solution of 2-ethyl-6-nitrophenol (5.0g) obtained in above-mentioned process in nitrobenzene (6.5ml), and the mixture was stirred at 90°C for 6hrs and at room temperature still for 12hrs. The reaction mixture was dissolved in ethyl acetate, the solution was poured in the ice water, and the organic layer was extracted. Also, the aqueous layer was extracted with ethyl acetate, and the combined organic extracts were washed with water and saturated sodium chloride aqueous solution, dried over sodium sulfate and concentrated in vacuo to give the residue. The obtained residue was purified by silica gel column chromatography (developing solvent; ethyl acetate: hexane = 1:10 to 1: 5) to give the title compound as a yellowish solid (8.15g, in 90% yield).

### Production Example 2

### Synthesis of 2,3-dimethoxy-5-(diethylsulfamoyl)-benzoicacid

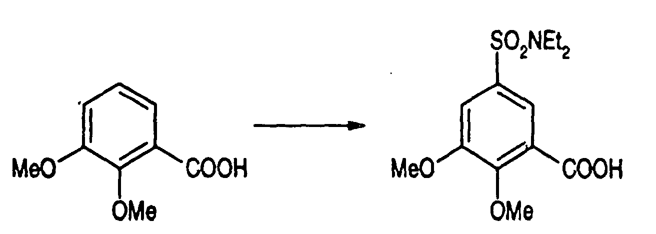

To 2,3-dimethoxybenzoic acid (4.47g) was added chlorosulfonic acid (15ml). The mixture was stirred at room temperature overnight, and to the reaction mixture was added water and the deposited crystals were obtained by filtration. The crystals were washed with water, dried in vacuo to give the crude product as light red powders. Diethyl amine (10ml) was added under cooling with ice to a solution of the crude product in tetrahydrofuran (50ml), and the mixture was stirred at room temperature overnight, 1N Hydrochloric acid was added to acidify the mixture, and the mixture was extracted with ethyl acetate. The organic layer was washed with water and saturated sodium chloride aqueous solution, and dried over sodium sulfate. The obtained residue was concentrated in vacuo to give the title compound as a light brown solid (4.44g, the crude product).

### Production Example 3

### Synthesis of 3,5-dichloro-4-(benzyloxy)benzoyl chloride

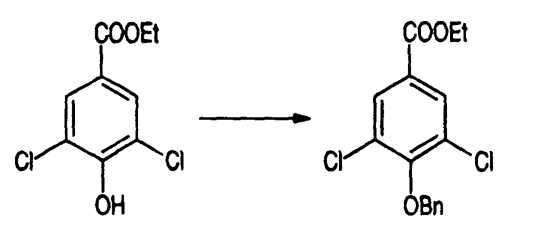

(1) To a solution of ethyl 3,5-dichloro-4-hydroxybenzoate (50.0g) in dimethylformamide (300ml) were added potassium carbonate (55.2g) and benzylbromide (50ml). The mixture was stirred at 70°C overnight and concentrated. To the reaction mixture was added water to deposit crystals. The crystals were collected by filtration, washed with water, and dried in vacuo to give ethyl 3,5-dichloro-4-(benzyloxy)benzoate (68.9g,quantitative) as white crystals. 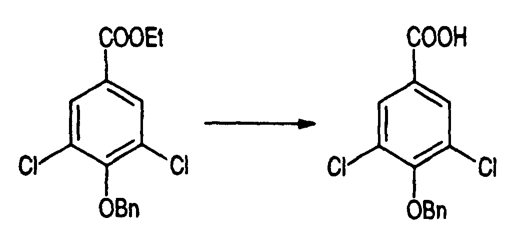
(2) To a solution of the compound (68.9g) obtained in the above-mentioned (1) in a mixture of tetrahydrofuran (400ml) and methanol (400ml) was added 4N lithium hydroxide aqueous solution (200ml). And the solution was stirred at room temperature overnight. After that, hydrochloric acid was added to acidify the reaction mixture. And the deposited crystals were collected by filtration. The obtained crystals were washed with water, dried in vacuo to give 3,5-dichloro-4-(benzyloxy)benzoic acid (62.9g, quantitative) as white crystals. 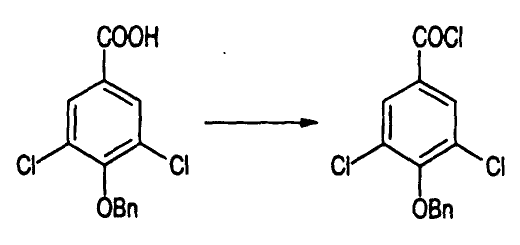
(3) Oxalyl chloride (28ml) and dimethylformamide(0.5ml) were added under cooling with ice to a solution of the compound (62.9g) obtained in the above-mentioned (2) in dichloromethane (400ml), and the mixture was stirred at room temperature overnight. The reaction mixture was concentrated in vacuo, made azeotropy with toluene, and dried in vacuo to give the title compound as a light yellowish solid (66.8g, the crude product).

### Production Example 4

### Synthesis of 3-chloro-4-hydroxy-5-nitrobenzoylchloride

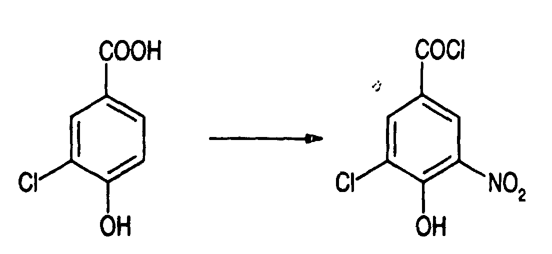

(1) Fuming nitric acid (3ml) was added under cooling with ice to a solution of 3-chloro-4-hydroxybenzoic acid 1/2hydrate (4.06g) in acetic acid (30ml). And the mixture was stirred at room temperature for 1.5 hrs. After that, to reaction mixture was added ice water to deposit crystals. The deposited crystals were collected by filtration. The obtained crystals were washed with water, dried in vacuo to give 3-chloro-4-hydroxy-5-nitrobenzoic acid (4.27g, crude product) as light yellowish solid. 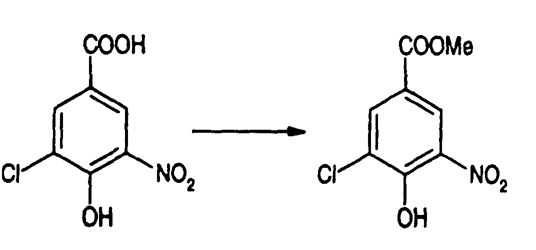
(2) To a solution of the crude product (4.27g) obtained in the above-mentioned (1) in methanol (50ml) were added dimethylaminopyridine (256mg) and WSC-HCl (4.53g). And the mixture was stirred at room temperature overnight, concentrated in vacuo, dissolved in chloroform, washed with water and saturated sodium chloride aqueous solution, and dried over sodium sulfate. After that, the dried product was concentrated in vacuo to give the crude residue. The crude residue was purified by silica gel column chromatography (developing solvent; ethyl acetate: hexane = 1: 2 to 1: 1) to give methyl 3-chloro-4-hydroxy-5-nitrobenzoate (1.02g, in 20% yield) as yellowish solid. 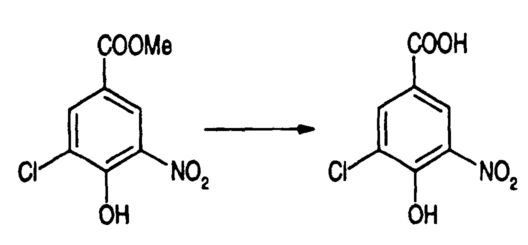
(3) To a solution of the compound (1.02g) obtained in the above-mentioned (2) in a mixture of tetrahydrofuran (20ml) and methanol (20ml) was added 4N lithium hydroxide solution (5ml). The solution was heated to reflux overnight. After that, to the reaction mixture was added hydrochloric acid to acidify the mixture. The deposited crystals were collected by filtration, washed with water, and dried in vacuo to give 3-chloro-4-hydroxy-5-nitrobenzoic acid (876mg, in 91% yield) as light yellowish solid. 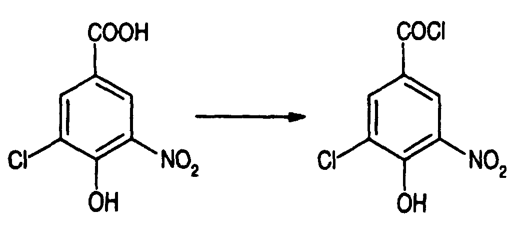
(4) To a solution of the compound (500mg) obtained in the above-mentioned (3) in toluene (3ml) was added thionyl chloride (1ml). And the solution was stirred at 100°C overnight. After that, the reaction mixture was concentrated in vacuo, made azeotropy with toluene, and dried in vacuo to give the title compound as brown solid (541mg, crude product).

### Production Example 5

### Synthesis of N,N-diethyl-3-(dimethylamino)-4-methoxy-5-nitrobenzene-sulfonamide

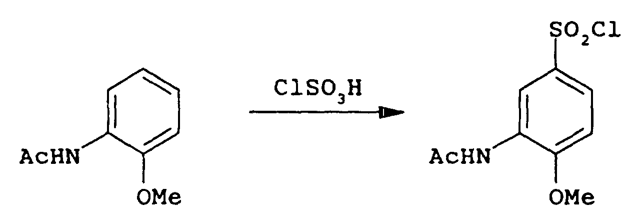

(1) Chlorosulfonic acid (80ml) was dropwise added under cooling with ice to o-acetoanisidide(20.0g). And the mixture was stirred at room temperature overnight. After that, to an ice water was added reaction mixture dropwise to deposit crystals. The deposited crystals were collected by filtration, washed with water and dried in vacuo to give 3-(acetylamino)-4-methoxybenzensulfonyl chloride (29.8g, in 93% yield) as light peachy crystals. 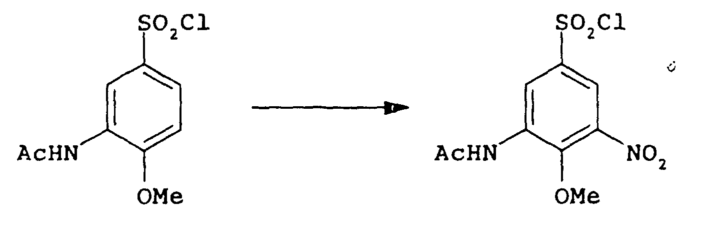
(2) The compound (29.8g) obtained in the above-mentioned (1) was dissolved under cooling with ice in concentrated sulfuric acid (200ml), and to the solution was added the mixture of fuming nitric acid (9.5ml) and concentrated sulfuric acid (19ml) dropwise. And the mixture was stirred under cooling with ice for 30 minutes. To ice water was added this reaction mixture dropwise. And then the reaction mixture was extracted with ethyl acetate, washed with water and saturated sodium chloride aqueous solution, and dried over sodium sulfate. The obtained residue was concentrated in vacuo to give 3-(acetylamino)-4-methoxy-5-nitrobenzensulfonyl chloride (32.6g) as brown oily crude product. 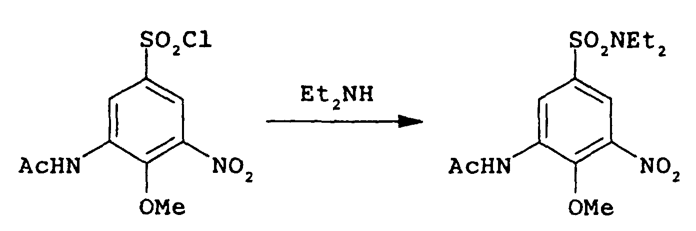
(3) Diethylamine (25.7ml) was dropwise added under cooling with ice to a solution of the crude product (32.6g) obtained in the above-mentioned (2) in tetrahydrofuran (350ml). And the mixture was stirred at room temperature for 40 minutes. The reaction mixture was concentrated in vacuo. And then the residue was dissolved in chloroform, washed with water and saturated sodium chloride aqueous solution, and dried over sodium sulfate. The obtained crude residue was concentrated in vacuo, washed with ethyl acetate slurry wise, filtered, and dried in vacuo to give N,N-diethyl-3-(acetylamino)-4-methoxy-5-nitrobenzensulfonam ide (27.6g, in 71% yield) as light yellowish crystals. 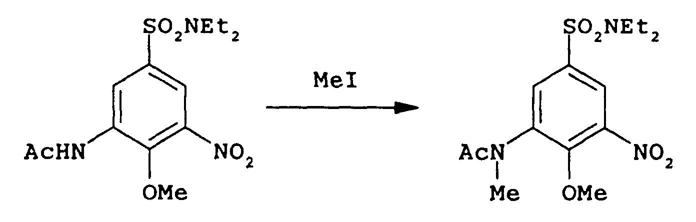
(4) Methyl iodide (85µl) and sodium hydride (44mg) were added under cooling with ice to a solution of the compound (315mg) obtained in the above-mentioned (3) in dimethylformamide (4ml). The mixture was stirred at room temperature for 1hr. After that, to the solution was added 10% citric acid aqueous solution. And the reaction mixture was extracted with ethyl acetate. The organic layer was washed with water and saturated sodium chloride aqueous solution, dried over sodium sulfate, and concentrated in vacuo. The obtained residue was purified by silica gel column chromatography (developing solvent; ethyl acetate: hexane = 1: 3) to give N,N-diethyl-3-(N'-acetyl-N'-methylamino)-4-methoxy-5-nitrob enzenesulfonamide (271mg, in 83% yield) as light yellowish solid. 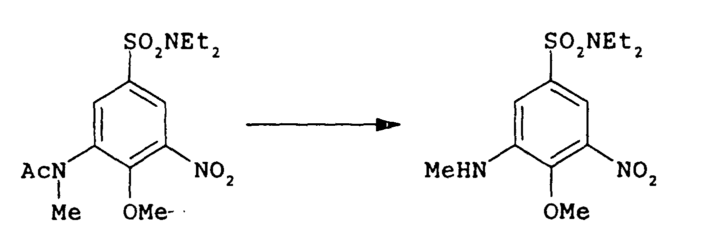
(5) To a solution of the compound (271mg) obtained in the above-mentioned (4) in the mixture of ethanol (5ml) and water (1.3ml) was added 6N hydrochloric acid (1.3ml). The solution was heated to reflux for 6hrs. After cooling by standing at room temperature, the reaction mixture was concentrated in vacuo and extracted with chloroform after adding saturated sodium bicarbonate aqueous solution. The organic layer was washed with 0.1N sodium hydroxide aqueous solution, water and saturated sodium chloride aqueous solution, dried over sodium sulfate, filtered and concentrated in vacuo. The obtained residue was purified by silica gel column chromatography (developing solvent; ethyl acetate: hexane = 1: 1) to give N,N-diethyl-3-(methylamino)-4-methoxy-5-nitrobenzenesulfona mide (195mg, in 82% yield) as yellowish oil. 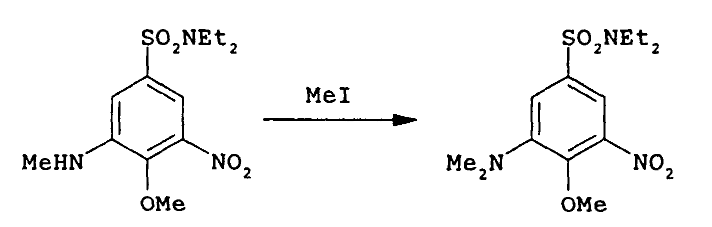
(6) Methyl iodide (54µl) and sodium hydride (28mg) were added under cooling with ice to a solution of the compound (183mg) obtained in the above-mentioned (5) in dimethylformamide (4ml), and the mixture was stirred at room temperature for 30 minutes. After that, to the solution was added 10% citric acid aqueous solution, and the reaction mixture was extracted with ethyl acetate. Then the extract was washed with water and saturated sodium chloride aqueous solution, dried over sodium sulfate, filtered, and concentrated in vacuo. The obtained residue was purified by silica gel column chromatography (developing solvent; ethyl acetate: hexane = 1: 3) to give the title compound as yellowish oil (148mg, in 77% yield).

As described above, the compounds obtained in Production Example 1 to 5 were shown in table 1 as follow. By the way, Me means methyl group, Et means ethyl group and Bn means benzyl group, respectively, in Production Examples 1 to 5 and table 1.

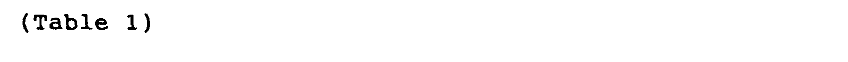

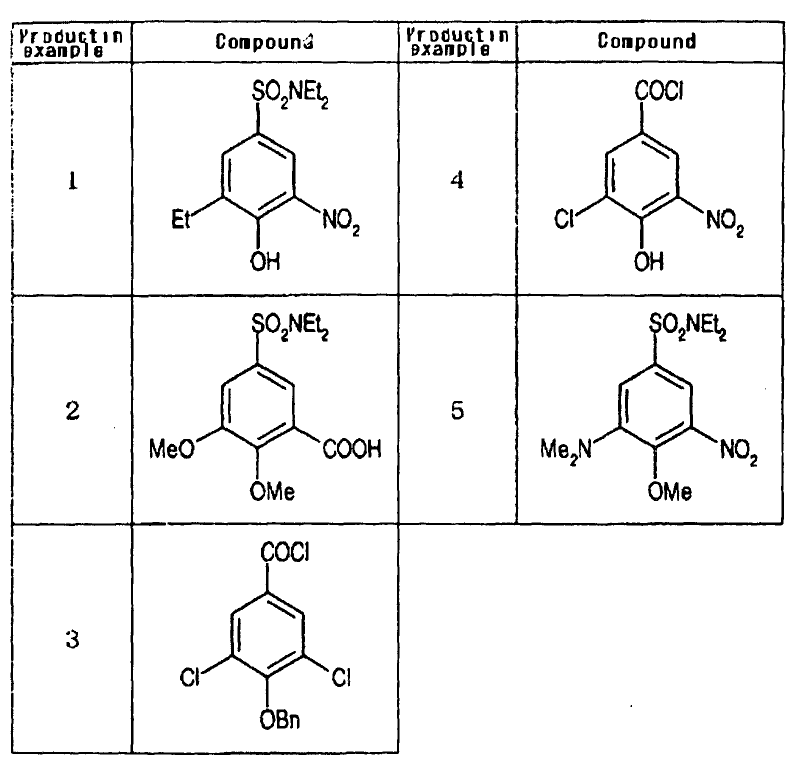

### Example 6

### Synthesis of 3,5-dichloro-N-(3-ethyl-5-diethylsulfamoyl-2-methoxyphenyl)-4-hydroxybenzamide

### Process (1)

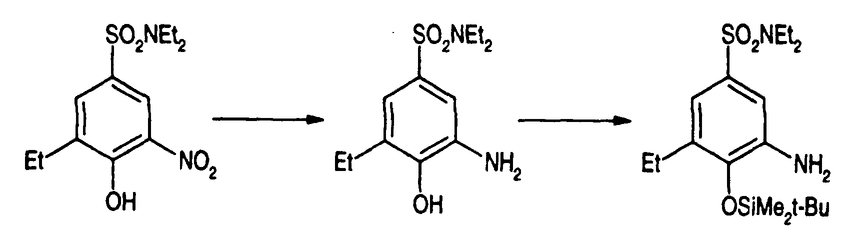

(1) To a solution of the compound (12.5g) obtained in the above-mentioned Production Example 1 in tetrahydrofuran (130ml) was added 7.5% palladium carbon (2.0g) under argon gas. The solution was subjected to 1 atmospheric hydrogen gas at room temperature, and the mixture was stirred overnight. The reaction mixture was filtered through cerite and concentrated in vacuo. The obtained residue was purified by silica gel column chromatography (developing solvent; ethyl acetate: hexane = 1: 1) to give the title compound as brown solid (11.3g, quantitative).
(2) To a solution of the compound (6.04g) obtained in the above-mentioned (1) in dimethylformamide (120ml) were added imidazole (4.98g) and tert-butyldimethylsilyl chloride (9.69g). The mixture was stirred at room temperature overnight. To the reaction mixture was added water, and the reaction mixture was extracted with ethyl acetate, washed with water and saturated sodium chloride aqueous solution, and dried over sodium sulfate. After drying, the obtained residue was purified by silica gel column chromatography (developing solvent; ethyl acetate: hexane = 1: 6) to give the compound as light yellowish solid (7.88g, in 92% yield).

### Process (2)

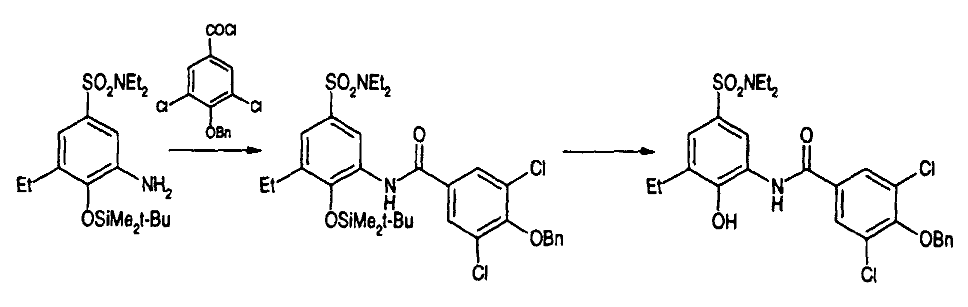

(1) Pyridine (1.96ml) and the crude product (8.40g) obtained in the above-mentioned Production Example 3 were added under cooling with ice to a solution of the compound (7.84g) obtained in the above-mentioned Process (1) in chloroform (150ml), and the mixture was stirred at room temperature overnight. To the reaction mixture was added water, and the organic layer was extracted. Also, the aqueous layer was extracted with chloroform, and the combined organic extracts were washed with water, 10% citric acid aqueous solution, water and saturated sodium chloride aqueous solution sequentially, dried over sodium sulfate and concentrated in vacuo to give the crude product.
(2) To a solution of the crude product obtained in the above-mentioned (1) in dimethylformamide (140ml) was added potassium carbonate (14.0g), and the mixture was stirred at 60°C for 2.5 hrs. To the reaction mixture was added 1N hydrochloric acid to acidify, and reaction mixture was extracted with ethyl acetate. The organic layer was washed with water and saturated sodium chloride aqueous solution, dried over sodium sulfate, and concentrated in vacuo. The thus obtained residue was purified by silica gel column chromatography (developing solvent; ethyl acetate: hexane = 1: 6) to give the compound as light yellowish solid (9.39g, in 84% yield).

### Process (6)

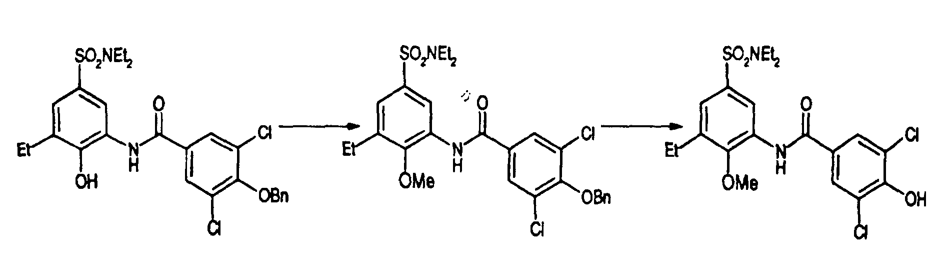

(1) To a solution of the compound (3.04g) obtained in the above-mentioned Process (2) in dimethylformamide (30ml) were added potassium carbonate (912mg) and methyl iodide (0.52ml), and the mixture was stirred at 60°C for 4 hrs. To the reaction mixture was added water, and the reaction mixture was extracted with ethyl acetate. The organic layer was washed with water and saturated sodium chloride aqueous solution, dried over sodium sulfate, and concentrated in vacuo. The obtained residue was purified by silica gel column chromatography (developing solvent; ethyl acetate: hexane = 1: 4) to give the compound as light yellowish solid (2.26g, in 73% yield).
(2) To a solution of the compound (2.24g) obtained in the above-mentioned (1) in tetrahydrofuran (30ml) was added 7.5% palladium carbon (0.23g) under argon gas. The mixture was subjected to 1 atmospheric hydrogen gas at room temperature, and stirred for 1 hr. The reaction mixture was filtered through cerite, the filtrate was concentrated in vacuo, and the obtained residue was recrystallized by ethyl acetate- hexane to give the title compound as white crystals (1.77g, in 94 % yield).

### Example 7

### Synthesis of potassium 2,6-dichloro-4-(N-(3-ethyl-5-diethylsulfamoyl-2-methoxyphenyl)carbamoyl)phenoxide

### Process (7)

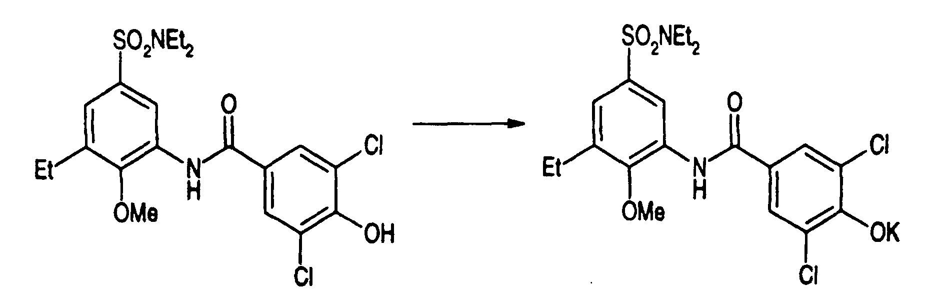

To a solution of the compound (217mg) obtained in the above-mentioned Example 6 in a mixed solvent of tetrahydrofuran (6ml) and water (4ml) was added potassium hydrogen carbonate (46mg). The mixture was stirred at room temperature for 1 hr, and the reaction mixture was concentrated in vacuo, and dried to give the title compound (234mg, quantitative) as a white amorphous compound.

### Example 8

### Synthesis of sodium 2,6-dichloro-4-(N-(3-ethyl-5-diethylsulfamoyl-2-methoxyphenyl)carbamoyl)phenoxide

### Process (7)

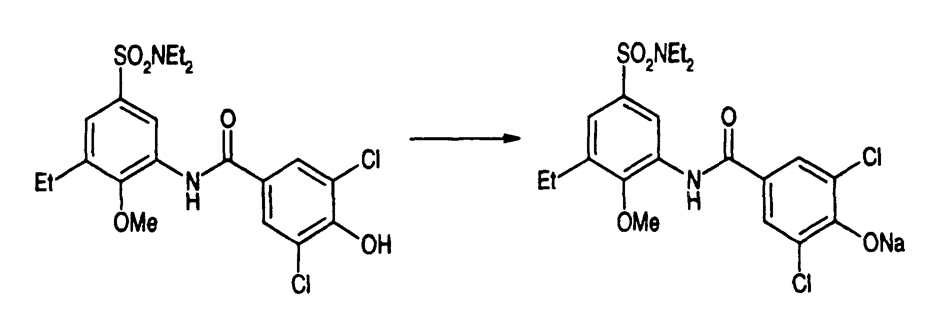

To a solution of the compound (71mg) obtained in the above-mentioned Example 6 in a mixed solvent of tetrahydrofuran (6ml) and water (4ml) was added sodium hydrogen carbonate (12mg). The mixture was stirred at room temperature for 1 hr, and the reaction mixture was concentrated in vacuo, and dried to give the title compound (74mg, quantitative) as a white amorphous compound.

### Example 9

### Synthesis of phosphate 2,6-dichloro-4-(N-(3-ethyl-5-diethylsulfamoyl-2-methoxyphenyl)carbamoyl)phenylphosphate

### Process (7)

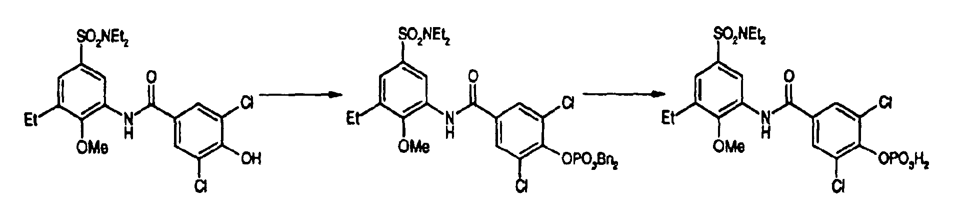

(1) To a solution of the compound (74mg) obtained in the above-mentioned Example 6 in tetrahydrofuran (5ml) were added sodium hydride (20mg) and tetrabenzylpyrophosphate (171mg). The mixture was stirred at room temperature overnight. To the reaction mixture was added water, and the reaction mixture was extracted with ethyl acetate. The organic layer was washed with water and saturated sodium chloride aqueous solution, dried over sodium sulfate, concentrated in vacuo. The obtained residue was purified by preparative thin-layer chromatography (PTLC) (developing solvent; ethyl acetate: hexane = 1: 1) to give the compound as light yellowish oil (7.88g, in 61% yield).
(2) To a solution of the compound (66mg) obtained in the above-mentioned (1) in tetrahydrofuran (5ml) was added 7.5% palladium carbon (7mg) under argon gas. The mixture was subjected to 1 atmospheric hydrogen gas at room temperature, and stirred for 30 minutes. The reaction mixture was filtered through cerite, and concentrated in vacuo. The obtained residue was recrystallized from chloroform- ethyl acetate- hexane to give the title compound as light peachy solid (34mg, in 68% yield).

### Example 10

### Synthesis of 3,5-dichloro-N-(5-diethylsulfamoyl-2,3-dimethoxyphenyl)-4-hydroxybenzamide

### Process (3)

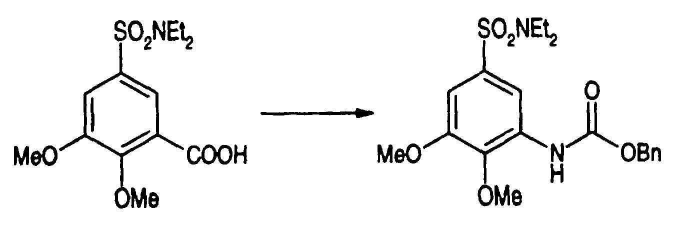

To a solution of the crude product (4.44g) obtained in the above-mentioned Production Example 2 in toluene (30ml) were added triethylamine (2.2ml) and diphenylphosphorylazide (3.3ml). The solution was heated to reflux for 2hrs. After cooling by standing the mixture at room temperature, to the mixture was added benzyl alcohol (1.91ml), and the solution was heated to reflux overnight. After cooling at the room temperature, to the reaction mixture was added water, and the reaction mixture was extracted with ethyl acetate. The organic layer was washed with 1N sodium hydroxide solution, water, and saturated sodium chloride aqueous solution sequentially, dried over sodium sulfate, concentrated in vacuo, and the obtained residue was purified by silica gel column chromatography (developing solvent; ethyl acetate: hexane = 1: 4) to give the compound as light yellowish oil(4.58g, in 20% yield).

### Process (4)

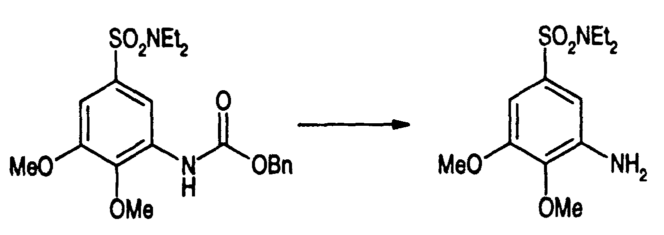

To a solution of the compound (1.67g) obtained in the above-mentioned Process(3) in ethyl acetate (30ml) was added 10% palladium carbon (162mg) under argon gas. The solution was subjected to 1 atmospheric hydrogen gas at room temperature and stirred for 3 hrs, and the reaction mixture was filtered through cerite, and the filtrate was concentrated in vacuo to give the compound as white solid (995mg, in 87% yield).

### Process (2)

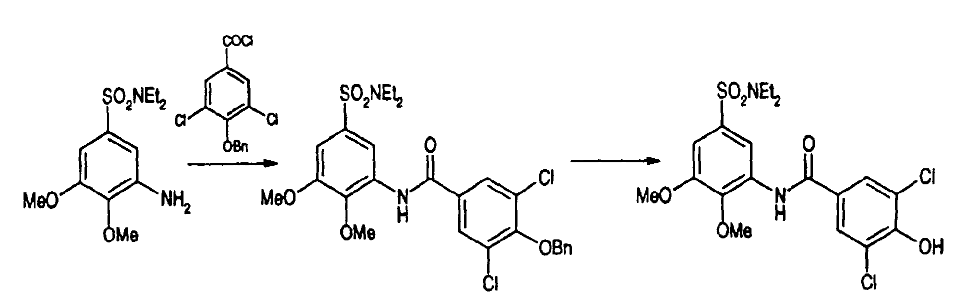

(1) Pyridine (0.15ml) and the crude product (257mg) obtained in the above-mentioned Production Example 3 were added under cooling with ice to a solution of the compound (179mg) obtained in the above-mentioned Process (4) in dichloromethane (15ml). The mixture was stirred at room temperature overnight. To the reaction mixture was added water, and the organic layer was extracted. Also, the aqueous layer was extracted with chloroform, and the combined extracts were washed with 10% citric acid aqueous solution, water, 1N sodium hydroxide solution and saturated sodium chloride aqueous solution, dried over sodium sulfate, and concentrated in vacuo. The obtained residue was purified by silica gel column chromatography (developing solvent; ethyl acetate: hexane = 1: 5 to 1: 2) to give the compound as white solid (287mg, in 82% yield).
(2) To a solution of the compound (284mg) obtained in the above-mentioned Process (1) in tetrahydrofuran (30ml) was added 10% palladium carbon (62mg) under argon gas. The solution was subjected to 1 atmospheric hydrogen gas at room temperature and stirred for 10 minutes, and the reaction mixture was filtered through cerite, and the filtrate was concentrated in vacuo to give the compound as white solid (223mg, in 93% yield).

### Example 11

### Synthesis of 3-chloro-N-(5-diethylsulfamoyl-2,3-dimethoxyphenyl)-4-hydroxy-5-nitrobenzamide

### Process (2)

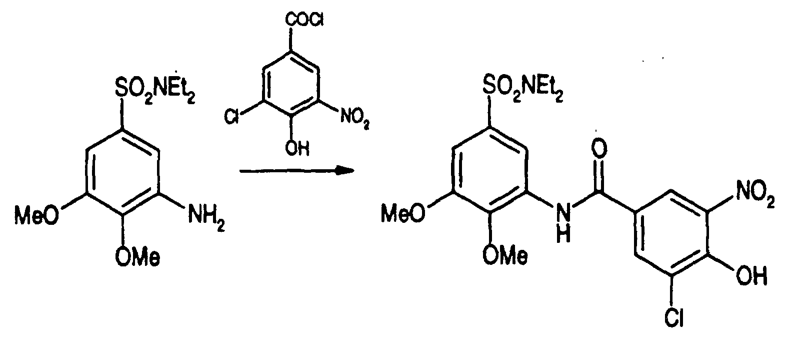

To a solution of the compound (661mg) obtained in the above-mentioned Process (4) in Example 10 in chloroform (10ml) was added the crude product (541mg) obtained in the above-mentioned Production Example 4. The mixture was heated to reflux for 2hrs. The reaction mixture was diluted with chloroform, washed with water and saturated sodium chloride aqueous solution, dried over sodium sulfate, and concentrated in vacuo. The obtained residue was recrystallized from ethyl acetate- hexane to give the title compound as light yellowish crystals (986mg, in 88% yield).

### Example 12

### Synthesis of 3,5-dichloro-N-(5-diethylsulfamoyl-3-(dimethylamino)-2-methoxyphenyl)-4-hydroxybenzamide.

### Process (1)

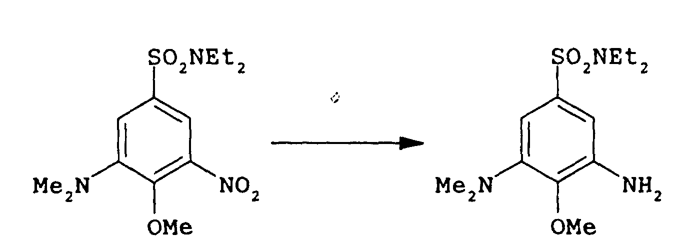

(1) To a solution of the compound (148mg) obtained in the above-mentioned Production Example 5 in tetrahydrofuran (5ml) was added 7.5% palladium carbon (15mg) under argon gas. The mixture was subjected to 1 atmospheric hydrogen gas at room temperature, and stirred for 24 hrs. The reaction mixture was filtered through cerite, and the filtrate was concentrated in vacuo to give crude reaction product as achromatic oil(156mg).

### Process (2)

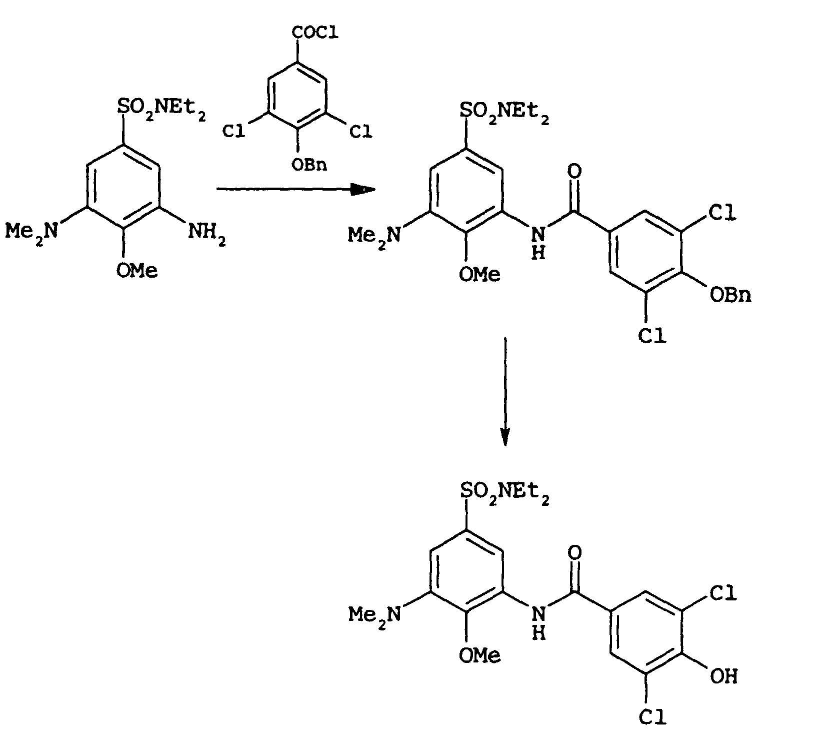

(1) Pyridine (0.45ml) and the crude product (672mg) obtained in the above-mentioned Production Example 3 were added under cooling with ice to a solution of the compound (540mg) obtained in the above-mentioned Process (1) in dichloromethane (50ml). The mixture was stirred at room temperature overnight. To the reaction mixture was added saturated sodium bicarbonate aqueous solution, and the organic layer was extracted. Also, the aqueous layer was extracted with chloroform, and the combined organic extracts were washed with 10% citric acid aqueous solution, water, saturated sodium bicarbonate aqueous solution and saturated sodium chloride aqueous solution sequentially, dried over sodium sulfate, filtered, and concentrated in vacuo. The obtained residue was purified by silica gel column chromatography (developing solvent; ethyl acetate: hexane = 1: 3) to give the achromatic amorphous compound (1.01g, in 97% yield).
(2) To a solution of the compound (1.01g) obtained in the above-mentioned (2) in toluene (20ml) was added trifluoroacetic acid (20ml). The mixture was stirred at 80°C for 2 hrs. The reaction mixture was concentrated in vacuo, and made azeotropy with toluene. The obtained residue was recrystalized from ethyl acetate- hexane to give the title compound as white solid (812mg, in 95% yield).

As described above, these compounds obtained in Example 6 to 12 are shown in Table 2 and 3.

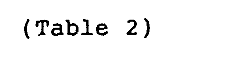

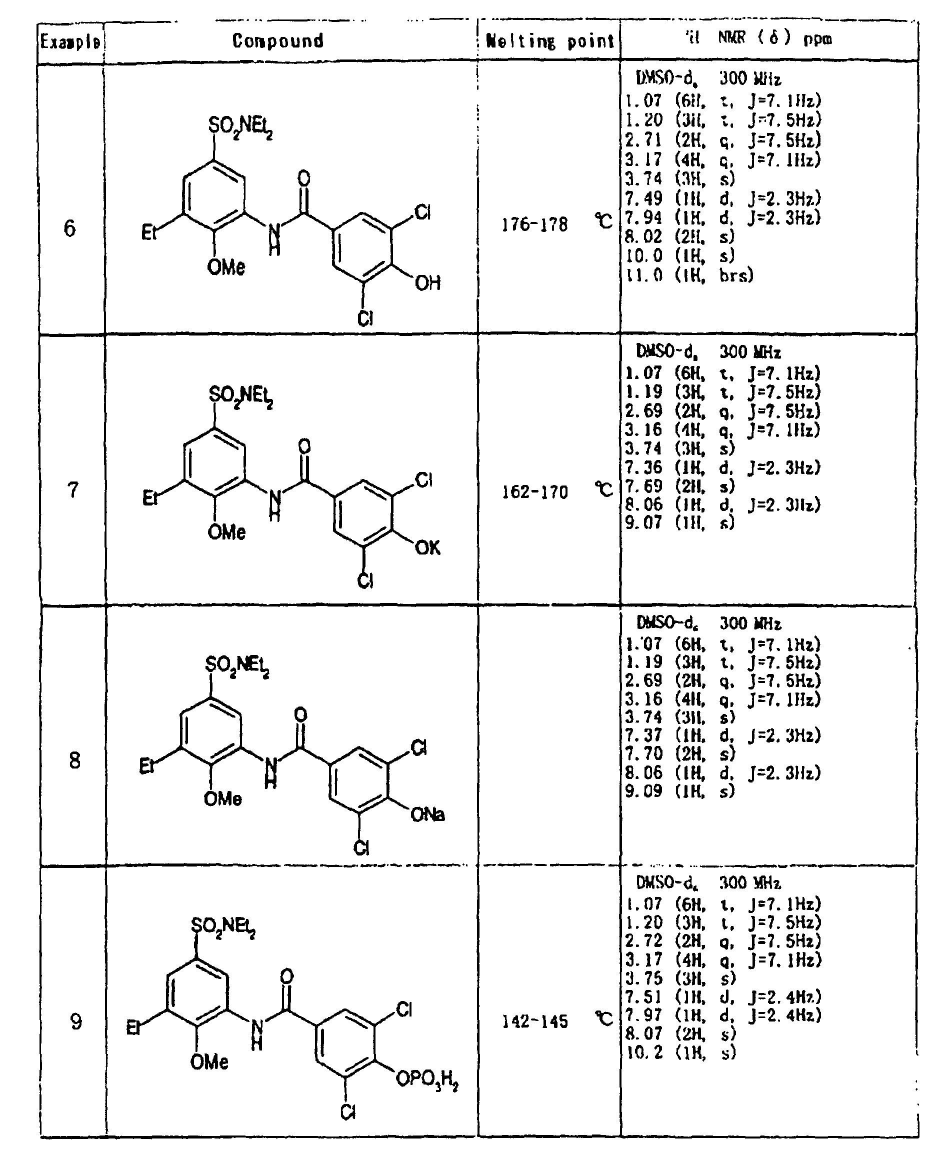

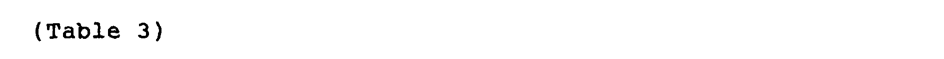

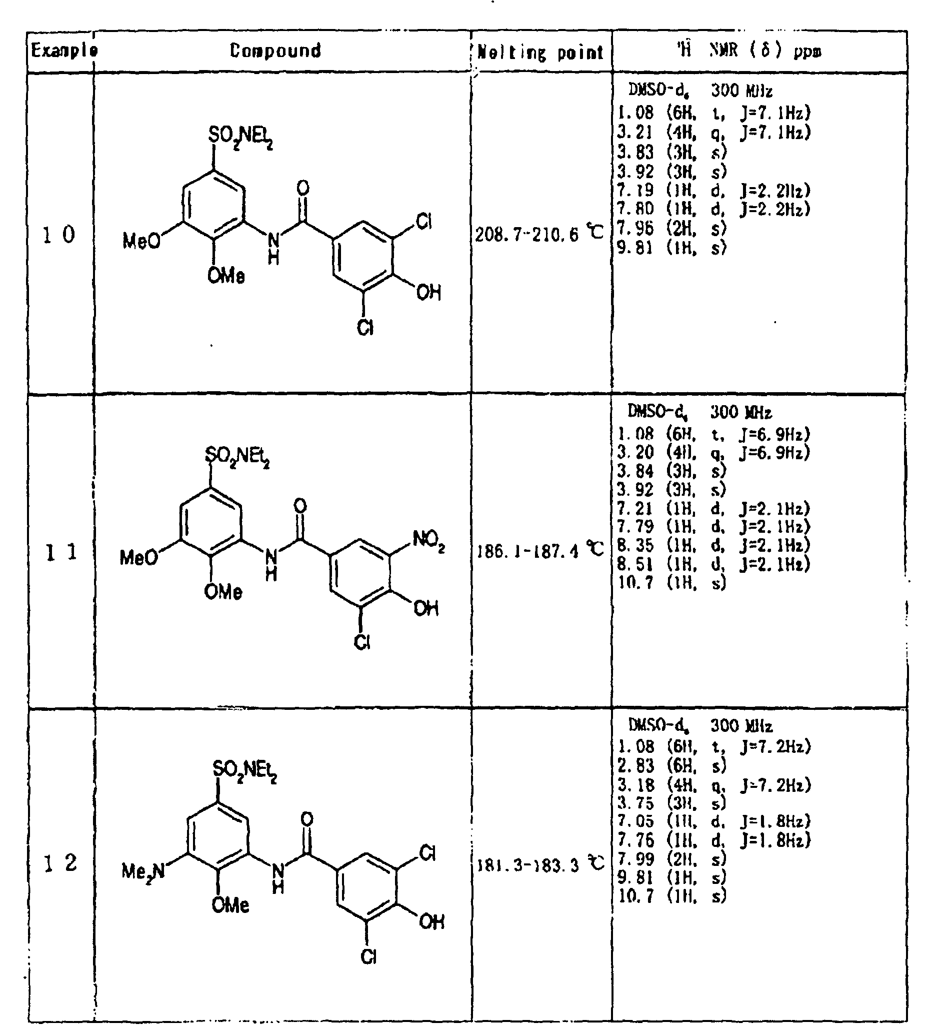

The experiment results carried out regarding CRF receptor antagonism of compounds in the present invention are shown as follow.

### (Experiment)

### Experiment 1 : in vitro Binding Assay using membrane fraction of recombinant cell (6EF2B) with expression of CRF receptor gene

### (SPA method)

### Preparation of membrane fraction:

Recombinant cells 6EF2B (derived from Hela cell), which express CRF type I(CRF-I) receptor gene stably, were cultivated in Eagle's MEM medium(10%FCS, 400ug/ml G418.1/100 penicillin/streptomycin). After cultivation, cells were suspended in 50mM Tris-HCl (pH7.2) solution containing 1mM EDTA and incubated at 25°C for 15 to 30 minutes. After that, every step was carried out at 4°C. Treated cells were suspended in 50mM Tris-HCl(pH7.2) containing 10% sucrose and homogenized by a Polytron(PT10-35 model). This step of homogenization was carried out at 25,000 rpm for 15 to 20 seconds and repeated twice or three times. In the course of this step the homogenates were inspected under a microscope. Homogenates were centrifuged at 600 x g for 5 minutes and the supernatant was centrifuged again at 40,000 x g for 30 minutes. The obtained pellet was resuspended in 50mM Tris-HCl(10mM MgCl₂, 2mM EGTA) and the suspension was centrifuged again at 40,000 x g for 30minutes. The obtained pellet was used for further experiments as membrane fraction. The membrane fraction was diluted with 50mM Tris-HCl(10mM MgCl₂, 2mM EGTA, 0.1%BSA, 100 kallikrein units/ml aprotinin, 0.1mM bacitracin, pH7.2) to have the concentration of 1mg/ml and then stored at -80°C. DC protein assay kit (produced by Bio-RAD) was used to determine the protein concentration of the membrane fraction.

### Binding Assay using CRF-I receptor by means of SPA method:

Compounds to be evaluated were stored at the concentration of 10mM in DMSO. The stored solution was diluted with DMSO to have the concentration of 1mM and diluted again with assay buffer to have the appropriate concentration just before experiments (The final concentration of DMSO was not more than 1%, at which DMSO did not cause adverse effect on measurement.). The assay buffer consisted of 50mM Tris-HCl (pH7.7) containing 100mM NaCl, 5mM KCl, 2mM CaCl₂, 0.1% BSA, 0.1% bacitracin, 0.05% FCS and 5% horse serum. The reactions were carried out in 96-well Optiplate(Packard) in which to 50ul of the test substance solution, were added 50ul of WGA-beads & membrane fraction (50 to 100ug membrane fraction/10mg beads/ml) pre-coupled by stirring (rotating with 10rpm) at room temperature for 2 hours on the day of experiment, and 100ul of 0.4nM ¹²⁵I-Tyr⁰-human/rat CRF(NEX-216, Dupont NEN), to be made up 200ul as total volume, and the mixture was incubated at 25°C for 3 hours. By the way, the step of pre-coupling was carried out by using beads solution (20mg/ml) suspended in 50mM Tris-HCl buffer solution (pH7.2) and after stirring beads were washed twice with Tris-HCl buffer and once with assay buffer by centrifugation (3000 rpm, 5 minutes). In order to measure total binding and non-specific binding activity, assay buffer solution and 1uM(final conc.) cold human /rat CRF(C-3042; SIGMA) solution were used respectively in place of test substance solution. After incubation, the mixture was centrifuged at 23 °C, 2,500 rpm for 5 minutes and the scintillation was counted with Top Count (Packard). An influence of color quench was corrected by standard curve made by using tartrazine as a quencher. Evaluation of samples was carried out several times. The result is shown in table 4.

### Experiment 2: Model of surgical stress in mouse (Inhibitory action of rise in releasing of adrenocorticotropic hormone(ACTH))

### Animals:

ddY male mice(SLC, 7 to 8 weeks of age: weight 34 to 42g)were used for experiments. Mice were housed before use under controlled lighting condition (12hours light and 12hours dark cycle(lights on from 8:00 to 20:00) at 23±3 °C and 55±15% humidity, and maintained with basal diet and water ad labium. On the experimental day, mice were moved to experimental room and habituated before experiment for more than 1 hour. On the day before experiment, mice were previously handled (by being fastened up for 10 to 15 seconds like the case of i.p. injections) to habituate to the stress caused by handling.

### Medicine and administration:

Test compound to be evaluated was dissolved in and diluted with physiological saline(Otsuka), 5% mannitol or 0.1N NaHCO₃/saline. Then the test sample solution was given into veins of mice at a dose of 10ml/Kg body weight. The same solvent alone mentioned above was given into veins of control mice group at a dose of 10ml/Kg weight.

### Experimental procedure:

Mice were anesthetized by injection of pentobarbital Na (2.5mg/mouse i.p.) and after 15 minutes the solvent alone or the solution containing test sample to be evaluated were intravenously injected on the tails of mice at a dose of 10ml/ Kg weight of mouse. After more 10 minutes a midline incision was made in the abdomen and in two minutes bowel was draped externally. Then 2.5 minutes after incision 0.6ml of blood samples were collected from main vein abdominalis for 1 minute gradually by using prechilled syringes(needle size 25G), which were wetted with 100mM EDTA solution. Control mice not subjected to surgical stress were decapitated and blood samples were collected at 13 to 14 minutes after medication of solvent alone and to them added 20ul of 100mM EDTA. Blood samples collected were put into siliconized tubes and centrifuged at 10,000rpm and 4°C for 5 minutes. The concentration of ACTH in supernatant was determined by IRMA method using Allegro ACTH kit (Nihon Medi+Physics Co.,Ltd.).

### Statistical analysis:

One way analysis of variance was done for statistical analysis, followed by post hoc analysis of Fisher's PLSD test using Super ANOVA v.1.11 software. The result is shown in table 4.

### Experiment 3: Model of surgical stress in mouse (Inhibitory action of rise in releasing of corticosterone(CORT))

### Animals:

ddY male mice(SLC, 7 weeks of age: weight 35 to 41g)were used. Mice were housed before use under controlled lighting condition (12hours light and 12hours dark cycle(lights on from 8:00 to 20:00) at 23±3 °C and humidity 55±15%, and maintained with basal diet and water ad labium. On the experimental day, after mice were weighted, diet was removed from mice just before experiments to eliminate the effects of food intake on experiments. Then, mice were moved to experimental room and habituated before experiment for more than 1 hour. On the day before experiment, mice were previously handled (by being fastened up for 10 to 15 seconds like the case of i.p. injections) to habituate to the stress caused by handling. Mice were divided into groups according to their weights on the experimental day.

### Medicine applied and method of administration:

Compound of the Example 7 was dissolved in and diluted with the solution of 0.1N NaHCO₃/saline (Otsuka), and the compound solution in 0.1N NaHCO₃/saline was given into veins of mice at a dose of 10ml/Kg weight of mouse. The same solvent alone mentioned above was given into veins of control mice group at a dose of 10ml/Kg weight of mouse.

### Experimental procedure:

Mice were anesthetized by injections of pentobarbital Na (2.5mg/mouse i.p.) and after 20 minutes the solvent alone or the solution containing test sample to be evaluated were intravenously injected into the tail veins of mice at a dose of 10ml/Kg weight. After more 5 minutes a midline incision was made in the abdomen, and bowel was draped externally in one to two minutes and then abdominal part was covered with wet absorbent cotton with saline to prevent from drying. In 30 minutes after incision in the abdomen, mice were decapitated and blood samples were collected into the siliconized tubes, prechilled on ice, which contained 20ul of 100mM EDTA. Blood samples were centrifuged at 10,000rpm and 4°C for 5 minutes and the obtained supernatant was collected and stored at -30 °C. Control mice not subjected to surgical stress were decapitated 35 minutes after medication of the solvent alone and blood samples were collected.

The concentration of CORT was determined by using Corticosterone Double Antibody (RIA measurement kit of CORT; ICN Pharmaceuticals, Inc.).

### Statistical analysis:

One way analysis of variance was done for statistical analysis followed by post hoc analysis of Fisher's PLSD test using Super ANOVA v.1.11 software with respect to each solvent. The result is shown in table 4.

**(Table 4)**

| Examples | | | |
|---|---|---|---|
| Example | IC₅₀(nM) [experiment 1] | Inhibitory action on ACTH release (mg/kg) [experiment 2] | Inhibitory action on CORT release (mg/kg) [experiment 3] |
| 6 | 218 | | |
| 7 | 263 | 10-30 | 10-30 |
| 8 | 195 | 10-30 | |
| 9 | 278 | 10-30 | |
| 10 | 165 | | |
| 11 | 123 | | |
| 12 | 57 | | |

### Test 1 Effects on a rise in releasing of stress hormone caused by surgical stress - effects on a rise in releasing of adrenocorticotropic hormone (ACTH)

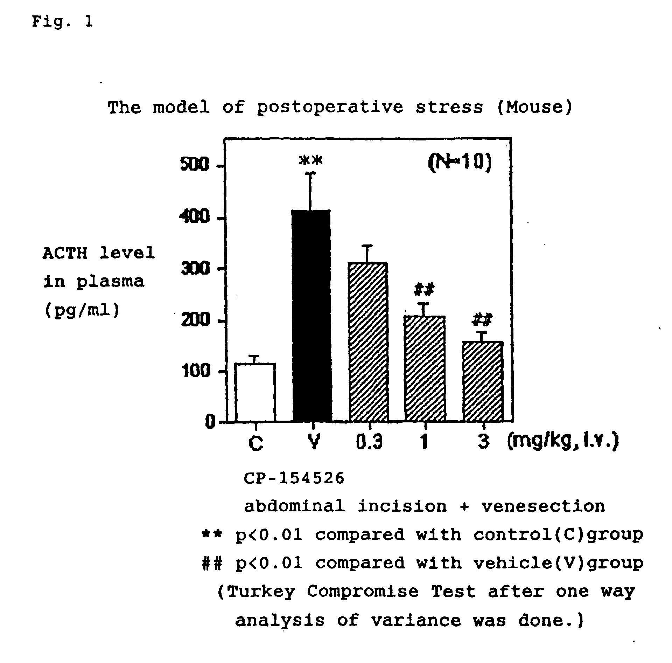

Mice (ddY Slc male, 7 weeks of age) handled by being fastened up before the day of experiment were divided equally into groups according to their weights on the experimental day (the number of mouse in one group was 10). Mice were anesthetized by i.p. injection of 2.5mg/mouse pentobarbital Na (Nembutal(brand name), Dainihonseiyaku) and, 5 minutes later, the solvent(0.1N HCl/saline)alone or test medicine CP154,526HCl(0.3, 1 or 3 mg/kg)were intravenously injected into the tail veins of mice at a dose of 10ml/Kg weight of mouse. 10 minutes after injection, a midline incision was made in the abdomen and in about two minutes movable bowel was draped externally. Then 25 minutes after incision, 0.6ml of blood sample was collected from aorta abdominalis for 1 minute gradually by using prechilled syringes (needle size 25G) which were wetted with 100mM EDTA solution. Control mice not subjected to surgical stress were given medication by the injection of the solvent alone 5 minutes after anesthesia. Then, 13 to 14 minutes after injection, mice were decapitated and blood samples were collected. Blood samples were centrifuged at 7,000 x g and 4°C for 20 minutes and the concentration of ACTH in supernatant was determined by IRMA method using Allegro ACTH kit (Nihon Medi+Physics Co., Ltd.). One way analysis of variance was done for statistical analysis followed by post hoc analysis of Turkey compromise test. The result is shown in Fig. 1.

The ACTH level in plasma increased significantly by surgical stress which was caused by incision in the abdomen and external draping of movable bowel. However, the increase of ACTH concentration in plasma was inhibited by administration of test medicine CP-154,526 HCl dose-dependently and it was clear from Fig. 1 that the dose of not less than 1mg/Kg weight of mouse showed a statistically significance for inhibitory action.

The inhibitory action of ACTH production and secretion by administration of test medicine lasts even after surgical stress. And moreover secretion of physiologically active substance (adrenocortical hormone), which is produced by the stimulation of ACTH on adrenal cortex and has negative effects on human body, is inhibited. Therefore, the secretion of physiologically active substance is inhibited by administration of test medicine, so it is clear that various symptoms of stress is suppressed.

### Test 2 Effects on a rise in releasing of stress hormone caused by surgical stress - effects on a rise in releasing of corticosterone, adrenocortical hormone.

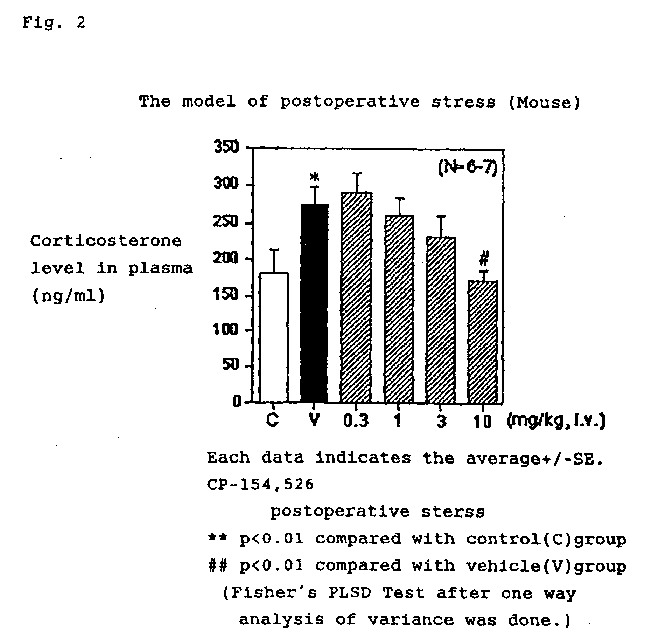

Mice (ddY Slc male, 7weeks of age) handled by being fastened up before the day of experiment were divided equally into groups according to their weights on the experimental day (the number of mouse in one group was 6 to 7). Mice were anesthetized by i.p. injections of 2.5mg/mouse pentobarbital Na(Nembutal(brand name)), Dainihonseiyaku) and, 20 minutes later, the solvent(0.1N HCl/saline) or test medicine CP154,526HCl(0.3, 1, 3 or 10 mg/kg) were intravenously injected into the tail veins of mice at a dose of 10ml/Kg weight of mouse. 5 Minutes after injection, a midline incision was made in the abdomen, and in about one to two minutes movable bowel was draped externally, and then, abdominal part was covered with wet absorbent cotton with saline to prevent from drying. In 30 minutes after incision, while the bowel was draped externally, mice were decapitated and blood samples were collected into siliconized tubes, prechilled on ice, which contained 20ul of 100mM EDTA. Blood samples were centrifuged at 7,000 x g and 4°C for 20 minutes and the supernatant was obtained. Negative control mice not subjected to surgical stress were decapitated and blood samples were collected in 35 minutes after injection of the solvent alone. Plasma was once stored at -30°C and thawed later to determine the concentration of corticosterone in plasma by RIA method using Corticosterone Double Antibody (ICN Pharmaceuticals, Inc). One way analysis of variance was done for statistical analysis followed by post hoc analysis of Fisher's PLSD test. The result is shown in Fig. 2.

The level of corticosterone in plasma increased significantly owing to surgical stress caused by incision in the abdomen and external draping of movable bowel. However, the increase of corticosterone concentration in plasma was inhibited by administration of test medicine CP-154,526 HCl dose-dependently and it was clear from Fig. 2 that the dose of not less than 10 mg/Kg weight of mouse showed a statistically significance for inhibitory action.

The secretion of physiologically active adrenocortical hormone is inhibited by administration of the test medicine, so it is obvious that various symptoms of stress caused by this releasing of adrenocortical hormone is suppressed.

### Test 3 Effects of CRF antagonist on model of Major surgical stress in mouse

ddY male mice(SLC, 8 to 9 weeks of age) were housed before use under controlled lighting condition (12hours light/12hours dark cycle(lights on from 8:00 to 20:00)) at 23±3 °C and 55±15% humidity, and maintained with basal diet and water ad labium. On the experimental day, mice were moved to experimental room and habituated before experiment for more than 1 hour. Mice were weighted and divided into groups to adjust mean weight in each group almost equal according to their weights on the experimental day.

Mice in each group were anesthetized by ether, and test compounds [(3-chloro-N-(5- diethylsulfamoyl-2,3-dimethoxyphenyl)-4-hydroxy-5-nitrobenzamide, and 3,5-dichloro-N-[5-diethylsulfamoyl-3-(dimethylamino)-2-methoxyphenyl] - 4-hydorxybenzamide and so on), which were dissolved in and diluted with solvents of 0.1N NaHCO₃/saline or 10% DMF in 0.1N NaHCO₃/saline just before surgery, were intravenously injected into the tail veins or subcutaneously at a dose of 10ml/Kg weight of mouse in the anesthetized condition.

Control mice not subjected to surgery were given anesthesia and injections of the solvents alone. After injection, a midline incision was made in the abdomen. Movable bowel was draped externally and left lobe of liver was tied tightly with thread and removed off. Externally draped bowel was brought back into abdomen and the cut was stitched up with thread (3 to 4 stitches / mouse). Then, 1.5 hours or 4.5 hours later mice were decapitated and blood samples were collected into siliconized tubes, prechilled on ice, which contained 25ul of 100 mmol/l EDTA, and blood samples were centrifuged at 10,000 rpm and 4°C for 5 minutes and the obtained plasma was stored at -30 °C.

The level of IL-6 in this plasma was determined by enzyme immuno assay (EIA) using Quantikine mice IL-6 measurement kit (R&D system company) and concentration of corticosterone was determined by radio immuno assay (RIA) using Corticosterone Double Antibody(ICN biochemicals company). Catecholamine and others were determined by means of common method. Dunnett analysis and so on were done for statistical analysis of the observed data.

As the result, the compounds used in this invention were proved to be excellent CRF antagonists and useful for treatments of postoperative stress.

### Test 4 Effects of CRF receptor antagonists on model of postoperative stress in rat

### (Dose-finding study)

Male SD rats were divided into three groups, a solvent-administration group, a methylprednisolone-administration group (positive control group) and a test compound-administration group. In a solvent-administration group, solvent alone (2.5% polysorbate80, 5% sorbitol; pH9.5) was injected, in a methylprednisolone-administration group methylprednisolone dissolved in and diluted with the solvent described above as a reference was injected, and in a test compound-administration group test compound dissolved in and diluted with the solvent described above was injected into mouse. Then mice in each group were subjected to abdominal operations in the ether anesthesia condition and models of cecum ligation puncture (CLP) were made. By the way, an untreated group was formed as a reference of CLP model and mice in this group were given injection of the solvent alone and not subjected to abdominal operations. After surgery mice were maintained under fasting state and administration in each group were carried out as follows. In a solvent-administration and a test compound-administration group, injections were carried out just before making of CLP model and after that appropriately at fixed intervals during the test period. In a methylprednisolone-administration group (positive control group), injections were carried out just before making of CLP model only and after that the solvent alone was injected at the same intervals as those in a solvent-administration and a test compound-administration group. A few hours after making of CLP models, mice were subjected to abdominal operation in the nembutal anesthesia condition deeply and blood samples were collected from aorta. ATCH, cortisol, catecholamine, IL-6, parameters in blood (red blood cells, white blood cells, platelets, hematocrit, hemoglobin), biochemical parameters in plasma (total protein, albumin, ALT, AST, creatinine, BUN, TG, cholesterol, glucose, Na⁺/K⁺/Cl) were determined.

On the basis of observed data, the effective dose in the following tests was decided.

### (Effects of CRF antagonists on prognosis after CLP)

Male SD rats were divided into three groups, a solvent-administration group, a methylprednisolone-administration group (positive control group) and a test compound-administration group. In a solvent-administration group, the solvent alone (2.5% polysorbate80, 5% sorbitol; pH9.5) was injected, in a methylprednisolone-administration group methylprednisolone dissolved in and diluted with the solvent described above as a reference was injected, and in a test compound-administration group test compound dissolved in and diluted with the solvent described above was injected into mouse. Then mice in each group were subjected to abdominal operations in the ether anesthesia condition and models of cecum ligation puncture (CLP) were made. By the way, an untreated group was formed as a reference of CLP model and mice in this group were given injection of the solvent alone and not subjected to abdominal operations. After surgery mice were maintained under fasting state and administrations in each group were carried out as follows. In a solvent-administration and test compound- administration group, injections were carried out just before making of CLP model and after that appropriately at fixed intervals. In a methylprednisolone-administration group (positive control group), injections were carried out just before making of CLP model only and after that the solvent alone was injected out at the same intervals as those in a solvent-administration and a test compound-administration group. Measurement of weight, amount of food intake and judgment of life and death were carried out once a day and effects of CRF antagonists on prognosis after CLP are observed.

As a result, compounds used in this invention were proved to be fine CRF antagonists and useful for treatment of postoperative stress.

### Industrial Applicability

Compounds which have antagonistic effects on CRF receptor, in other words CRF antagonists, were useful as preventives and/or therapeutic agents against postoperative stress.

## Claims

1. A pharmaceutical composition for prophylaxis or therapy of a postoperative stress, which comprises a compound having CRF antagonist activating effect.

2. A pharmaceutical composition for prophylaxis or therapy of a postoperative stress as claimed in claim 1, wherein the compound having CRF antagonist activating effect is a 5 to 6 membered nitrogen-containing heterocyclic compound, its fused ring derivative or a salt thereof which has CRF antagonist activating effect.

3. A pharmaceutical composition for prophylaxis or therapy of a postoperative stress as claimed in claim 1 or claim 2,
wherein the compound having CRF antagonist activating effect is a compound represented by the formula I 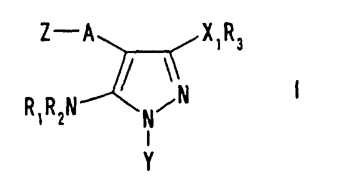 wherein A is CH₂; R₁, R₂ and R₃ are each independently linear C₁-C₆ alkyl, branched C₃-C₈ alkyl, C₃-C₈ alkenyl, wherein the double bond is not adjacent to the N or X₁ when X₁ is oxygen or sulfur, or C₃-C₇ cycloalkyl -(CH₂)ₙ- wherein n is 0, 1, 2, 3, 4; or R₁ and R₂ may be taken together with the nitrogen to form a saturated 4, 5 or 6 membered ring optionally condensed with benzene ring; and R₃ may also be (CH₂)_{q}Q₁R₁₉ wherein q is 0, 1 or 2 and Q₁ is oxygen, sulfur, NH, N(C₁-C₆ alkyl), or a covalent bond when X₁ is not a covalent bond, and R₁₉ may be hydrogen, linear C₁-C₆ alkyl, branched C₃-C₈ alkyl, C₃-C₈ alkenyl, C₃-C₈ cycloalkyl, C₃-C₈ cycloalkyl-(CH₂)ₙ- wherein n is 1 to 4; X₁ is a covalent bond, CH₂, NR, wherein R is hydrogen or linear C₁-C₆ alkyl, oxygen or sulfur; Y is phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, or piperidinyl, each of the above groups may be substituted with one to three of any substitutents of fluoro, chloro, bromo or methyl, or one of trifluoromethyl, with the proviso that Y is not unsubstituted phenyl; and Z is
(a) the formula II 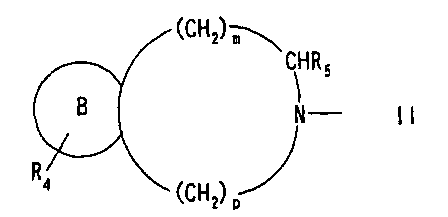 wherein the B ring is phenyl, naphthyl, pyridyl, pyrimidinyl, pyrazinyl, pyridazinyl, triazilyl, pyrrolyl, pyrazolyl, imidazolyl, triazolyl, thienyl, or indolyl, each of the above groups maybe substituted with methyl, methoxy, fluoro, chloro, bromo or iodo; or a saturated 5 or 6 membered carbocyclic ring or a partially unsaturated ring having one or two double bonds; R₄ is hydrogen, C₁-C₆ alkyl, C₁-C₆ alkoxy, or hydroxy, fluoro, chloro, bromo, iodo or trifluoromethyl; R₅ is hydrogen, linear C₁-C₆ alkyl, branched C₃-C₈ alkyl, C₃-C₈ alkenyl or (CH₂)ₒ-X₂-(CH₂)ᵣ-Q₂-R₆; R₆ is hydrogen, linear C₁-C₆ alkyl, branched C₃-C₈ alkyl, C₃-C₈ alkenyl; X₂ and Q₂ are each independently oxygen, sulfur, NH, N(C₁₋C₆ alkyl), or one of X₂ and Q₂ may be a covalent bond; m is 0 or 1; o is 1 or 2; p is 1 or 2; r is 0, 1, or 2;
(b) the formula III 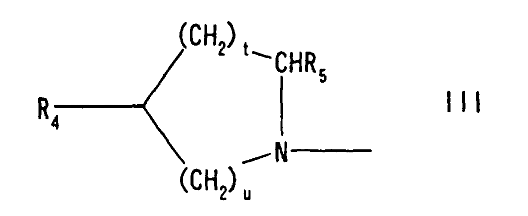 wherein R₄ and R₅ are as defined above, and t and u are each independently 1 or 2;
(c) -NR₇R₈ wherein R₇ and R₈ are each independently hydrogen, linear C₁-C₆ alkyl, branched C₃-C₈ alkyl, C₃-C₈ alkenyl, (CH₂)ᵥCH₂OH, (CH₂)ᵥNR₉R₁₀ wherein v is 0 to 3, and R₉ and R₁₀ are each independently hydrogen, or linear C₁-C₆ alkyl, C₃-C₁₂ cycloalkyl, C₃-C₁₂ cycloalkyl(CH₂)ₙ, C₆-C₁₀ bicycloalkyl(CH₂)ₙ, wherein n is 0 to 4, C₃-C₆ cycloalkyl group fused with benzene ring, C₁-C₆ hydroxyalkyl, phenyl, C₁-C₃ phenylalkylene, each of the above groups may be substituted with one or two of hydroxy, fluoro, chloro, bromo, C₁-C₅ alkyl or C₁-C₅ alkoxy; or R₇ and R₈ may be taken together with the nitrogen to form a saturated or partially unsaturated 5 to 7 membered ring which may contain one of oxygen, sulfur, NH, or N(C₁₋C₆ alkyl) and which may be substituted with C₁-C₆ alkyl, hydroxyl or phenyl wherein any double bond are not adjacent to any heteroatoms;
(d) the formula IV 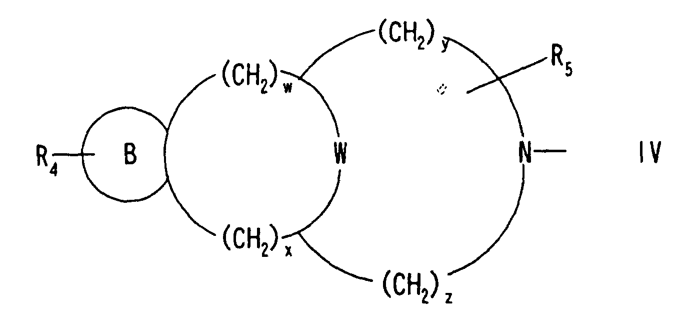 wherein B, R₄ and R₅ are as defined above, w, x, y and z are each independently 1 or 2 and W is (CH₂)_{q} wherein q is as defined above, N(C₁₋C₆ alkyl), or oxygen;
(e) the formula V 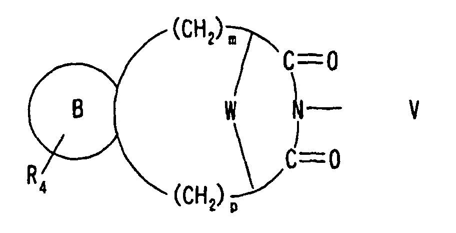 wherein B, R₄, m and p are as defined above;
(f) the formula VI 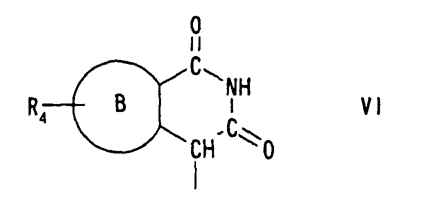 wherein B and R₄ are as defined above; or
(g) O(CH₂)ᵥR₁₁ₐ wherein v is 0 to 3 and R₁₁ₐ is linear C₁-C₆ alkyl, branched C₃-C₈ alkyl, phenyl, naphthyl, 1 2,3,4-tetrahydronaphthyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, piperidinyl, or thienyl, each of the above groups may be substituted with one or two of any substitutents of fluoro, chloro, bromo, methyl or trifluoromethyl,
or its pharmaceutically acceptable acid addition salt.

4. A pharmaceutical composition for prophylaxis or therapy of a postoperative stress as claimed in claim 1 or claim 2, wherein the compound having CRF antagonist activating effect is a compound represented by the formula VII 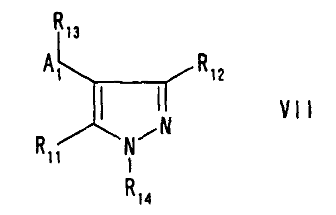 wherein A₁ is C=O or SO₂ or A₁ and R₁₁ taken together with the carbons to which they are attached form 5-pyridyl or pyrimidinyl which may be substituted with R₁₅, wherein R₁₅ is hydrogen, C₁-C₆ alkyl, fluoro, chloro, bromo, hydroxy, amino, O(C₁-C₆ alkyl), NH(C₁-C₆ alkyl), N(C₁-C₆ alkyl)(C₁-C₆ alkyl), SH, S(O)ₙ(C₁-C₆ alkyl) wherein n is 0, 1 or 2, wherein said C₁-C₆ alkyl or C₁-C₆ alkyl moieties may be substituted with from one to three substituents R₁₆ which is hydroxy, amino, C₁-C₃ alkoxy, dimethylamino, diethylamino, methylamino, ethylamino, NH(C=O)CH₃, fluoro, chloro, bromo or C₁-C₃ thioalkyl; R₁₁ is hydrogen, C₁-C₆alkyl, amino, O(C₁-C₆ alkyl), NH(C₁-C₆ alkyl), N(C₁-C₆ alkyl)(C₁-C₆ alkyl), wherein said C₁-C₆ alkyl or C₁-C₆ alkyl moieties may be substituted with from one to three substituents R₁₆ as defined above; R₁₂ is hydrogen, C₁-C₆ alkyl, hydroxy, amino, O(C₁-C₆ alkyl), NH(C₁-C₆ alkyl), N(C₁-C₆ alkyl)(C₁-C₆ alkyl), SH, S(O)ₙ(C₁-C₆ alkyl) wherein n is 0,1 or 2, cyano, hydroxy, carboxy or amido, wherein said alkyl may be substituted with one to three of hydroxy, amino, carboxy, amido, NH(C=O) (C₁-C₆ alkyl), N(C₁-C₆ alkyl)(C₁-C₆ alkyl), (C=O)O(C₁-C₆ alkyl), C₁-C₃ alkoxy, C₁-C₃ thioalkyl, fluoro, bromo, chloro, iodo, cyano or nitro; R₁₃ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, azaindolyl, benzoxazolyl, oxazolyl, pyrrolidinyl, thiazolidinyl, morpholinyl, pyridinyl, tetrazolyl, or 9 to 12 membered bicycloalkyl, optionally containing one to three of oxygen, sulfur or N-Z₀ wherein Z₀ is hydrogen, C₁₋C₄ alkyl, C₁₋C₄ alkanoyl, phenyl or phenylmethyl, wherein each one of the above groups may be substituted independently with from one to three of fluoro, chloro, bromo, C₁-C₆ alkyl, C₁-C₆ alkoxy or trifluoromethyl, or one of cyano, nitro, amino, NH(C₁₋₆ alkyl), N(C₁-C₄ alkyl)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NHSO₂(C₁-C₄ alkyl), S(C₁-C₆ alkyl) or SO₂(C₁-C₆ alkyl), wherein said C₁₋C₄ alkyl or C₁₋C₄ alkyl moieties and C₁₋C₆ alkyl or C₁₋C₆ alkyl moieties may be substituted with one or two of fluoro, chloro, hydroxy, amino, methylamino, dimethylamino or acetyl; and R₁₄ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, azaindolyl, benzoxazolyl, oxazolyl, pyrrolidinyl, thiazolidinyl, morpholinyl, pyridinyl, tetrazolyl, or 3 to 8 membered cycloalkyl or 9 to 12 membered bicycloalkyl, optionally containing one to three of oxygen, sulfur or N-Z₀ wherein Z₀ is hydrogen, C₁₋C₄ alkyl, C₁-C₄ alkanoyl, phenyl or phenylmethyl, wherein each of the above groups may be substituted independently with from one to three of fluoro, chloro, bromo, trifluoromethyl, C₁-C₆ alkyl or C₁-C₆ alkoxy, or one of cyano, nitro, amino, NH(C₁-C₆ alkyl), N(C₁-C₄ alkyl)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NH₂SO₂(C₁-C₄ alkyl), S(C₁-C₆ alkyl) or SO₂(C₁-C₆ alkyl), wherein said C₁-C₄ alkyl or C₁-C₄ alkyl moieties and C₁-C₆ alkyl or C₁-C₆ alkyl moieties may be substituted with one or two of fluoro, chloro, hydroxy, amino, methylamino, dimethylamino or acetyl; with the proviso that (1) R₁₄ is not unsubstituted phenyl; (2) when R₁₁ is amino, R₁₂ is methylthio, R₁₄ is 2,4,6-trichlorophenyl, and A is C=O, then R₁₃ is not 2-chlorophenyl; and (3) R₁₁ and R₁₂ is not both hydrogen, or its pharmaceutically acceptable acid addition salt.

5. A pharmaceutical composition for prophylaxis or therapy of a postoperative stress as claimed in claim 1 or claim 2, wherein the compound having CRF antagonist activating effect is a compound represented by the formula VIII 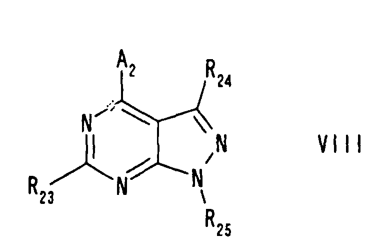 wherein A₂ is NR₂₁R₂₂, CR₂₁R₂₂R₂₁₁, C(=CR₂₁R₂₁₂)R₂₂, NHCR₂₁R₂₂R₂₁₁, OCR₂₁R₂₂R₂₁₁, SCR₂₁R₂₂R₂₁₁, NHNR₂₁R₂₂, CR₂₂R₂₁₁NHR₂₁, CR₂₂R₂₁₁OR₂₁, CR₂₂R₂₁₁SR₂₁ or C(O)R₂₂;
R₂₁ is hydrogen, or C₁-C₆ alkyl which may be substituted with one or two substituents R₂₆ independently selected from the group consisting of hydroxy, fluoro, chloro, bromo, iodo, C₁-C₆ alkoxy, O-C(=O)-(C₁-C₆ alkyl), O-C(=O)-N(C₁-C₄ alkyl)(C₁-C₂ alkyl), amino, NH(C₁-C₄ alkyl), N(C₁-C₄ alkyl)(C₁-C₂ alkyl), S(C₁-C₆ alkyl), OC(O)NH(C₁-C₄, alkyl), N(C₁-C₂ alkyl)C(O)(C₁-C₄ alkyl), NHC(=O)(C₁-C₄ alkyl), COOH, C(=O)O(C₁-C₄, alkyl), C(=O)NH(C₁-C₄, alkyl), C(=O)N(C₁-C₄ alkyl)(C₁-C₂ alkyl), SH, CN, NO₂, SO(C₁-C₄ alkyl), SO₂(C₁-C₄ alkyl), SO₂NH(C₁-C₄ alkyl) and SO₂N(C₁-C₄ alkyl)(C₁-C₂ alkyl), and wherein said C₁-C₆ alkyl may contain one or two double or triple bonds;
R₂₂ is C₁-C₁₂ alkyl, aryl or (C₁-C₁₀ alkylene) aryl wherein said aryl is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, azaindolyl, oxazolyl, or benzoxazolyl, or 3 to 8 membered cycloalkyl or (C₁-C₆ alkylene)cycloalkyl, wherein said cycloalkyl may contain one or two of oxygen, sulfur or N-Z₁ wherein Z₁ is hydrogen, C₁-C₄ alkyl, benzyl or C₁-C₄ alkanoyl, wherein R₂₂ may be substituted independently with from one to three of chloro, fluoro or C₁-C₄ alkyl, or one of hydroxy, bromo, iodo, C₁-C₆ alkoxy, O-C(=O)-(C₁-C₆ alkyl), O-C-N(C₁-C₄ alkyl)(C₁-C₂ alkyl), S(C₁-C₆ alkyl), NH₂, NH(C₁-C₂ alkyl), N(C₁-C₄ alkyl)(C₁-C₂ alkyl), N(C₁-C₄ alkyl) -C(=O)(C₁-C₄ alkyl), NHC(=O)(C₁-C₄ alkyl), COOH, C(=O)O(C₁-C₄ alkyl), C(=O)NH(C₁-C₄ alkyl), C(=O)N(C₁-C₄ alkyl)(C₁-C₂ alkyl), SH, CN, NO₂, SO(C₁-C₄ alkyl), SO₂(C₁-C₄ alkyl), SO₂NH(C₁-C₄ alkyl) or SO₂N(C₁-C₄ alkyl) (C₁-C₂ alkyl), and wherein said C₁-C₁₂ alkyl or C₁-C₁₀ alkylene may contain one to three double or triple bonds; or NR₂₁R₂₂ or CR₂₁R₂₂R₂₁₁ may form a 4 to 8 membered ring optionally containing one or two double bonds or one or two of oxygen, sulfur or N-Z₁ wherein Z₁ is hydrogen, C₁-C₄ alkyl, benzyl or C₁-C₄ alkanoyl;
R₂₃ is hydrogen, C₁-C₆ alkyl, fluoro, chloro, bromo, iodo, hydroxy, amino, O(C₁-C₆ alkyl), NH(C₁-C₆ alkyl), N(C₁-C₄ alkyl)(C₁-C₂ alkyl), SH, S(C₁-C₄ alkyl), SO(C₁-C₄ alkyl), or SO₂(C₁-C₄ alkyl), wherein said C₁-C₄ alkyl or C₁-C₄ alkyl moieties and C₁-C₆ alkyl or C₁-C₆ alkyl moieties may contain one or two double or triple bonds and may be substituted with from one to three substituents R₂₇ independently selected from the group consisting of hydroxy, amino, C₁-C₃ alkoxy, dimethylamino, diethylamino, methylamino, ethylamino, NHC(=O)CH₃, fluoro, chloro and C₁-C₃ thioalkyl;
R₂₄ is hydrogen, C₁-C₆ alkyl, fluoro, chloro, bromo, iodo, C₁-C₆ alkoxy, amino, NH(C₁-C₆ alkyl), N(C₁-C₆ alkyl) (C₁-C₂ alkyl), SOₙ(C₁-C₆ alkyl) wherein n is 0, 1 or 2, cyano, hydroxy, carboxy or amido, wherein said C₁-C₆ alkyl or C₁-C₆ alkyl moieties may be substituted with one to three of hydroxy, amino, carboxy, amido, NH(C=O)(C₁-C₄ alkyl), NH(C₁-C₄ alkyl), N(C₁-C₄ alkyl)(C₁-C₂ alkyl), (C=O)O(C₁-C₄ alkyl), C₁-C₃ alkoxy, C₁-C₃ thioalkyl, fluoro, bromo, chloro, iodo, cyano or nitro;
R₂₅ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, pyrrolopyridyl, benzoxazolyl, oxazolyl, pyrrolidinyl, thiazolidinyl, piperazinyl, piperidinyl, tetrazolyl, or 3 to 8 membered cycloalkyl or 9 to 12 membered bicycloalkyl, optionally containing one or two of oxygen, sulfur or N-Z₁ wherein Z₁ is hydrogen, C₁-C₄ alkyl, C₁-C₄ alkanoyl, phenyl or benzyl, wherein each one of the above groups may be substituted independently with from one to three of fluoro, chloro, bromo, formyl, C₁-C₆ alkyl, C₁-C₆ alkoxy or trifluoromethyl, or one of hydroxy, iodo, cyano, nitro, amino, cyclopropyl, NH(C₁-C₄ alkyl), N(C₁-C₄ alkyl)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NHSO₂(C₁-C₄ alkyl), S(C₁-C₆ alkyl), SO₂(C₁-C₆ alkyl), wherein said C₁-C₄ alkyl or C₁-C₄ alkyl moieties and C₁-C₆ alkyl or C₁-C₆ alkyl moieties may have one double or triple bond and may be substituted with one or two of fluoro, chloro, hydroxy, amino, methylamino, dimethylamino or acetyl; with the proviso that R₂₅ is not unsubstituted phenyl; R₂₁₁ is hydrogen, hydroxy, fluoro, chloro, COO(C₁-C₂ alkyl), cyano or CO(C₁-C₂ alkyl); and R₂₁₂ is hydrogen or C₁-C₄ alkyl;
(a) A₂ is not linear C₁-C₁₂ alkyl;
(b) R₂₅ is not a sugar group;
(c) when R₂₃ and R₂₄ are hydrogen and R₂₅ is chlorophenyl, then A₂ is not NH-CH(CH₃)-(CH₂)₃-N(C₂H₅)₂;
(d) when R₂₃ and R₂₄ are hydrogen and A₂ is NR₂₁R₂₂ wherein R₂₁ is C₃-C₇ cycloalkyl and R₂₂ is C₂-C₆ alkenyl, phenyl-(C₁-C₆ alkylene) or hetero-(C₁-C₆ alkylene) wherein the hetero radical is furyl, thienyl or pyridinyl, and wherein said phenyl may be substituted with fluoro, chloro, bromo or iodo, then R₂₅ is not tetrahydrofuranyl or tetrahydropyranyl;
(e) when R₂₃ is methoxy, methylthio, or methylsulfonyl, R₂₄ is hydrogen, and R₂₅ is tetrahydrofuranyl or tetrahydropyranyl, then A₂ is not NHC₂H₄C₆H₅, NH(C₁-C₂ alkyl), morpholinyl, hydrazino, which may be substituted with one methyl or two methoxy;
(f) when R₂₃ is hydrogen, C₁-C₆ alkyl, hydrazino, chloro, bromo, SH, or S(C₁-C₄ alkyl), R₂₄ is hydrogen, and R₂₅ is C₃-C₈ cycloalkyl, then A₂ is not hydrazino, NH(C₁-C₂ alkyl) or N(C₁-C₆ alkyl)(C₁-C₁₂ alkyl);
(g) when R₂₃ and R₂₄ are hydrogen and A₂ is NH(CH₂)ₘCOOH, wherein m is 1 to 12, then R₂₅ is not phenyl substituted with one of fluoro, chloro, bromo or iodo;
(h) when R₂₃ is hydrogen, hydroxy, methylthio, chloro or NHbenzyl, R₂₄ is hydrogen, and R₂₅ is chlorophenyl or bromophenyl, then A₂ is not NH(C₁-C₁₂ alkyl), NHallyl, or N(C₁-C₆ alkyl) (C₁-C₁₂ alkyl), wherein said C₁-C₁₂ alkyl may be substituted with NHbenzyl or NC₂H₅ which may be substituted with one or two of bromo, chloro, fluoro, NC₂H₅phenol or morpholinopropyl;
(i) when R₂₃ and R₂₄ are hydrogen and R₂₅ is nitrophenyl, then A₂ is not NHR₂₂ wherein R₂₂ is C₁-C₁₂ alkyl which may be substituted with two hydroxy, or R₂₂ is phenyl or benzyl;
(j) when R₂₃ is chloro or O(C₁-C₆ alkyl), R₂₄ is hydrogen, and A₂ is NR₂₁R₂₂, wherein R₂₁ and R₂₂ are independently hydrogen or C₁-C₆ alkyl, then R₂₅ is not chlorophenyl; and
(k) when R₂₃ is hydrogen, A₂ is benzyl or phenethyl, and R₂₄ is fluoro, chloro, bromo or iodo, then R₂₅ is not 5'-deoxy-ribofuranosyl or 5'-amino-5'-deoxy-ribofuranosyl, or its pharmaceutically acceptable acid addition salt.

6. A pharmaceutical composition for prophylaxis or therapy of a postoperative stress as claimed in claim 1 or claim 2,
wherein the compound having CRF antagonist activating effect is a compound represented by the formula IX 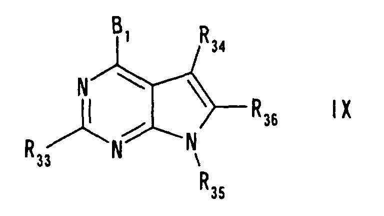 wherein B₁ is NR₃₁R₃₂, CR₃₁R₃₂R₃₁₁, C(=CR₃₂R₃₁₂)R₃₁, NHCR₃₁R₃₂R₃₁₁, OCR₃₁R₃₂R₃₁₁, SCR₃₁R₃₂R₃₁₁, NHNR₃₁R₃₂, CR₃₂R₃₁₁NHR₃₁, CR₃₂R₃₁₁OR₃₁, CR₃₂R₃₁₁SR₃₁, or C(O)R₃₂;
R₃₁ is hydrogen, or C₁-C₆ alkyl which may be substituted with one or two substituents R₃₇ independently selected from the group consisting of hydroxy, fluoro, chloro, bromo, iodo, C₁-C₈ alkoxy, O-C(=O)-(C₁-C₆ alkyl), O-C(=O)NH(C₁-C₄ alkyl), O-C(=O)-N(C₁-C₄ alkyl) (C₁-C₂ alkyl), amino, NH(C₁-C₄ alkyl), N(C₁-C₄ alkyl)(C₁-C₂ alkyl), S(C₁-C₆ alkyl), N(C₁-C₄ alkyl)C(=O)(C₁-C₄ alkyl), NHC(=O) (C₁-C₄ alkyl), COOH, C(=O)O(C₁-C₄ alkyl), C(=O)NH (C₁-C₄ alkyl), C(=O)N(C₁-C₄ alkyl)(C₁-C₂ alkyl), SH, CN, NO₂, SO (C₁-C₄ alkyl), SO₂(C₁-C₄ alkyl), SO₂NH(C₁-C₄ alkyl) and SO₂N(C₁-C₄ alkyl)(C₁-C₂ alkyl) wherein said C₁-C₆ alkyl may contain one or two double or triple bonds;
R₃₂ is C₁-C₁₂ alkyl, aryl or (C₁-C₁₀ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, pyrrolopyridyl, oxazolyl or benzoxazolyl, or 3 to 8 membered cycloalkyl or (C₁-C₆ alkylene) cycloalkyl, wherein said cycloalkyl may contain one or two of oxygen, sulfur or N-Z₂ wherein Z₂ is hydrogen, C₁-C₄ alkyl, benzyl or C₁-C₄ alkanoyl, wherein R₃₂ may be substituted independently with from one to three of chloro, fluoro or C₁-C₄ alkyl, or one of hydroxy, bromo, iodo, C₁-C₆ alkoxy, O-C(=O)-(C₁-C₆ alkyl), O-C(=O)-N(C₁-C₄ alkyl)(C₁-C₂ alkyl), S(C₁-C₆ alkyl), NH₂, NH(C₁-C₂ alkyl), N(C₁-C₄ alkyl)(C₁-C₂ alkyl), N(C₁-C₄ alkyl)-C(=O)(C₁-C₄ alkyl), NHC(=O)(C₁-C₄ alkyl), COOH, C(=O)O(C₁-C₄ alkyl), C(=O)NH(C₁-C₄ alkyl), C(=O)N (C₁-C₄ alkyl)(C₁-C₂ alkyl), SH, CN, NO₂, SO(C₁-C₄ alkyl), SO₂(C₁-C₄ alkyl), SO₂NH(C₁-C₄ alkyl), SO₂N(C₁-C₄ alkyl)(C₁-C₂ alkyl), and wherein said C₁-C₁₂ alkyl or C₁-C₁₀ alkylene moieties may contain one to three double or triple bonds; or
NR₃₁R₃₂ or CR₃₁R₃₂R₃₁₁ may form a saturated 3 to 8 membered ring of which the 5 to 8 membered ring may contain one or two double bonds or one or two of oxygen, sulfur or N-Z₂ wherein Z₂ is hydrogen, C₁-C₄ alkyl, benzyl or C₁-C₄ alkanoyl;
R₃₃ is hydrogen, C₁-C₆ alkyl, fluoro, chloro, bromo, iodo, hydroxy, amino, O(C₁-C₆ alkyl), NH(C₁-C₆ alkyl), N(C₁-C₄ alkyl)(C₁-C₂ alkyl), SH, S(C₁-C₄ alkyl), SO(C₁-C₄ alkyl), or SO₂(C₁-C₄ alkyl), wherein said C₁-C₄ alkyl moieties and C₁-C₆ alkyl or C₁-C₆ alkyl moieties may contain one double or triple bond and may be substituted with from one to three substituents R₃₈ independently selected from the group consisting of hydroxy, C₁-C₃ alkoxy, fluoro, chloro and C₁-C₃ thioalkyl;
R₃₄ is hydrogen, C₁-C₆ alkyl, fluoro, chloro, bromo, iodo, C₁-C₆ alkoxy, amino, NH(C₁-C₆ alkyl), N(C₁-C₆ alkyl)(C₁-C₂ alkyl), SOₙ(C₁-C₆ alkyl) wherein n is 0, 1 or 2, cyano, hydroxy, carboxy or amido, wherein said C₁-C₆ alkyl or C₁-C₆ alkyl moieties may be substituted with one of hydroxy, trifluoromethyl, amino, carboxy, amido, NH(C=O)(C₁-C₄ alkyl), NH(C₁-C₄ alkyl), N(C₁-C₄ alkyl)(C₁-C₂ alkyl), (C=O)O(C₁-C₄ alkyl), C₁-C₃ alkoxy, C₁-C₃ thioalkyl, fluoro, bromo, chloro, iodo, cyano or nitro;
R₃₅ is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, pyrrolopyridyl, benzoxazolyl, oxazolyl, pyrrolidinyl, thiazolidinyl, morpholinyl, piperizinyl, piperazinyl, tetrazolyl, or 3 to 8 membered cycloalkyl or 9 to 12 membered bicycloalkyl, optionally containing one or two of oxygen, sulfur or N-Z₂ wherein Z₂ is hydrogen, C₁-C₄ alkyl, C₁-C₄ alkanoyl, phenyl or phenylmethyl, wherein each one of the above groups may be substituted independently with from one to three of fluoro, chloro, bromo, formyl, C₁-C₆ alkyl, C₁-C₆ alkoxy or trifluoromethyl, or one of hydroxy, iodo, cyano, nitro, amino, NH(C₁-C₄ alkyl), N(C₁-C₄)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NHSO₂(C₁-C₄ alkyl), S(C₁-C₆ alkyl), SO₂(C₁-C₆ alkyl), wherein said C₁-C₄ alkyl or C₁-C₄ alkyl moieties and C₁-C₆ alkyl or C₁-C₆ alkyl moieties may be substituted with one or two of fluoro, chloro, hydroxy, C₁-C₄ alkoxy, amino, methylamino, dimethylamino or acetyl, wherein said C₁-C₄ alkyl or C₁-C₄ alkyl moieties and C₁-C₆ alkyl or C₁-C₆ alkyl moieties may contain one double or triple bond; with the proviso that R₃₅ is not unsubstituted phenyl;
R₃₆ is hydrogen, C₁-C₆ alkyl, fluoro, chloro, bromo, iodo, C₁-C₆ alkoxy, formyl, amino, NH(C₁-C₆ alkyl), N(C₁-C₆ alkyl)(C₁-C₂ alkyl), SOₙ(C₁-C₆ alkyl) wherein n is 0, 1 or 2, cyano, carboxy, or amido, wherein said C₁-C₆ alkyl may be substituted with one of hydroxy, trifluoromethyl, amino, carboxy, amido, NH(C=O)(C₁-C₄ alkyl), NH(C₁-C₄ alkyl), N(C₁-C₄ alkyl)(C₁-C₂ alkyl), (C=O)O(C₁-C₄ alkyl), C₁-C₃ alkoxy, C₁-C₃ thioalkyl, fluoro, bromo, chloro, iodo, cyano or nitro;
R₃₁₁ is hydrogen, hydroxy, fluoro, chloro, COO(C₁-C₂ alkyl), cyano or CO(C₁-C₂ alkyl); and
R₃₁₂ is hydrogen or C₁-C₄ alkyl; with the proviso that (1) B₁ is not linear C₁-C₁₂ alkyl, (2) when R₃₅ is unsubstituted cycloakyl, R₃₃ and R₃₄ are hydrogen, and R₃₆ is hydrogen or methyl, then B₁ is not NHR₃₂ wherein R₃₂ is benzyl or thienylmethyl, and (3) when R₃₅ is p-bromophenyl and R₃₃, R₃₄, and R₃₆ are methyl, then B₁ is not methylamino or hydroxyethylamino,
or its pharmaceutically acceptable acid addition salt.

7. A pharmaceutical composition for prophylaxis or therapy of a postoperative stress as claimed in claim 1 or claim 2, wherein the compound having CRF antagonist activating effect is a compound represented by the formula Ia 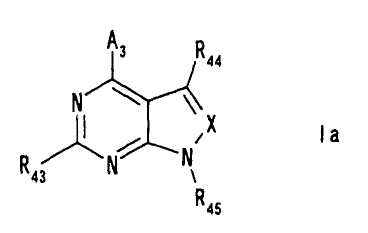 wherein X is nitrogen or -CR₄₆; A₃ is -NR₄₁R₄₂, -CR₄₁R₄₂R₄₁₁, -C(=CR₄₂R₄₁₂)R₄₁, -NHCR₄₁R₄₂R₄₁₁, -OCR₄₁R₄₂R₄₁₁, -SCR₄₁R₄₂R₄₁₁, -NHNR₄₁R₄₂, -CR₄₂R₄₁₁NHR₄₁, -CR₄₂R₄₁₁OR₄₁, -CR₄₂R₄₁₁SR₄₁ or -C(O)R₄₂;
R₄₁ is hydrogen, or C₁-C₆ alkyl which may optionally be substituted with from one or two substituents independently selected from the group consisting of hydroxy, fluoro, chloro, bromo, iodo, C₁-C₆ alkoxy, -OC(=O)-(C₁-C₆ alkyl), -OC(=O)NH(C₁-C₄ alkyl), -OC(=O)-N(C₁-C₄ alkyl)(C₁-C₂ alkyl), amino, -NH(C₁-C₄ alkyl), -N(C₁-C₂ alkyl)(C₁-C₄ alkyl), -S(C₁-C₆ alkyl), -N(C₁-C₄ alkyl)C(=O)(C₁-C₄ alkyl), -NHC(=O)(C₁-C₄ alkyl), -COOH, -C(=O)O(C₁-C₄ alkyl), -C(=O)NH(C₁-C₄ alkyl), -C(=O)N(C₁-C₄ alkyl)(C₁-C₂ alkyl), -SH, -CN, -NO₂, -SO(C₁-C₄ alkyl), -SO₂(C₁-C₄ alkyl) and -SO₂NH(C₁-C₄ alkyl) -SO₂N(C₁-C₄ alkyl)(C₁-C₂ alkyl), and wherein each of the foregoing C₁-C₆ alkyl moieties in the definition of R₄₁ may contain one or two double or triple bonds;
R₄₂ is C₁-C₁₂ alkyl, aryl or -(C₁-C₁₀ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, pyrrolopyridyl, oxazolyl or benzoxazolyl, or 3 to 8 membered cycloalkyl or -(C₁-C₆ alkylene)cycloalkyl, wherein one or two of the carbon atoms of any of said cycloalkyl moieties may optionally be replaced independently by oxygen, sulfur or N-Z₃ wherein Z₃ is hydrogen, C₁-C₄ alkyl or C₁-C₄ alkanoyl, and wherein R₄₂ may optionally be substituted with from one to three substituents independently selected from chloro, fluoro and C₁-C₄ alkyl, or one substituent selected from hydroxy, bromo, iodo, C₁-C₆ alkoxy, -OC(=O)-(C₁-C₆ alkyl), -OC(=O)N(C₁-C₄ alkyl)(C₁-C₂ alkyl), -S(C₁-C₆ alkyl), NH₂, -NH(C₁-C₂ alkyl), -N(C₁-C₂ alkyl)(C₁-C₄ alkyl), -N(C₁-C₄ alkyl)-C(=O)-(C₁-C₄ alkyl), -NHC(=O)(C₁-C₄ alkyl), -COOH, -C(=O)O(C₁-C₄ alkyl), -C(=O)NH(C₁-C₄ alkyl), -C(=O)N(C₁-C₄ alkyl)(C₁-C₂ alkyl), -SH, -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl), and wherein each of the foregoing C₁-C₁₂ alkyl and C₁-C₁₀ alkylene moieties in the definition may optionally contain one to three double or triple bonds; or
R₄₁ and R₄₂ taken together with the atom to which they are attached may form a saturated 3 to 8 membered ring which, if it is a 5 to 8 membered ring, may optionally contain one or two double bonds, and wherein one or two of the carbon atoms of said 5 to 8 membered ring may optionally be replaced independently by oxygen, sulfur or N-Z₃ wherein Z₃ is hydrogen, C₁-C₄ alkyl, C₁-C₄ alkanoyl or benzyl;
R₄₃ is hydrogen, C₁-C₆ alkyl, fluoro, chloro, bromo, iodo, hydroxy, amino, -O(C₁-C₆ alkyl), -NH(C₁-C₆ alkyl), -N(C₁-C₄ alkyl)(C₁-C₂ alkyl), -SH, -S(C₁-C₄ alkyl), -SO(C₁-C₄ alkyl) or -SO₂(C₁-C₄ alkyl), wherein each of the foregoing C₁-C₄ alkyl and C₁-C₆ alkyl moieties in the definition of R₄₃ may contain one double or triple bond and may optionally be substituted with from one to three substituents independently selected from the group consisting of hydroxy, C₁-C₃ alkoxy, fluoro, chloro and C₁-C₃ thioalkyl;
R₄₄ is hydrogen, C₁-C₆ alkyl, fluoro, chloro, bromo, iodo, C₁-C₆ alkoxy, formyl, -NH(C₁-C₆ alkyl), -N(C₁-C₆ alkyl)(C₁-C₂ alkyl), -SOₙ(C₁-C₆ alkyl) wherein n is 0, 1 or 2, cyano, hydroxy, carboxy or amido, wherein each of the foregoing C₁-C₆ alkyl moieties in the definition of R₄₄ may optionally be substituted with one substituent selected from hydroxy, trifluoromethyl, amino, carboxy, amido, -NHC(=O)(C₁-C₄ alkyl), -NH(C₁-C₄ alkyl), -N(C₁-C₄ alkyl)(C₁-C₂ alkyl), -C(=O)O(C₁-C₄ alkyl), C₁-C₃ alkoxy, C₁-C₃ thioalkyl, fluoro, bromo, chloro, iodo, cyano and nitro;
R₄₅ is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, pyrrolopyridyl, benzoxazolyl, oxazolyl, pyrrolidinyl, thiazolidinyl, morpholinyl, piperidinyl, piperazinyl, tetrazolyl, or 3 to 8 membered cycloalkyl or 9 to 12 membered bicycloalkyl ring, wherein one or two of the carbon atoms in said ring may optionally be replaced independently by oxygen, sulfur or N-Z₃ wherein Z₃ is hydrogen, C₁-C₄ alkyl, C₁-C₄ alkanoyl, phenyl or benzyl, and wherein each of the above R₄₅ groups may optionally be substituted with one or more substituents, preferably with two or three substituents, independently selected from fluoro, chloro, bromo, formyl, C₁-C₆ alkyl, C₁-C₆ alkoxy and trifluoromethyl, or one substituent selected from hydroxy, iodo, cyano, nitro, amino, -NH(C₁-C₄ alkyl), -N(C₁-C₄ alkyl)(C₁-C₂ alkyl), -COO(C₁-C₄ alkyl), -CO(C₁-C₄ alkyl), -SO₂NH(C₁-C₄ alkyl), -SO₂N(C₁-C₄ alkyl)(C₁-C₂ alkyl), -SO₂NH₂, -NHSO₂(C₁-C₄ alkyl), -S(C₁-C₆ alkyl) and -SO₂(C₁-C₆ alkyl), and wherein each of the foregoing C₁-C₄ alkyl and C₁-C₆ alkyl moieties in the definition of R₄₅ may optionally be substituted with from one or two substituents independently selected from fluoro, chloro, hydroxy, C₁-C₄ alkoxy, amino, methylamino, dimethylamino and acetyl, and wherein each of the foregoing C₁-C₄ alkyl and C₁-C₆ alkyl moieties in the definition of R₄₅ may optionally contain one double or triple bond; with the proviso that R₄₅ is not unsubstituted phenyl;
R₄₆ is hydrogen, C₁-C₆ alkyl, fluoro, chloro, bromo, iodo, C₁-C₆ alkoxy, formyl, amino, -NH(C₁-C₆ alkyl), N(C₁-C₆ alkyl)(C₁-C₂ alkyl), -SOₙ(C₁-C₆ alkyl) wherein n is 0, 1 or 2, cyano, carboxy or amido, and wherein each of the foregoing C₁-C₆ alkyl moieties in the definition of R₄₆ may be optionally substituted with one substituent selected from hydroxy, trifluoromethyl, amino, carboxy, amido, -NHC(=O)(C₁-C₄ alkyl), -NH(C₁-C₄ alkyl), -N(C₁-C₄ alkyl)(C₁-C₂ alkyl), -C(=O)O(C₁-C₄ alkyl), C₁-C₃ alkoxy, C₁-C₃ thioalkyl, fluoro, bromo, chloro, iodo, cyano and nitro;
R₄₁₁ is hydrogen, hydroxy, fluoro, chloro, -COO(C₁-C₂ alkyl), cyano or -C(=O)(C₁-C₂ alkyl; and
R₄₁₂ is hydrogen or C₁-C₄ alkyl; with the proviso that: (1) when X is -CR₄₆, A₃ is not linear alkyl, (2) when X is -CR₄₆, R₄₅ is unsubstituted cycloalkyl, R₄₃ and R₄₄ are both hydrogen, and R₄₆ is hydrogen or methyl, then A₃ is not -NHR₄₂ wherein R₄₂ is benzyl or thienylmethyl, and (3) when X is -CR₄₆, R₄₅ is p-bromophenyl, and R₄₃, R₄₄ and R₄₆ are methyl, then A₃ is not methylamino or hydroxyethylamino;
and when X is nitrogen, with the further proviso that:
(a) A₃ is not linear C₁-C₁₂ alkyl;
(b) R₄₅ is not a sugar group;
(c) when R₄₃ and R₄₄ are hydrogen and R₄₅ is chlorophenyl, then A₃ is not -NH-CH(CH₃)-(CH₂)₃-N(C₂H₅)₂;
(d) when R₄₃ and R₄₄ are hydrogen and A₃ is -NR₄₁R₄₂ wherein R₄₁ is C₃-C₇ cycloalkyl and R₄₂ is C₂-C₆ alkenyl, phenyl-(C₁-C₆ alkylene) or hetero-(C₁-C₆ alkylene) wherein the hetero radical is furyl, thienyl or pyridinyl, and wherein said phenyl may be substituted with fluoro, chloro, bromo or iodo, then R₄₅ is not tetrahydrofuranyl or tetrahydropyranyl;
(e) when R₄₃ is methoxy, methylthio or methylsulfonyl, R₄₄ is hydrogen, and R₄₅ is tetrahydrofuranyl or tetrahydropyranyl, then A₃ is not -NH(C₁-C₂ alkyl), morpholinyl, hydrazino or -NHC₂H₄C₆H₅, wherein the phenyl may be substituted with one methyl or two methoxy;
(f) when R₄₃ is hydrogen, C₁-C₆ alkyl, chloro, bromo, -SH or -S-(C₁-C₄ alkyl), R₄₄ is hydrogen, and R₄₅ is C₃-C₈ cycloalkyl, then A₃ is not hydrazino, -NH(C₁-C₂ alkyl) or -N(C₁-C₆ alkyl)(C₁-C₁₂ alkyl);
(g) when R₄₃ and R₄₄ are hydrogen and A₃ is -NH(CH₂)ₘCOOH wherein m is 1 to 12, then R₄₅ is not phenyl substituted with one of fluoro, chloro, bromo or iodo;
(h) when R₄₃ is hydrogen, hydroxy, methylthio, chloro or -NHbenzyl, R₄₄ is hydrogen, and R₄₅ is chlorophenyl or bromophenyl, then A₃ is not -NH(C₁-C₁₂ alkyl), -NHallyl, or -N(C₁-C₆ alkyl) (C₁-C₁₂ alkyl) wherein said C₁-C₁₂ alkyl may be substituted with -NC₂H₅, or -NH benzyl which may be substituted with one or two of bromo, chloro, fluoro, -NC₂H₅ phenyl or morpholinopropyl;
(i) when R₄₃ and R₄₄ are hydrogen and R₄₅ is nitrophenyl, then A₃ is not -NHR₄₂ wherein R₄₂ is phenyl, benzyl or C₁-C₁₂ alkyl which may be substituted by one or two of hydroxy;
(j) when R₄₃ is chloro or -O(C₁-C₆ alkyl), R₄₄ is hydrogen, and A₃ is -NR₄₁R₄₂ wherein R₄₁ and R₄₂ are independently hydrogen or C₁-C₆ alkyl, then R₄₅ is not chlorophenyl; and
(k) when R₄₃ is hydrogen, A₃ is benzyl or phenethyl, and R₄₄ is fluoro, chloro, bromo or iodo, then R₄₅ is not 5'-deoxy-ribofuranosyl or 5'-amino-5'-deoxyribofuranosyl, or its pharmaceutically acceptable salt.

8. A pharmaceutical composition for prophylaxis or therapy of a postoperative stress as claimed in claim 1 or claim 2, wherein the compound having CRF antagonist activating effect is a compound represented by the formula Ib 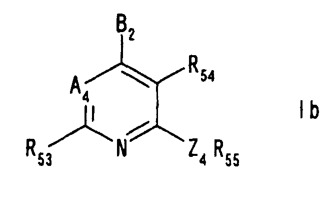 or IIb 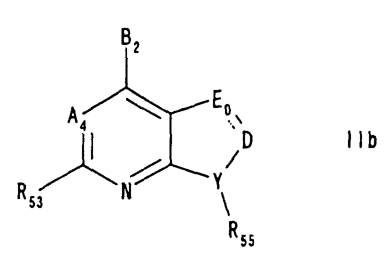 wherein the dashed line represents an optional double bond;
A₄ is -CR₅₇ or nitrogen;
B₂ is -NR₅₁R₅₂, -CR₅₁R₅₂R₅₁₁, -C(=CR₅₁R₅₁₂)R₅₂, -NHCR₅₁₁R₅₁R₅₂, -OCR₅₁₁R₅₁R₅₂, -SCR₅₁₁R₅₁R₅₂, -CR₅₁₁R₅₂OR₅₁, -CR₅₁₁R₅₂SR₅₁, -C(S)R₅₂, -NHNR₅₁R₅₂, -CR₅₂R₅₁₁NHR₅₁ or -C(O)R₅₂;
D is (i) nitrogen or -CR₅₁₀ when a double bond connects E₀ and D and E₀ is -CR₅₄; (ii) -CR₅₁₀ when a double bond connects E₀ and D and E₀ is nitrogen; or (iii) -CR₅₈R₅₉, -CHR₅₁₀, -C=O, -C=S, -C=NH or -C=NCH₃ when a single bond connects E₀ and D;
E₀ is -CR₅₄ or nitrogen when a double bond connects E₀ and D, and E₀ is -CR₅₄R₅₆ or -NR₅₆ when a single bond connects E₀ and D;
Y is nitrogen or -CH;
Z₄ is NH, oxygen, sulfur, -N(C₁-C₂ alkyl) or -CR₅₁₂R₅₁₃, wherein R₅₁₂ and R₅₁₃ are each independently hydrogen, trifluoromethyl or methyl, or one of R₅₁₂ and R₅₁₃ is cyano and the other is hydrogen or methyl;
R₅₁ is hydrogen or C₁-C₆ alkyl which may optionally be substituted with one or two substituents independently selected from hydroxy, cyano, nitro, fluoro, chloro, bromo, iodo, CF₃, C₁-C₄ alkoxy, -O-CO-(C₁-C₄ alkyl), -O-CO-NH(C₁-C₄ alkyl), -O-CO-N(C₁-C₄ alkyl)(C₁-C₂ alkyl), -NH(C₁-C₄ alkyl), -N(C₁-C₂ alkyl)(C₁-C₄ alkyl), -S(C₁-C₄ alkyl), -N(C₁-C₄ alkyl)CO(C₁-C₄ alkyl), -NHCO(C₁-C₄ alkyl), -CO₂(C₁-C₄ alkyl), -CONH(C₁-C₄ alkyl), -CON(C₁-C₄ alkyl)(C₁-C₂ alkyl), (C₁-C₄ alkyl)sulfinyl, (C₁-C₄ alkyl)sulfonyl and (C₁-C₄ alkyl)sulfanyl, and wherein said C₁-C₆ alkyl, C₁-C₄ alkoxy and C₁-C₄ alkyl moieties in the foregoing R₅₁ groups optionally contain one double or triple bond;
R₅₂ is C₁-C₆ alkyl, aryl or (aryl)(C₁-C₄ alkyl) wherein said aryl and aryl moieties of said (aryl)(C₁-C₄ alkyl) are selected from the group consisting of phenyl, naphthyl, thienyl, benzothienyl, pyridyl, quinolyl, pyrazinyl, pyrimidyl, imidazolyl, furanyl, benzofuranyl, benzothiazolyl, thiazolyl, isothiazolyl, benzisothiazolyl, benzisoxazolyl, benzimidazolyl, indolyl and benzoxazolyl; or R₅₂ is C₃-C₈ cycloalkyl or (C₃-C₈ cycloalkyl)(C₁-C₆ alkyl), wherein one or two of the ring carbons of said cycloalkyl moieties of said (C₃-C₈ cycloalkyl )(C₁-C₆ alkyl) having at least 4 ring members and cycloalkyl having at least 4 ring members is optionally replaced by oxygen, sulfur or -NR₅₁₄ wherein R₅₁₄ is hydrogen or C₁-C₄ alkyl; and wherein each of the foregoing R₅₂ groups is optionally substituted with from one to three substituents independently selected from chloro, fluoro and C₁-C₄ alkyl, or one substituent selected from bromo, iodo, cyano, nitro, C₁-C₆ alkoxy, -O-CO-(C₁-C₄ alkyl), -O-CO-N(C₁-C₄ alkyl)(C₁-C₂ alkyl), -CO₂(C₁-C₄ alkyl), (C₁-C₄ alkyl)sulfanyl, (C₁-C₄ alkyl)sulfinyl and (C₁-C₄ alkyl)sulfonyl, and wherein said C₁-C₆ alkyl, C₁-C₄ alkyl moieties and C₁-C₆ alkyl moieties of the foregoing R₅₂ groups optionally contain one carbon-carbon double or triple bond; or R₅₁ and R₅₂ of said -NR₅₁R₅₂ and said -CR₅₁R₅₂R₅₁₁ are taken together to form a saturated 5 to 8 member ring, wherein said ring optionally contains one or two carbon-carbon double bonds, and wherein one or two of the ring carbons is optionally replaced by oxygen or sulfur;
R₅₃ is hydrogen, C₁-C₆ alkyl, fluoro, chloro, bromo, iodo, hydroxy, amino, SH, -NH(C₁-C₄ alkyl), -N(C₁-C₄ alkyl)(C₁-C₂ alkyl), -CH₂OH, -CH₂OCH₃, -O(C₁-C₄ alkyl), (C₁-C₄ alkyl)sulfanyl, (C₁-C₄ alkyl)sulfonyl, or (C₁-C₄ alkyl)sulfinyl, wherein said C₁-C₆ alkyl and C₁-C₄ alky moieties of the foregoing R₅₃ groups optionally contain one double or triple bond and are optionally substituted with from one to three substituents independently selected from hydroxy, amino, C₁-C₃ alkoxy, -NH(C₁-C₂ alkyl), -N(C₁-C₂)₂, -NHCOCH₃, fluoro, chloro and C₁-C₃ thioalkyl;
R₅₄ is hydrogen, C₁-C₆ alkyl, fluoro, chloro, bromo, iodo, C₁-C₆ alkoxy, formyl, trifluoromethoxy, -CH₂OCH₃, -CH₂OCH₂CH₃, -CH₂CH₂OCH₃, -CH₂CF₃, CF₃, amino, nitro, -NH(C₁-C₄ alkyl), -N(CH₃)₂, -NHCOCH₃, -NMCONHCH₃, (C₁-C₄ alkyl)sulfanyl, (C₁-C₄ alkyl)sulfinyl, (C₁-C₄ alkyl)sulfonyl, cyano, hydroxy, -CO(C₁-C₄ alkyl), -CHO, or -CO₂ (C₁-C₄ alkyl), wherein said C₁-C₆ alkyl, C₁-C₆ alkoxy and the C₁-C₄ alkyl moieties of the foregoing R₅₄ groups optionally contain one double or triple bond and are optionally substituted with one substituent selected from hydroxy, amino, -NHCOCH₃, -NH(C₁-C₂ alkyl), -N(C₁-C₂ alkyl)₂, -CO₂(C₁-C₄ alkyl), -CO(C₁-C₄ alkyl), C₁-C₃ alkoxy, (C₁-C₃ alkyl)sulfany, fluoro, chloro, cyano and nitro;
R₅₅ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, azaindolyl, benzoxazolyl, oxazolyl, pyrrolidinyl, thiazolidinyl, morpholinyl, pyridinyl, tetrazolyl, or 3 to 8 membered cycloalkyl or 9 to 12 membered bicycloalkyl, wherein said cycloalkyl and bicycloalkyl optionally contain one or two of oxygen, sulfur or -N-G₀ wherein G₀ is hydrogen, C₁-C₄ alkyl, C₁-C₄ alkanoyl, phenyl or benzyl, wherein each of the above R₅₅ groups is optionally substituted with from one to three substituents independently selected from fluoro, chloro, C₁-C₆ alkyl, C₁-C₆ alkoxy and trifluoromethyl, or one substituent selected from bromo, iodo, cyano, nitro, amino, - NH(C₁-C₄ alkyl), -N(C₁-C₄ alkyl)(C₁-C₂ alkyl), -CO₂(C₁-C₄ alkyl), -CO(C₁-C₄ alkyl), -SO₂NH(C₁-C₄ alkyl), -SO₂N(C₁-C₄ alkyl)(C₁-C₂ alkyl), -SO₂NH₂, -NHSO₂(C₁-C₄ alkyl), -S(C₁-C₄ alkyl) and -SO₂(C₁-C₄ alkyl), wherein said C₁-C₄ alkyl moieties and C₁-C₆ alkyl moieties of the foregoing R₅₅ groups optionally contain one double or triple bond and are optionally substituted with one or two substituents independently selected from fluoro, chloro, hydroxy, amino, methylamino, dimethylamino and acetyl;
R₅₆ is hydrogen or C₁-C₆ alkyl, wherein said C₁-C₆ alkyl is optionally substituted with one of hydroxy, methoxy, ethoxy or fluoro;
R₅₇ is hydrogen, C₁-C₄ alkyl, fluoro, chloro, bromo, iodo, cyano, hydroxy, C₁-C₄ alkoxy, -CO(C₁-C₄ alkyl), -CO₂(C₁-C₄ alkyl), -OCF₃, CF₃, -CH₂OH, -CH₂OCH₃ or -CH₂OCH₂CH₃;
R₅₈ and R₅₉ are each independently hydrogen, hydroxy, methyl, ethyl, methoxy or ethoxy; or R₅₈ and R₅₉ taken together form an oxo (=O) group;
R₅₁₀ is hydrogen, C₁-C₆ alkyl, fluoro, chloro, bromo, iodo, C₁-C₆ alkoxy, formyl, amino, -NH(C₁-C₄ alkyl), -N(C₁-C₄ alkyl)(C₁-C₂ alkyl), -SOₙ(C₁-C₄ alkyl) wherein n is 0, 1 or 2, cyano, carboxy or amido, wherein said C₁-C₆ alkyl and C₁-C₄ alkyl moieties of the foregoing R₅₁₀ groups are optionally substituted with one of hydroxy, trifluoromethyl, amino, carboxy, amido, -NHCO(C₁-C₄ alkyl), -NH(C₁-C₄ alkyl), -N(C₁-C₄ alkyl)(C₁-C₂ alkyl), -CO₂(C₁-C₄ alkyl), C₁-C₃ alkoxy, C₁-C₃ thioalkyl, fluoro, bromo, chloro, iodo, cyano or nitro; and,
R₅₁₁ is hydrogen, hydroxy, fluoro, or methoxy, or its pharmaceutically acceptable salt.

9. A pharmaceutical composition for prophylaxis or therapy of a postoperative stress as claimed in claim 1 or claim 2, wherein the compound having CRF antagonist activating effect is a compound represented by the formula Ic, IIc or IIIc 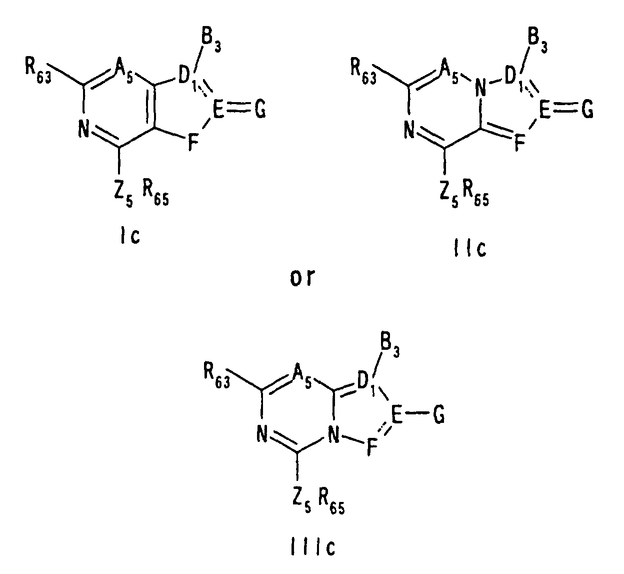 wherein the dashed lines represent optional double bonds;
A₅ is nitrogen or CR₆₇;
B₃ is -NR₆₁R₆₂, -CR₆₁R₆₂R₆₁₀, -C(=CR₆₂R₆₁₁)R₆₁, -NHCR₆₁R₆₂R₆₁₀, -OCR₆₁R₆₂R₆₁₀, -SCR₆₁R₆₂R₆₁₀, -CR₆₂R₆₁₀NHR₆₁, -CR₆₂R₆₁₀OR₆₁, -CR₆₂R₆₁₀SR₆₁ or -COR₆₂;
D₁ is nitrogen and is single bonded to all atoms to which it is attached, or D₁ is carbon and is either double bonded to E in formulas Ic and IIc or double bonded to the adjacent carbon to both fused rings in formulas IIIc, or D₁ is CH and is single bonded to E in formula Ic and IIc;
E is nitrogen, CH or carbon;
F is oxygen, sulfur, CHR₆₄ or NR₆₄ when it is single bonded to E, and F is nitrogen or CR₆₄ when it is double bonded to E;
G, when it is single bonded to E, is hydrogen, C1-C4 alkyl, -S(C₁-C₄ alkyl), -O(C₁-C₄ alkyl), NH₂, -NH(C₁-C₄ alkyl) or -N(C₁-C₂ alkyl)(C₁-C₄ alkyl), wherein each of the C₁-C₄ alkyl groups of G may optionally be substituted with one of hydroxy, -O(C₁-C₂ alkyl) or fluoro; G, when it is double bonded to E, is oxygen, sulfur or NH; and G, when E is nitrogen and double bonded to D₁ or F, is absent;
R₆₁ is hydrogen, C₁-C₆ alkyl optionally substituted with one or two substituents R₆₈ independently selected from hydroxyl, fluoro, chloro, bromo, iodo, C₁-C₄ alkoxy, CF₃, -C(=O)O-(C₁-C₄) alkyl, -OC(=O)(C₁-C₄ alkyl), -OC(=O)N(C₁-C₄ alkyl)(C₁-C₂ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -S(C₁-C₄ alkyl), -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl), and wherein each of the C₁-C₄ alkyl groups in the foregoing R₆₁ groups may optionally contain one or two double or triple bonds;
R₆₂ is C₁-C₁₂ alkyl which may optionally contain from one to three double or triple bonds, aryl or (C₁-C₄ alkylene)aryl, wherein said aryl and aryl moiety of said (C₁-C₄ alkylene)aryl is selected from phenyl, naphthyl, thienyl, benzothienyl, pyridyl, quinolyl, pyrazinyl, pyrimidyl, imidazolyl, furanyl, benzofuranyl, benzothiazolyl, isothiazolyl, pyrazolyl, pyrrolyl, indolyl, pyrrolopyridyl, oxazolyl and benzoxazolyl, or C₃-C₈ cycloalkyl or (C₁-C₆ alkylene)(C₃-C₈ cycloalkyl), wherein one or two of the carbon atoms of said cycloalkyl and 5 to 8 membered cycloalkyl moieties of said (C₁-C₆ alkylen)(C₃-C₈ cycloalkyl) may optionally and independently be replaced by oxygen, sulfur or NZ₆₂ wherein Z₆₂ is selected from hydrogen, C₁-C₄ alkyl, benzyl and C₁-C₄ alkanoyl, and wherein each of the foregoing R₆₂ groups may optionally be substituted with from one to three substituents independently selected from chloro, fluoro, hydroxyl and C₁-C₄ alkyl, or one substituent selected from bromo, iodo, C₁-C₆ alkoxy, -OC(=O)(C₁-C₆ alkyl), -OC(=O)N(C₁-C₄ alkyl)(C₁-C₂ alkyl), -S(C₁-C₆ alkyl), amino, -NH(C₁-C₂ alkyl), -N(C₁-C₂ alkyl)(C₁-C₄ alkyl), -N(C₁-C₄ alkyl)-CO-(C₁-C₄ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -SH, -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl); -NR₆₁R₆₂ or CR₆₁R₆₂R₆₁₀ may form a saturated 3 to 8 membered carbocyclic ring which may optionally contain from one to three double bonds and wherein one or two of the ring carbon atoms of such 5 to 8 membered rings may optionally and independently be replaced by oxygen, sulfur or NZ₆₃ wherein Z₆₃ is hydrogen, C₁-C₄ alkyl, benzyl or C₁-C₄ alkanoyl;
R₆₃ is hydrogen, C₁-C₄ alkyl, -O(C₁-C₄ alkyl), chloro, fluoro, bromo, iodo, -CN, -S(C₁-C₄ alkyl) or -SO₂(C₁-C₄ alkyl) wherein each of the (C₁∼C₄ alkyl) moieties in the foregoing R₆₃ groups may optionally be substituted with one substituent R₆₉ selected from hydroxy, fluoro, (C₁-C₂ alkoxy);
R₆₄ is each independently hydrogen, (C₁-C₆ alkyl), fluoro, chloro, bromo, iodo, hydroxy, cyano, amino, nitro, -O(C₁-C₄ alkyl), -N(C₁-C₄ alkyl)(C₁-C₂ alkyl), -S(C₁-C₄ alkyl), -SO(C₁-C₄ alkyl), -SO₂(C₁-C₄ alkyl), -CO(C₁-C₄ alkyl), -C(=O)H or -C(=O)O(C₁-C₄ alkyl), wherein each of the (C₁-C₆ alkyl) and (C₁-C₄ alkyl) moieties in the foregoing R₆₄ groups may optionally contain one or two double or triple bonds and may optionally be substituted with one or two substituents independently selected from hydroxy, amino, C₁-C₃ alkoxy, dimethylamino, methylamino, ethylamino, -NHC(=O)CH₃, fluoro, chloro, C₁-C₃ thioalkyl, -CN, -COOH, -C(=O)O(C₁-C₄ alkyl), -C(=O)(C₁-C₄ alkyl) and -NO₂;
R₆₅ is phenyl, naphthyl, thienyl, benzothienyl, pyridyl, quinolyl, pyrazinyl, furanyl, benzofuranyl, benzothiazolyl, benzisothiazolyl, benzisoxazolyl, benzimidazolyl, indolyl, benzoxazolyl or C₃-C₈ cycloalkyl, wherein one or two of the carbon atoms of said cycloalkyl rings that contain at least 5 ring members may optionally and independently be replaced by oxygen, sulfur or NZ₆₄ wherein Z₆₄ is hydrogen, C₁-C₄ alkyl or benzyl; and wherein each of the foregoing R₆₅ groups may optionally be substituted with from one to four substituents R₆₁₂, wherein one to three of said substituents may be selected independently from chloro, C₁-C₆ alkyl and -O(C₁-C₆ alkyl) and one of said substituents may be selected from bromo, iodo, formyl, -CN, -CF₃, -NO₂, -NH₂, -NH(C₁-C₄ alkyl), -N(C₁-C₂ alkyl)(C₁-C₆ alkyl), -C(=O)O(C₁-C₄ alkyl), -C(=O)(C₁-C₄ alkyl), -COOH, -SO₂NH(C₁-C₄ alkyl), -SO₂N(C₁-C₂ alkyl)(C₁-C₄ alkyl), -SO₂NH₂, -NMSO₂(C₁-C₄ alkyl), -S(C₁-C₆ alkyl) and -SO₂(C₁-C₆ alkyl), and wherein each of the C₁-C₄ alkyl and C₁-C₆ alkyl moieties in the foregoing R₆₅ groups may optionally be substituted with one or two substituents independently selected from fluoro, hydroxyl, amino, methylamino, dimethylamino and acetyl;
R₆₇ is hydrogen, C₁-C₄ alkyl, halo, cyano, hydroxy, -O(C₁-C₄ alkyl), -C(=O)(C₁-C₄ alkyl), C(=O)O(C₁-C₄ alkyl), -OCF₃, -CF₃, -CH₂OH, -CH₂O(C₁-C₄ alkyl);
R₆₁₀ is hydrogen, hydroxy, methoxy or fluoro;
R₆₁₁ is hydrogen or C₁-C₄ alkyl; and
Z₅ is NH, oxygen, sulfur, -N(C₁-C₄ alkyl), -NC(=O)(C₁-C₂ alkyl), NC(=O)O(C₁-C₂ alkyl) or CR₆₁₃R₆₁₄ wherein R₆₁₃ and R₆₁₄ are independently selected from hydrogen, trifluoromethyl and methyl with the exception that one of R₆₁₃ and R₆₁₄ can be cyano; with the proviso that (a) in the five membered rings of structures Ic, IIc and IIIc, there can not be two double bonds adjacent to each other; and (b) when R₆₄ is attached to nitrogen, it is not halo, cyano or nitro,
or its pharmaceutically acceptable salt.

10. A pharmaceutical composition for prophylaxis or therapy of a postoperative stress as claimed in claim 1 or claim 2, wherein the compound having CRF antagonist activating effect is a compound represented by the formula Id 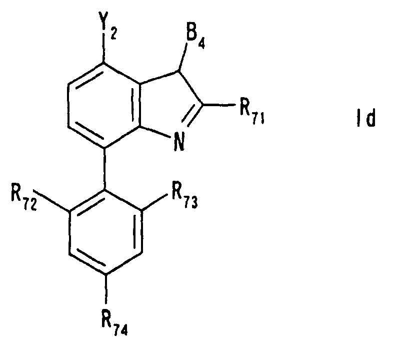 wherein B₄ is hydrogen or (C₁-C₁₀) linear or branched alkyl;
Y₂ is hydrogen or methyl;
R₇₁ is hydrogen or (C₁-C₁₀) linear or branched alkyl, wherein said alkyl may optionally be substituted with one or more substituents selected from halo, -S(C₁-C₄)alkyl, amino, -NH(C₁-C₄)alkyl and -N((C₁-C₄)alkyl))₂;
R₇₂, R₇₃ and R₇₄ are selected independently from hydrogen, fluoro, chloro, (C₁-C₆)alkyl and (C₁-C₆)alkoxy; or two of R₇₂, R₇₃, and R₇₄ are hydrogen and the other is selected from hydroxy, iodo, bromo, formyl, cyano, nitro, trifluoromethyl, amino, (C₁-C₆)alkyl-O-(C₁-C₆)alkyl, -NHCH₃, -N(CH₃)₂, -COCH, -COO(C₁-C₄)alkyl, -CO(C₁-C₄)alkyl, -SO₂-NH(C₁-C₆)alkyl, -SO₂-N((C₁-C₄)alkyl)₂, -SO₂NH₂, -NHSO₂-(C₁-C₄)alkyl, -S(C₁-C₆)alkyl and -SO₂-(C₁-C₆)alkyl, and wherein said C₁-C₄ moieties and C₁-C₆ alkyl moieties in the foregoing R₇₂, R₇₃ and R₇₄ groups may optionally be substituted with one or two of fluoro or one substituent selected from hydroxy, amino, methylamino, dimethylamino and acetyl,
or its pharmaceutically acceptable salt.

11. A pharmaceutical composition for prophylaxis or therapy of a postoperative stress as claimed in claim 1 or claim 2, wherein the compound having CRF antagonist activating effect is a compound represented by the formula Ie 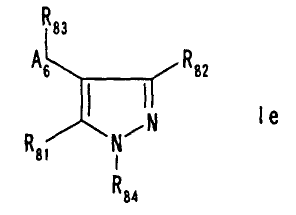 wherein A₆ is C=O or SO₂, or A₆ and R₈₁ together with the carbons to which they are attached form pyrimidinyl or 5-pyridyl which may be substituted with R₈₅ wherein R₈₅ is hydrogen, C₁-C₆ alkyl, fluoro, chloro, bromo, hydroxy, amino, O(C₁-C₆ alkyl), NH(C₁-C₆ alkyl), N(C₁-C₆ alkyl)(C₁-C₆ alkyl), SH or S(O)ₙ(C₁-C₆ alkyl) wherein n is 0, 1 or 2, and said C₁-C₆ alkyl may contain one or two double or triple bonds and may be substituted with from one to three substituents R₈₆, wherein R₈₆ is hydroxy, amino, C₁-C₃ alkoxy, dimethylamino, diethylamino, methylamino, ethylamino, NH(C=O)CH₃, fluoro, chloro, bromo or C₁-C₃ thioalkyl;
R₈₁ is hydrogen, C₁-C₆ alkyl, amino, O(C₁-C₆ alkyl), NH(C₁-C₆ alkyl), N(C₁-C₆ alkyl)(C₁-C₆ alkyl), wherein said C₁-C₆ alkyl may optionally contain one or two double or triple bonds and may be substituted with from one to three substituents R₈₆ as defined above;
R₈₂ is hydrogen, C₁-C₆ alkyl, hydroxy, amino, O(C₁-C₆ alkyl), NH(C₁-C₆ alkyl), N(C₁-C₆ alkyl)(C₁-C₆ alkyl), SH, S(O)ₙ(C₁-C₆ alkyl) wherein n is 0, 1, or 2, cyano, hydroxy, carboxy or amido, wherein said alkyl may be substituted with one to three of hydroxy, amino, carboxy, amido, NH(C=O)(C₁-C₆ alkyl), N(C₁-C₆ alkyl)(C₁-C₆ alkyl), (C=O)O(C₁-C₆ alkyl), C₁-C₃ alkoxy, C₁-C₃ thioalkyl, fluoro, bromo, chloro, iodo, cyano or nitro;
R₈₃ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, azaindolyl, benzoxazolyl, oxazolyl, pyrrolidinyl, thiazolidinyl, morpholinyl, pyridinyl, tetrazolyl, or 3 to 8 membered cycloalkyl or 9 to 12 membered bicycloalkyl, optionally containing one to three of oxygen, sulfur or N-Z₆ wherein Z₆ is hydrogen, C₁-C₄ alkyl, C₁-C₄ alkanoyl, phenyl or phenylmethyl, wherein each one of the above groups may be substituted independently with from one to three of fluoro, chloro, bromo, trifluoromethyl, C₁-C₆ alkyl or C₁-C₆ alkoxy, or one or two of cyano, nitro, amino, NH(C₁-C₆ alkyl), N(C₁-C₄ alkyl)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NHSO₂ (C₁-C₄ alkyl), S(C₁-C₆ alkyl) or SO₂(C₁-C₆ alkyl), wherein said C₁-C₄ alkyl and C₁-C₆ alkyl may be substituted with fluoro, chloro, hydroxy, amino, methylamino, dimethylamino or acetyl; and
R₈₄ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, azaindolyl, benzoxazolyl, oxazolyl, pyrrolidinyl, thiazolidinyl, morpholinyl, pyridinyl, tetrazolyl, or 9 to 12 membered bicycloalkyl, optionally containing one to three of oxygen, sulfur or N-Z₆ wherein Z₆ is hydrogen, C₁-C₄ alkyl, C₁-C₄ alkanoyl, phenyl or phenylmethyl, wherein each of the above groups may be substituted independently with from one to three of fluoro, chloro, bromo, trifluoromethyl, C₁-C₆ alkyl or C₁-C₆ alkoxy, or one of cyano, nitro, amino, NH(C₁-C₆ alkyl), N(C₁-C₄ alkyl)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NHSO₂(C₁-C₄ alkyl), S(C₁-C₆ alkyl) or SO₂(C₁-C₆ alkyl), wherein said C₁-C₄ alkyl and C₁-C₆ alkyl may be substituted with one or two of fluoro, chloro, hydroxy, amino, methylamino, dimethylamino or acetyl; with the proviso that (1) R₈₄ is not unsubstituted phenyl; (2) when R₈₁ is amino, R₈₂ is methylthio, R₈₄ is 2,4,6-trifluorophenyl, and A₆ is C=O, then R₈₃ is not 2-chloropheny,
or its pharmaceutically acceptable salt.

12. A pharmaceutical composition for prophylaxis or therapy of a postoperative stress as claimed in claim 1 or claim 2, wherein the compound having CRF antagonist activating effect is a compound represented by the formula If 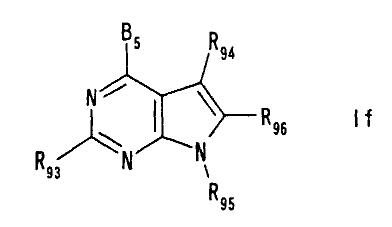 wherein B₅ is NR₉₁R₉₂, CR₉₁R₉₂R₉₁₁, C(=CR₉₂R₉₁₂)R₉₁, NHCR₉₁R₉₂R₉₁₁, OCR₉₁R₉₂R₉₁₁, SCR₉₁R₉₂R₉₁₁, NHNR₉₁R₉₂, CR₉₂R₉₁₁NHR₉₁, CR₉₂R₉₁₁OR₉₁, CR₉₂R₉₁₁SR₉₁ or C(O)R₉₂;
R₉₁ is hydrogen, or C₁-C₆ alkyl which may be substituted with one or two substituents R₉₇ independently selected from the group consisting of hydroxy, fluoro, chloro, bromo, iodo, C₁-C₈ alkoxy, O-C(=O)-(C₁₋₆ alkyl), O-C(=O)-NH(C₁₋₄ alkyl), O-C(=O)-N(C₁₋₄ alkyl)(C₁₋₂ alkyl), amino, NH(C₁₋₄ alkyl), N(C₁₋₂ alkyl)(C₁₋₄ alkyl), S(C₁₋₆ alkyl), N(C₁₋₄ alkyl)C(=O)(C₁₋₄ alkyl), NHC(=O)(C₁₋₄ alkyl), COOH, C(=O)O(C₁₋₄ alkyl), C(=O)NH(C₁₋₄ alkyl), C(=O)N(C₁₋₄ alkyl)(C₁₋₂ alkyl), SH, CN, NO₂, SO(C₁₋₄ alkyl), SO₂(C₁₋₄ alkyl), SO₂NH(C₁₋₄ alkyl) and SO₂N(C₁₋₄ alkyl)(C₁₋₂ alkyl), and wherein said C₁-C₆ alkyl may contain one or two double or triple bonds;
R₉₂ is C₁-C₁₂ alkyl, aryl or (C₁-C₁₀ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiolyl, thiazolyl, isoxazolyl, benzisoxazolyl, benzimidazolyl, triazolyl, pyrazolyl, pyrrolyl, indolyl, pyrrolpyridyl, oxazolyl or benzoxazolyl, or 3 to 8 membered cycloalkyl or (C₁-C₆ alkylene) cycloalkyl, wherein said cycloalkyl may contain one or two of oxygen, sulfur or N-Z₇ wherein Z₇ is hydrogen, C₁-C₄ alkyl, benzyl or C₁-C₄ alkanoyl, wherein R₉₂ may be substituted independently by from one to three of chloro, fluoro, or C₁-C₄ alkyl, or one of hydroxy, bromo, iodo, C₁-C₆ alkoxy, O-C(=O)-(C₁₋C₆ alkyl), O-C(=O)-N(C₁₋C₄ alkyl)(C₁₋C₂ alkyl), S(C₁₋C₆ alkyl), NH₂, NH(C₁₋C₂ alkyl), N(C₁₋C₂ alkyl)(C₁₋C₄ alkyl) N(C₁₋C₄ alkyl)C(=O)(C₁₋C₄ alkyl), NHC(=O)(C₁₋C₄ alkyl), COOH, C(=O)O(C₁₋C₄ alkyl), C(=O)NH(C₁₋C₄ alkyl), C(=O)N(C₁₋C₄ alkyl)(C₁₋C₂ alkyl), SH, CN, NO₂, SO(C₁₋C₄ alkyl), SO₂(C₁₋C₄ alkyl), SO₂NH(C₁₋C₄ alkyl), SO₂N(C₁₋C₄ alkyl)(C₁₋C₂ alkyl), and wherein said C₁-C₁₂ alkyl or C₁-C₁₀ alkylene may contain one to three double or triple bonds; or NR₉₁R₉₂ or CR₉₁R₉₂R₉₁₁ may form a saturated 3 to 8 membered carbocyclic ring of which the 5 to 8 membered ring may optionally contain one or two double bonds or one or two of O, S or N-Z₇ wherein Z₇ is hydrogen, C₁-C₄ alkyl, benzyl, or C₁-C₄ alkanoyl;
R₉₃ is hydrogen, C₁-C₆ alkyl, fluoro, chloro, bromo, iodo, hydroxy, amino, O(C₁₋C₆ alkyl), NH(C₁₋C₆ alkyl), N(C₁₋C₄ alkyl)(C₁₋C₂ alkyl), SH, S(C₁₋C₄ alkyl), SO(C₁₋C₄ alkyl) or SO₂(C₁₋C₄ alkyl), wherein said C₁-C₄ alkyl and C₁-C₆ alkyl may contain one double or triple bond and may be substituted with from one to three substituents R₉₈ independently selected from the group consisting of hydroxy, C₁-C₃ alkoxy, fluoro, chloro or C₁-C₃ thioalkyl;
R₉₄ is hydrogen, C₁-C₆ alkyl, fluoro, chloro, bromo, iodo, C₁-C₆ alkoxy, amino, NH(C₁-C₆ alkyl), N(C₁-C₆ alkyl)(C₁-C₂ alkyl), SOₙ(C₁-C₆ alkyl) wherein n is 0, 1 or 2, cyano, hydroxy, carboxy, or amido, wherein said C₁-C₆ alkyl may be substituted with one of hydroxy, trifluoromethyl, amino, carboxy, amido, NHC(=O)(C₁₋₄ alkyl), NH(C₁₋₄ alkyl), N(C₁₋₄ alkyl)(C₁₋₂ alkyl), C(=O)O(C₁₋₄ alkyl), C₁-C₃ alkoxy, C₁-C₄ thioalkyl, fluoro, bromo, chloro, iodo, cyano or nitro;
R₉₅ is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, pyrrolopyridyl, benzoxazolyl, oxazolyl, pyrrolidinyl, thiazolidinyl, morpholinyl, piperidinyl, piperazinyl, tetrazolyl, or 3 to 8 membered cycloalkyl or 9 to 12 membered bicycloalkyl, optionally containing one or two of oxygen, sulfur or N-Z₇ wherein Z₇ is hydrogen, C₁-C₄ alkyl, C₁-C₄ alkanoyl, phenyl or phenylmethyl, wherein each one of the above groups may be substituted independently with from one to three of fluoro, chloro, bromo, formyl, C₁-C₆ alkyl, C₁-C₆ alkoxy or trifluoromethyl, or one of hydroxy, iodo, cyano, nitro, amino, NH(C₁₋₄ alkyl), N(C₁₋₄ alkyl)(C₁₋₂ alkyl), COO(C₁₋₄ alkyl), CO(C₁₋₄ alkyl), SO₂NH(C₁₋₄ alkyl), SO₂N(C₁₋₄ alkyl)(C₁₋₂ alkyl), SO₂NH₂, NHSO₂(C₁₋₄ alkyl), S(C₁₋₆ alkyl), SO₂(C₁₋₆ alkyl), wherein said C₁-C₄ alkyl and C₁-C₆ alkyl may be substituted with one or two of fluoro, chloro, hydroxy, C₁-C₄ alkoxy, amino, methylamino, dimethylamino or acetyl, wherein said C1-C4 alkyl and C1-C6 alkyl may contain one double or triple bond; with the proviso that R₉₅ is not unsubstituted phenyl;
R₉₆ is hydrogen, C₁-C₆ alkyl, fluoro, chloro, bromo, iodo, C₁-C₆ alkoxy, formyl, amino, NH(C₁-C₆ alkyl), N(C₁-C₆ alkyl)(C₁-C₂ alkyl), SOₙ(C₁-C₆ alkyl) wherein n is 0, 1 or 2, cyano, carboxy or amido, wherein said C₁-C₆ alkyl may be substituted with one of hydroxy, trifluoromethyl, amino, carboxy, amido, NHC(=O)(C₁₋C₄ alkyl), NH(C₁₋C₄ alkyl), N(C₁₋C₄ alkyl)(C₁₋C₂ alkyl), C(=O)O(C₁₋C₄ alkyl), C₁-C₃ alkoxy, C₁-C₃ thioalkyl, fluoro, bromo, chloro, iodo, cyano or nitro;
R₉₁₁ is hydrogen, hydroxy, fluoro, COO(C₁-C₂ alkyl), cyano or CO(C₁-C₂ alkyl);
R₉₁₂ is hydrogen or C₁-C₄ alkyl, with the proviso that (1) B₅ is not C₁-C₁₂ linear alkyl; (2) when R₉₅ is unsubstituted cycloalkyl, R₉₃ and R₉₄ are hydrogen, and when R₉₆ is hydrogen or methyl, then B₅ is not NHR₉₂ wherein R₉₂ is benzyl or thienylmethyl; and (3) when R₉₅ is p-bromophenyl, and R₉₃, R₉₄ and R₉₆ are methyl, then B₅ is not methylamino or hydroxyethylamino,
or its pharmaceutically acceptable acid addition salt.

13. A pharmaceutical composition for prophylaxis or therapy of a postoperative stress as claimed in claim 1 or claim 2, wherein the compound having CRF antagonist activating effect is a compound represented by the formula Ig 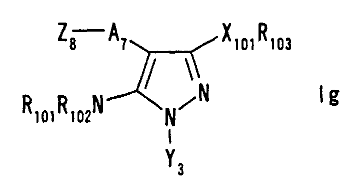 wherein A₇ is CH₂; X₁₀₁ is covalent bond, CH₂, NR₁₀₀ wherein R₁₀₀ is hydrogen, linear C₁-C₆ alkyl or branched C₃-C₈ alkyl, oxygen or sulfur;
R₁₀₁, R₁₀₂ and R₁₀₃ are each independently linear C₁-C₆ alkyl, branched C₃-C₈ alkyl, C₃-C₈ alkenyl wherein the double bond is not adjacent to nitrogen or X₁₀₁ when X₁₀₁ is oxygen or sulfur, or C₃-C₇ cycloalkyl (CH₂)ₙ wherein n is 0, 1, 2, 3 or 4; or R₁₀₁ and R₁₀₂ may be taken together with the nitrogen to form a saturated 4, 5 or 6 membered ring which is optionally bonded with benzo, R₁₀₃ may also be (CH₂)_{q}Q₁₀₁R₁₀₁₉ wherein q is 0, 1 or 2 and Q₁₀₁ is oxygen, sulfur, NH, N(C₁-C₆ alkyl), or a covalent bond when X₁₀₁ is not a covalent bond, and R₁₀₁₉ is hydrogen, linear C₁-C₆ alkyl, branched C₃-C₈ alkyl, C₃-C₈ alkenyl or C₃-C₆ cycloalkyl(CH₂)ₙ wherein n is 0 to 4;
Y₃ is phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl or piperidinyl, wherein each of the above groups may be substituted with one to three of any one of fluoro, chloro, bromo, or methyl, or one of trifluoromethyl; with the proviso that Y₃ is not unsubstituted phenyl; and
Z₈ is IIg
(a) 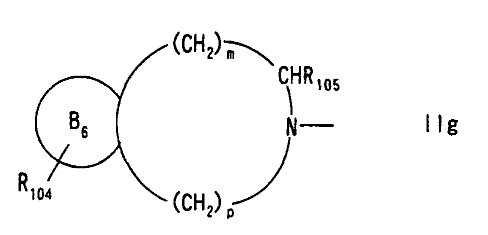 wherein the B₆ ring is phenyl, naphthyl, pyridyl, pyrimidinyl, pyrazinyl, pyridazinyl, triazilyl, pyrrolyl, pyrazolyl, imidazolyl, triazolyl, thienyl, or indolyl, wherein each of the above groups may be substituted with methyl, methoxy, fluoro, chloro, bromo or iodo; or a saturated 5 or 6 membered carbocyclic ring or a partially unsaturated ring having one or two double bonds;
R₁₀₄ is hydrogen, C₁-C₆ alkyl, C₁-C₆ alkoxy, or hydroxy, fluoro, chloro, bromo, iodo or trifluoromethyl;
R₁₀₅ is hydrogen, linear C₁-C₆ alkyl, branched C₃-C₈ alkyl, C₃-C₈ alkenyl or (CH₂)ₒ-X₁₀₂-(CH₂)ᵣ-Q₁₀₂-R₁₀₆;
R₁₀₆ is hydrogen, linear C₁-C₆ alkyl, branched C₃-C₈ alkyl or C₃-C₈ alkenyl;
X₁₀₂ and Q₁₀₂ are each independently oxygen, sulfur, NH or N(C₁-C₆ alkyl), or one of X₁₀₂ and Q₁₀₂ may be a covalent bond;
m is 0 or 1;
o is 1 or 2;
p is 1 or 2;
r is 0, 1, or 2;
(b) 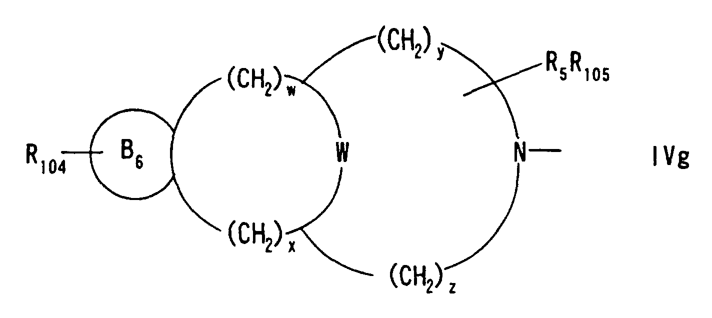 wherein R₁₀₄ and R₁₀₅ are as defined above, and t and u are each independently 1 or 2;
(c) -NR₁₀₇R₁₀₈ wherein R₁₀₇ and R₁₀₈ are each independently hydrogen or C₁-C₆ linear alkyl, branched C₃-C₈ alkyl, C₃-C₈ alkenyl, (CH₂)ᵥCH₂OH, (CH₂)ᵥNR₁₀₉R₁₀₁₀ wherein v is 0 to 3 and R₁₀₉ and R₁₀₁₀ are each independently hydrogen or linear C₁-C₆ alkyl, C₃-C₁₂ cycloalkyl, (C₃-C₁₂ cycloalkyl)(CH₂)ₙ, (C₆-C₁₀ bicycloalkyl)(CH₂)ₙ, wherein n is 0 to 4, benzofused C₃-C₆ cycloalkyl, C₁-C₆ hydroxyalkyl, phenyl, phenyl (C₁-C₃ alkylene), wherein each of the above groups may be substituted with one or two of hydroxy, fluoro, chloro, bromo, C₁-C₅ alkyl or C₁-C₅ alkoxy; or R₁₀₇ and R₁₀₈ may be taken together with the nitrogen to form a saturated or partially unsaturated 5 to 7 membered ring which may contain one of oxygen, sulfur, NH or N(C₁-C₆ alkyl) and which may be substituted by C₁-C₆ alkyl, hydroxy or phenyl, wherein any double bonds are not adjacent to any heteroatoms;
(d) 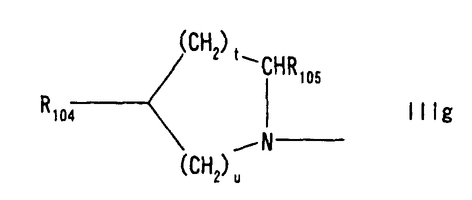 wherein B₆, R₁₀₄ and R₁₀₅ are as defined above, w, x, y and z are each independently 1 or 2, and W₁ is (CH₂)_{q} wherein q is as defined above, N(C₁-C₆ alkyl), or oxygen;
(e) 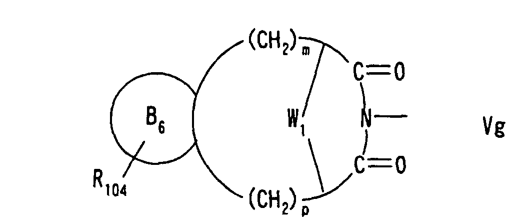 wherein B₆, W₁, R₁₀₄, m and p are as defined above;
(f) 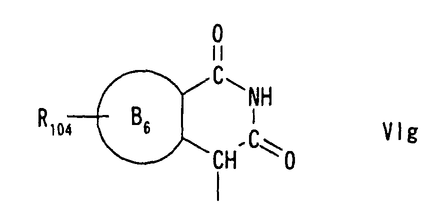 wherein B₆ and R₁₀₄ are as defined above;
(g) O(CH₂)ᵥR₁₀₁₁
wherein v is 0 to 3 and R₁₀₁₁ is linear C₁-C₆ alkyl, branched C₃-C₈ alkyl, phenyl, naphthyl, 1,2,3,4-tetrahydronaphthyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, piperidinyl, or thienyl, wherein each of the above groups may be substituted with one or two of any one of fluoro, chloro, bromo, methyl or trifluoromethyl;
(h) 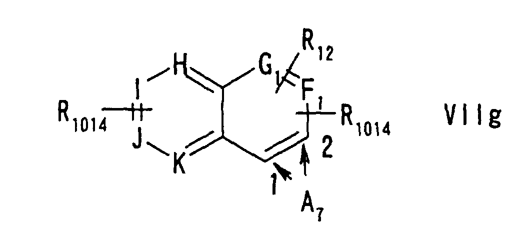 wherein A₇ is as defined above and is attached to position 1 or 2 while R₁₀₁₄ is attached to position 2 or 1 respectively; F₁, G₁, H, I, J and K are independently carbon or nitrogen, with the proviso that not more than three of H, I, J and K are nitrogen with not more than two adjacent nitrogens; R₁₀₁₂ and R₁₀₁₃ each independently are hydrogen, linear C₁-C₆ alkyl, branched C₃-C₈ alkyl, C₃-C₈ alkenyl, fluoro, chloro, bromo, trifluoromethyl, hydroxy, thiol, C₁-C₁₂ alkoxy, C₁-C₁₂ thioalkanyl, or C₃-C₁₂ alkenoxy or C₃-C₁₂ thioalkenyl wherein the double bond is not adjacent to oxygen or sulfur; and R₁₀₁₄ is hydroxy, C₁-C₁₂ alkoxy, C₃-C₁₂ alkenoxy, wherein the double bond is not adjacent to oxygen, or -X₁₀₂-(CH₂)ᵣQ₁₀₂R₁₀₆ wherein X₁₀₂, r, Q₁₀₂ and R₁₀₆ are as defined above in paragraph (a) except that Q₁₀₂ is not sulfur, or R₁₀₁₄ is NR₁₀₁₅R₁₀₁₆ wherein R₁₀₁₅ and R₁₀₁₆ are each independently hydrogen, linear C₁-C₆ alkyl, branched C₃-C₈ alkyl, C₃-C₈ alkenyl, wherein the double bond is not adjacent to nitrogen, or C₃-C₇ cycloalkyl-(CH₂)ₙ wherein n is as defined above, or R₁₀₁₅ and R₁₀₁₆ may be taken together with the nitrogen to form a saturated 5 or 6 membered ring optionally condensed with benzo; or
(i) 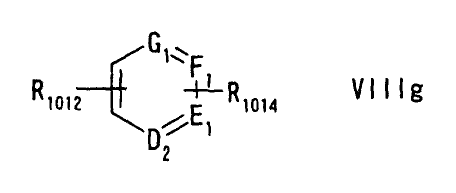 wherein D₂, E₁, F₁ and G₁ are independently carbon or nitrogen, with the proviso that not more than two of D₂, E₁, F₁ and G₁ are nitrogen, R₁₀₁₂ and R₁₀₁₄ are as defined above (h) and A₇ which is defined above is attached to a carbon in formula VIIIg, and R₁₀₁₄ is attached to a carbon located adjacent to the carbon to which A₇ is attached,
or its pharmaceutically acceptable acid addition salt.

14. A pharmaceutical composition for prophylaxis or therapy of a postoperative stress as claimed in claim 1 or claim 2, wherein the compound having CRF antagonist activating effect is a compound represented by the formula Ih 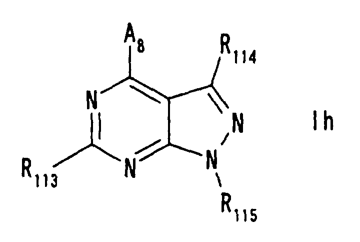 wherein A₈ is NR₁₁₁R₁₁₂, CR₁₁₁R₁₁₂R₁₁₁₁, C(=CR₁₁₁R₁₁₁₂)R₁₁₂, NHCR₁₁₁R₁₁₂R₁₁₁₁, OCR₁₁₁R₁₁₂R₁₁₁₁, SCR₁₁₁R₁₁₂R₁₁₁₁, NHNR₁₁₁R₁₁₂, CR₁₁₂R₁₁₁₁NHR₁₁₁, CR₁₁₂R₁₁₁₁OR₁₁₁, CR₁₁₂R₁₁₁₁SR₁₁₁ or C(O)R₁₁₂;
R₁₁₁ is hydrogen, or C₁-C₆ alkyl which may be substituted with one or two substituents R₁₁₆ independently selected from the group consisting of hydroxy, fluoro, chloro, bromo, iodo, C₁-C₆ alkoxy, O-C(=O)-(C₁-C₆ alkyl), O-C(=O)-N(C₁-C₄ alkyl)(C₁-C₂ alkyl), amino, NH(C₁-C₄ alkyl), N(C₁-C₂ alkyl)(C₁-C₄ alkyl), S(C₁-C₆ alkyl), OC(O)NH(C₁-C₄ alkyl), N(C₁-C₂ alkyl)C(O)(C₁-C₄ alkyl), NHC(=O)(C₁-C₄ alkyl), COOH, C(=O)O(C₁-C₄ alkyl), C(=O)NH(C₁-C₄ alkyl), C(=O)N(C₁-C₄ alkyl)(C₁-C₂ alkyl), SH, CN, NO₂, SO(C₁-C₄ alkyl), SO₂(C₁-C₄ alkyl), SO₂NH(C₁-C₄ alkyl) and SO₂N(C₁-C₄ alkyl)(C₁-C₂ alkyl), and wherein said C₁-C₆ alkyl may contain one or two double or triple bonds;
R₁₁₂ is C₁-C₁₂ alkyl, aryl or (C₁-C₁₀ alkylene)aryl wherein said aryl is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, azaindolyl, oxazolyl, or benzoxazolyl, or 3 to 8 membered cycloalkyl or (C₁-C₆ alkylene)cycloalkyl, wherein said cycloalkyl may contain one or two of oxygen, sulfur or N-Z₉ wherein Z₉ is hydrogen, C₁-C₄ alkyl, benzyl or C₁-C₄ alkanoyl, wherein R₁₁₂ may be substituted independently with from one to three of chloro, fluoro, or C₁-C₄ alkyl, or one of hydroxy, bromo, iodo, C₁-C₆ alkoxy, O-C(=O)-(C₁-C₆ alkyl), O-C(=O)-N(C₁-C₄ alkyl)(C₁-C₂ alkyl), S(C₁-C₆ alkyl), NH₂, NH(C₁-C₂ alkyl), N(C₁-C₂ alkyl)(C₁-C₄ alkyl), N(C₁-C₄ alkyl)C(=O)(C₁-C₄ alkyl), NHC(=O)(C₁-C₄ alkyl), COOH, C(=O)O(C₁-C₄ alkyl), C(=O)NH(C₁-C₄ alkyl),C(=O)N(C₁-C₄ alkyl)(C₁-C₂ alkyl), SH, CN, NO₂, SO(C₁-C₄ alkyl), SO₂(C₁-C₄ alkyl), SO₂NH(C₁-C₄ alkyl) or SO₂N(C₁-C₄ alkyl)(C₁-C₂ alkyl), and wherein said C₁-C₁₂ alkyl or C₁-C₁₀ alkylene may contain one to two double or triple bonds; or
NR₁₁₁R₁₁₂ or CR₁₁₁R₁₁₂R₁₁₁₁ may form a saturated 4 to 8 membered ring optionally containing one or two double bonds or one or two of oxygen, sulfur or N-Z₉ wherein Z₉ is hydrogen, C₁-C₄ alkyl, benzyl, or C₁-C₄ alkanoyl;
R₁₁₃ is hydrogen, C₁-C₆ alkyl, fluoro, chloro, bromo, iodo, hydroxy, amino, O(C₁-C₆ alkyl), NH(C₁-C₆ alkyl), N(C₁-C₄ alkyl)(C₁-C₂ alkyl), SH, S(C₁-C₄ alkyl), SO(C₁-C₄ alkyl) or SO₂(C₁-C₄ alkyl), wherein said C₁-C₄ alkyl and C₁-C₆ alkyl may contain one or two double or triple bonds and may be substituted with from one to three substituents R₁₁₇ independently selected from the group consisting of hydroxy, amino, C₁-C₃ alkoxy, dimethylamino, diethylamino, methylamino, ethylamino, NHC(=O)CH₃, fluoro, chloro or C₁-C₃ thioalkyl;
R₁₁₄ is hydrogen, C₁-C₆ alkyl, fluoro, chloro, bromo, iodo, C₁-C₆ alkoxy, amino, NH(C₁-C₆ alkyl), N(C₁-C₆ alkyl) (C₁-C₂ alkyl), SOₙ(C₁-C₆ alkyl) wherein n is 0, 1 or 2, cyano, hydroxy, carboxy, or amido, wherein said C₁-C₆ alkyl may be substituted with one to three of hydroxy, amino, carboxy, amido, NHC(=O)(C₁-C₄ alkyl), NH(C₁-C₄ alkyl), N(C₁-C₄ alkyl)(C₁-C₂ alkyl), C(=O)O(C₁-C₄ alkyl), C₁-C₃ alkoxy, C₁-C₃ thioalkyl, fluoro, bromo, chloro, iodo, cyano or nitro;
R₁₁₅ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, pyrrolopyridyl, benzoxazolyl, oxazolyl, pyrrolidinyl, thiazolidinyl, piperazinyl, piperidinyl, tetrazolyl, or 3 to 8 membered cycloalkyl or 9 to 12 membered bicycloalkyl, optionally containing one or two of oxygen, sulfur or N-Z₉ wherein Z₉ is hydrogen, C₁-C₄ alkyl, C₁-C₄ alkanoyl, phenyl or benzyl, wherein each one of the above groups may be substituted independently with from one to three of fluoro, chloro, bromo, formyl, C₁-C₄ alkyl, C₁-C₄ alkoxy or trifluoromethyl, or one of hydroxy, iodo, cyano, nitro, amino, cyclopropyl, NH(C₁-C₄ alkyl), N(C₁-C₄ alkyl)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NHSO₂(C₁-C₄ alkyl), S(C₁-C₆ alkyl), SO₂(C₁-C₆ alkyl), wherein said C₁-C₄ alkyl and C₁-C₆ alkyl may contain one or two double or triple bond and may be substituted with one or two of fluoro, chloro, hydroxy, amino, methylamino, dimethylamino or acetyl; with the proviso that R₁₁₅ is not unsubstituted phenyl;
R₁₁₁₁ is hydrogen, hydroxy, fluoro, chloro, COO(C₁-C₂ alkyl), cyano or CO(C₁-C₂ alkyl); and
R₁₁₁₂ is hydrogen or C₁-C₄ alkyl; with the proviso that
(a) A₈ is not linear C₁-C₁₂ alkyl;
(b) R₁₁₅ is not a sugar group;
(c) when R₁₁₃ and R₁₁₄ are hydrogen and R₁₁₅ is chlorophenyl, then A₈ is not NHCH(CH₃)-(CH₂)₃-N(C₂H₅)₂;
(d) when R₁₁₃ and R₁₁₄ are hydrogen and A₈ is NR₁₁₁R₁₁₂, wherein R₁₁₁ is C₃-C₇ cycloalkyl and R₁₁₂ is C₂-C₆ alkenyl, phenyl(C₁-C₆ alkylene) or hetero(C₁-C₆ alkylene) wherein the hetero radical is furyl, thienyl or pyridinyl, and wherein said phenyl may be substituted with fluoro, chloro, bromo or iodo, then R₁₁₅ is not tetrahydrofuranyl or tetrahydropyranyl;
(e) when R₁₁₃ is methoxy, methylthio or methylsulfonyl, R₁₁₄ is hydrogen, and R₁₁₅ is tetrahydrofuranyl or tetrahydropyranyl, then A₈ is not NH(C₁-C₂ alkyl), morpholinyl or hydrazino, or NHC₂H₄C₆H₅ which may be substituted with one methyl or two methoxy;
(f) when R₁₁₃ is hydrogen, C₁-C₆ alkyl, hydrazino, chloro, bromo, SH, or S(C₁-C₄ alkyl), R₁₁₄ is hydrogen, and R₁₁₅ is C₃-C₈ cycloalkyl, then A₈ is not hydrazino, NH(C₁-C₂ alkyl) or N(C₁-C₆ alkyl)(C₁-C₁₂ alkyl);
(g) when R₁₁₃ and R₁₁₄ are hydrogen and A₈ is NH(CH₂)ₘCOOH wherein m is 1 to 12, then R₁₁₅ is not phenyl substituted by one of fluoro, chloro, bromo or iodo;
(h) when R₁₁₃ is hydrogen, hydroxy, methylthio, chloro or NHbenzyl, R₁₁₄ is hydrogen, and R₁₁₅ is chlorophenyl or bromophenyl, then A₈ is not NH(C₁-C₁₂ alkyl), NHaryl, or N(C₁-C₆ alkyl)(C₁-C₁₂ alkyl), wherein said C₁-C₁₂ alkyl may be substituted with NC₂H₅, or NHbenzyl which may be substituted with one or two of bromo, chloro, fluoro, NC₂H₅ phenyl or morpholinopropyl;
(i) when R₁₁₃ and R₁₁₄ are hydrogen and R₁₁₅ is nitrophenyl, then A₈ is not NHR₁₁₂, wherein R₁₁₂ is C₁-C₁₂ alkyl which may be substituted with two hydroxy, or R₁₁₂ is phenyl or benzyl;
(j) when R₁₁₃ is chloro or O(C₁-C₆ alkyl), R₁₁₄ is hydrogen, and A₈ is NR₁₁₁R₁₁₂, wherein R₁₁₁ and R₁₁₂ are independently hydrogen or C₁-C₆ alkyl, then R₁₁₅ is not chlorophenyl; and
(k) when R₁₁₃ is hydrogen, A₈ is benzyl or phenethyl, and R₁₁₄ is fluoro, chloro, bromo or iodo, then R₁₁₅ is not 5'-deoxy-ribofuranosyl or 5'-amino-5'-deoxy-ribofuranosyl, or its pharmaceutically acceptable acid addition salt.

15. A pharmaceutical composition for prophylaxis or therapy of a postoperative stress as claimed in claim 1 or claim 2, wherein the compound having CRF antagonist activating effect is a compound represented by the formula Ii 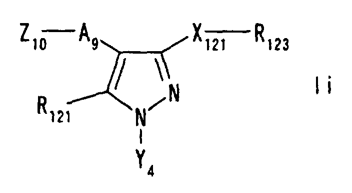 wherein A₉ is CH₂, R₁₂₁ is hydrogen, linear or branched C₁-C₆ alkyl, C₃-C₈ alkyl containing one or not adjacent two double bonds, hydroxy, O(C₁-C₆ alkyl), SH, S(C₁-C₆ alkyl), C₃-C₆ cycloalkyl, morpholinyl, piperidinyl or aryl, wherein said aryl may be substituted with one to three of fuloro, chloro, bromo, hydroxy, O(C₁-C₆ alkyl), SH, S(C₁-C₆ alkyl), amino, NH(C₁-C₆ alkyl) or N(C₁-C₆ alkyl), or one of iodo, nitro or cyano and is selected from the group consisting of phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl and thiazolidinyl;
R₁₂₃ is linear C₁-C₆ alkyl, branched C₃-C₈ alkyl, C₃-C₈ alkenyl wherein the double bond is not adjacent to X₁₂₁ when X₁₂₁ is heteroatom, C₃-C₇ cycloalkyl(CH₂)ₙ wherein n is 0 to 4, or (CH₂)_{q}Q₁₂₁R₁₂₁₉ wherein q is 0, 1 or 2 and Q₁₂₁ is oxygen, sulfur, NH, N(C₁-C₆ alkyl), or covalent bond when X₁₂₁ is not covalent bond, R₁₂₁₉ is hydrogen, linear C₁-C₆ alkyl, branched C₃-C₈ alkyl, C₃-C₈ alkenyl, C₃-C₆ cycloalkyl or C₃-C₆ cycloalkyl-(CH₂), with the proviso that X₁₂₁ and Q₁₂₁ are not both heteroatom when q is 1;
X₁₂₁ is covalent bond, CH₂, oxygen, sulfur or NR₁₂₀ wherein R₁₂₀ is hydrogen, linear C₁-C₆ alkyl or branched C₃-C₈ alkyl;
Y₄ is phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl or piperidinyl, wherein each of the above groups may be substituted with one to three of any one of fluoro, chloro, bromo, or methyl, or one of trifluoromethyl; with the proviso that Y₄ is not unsubstituted phenyl; and
Z₁₀ is
(a) 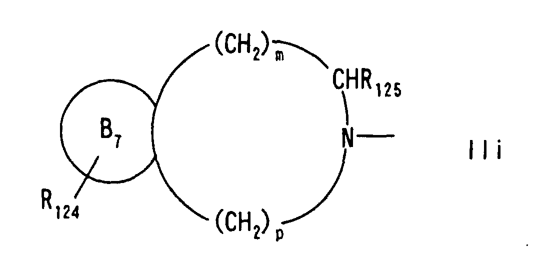 wherein the B₇ ring is phenyl, naphthyl, pyridyl, pyrimidinyl, pyrazinyl, pyridazinyl, triazolyl, pyrrolyl, pyrazolyl, imidazolyl, thienyl or indolyl, wherein each of the above groups may be substituted with methyl, methoxy, trifluoromethoxy, fluoro, chloro, bromo or iodo, or a saturated 5 or 6 membered carbocyclic ring or a partially unsaturated ring having one or two double bonds;
R₁₂₄ is hydrogen, C₁-C₆ alkyl, C₁-C₆ alkoxy, hydroxy, fluoro, chloro, bromo, iodo or trifluoromethyl;
R₁₂₅ is hydrogen, linear C₁-C₆ alkyl, branched C₃-C₈ alkyl, C₃-C₈ alkeny, or (CH₂)ₒ-X₁₂₂-(CH₂)ᵣ-Q₁₂₂-R₁₂₆;
X₁₂₂ and Q₁₂₂ are each independently oxygen, sulfur, NH, N(C₁-C₆ alkyl), or one of X₁₂₂ and Q₁₂₂ may be a covalent bond;
R₁₂₆ is hydrogen, linear C₁-C₆ alkyl, branched C₃-C₈ alkyl, or C₃-C₈ alkenyl;
m is 0 or 1;
o is 1 or 2;
p is 1 or 2;
r is 0, 1, or 2;
(b) 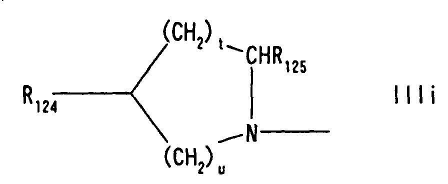 wherein R₁₂₄ and R₁₂₅ are as defined above, and t and u are each independently 1 or 2;
(c) -NR₁₂₇R₁₂₈ wherein R₁₂₇ and R₁₂₈ are each independently hydrogen or linear C₁-C₆ alkyl, branched C₃-C₈ alkyl, C₃-C₈ alkenyl, (CH₂)ᵥCH₂OH, (CH₂)ᵥNR₁₂₉R₁₂₁₀ wherein v is 0 to 3, and R₁₂₉ and R₁₂₁₀ are each independently hydrogen or linear C₁-C₆ alkyl, (C₃-C₁₂ cycloalkyl)(CH₂)ₙ, (C₆-C₁₀ bicycloalkyl)(CH₂)ₙ, benzofused C₃-C₆ cycloalkyl, C₁-C₆ hydroxyalkyl, phenyl(CH₂)ₙ wherein n is 0 to 4, wherein each of the above groups may be substituted with one or two of hydroxy, fluoro, chloro, bromo, C₁-C₅ alkyl or C₁-C₅ alkoxy, or R₁₂₇ and R₁₂₈ may be taken together with nitrogen to form a saturated or partially unsaturated 5 to 7 membered ring which may contain one of oxygen, sulfur, NH or N(C₁-C₆ alkyl) and which may be substituted with C₁-C₆ alkyl, hydroxy or phenyl, wherein any double bonds are not adjacent to any heteroatoms;
(d) 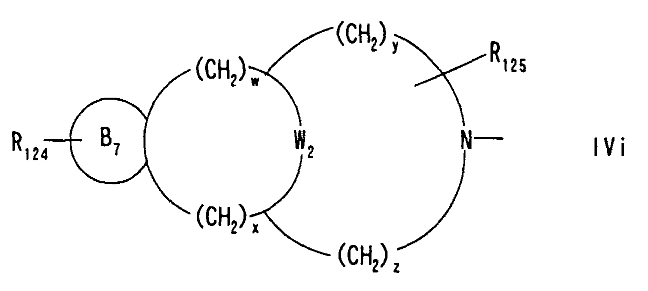 wherein B₇, W₂, R₁₂₄ and R₁₂₅ are as defined above, w, x, y and z are each independently 1 or 2, and W₂ is (CH₂)_{q} wherein q is as defined above, N(C₁-C₆ alkyl) or oxygen;
(e) 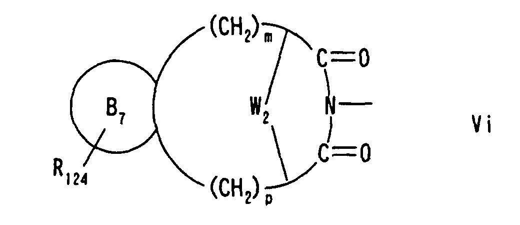 wherein B₇, R₁₂₄, m and p are as defined above;
(f) 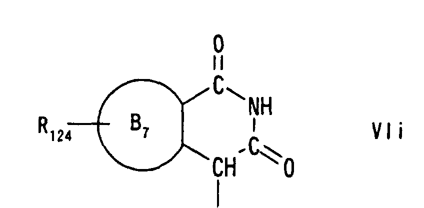 wherein B₇ and R₁₂₄ are as defined above;
(g) O(CH₂)ᵥR₁₂₁₁
wherein v is 0 to 3 and R₁₂₁₁ is linear C₁-C₆ alkyl, branched C₃-C₈ alkyl, phenyl, naphthyl, 1,2,3,4-tetrahydronaphthyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, piperidinyl, or thienyl, wherein each of the above groups may be substituted with one or two of any one of fluoro, chloro, bromo, methyl or trifluoromethyl;
(h) 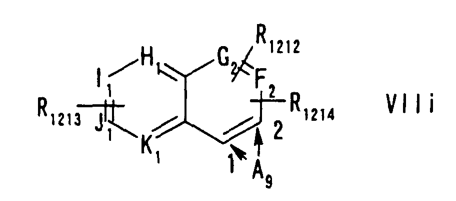 wherein A₉ is as defined above and is attached to position 1 or 2, while R₁₂₁₄ is attached to position 2 or 1 respectively; F₂, G₂, H₁, I₁, J₁ and K₁ are independently carbon or nitrogen, with the proviso that not more than three of H₁, I₁, J₁ and K₁ are nitrogen with not more than two adjacent nitrogens; R₁₂₁₂ and R₁₂₁₃ each independently are hydrogen, linear C₁-C₆ alkyl, branched C₃-C₈ alkyl, C₃-C₈ alkenyl, fluoro, chloro, bromo, trifluoromethyl, hydroxy, thiol, C₁-C₁₂ alkoxy, C₁-C₁₂ thioalkanyl, or C₃-C₁₂ alkenoxy or C₃-C₁₂ thioalkenyl, wherein the double bond is not adjacent to oxygen; and R₁₂₁₄ is hydroxy, C₁-C₁₂ alkoxy, C₃-C₁₂ alkenoxy, wherein the double bond is not adjacent to oxygen, or -X₁₂₂-(CH₂)ᵣQ₁₂₂R₁₂₆ wherein X₁₂₂, r, Q₁₂₂ and R₁₂₆ are as defined above in paragraph (a) except that Q₁₂₂ is not sulfur, or NR₁₂₁₅R₁₂₁₆ wherein R₁₂₁₅ and R₁₂₁₆ are each independently hydrogen, linear C₁-C₆ alkyl, branched C₃-C₈ alkyl, C₃-C₈ alkenyl, wherein the double bond is not adjacent to nitrogen, or C₃-C₇ cycloalkyl-(CH₂)ₙ wherein n is as defined above, or R₁₂₁₅ and R₁₂₁₆ may be taken together with the nitrogen to form a saturated 5 or 6 membered ring optionally condensed with benzo; or
(i) 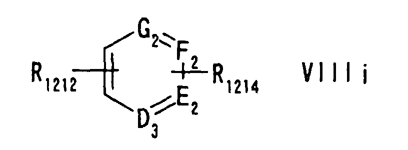 wherein D₃, E₂, F₂ and G₂ are independently carbon or nitrogen, with the proviso that not more than two of D₃, E₂, F₂ and G₂ are nitrogen, R₁₂₁₂ and R₁₂₁₄ are as defined above (h), the above-defined A₉ is attached to the formula VIIIi, and R₁₂₁₄ is attached to a carbon located adjacent to the carbon to which A₉ is attached,
or its pharmaceutically acceptable acid addition salt.

16. A pharmaceutical composition for prophylaxis or therapy of a postoperative stress as claimed in claim 1 or claim 2, wherein the compound having CRF antagonist activating effect is a compound represented by the formula Ij 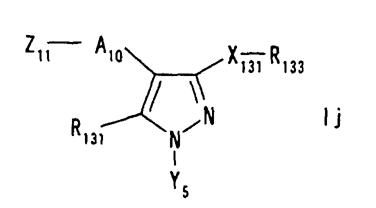 wherein A₁₀ is CH₂, R₁₃₁ is linear or branched C₁-C₆ alkyl, C₃-C₆ alkyl containing one or not adjacent two double bonds, hydroxy, O(C₁-C₆ alkyl), SH, S(C₁-C₆ alkyl), SO₂(C₁-C₆ alkyl), C₃-C₆ cycloalkyl, morpholinyl, piperidinyl or aryl, wherein said aryl may be substituted with one to three of fuloro, chloro, bromo, hydroxy, O(C₁-C₆ alkyl), SH, S(C₁-C₆ alkyl), amino, NH(C₁-C₆ alkyl), N(C₁-C₆ alkyl)₂, or one of iodo, nitro, cyano and is selected from the group consisting of phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl and thiazolidinyl;
R₁₃₃ is linear C₁-C₆ alkyl, branched C₃-C₈ alkyl, C₃-C₈ alkenyl, wherein the double bond is not adjacent to X₁₃₁ when X₁₃₁ is heteroatom, C₃-C₇ cycloalkyl(CH₂)ₙ wherein n is 0 to 4, or (CH₂)_{q}Q₁₃₁R₁₃₁₉ wherein q is 0, 1 or 2 and Q₁₃₁ is oxygen, sulfur, NH or N(C₁-C₆ alkyl), or covalent bond when X₁₃₁ is not covalent bond, R₁₃₁₉ is hydrogen, linear C₁-C₆ alkyl, branched C₃-C₈ alkyl, C₃-C₈ alkenyl, C₃-C₆ cycloalkyl or C₃-C₆ cycloalkyl(CH₂), with the proviso that X₁₃₁ and Q₁₃₁ is not both heteroatom when q is 1;
X₁₃₁ is covalent bond, CH₂, oxygen, sulfur or NR₁₃₀ wherein R₁₃₀ is hydrogen, linear C₁-C₆ alkyl or branched C₃-C₈ alkyl;
Y₅ is phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, or piperidinyl, wherein each of the above groups may be substituted with one to three of any one of fluoro, chloro, bromo, formyl, C₁-C₆ alkyl, branched C₃-C₈ alkyl, C₁-C₆ alkoxy or trifuloromethyl, or one of hydroxyl, iodo, cyano, nitro, amino, NH(C₁-C₄ alkyl), N(C₁-C₄ alkyl)(C₁-C₂ alkyl), COO(C₁-C₄ alkyl), CO(C₁-C₄ alkyl), SO₂NH(C₁-C₄ alkyl), SO₂N(C₁-C₄ alkyl)(C₁-C₂ alkyl), SO₂NH₂, NHSO₂(C₁-C₄ alkyl), S(C₁-C₆ alkyl) or SO₂(C₁-C₆ alkyl), wherein said C₁-C₄ alkyl and C₁-C₆ alkyl may be substituted with one or two of fluoro, chloro, hydroxy, C₁-C₄ alkoxy, amino, methylamino, dimethylamino or acetyl and may contain one double bond or triple bond, and wherein each of above Y₅ groups may be substituted with one to three of any one of fluoro, chloro, bromo or methyl, or one of trifluoromethyl; and
Z₁₁ is
(a) 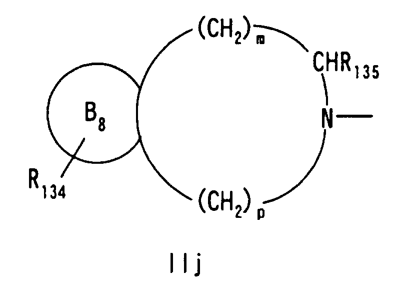 wherein the B₈ ring is phenyl, naphthyl, pyridyl, pyrimidinyl, pyrazinyl, pyridazinyl, triazolyl, pyrrolyl, pyrazolyl, imidazolyl, thienyl or indolyl, wherein each of the above groups may be substituted with methyl, methoxy, trifluoromethyl, fluoro, chloro, bromo or iodo, or a saturated 5 or 6 membered carbocyclic ring or a partially unsaturated ring having one or two double bonds;
R₁₃₄ is hydrogen, C₁-C₆ alkyl, C₁-C₆ alkoxy, hydroxy, fluoro, chloro, bromo, iodo or trifluoromethyl;
R₁₃₅ is hydrogen, linear C₁-C₆ alkyl, branched C₃-C₈ alkyl, C₃-C₈ alkenyl or (CH₂)ₒ-X₁₃₂-(CH₂)ᵣ-Q₁₃₂-R₁₃₆;
X₁₃₂ and Q₁₃₂ are each independently oxygen, sulfur, NH or N(C₁-C₆ alkyl), or one of X₁₃₂ and Q₁₃₂ may be a covalent bond;
R₁₃₆ is hydrogen, linear C₁-C₆ alkyl, branched C₃-C₈ alkyl or C₃-C₈ alkenyl;
m is 0 or 1;
o is 1 or 2;
p is 1 or 2;
r is 0, 1, or 2;
(b) 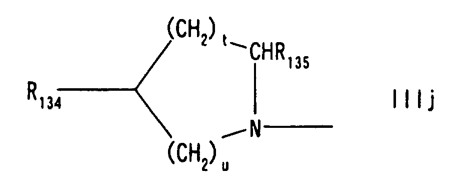 wherein R₁₃₄ and R₁₃₅ are as defined above, and t and u are each independently 1 or 2;
(c) -NR₁₃₇R₁₃₈ wherein R₁₃₇ and R₁₃₈ are each independently hydrogen, linear C₁-C₆ alkyl, branched C₃-C₈ alkyl, C₃-C₈ alkenyl, (CH₂)ᵥCH₂OH, (CH₂)ᵥNR₁₃₉R₁₃₁₀ wherein v is 0 to 3 and R₁₃₉ and R₁₃₁₀ are each independently hydrogen or linear C₁-C₆ alkyl, (C₃-C₁₂ cycloalkyl)(CH₂)ₙ, (C₆-C₁₀ bicycloalkyl)(CH₂)ₙ, benzofused C₃-C₈ cycloalkyl, C₁-C₆ hydroxyalkyl, phenyl(CH₂)ₙ, wherein each of the above groups may be substituted with one or two of hydroxy, fluoro, chloro, bromo, C₁-C₅ alkyl or C₁-C₅ alkoxy, or R₁₃₇ and R₁₃₈ may be taken together with the nitrogen to form a saturated or partially unsaturated 5 to 7 membered ring which may contain one of oxygen, sulfur, NH or N(C₁-C₆ alkyl) and may be substituted with C₁-C₆ alkyl, hydroxy or phenyl, and n is 0 to 4;
(d) 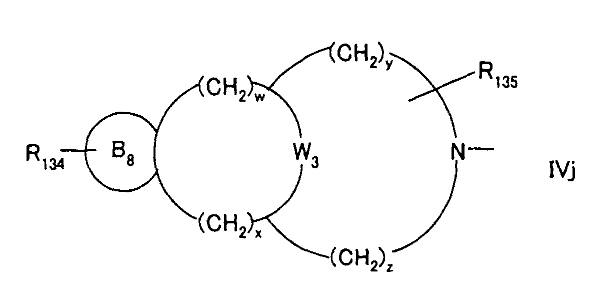 wherein B₈, W₃, R₁₃₄ and R₁₃₅ are as defined above, w, x, y and z are each independently 1 or 2, and W₃ is (CH2)_{q} wherein q is as defined above, N(C₁-C₆ alkyl) or oxygen;
(e) 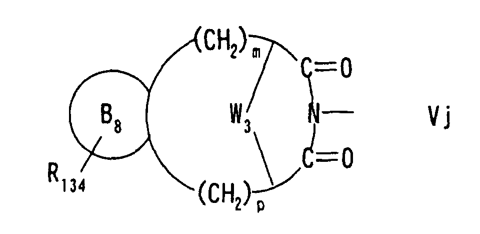 wherein B₈, R₁₃₄, m and p are as defined above;
(f) 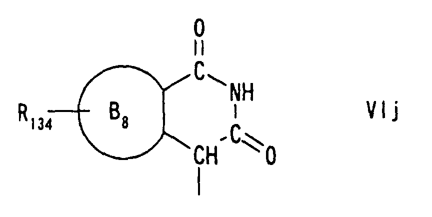 wherein B₈ and R₁₃₄ are as defined above;
(g) O(CH₂)ᵥR₁₃₁₁
wherein v is 0 to 3 and R₁₃₁₁ is linear C₁-C₆ alkyl, branched C₃-C₈ alkyl, phenyl, naphthyl, 1,2,3,4-tetrahydronaphthyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, piperidinyl, or thienyl, wherein each of the above groups may be substituted with one or two of any one of fluoro, chloro, bromo, methyl or trifluoromethyl;
(h) 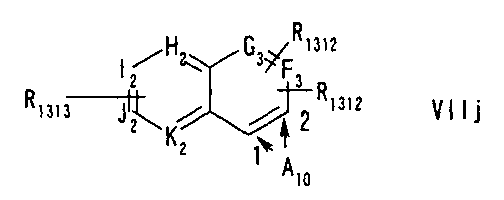 wherein A₁₀ is as defined above and is attached to position 1 or 2 while R₁₃₁₄ is attached to position 2 or 1 respectively; F₃, G₃, H₂, I₂, J₂ and K₂ are independently carbon or nitrogen, with the proviso that not more than three of H₂, I₂, J₂ and K₂ are nitrogen with not more than two adjacent nitrogens; R₁₃₁₂ and R₁₃₁₃ each independently are hydrogen, linear C₁-C₆ alkyl, branched C₃-C₈ alkyl, C₃-C₈ alkenyl, fluoro, chloro, bromo, trifluoromethyl, hydroxy, thiol, C₁-C₁₂ alkoxy, C₁-C₁₂ thioalkyl, or C₃-C₁₂ alkenoxy or C₃-C₁₂ thioalkenyl, wherein the double bond is not adjacent to oxygen; and R₁₃₁₄ is hydroxy, C₁-C₁₂ alkoxy, C₃-C₁₂ alkenoxy, wherein the double bond is not adjacent to oxygen, or -X₁₃₂-(CH₂)ᵣQ₁₃₂R₁₃₆ wherein X₁₃₂, r, Q₁₃₂ and R₁₃₆ are as defined above in paragraph (a) except that Q₁₃₂ is not sulfur, or R₁₃₁₄ is NR₁₃₁₅R₁₃₁₆, wherein R₁₃₁₅ and R₁₃₁₆ are each independently hydrogen, linear C₁-C₆ alkyl, branched C₃-C₈ alkyl, C₃-C₈ alkenyl, wherein the double bond is not adjacent to nitrogen or C₃-C₇ cycloalkyl(CH₂)ₙ wherein n is as defined above, or R₁₃₁₅ and R₁₃₁₆ may be taken together with the nitrogen to form a saturated 5 or 6 membered ring which may be condensed with benzo; or
(i) 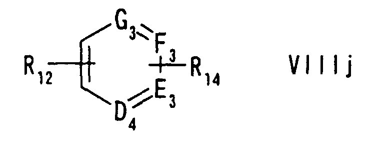 wherein D₄, E₃, F₃ and G₃ are independently carbon or nitrogen, with the proviso that not more than two of D₄, E₃, F₃ and G₃ are nitrogen, R₁₃₁₂ and R₁₃₁₄ are as defined above (h), A₁₀ which is defined above is attached to a carbon in formula VIIIj, and R₁₃₁₄ is attached to a carbon located adjacent to the carbon to which A₁₀ is attached,
or its pharmaceutically acceptable acid addition salt.

17. A pharmaceutical composition for prophylaxis or therapy of a postoperative stress as claimed in claim 1 or claim 2, wherein the compound having CRF antagonist activating effect is a compound represented by the formula Ik, IIk or IIIk 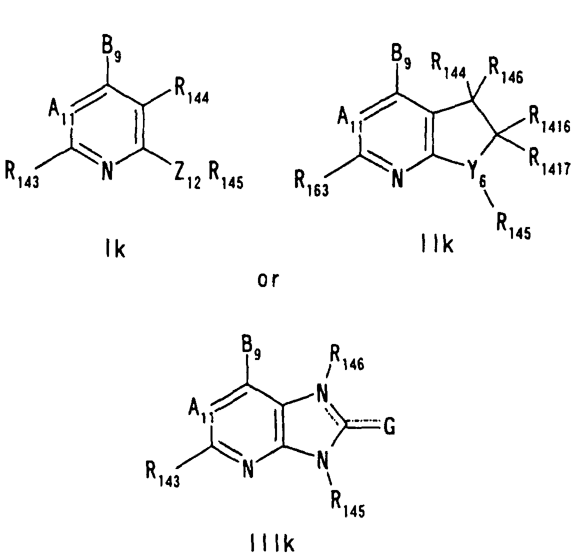 wherein the dashed lines represent optional double bonds;
A₁₁ is -CR₁₄₇ or nitrogen;
B₉ is -NR₁₄₁R₁₄₂, -CR₁₄₁R₁₄₂R₁₄₁₁, -C(=CR₁₄₂R₁₄₁₂)R₁₄₁, -NHCHR₁₄₁R₁₄₂, -OCHR₁₄₁R₁₄₂, -SCHR₁₄₁R₁₄₂, -CHR₁₄₂OR₁₄₁₂, -CHR₁₄₂SR₁₄₁₂, -C(S)R₁₄₂ or -C(O)R₁₄₂;
G₄ is oxygen, sulfur, NH, NH₃, hydrogen, methoxy, ethoxy, trifluoromethoxy, methyl, ethyl, thiomethoxy, NH₂, NHCH₃, N(CH₃)₂ or trifluoromethyl;
Y₆ is -CH or nitrogen;
Z₁₂ is NH, oxygen, sulfur, -N(C₁-C₂ alkyl) or -C(R₁₄₁₃R₁₄₁₄), wherein R₁₄₁₃ and R₁₄₁₄ are each independently hydrogen, trifluoromethyl or methyl, or one of R₁₄₁₃ and R₁₄₁₄ is cyano and the other is hydrogen or methyl;
R₁₄₁ is C₁-C₆ alkyl which may optionally be substituted with one or two substituents R₁₄₈ independently selected from the group consisting of hydroxy, fluoro, chloro, bromo, iodo, CF₃, C₁-C₄ alkoxy, -O-CO-(C₁-C₄ alkyl), -O-CO-NH-(C₁-C₄ alkyl), -O-CO-N(C₁-C₄ alkyl)(C₁-C₂ alkyl), -NH(C₁-C₄ alkyl), -N(C₁-C₂ alkyl)(C₁-C₄ alkyl), -S(C₁-C₄ alkyl), -N(C₁-C₄ alkyl)CO(C₁-C₄ alkyl), -NHCO(C₁-C₄ alkyl), -COO(C₁-C₄ alkyl), -CONH(C₁-C₄ alkyl), -CON(C₁-C₄ alkyl)(C₁-C₂ alkyl), CN, NO₂, -SO(C₁-C₄ alkyl) and -SO₂(C₁-C₄ alkyl), and wherein said C₁-C₆ alkyl and (C₁-C₄)alkyl moieties in the foregoing R₁₄₁ groups may optionally contain one carbon-carbon double or triple bond;
R₁₄₂ is C₁-C₁₂ alkyl, aryl or -(C₁-C₄ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, benzisoxazolyl, benzimidazolyl, indolyl or benzoxazolyl, or 3 to 8 membered cycloalkyl or -(C₁-C₆ alkylene)cycloalkyl, wherein one or two of the ring carbons of said cycloalkyl having at least 4 ring members and cycloalkyl moieties of said -(C₁-C₆ alkylene)cycloalkyl having at least 4 ring members may optionally be replaced by oxygen, sulfur or N-R₁₄₉ wherein R₁₄₉ is hydrogen or C₁-C₄ alkyl; and wherein each of the foregoing R₁₄₂ groups may optionally be substituted with from one to three substituents independently selected from chloro, fluoro and C₁-C₄ alkyl, or one substituent selected from bromo, iodo, C₁-C₆ alkoxy, -O-CO-(C₁-C₆ alkyl), -O-CO-N(C₁-C₄ alkyl)(C₁-C₂ alkyl), -S(C₁-C₆ alkyl), CN, NO₂, -SO(C₁-C₄ alkyl) and -SO₂(C₁-C₄ alkyl), and wherein said C₁-C₁₂ alkyl and C₁-C₄ alkylene moieties of said (C₁-C₄ alkylene)aryl may optionally contain one carbon-carbon double or triple bond;
or -NR₁₄₁R₁₄₂ or -CR₁₄₁R₁₄₂R₁₄₁₁ may form a saturated 5 to 8 membered carbocyclic ring which may optionally contain one or two carbon-carbon double bonds and in which one or two of the ring carbons may optionally be replaced by oxygen or sulfur;
R₁₄₃ is methyl, ethyl, fluoro, chloro, bromo, iodo, cyano, methoxy, OCF₃, methylthio, methylsulfonyl, CH₂OH or CH₂OCH₃;
R₁₄₄ is hydrogen, C₁-C₄ alkyl, fluoro, chloro, bromo, iodo, C₁-C₄ alkoxy, trifluoromethoxy, -CH₂OCH₃, -CH₂OCH₂CH₃, -CH₂CH₂OCH₃, -CH₂OF₃, CF₃, amino, nitro, -NH(C₁-C₄ alkyl), -N(CH₃)₂, -NHCOCH₃, -NHCONHCH₃, -SOₙ(C₁-C₄ alkyl) wherein n is 0, 1 or 2, cyano, hydroxy, -CO(C₁-C₄ alkyl), -CHO, cyano or -COO(C₁-C₄ alkyl), wherein said C₁-C₄ alkyl may optionally contain one double or triple bond and may optionally be substituted with one substituent selected from hydroxy, amino, -NHCOCH₃, -NH(C₁-C₂ alkyl), -N(C₁-C₂ alkyl)₂, -COO(C₁-C₄ alkyl), -CO(C₁-C₄ alkyl), C₁-C₃ alkoxy, C₁-C₃ thioalkyl, fluoro, chloro, cyano and nitro;
R₁₄₅ is phenyl, naphthyl, thienyl, benzothienyl, pyridyl, quinolyl, pyrazinyl, pyrimidyl, furanyl, benzofuranyl, benzothiazolyl or indolyl, wherein each of the above groups R₁₄₅ is substituted with from one to three substituents independently selected from fluoro, chloro, C₁-C₆ alkyl and C₁-C₆ alkoxy, or one substituent selected from hydroxy, iodo, bromo, formyl, cyano, nitro, trifluoromethyl, amino, -(C₁-C₆ alkyl)O(C₁-C₆)alkyl, -NHCH₃, -N(CH₃)₂, -COOH, -COO(C₁-C₄alkyl), -CO(C₁-C₄ alkyl), -SO₂NH(C₁-C₄ alkyl), -SO₂N(C₁-C₄ alkyl)(C₁-C₂ alkyl), -SO₂NH₂, -NHSO₂(C₁-C₄ alkyl), -S(C₁-C₆ alkyl) and -SO₂(C₁-C₆ alkyl), and wherein C₁-C₄ alkyl and C₁-C₆ alkyl moieties of the foregoing R₁₄₅ groups may optionally be substituted with one or two fluoro or one substituent selected from hydroxy, amino, methylamino, dimethylamino and acetyl;
R₁₄₆ is hydrogen or C₁-C₆ alkyl, wherein said C₁-C₆ alkyl may optionally be substituted with one of hydroxy, methoxy, ethoxy or fluoro;
R₁₄₇ is hydrogen, methyl, fluoro, chloro, bromo, iodo, cyano, hydroxy, -O(C₁-C₄ alkyl), -C(O)(C₁-C₄ alkyl), -C(O)O(C₁-C₄ alkyl), -OCF₃, CF₃, -CH₂OH, -CH₂OCH₃ or -CH₂OCH₂CH₃;
R₁₄₁₁ is hydrogen, hydroxy, fluoro, or methoxy;
R₁₄₁₂ is hydrogen or C₁-C₄ alkyl; and
R₁₄₁₆ and R₁₄₁₇ are each independently hydrogen, hydroxy, methyl, ethyl, methoxy or ethoxy, with the proviso that R₁₄₁₆ and R₁₄₁₇ are not both methoxy or ethoxy;
or R₁₄₁₆ and R₁₄₁₇ may be take together to form an oxo (=O) group; with the proviso that when G₄ is oxygen, sulfur, NH or NCH₃, G₄ is double bonded to 5 membered ring of structure IIIk, and with the further proviso that R₁₄₆ is absent when nitrogen to which it is attached is double bonded to an adjacent ring carbon atom,
or its pharmaceutically acceptable salt.

18. A pharmaceutical composition for prophylaxis or therapy of a postoperative stress as claimed in claim 1 or claim 2, wherein the compound having CRF antagonist activating effect is a compound represented by the formula Im 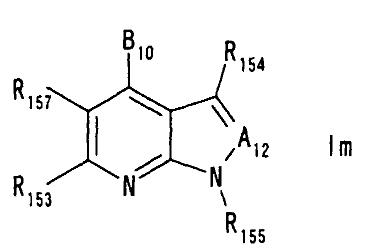 wherein A₁₂ is nitrogen or -CR₁₅₆;
B₁₀ is -NR₁₅₁R₁₅₂, -CR₁₅₁R₁₅₂R₁₅₁₁, -C(=CR₁₅₂R₁₅₁₂)R₁₅₁, -NHCHR₁₅₁R₁₅₂, -OCHR₁₅₁R₁₅₂, -SCHR₁₅₁R₁₅₂, -CHR₁₅₂OR₁₅₁₂, -CHR₁₅₂SR₁₅₁₂, -C(S)R₁₅₁ or -C(O)R₁₅₁;
R₁₅₁ is C₁-C₆ alkyl which may optionally be substituted with one or two substituents independently selected from the group consisting of hydroxy, fluoro, chloro, bromo, iodo, C₁-C₄ alkoxy, -O-CO-(C₁-C₄ alkyl), -O-CO-NH(C₁-C₄ alkyl), -O-CO-N(C₁-C₄ alkyl)(C₁-C₂ alkyl), -NH(C₁-C₄ alkyl), -N(C₁-C₂ alkyl)(C₁-C₄ alkyl), -S(C₁-C₄ alkyl), -N(C₁-C₄ alkyl)CO(C₁-C₄ alkyl), -NHCO(C₁-C₄ alkyl), -COO(C₁-C₄ alkyl), -CONH(C₁-C₄ alkyl), -CON(C₁-C₄ alkyl)(C₁-C₂ alkyl), CN, NO₂, -SO(C₁-C₄ alkyl) and -SO₂(C₁-C₄ alkyl), and wherein any of the foregoing C₁-C₄ alkyl and C₁-C₆ alkyl groups may optionally contain one carbon-carbon double or triple bond;
R₁₅₂ is C₁-C₁₂ alkyl, aryl, -(C₁-C₄ alkylene)aryl, wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzoisothiazolyl, thiazolyl, isoxazolyl, benzoisoxazolyl, benzoimidazolyl, triazolyl, pyrazolyl, pyrrolyl, indolyl, oxazolyl or benzoxazolyl, or 3 to 8 membered cycloalkyl or -(C₁-C₆ alkylene)cycloalkyl, wherein one or two of the ring carbons of said cycloalkyl having at least 4 ring members and cycloalkyl moieties of said -(C₁-C₆ alkylene)cycloalkyl having at least 4 ring members may optionally be replaced by oxygen, sulfur or N-Z₁₃ wherein Z₁₃ is hydrogen or C₁-C₄ alkyl, and wherein each of foregoing R₁₅₂ groups may optionally be substituted with from one to three substituents independently selected from chloro, fluoro and C₁-C₄ alkyl, or one substituent selected from bromo, iodo, C₁-C₆ alkoxy, -O-CO-(C₁-C₆ alkyl), -S(C₁-C₆ alkyl), -COO(C₁-C₄ alkyl), CN, NO₂, -SO(C₁-C₄ alkyl) and -SO₂(C₁-C₄ alkyl), and wherein said C₁-C₁₂ alkyl and C₁-C₄ alkylene moieties of said -(C₁-C₄ alkylene)aryl may optionally contain one carbon-carbon double or triple bond;
or -NR₁₅₁R₁₅₂ may form a saturated 5 to 8 membered heterocyclic ring, or -CHR₁₅₁R₁₅₂ may form a saturated 5 to 8 membered carbocyclic ring, wherein each of these rings may optionally contain one or two carbon-carbon double bonds and wherein one or two of the carbon atoms of each of these rings may optionally be replaced by sulfur or oxygen;
R₁₅₃ is C₁-C₄ alkyl, fluoro, chloro, bromo, iodo, -CH₂OH, -CH₂OCH₃, -O(C₁-C₃ alkyl), -S(C₁-C₃ alkyl) or -SO₂(C₁-C₃ alkyl), wherein said C₁-C₃ alkyl may optionally contain one carbon-carbon double or triple bond;
R₁₅₄ is hydrogen, C₁-C₆ alkyl, fluoro, chloro, bromo, iodo, C₁-C₄ alkoxy, amino, -NHCH₃, -N(CH₃)₂, -CH₂OH, -CH₂OCH₃ or -SOₙ(C₁-C₄ alkyl) wherein n is 0, 1 or 2 , cyano, hydroxy, -CO(C₁-C₄ alkyl), -CHO or -COO(C₁-C₄ alkyl) wherein said C₁-C₄ alkyl moieties in the foregoing R₁₅₄ groups may optionally contain one carbon-carbon double or triple bond;
R₁₅₅ is phenyl, naphthyl, thienyl, benzothienyl, pyridyl, pyrimidyl, benzofuranyl, pyrazinyl or benzothiazolyl, wherein each one of foregoing R₁₅₅ groups may optionally be substituted with from one to three substituents independently selected from fluoro, chloro, C₁-C₆ alkyl and C₁-C₆ alkoxy, or by one substituent selected from iodo, hydroxy, bromo, formyl, cyano, nitro, amino, trifluoromethyl, -NH(C₁-C₄ alkyl), -N(C₁-C₆)(C₁-C₂ alkyl), -COO(C₁-C₄ alkyl), -CO(C₁-C₄ alkyl), -COOH, -SO₂NH(C₁-C₄ alkyl), -SO₂N(C₁-C₄ alkyl)(C₁-C₂ alkyl), -SO₂NH₂, -NHSO₂(C₁-C₄ alkyl), -S(C₁-C₆ alkyl) and -SO₂(C₁-C₆ alkyl), wherein each of said C₁-C₄ alkyl moieties and C₁-C₆ alkyl moieties in the foregoing R₁₅₅ groups may optionally be substituted with one to three fluorine;
R₁₅₆ is hydrogen, C₁-C₄ alkyl, fluoro, chloro, bromo, iodo, -CH₂OH, -CH₂OCH₃ or C₁-C₄ alkoxy;
R₁₅₇ is hydrogen, C₁-C₄ alkyl, fluoro, chloro, bromo, iodo, -O(C₁-C₄ alkyl), cyano, -CH₂OH, -CH₂O(C₁-C₂ alkyl), -CO(C₁-C₂ alkyl) or -COO(C₁-C₂ alkyl);
R₁₅₁₁ is hydrogen, hydroxy, fluoro or methoxy; and
R₁₅₁₂ is hydrogen or C₁-C₄ alkyl; with the proviso that when A₁₂ is nitrogen, then: (a) B₁₀ is not unsubstituted alkyl; (b) R₁₅₅ is not unsubstituted phenyl or monosubstituted phenyl; and (c) R₁₅₃ is not unsubstituted alkyl,
or its pharmaceutically acceptable salt.

19. A pharmaceutical composition for prophylaxis or therapy of a postoperative stress as claimed in claim 1 or claim 2, wherein the compound having CRF antagonist activating effect is a compound represented by the formula In 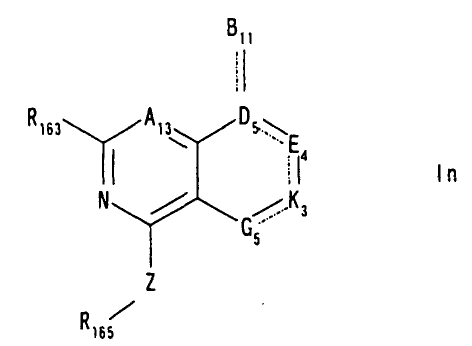 wherein the dashed lines represent optional double bonds;
A₁₃ is nitrogen or CR₁₆₇;
B₁₁ is -NR₁₆₁R₁₆₂, -CR₁₆₁R₁₆₂R₁₆₁₀, -C(=CR₁₆₂R₁₆₁₁)R₁₆₁, -NHCR₁₆₁R₁₆₂R₁₆₁₀, -OCR₁₆₁R₁₆₂R₁₆₁₀, -SCR₁₆₁R₁₆₂R₁₆₁₀, -CR₁₆₂R₁₆₁₀NHR₁₆₁, -CR₁₆₂R₁₆₁₀OR₁₆₁, -CR₁₆₂R₁₆₁₀SR₁₆₁ or -COR₁₆₂, and is single bonded to D₅, or B₁₁ is -CR₁₆₁R₁₆₂, and is double bonded to D₅ and D₅ is carbon;
D₅ is nitrogen or CR₁₆₄ and is single bonded to all atoms to which it is attached, or D₅ is carbon and is double bonded to E₄ or double bonded to B₁₁;
E₄ is oxygen, nitrogen, sulfur, C=O, C=S, CR₁₆₆R₁₆₁₂, NR₁₆₆ or CR₁₆₆, or E₄ is a two atom spacer, wherein one of the atoms is oxygen, nitrogen, sulfur, C=O, C=S, CR₁₆₆R₁₆₁₂, NR₁₆₆ or CR₁₆₆, and the other is CR₁₆₆R₁₆₁₂ or CR₁₆₉;
K₃ and G₅ are each independently C=O, C=S, sulfur, oxygen, CHR₁₆₈ or NR₁₆₈ when single bonded to both adjacent ring atoms, or nitrogen or CR₁₆₈ when it is double bonded to one adjacent ring atom; 6 or 7 membered ring that contains D₅, E₄, K₃ and G₅ may contain from one to three double bonds, from zero to two heteroatoms selected from oxygen, nitrogen and sulfur, and from zero to two C=O or C=S groups, wherein the carbon atoms of such groups are part of the ring and the oxygen and sulfur atoms are substituents on the ring;
R₁₆₁ is C₁-C₆ alkyl which may optionally be substituted with from one or two substituents independently selected from hydroxy, fluoro, chloro, bromo, iodo, C₁₋₄ alkoxy, CF₃,-C(=O)(C₁₋C₄ alkyl), -C(=O)-O-(C₁₋C₄ alkyl), -OC(=O)(C₁₋C₄ alkyl), -OC(=O)N(C₁₋C₄ alkyl)(C₁₋C₂ alkyl), -NHCO(C₁₋C₄ alkyl), -COOH, -COO(C₁₋C₄ alkyl), -CONH(C₁₋C₄ alkyl), -CON(C₁₋C₄ alkyl)(C₁₋C₂ alkyl), -S(C₁₋C₄ alkyl), -CN, -NO₂, -SO(C₁₋C₄ alkyl), -SO₂NH(C₁₋C₄ alkyl) and -SO₂N(C₁-C₄ alkyl)(C₁₋C₂ alkyl), wherein each of the C₁-C₄ alkyl groups in the foregoing R₁₆₁ groups may optionally contain one or two double or triple bonds;
R₁₆₂ is C₁-C₁₂ alkyl which may optionally contain from one to three double or triple bonds, aryl or (C₁-C₄ alkylene)aryl, wherein said aryl and aryl moieties of said (C₁-C₄ alkylene)aryl is selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl, or C₃-C₈ cycloalkyl or (C₁-C₆ alkylene)(C₃-C₈ cycloalkyl), wherein one or two of the carbon atoms of said cycloalkyl and one or two of the carbon atoms of 5 to 8 membered cycloalkyl moieties of said (C₁-C₆ alkylene)(C₃-C₈ cycloalkyl) may optionally and independently be replaced by oxygen or sulfur, and wherein each of the foregoing R₁₆₂ groups may optionally be substituted with from one to three substituents selected from chloro, fluoro and C₁-C₄ alkyl, or one substituent selected from C₁-C₆ alkoxy, -OC(=O)(C₁-C₆ alkyl), -OC(=O)N(C₁-C₄ alkyl)(C₁-C₂ alkyl), -S(C₁-C₆ alkyl), amino, -NH(C₁-C₂ alkyl), -N(C₁-C₄ alkyl)(C₁-C₂ alkyl), -N(C₁-C₄ alkyl)-CO-(C₁-C₄ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -SH, -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl);
-NR₁₆₁R₁₆₂ or CR₁₆₁R₁₆₂R₁₆₁₀ may form a ring selected from saturated 3 to 8 membered rings, and 5 to 8 membered rings of which may optionally contain one or two double bonds, and wherein one or two of the ring carbon atoms of such 5 to 8 membered rings may optionally and independently be replaced by oxygen, sulfur or NZ₁₆₃ wherein Z₁₆₃ is hydrogen or C₁-C₄ alkyl;
R₁₆₃ is hydrogen, C₁-C₄ alkyl, -O(C₁-C₄ alkyl), fluoro, chloro, bromo, iodo, -S(C₁-C₄ alkyl) or -SO₂(C₁-C₄ alkyl);
R₁₆₄ is hydrogen, C₁-C₂ alkyl, hydroxy or fluoro; each R₁₆₆, R₁₆₈ and R₁₆₉ that is attached to a carbon atom is selected independently from hydrogen, C₁-C₂ alkyl, fluoro, chloro, bromo, iodo, hydroxy, hydroxymethyl, formyl, trifluoromethyl, cyano, amino, nitro, -O(C₁-C₂ alkyl), -N(C₁-C₂ alkyl)(C₁-C₂ alkyl),-S(C₁-C₂ alkyl), -CO(C₁-C₂ alkyl), -C(=O)H and -C(=O)O(C₁-C₂alkyl), wherein each of the C₁-C₂ alkyl moieties in the foregoing R₁₆₆, R₁₆₈, and R₁₆₉ groups may optionally contain one double or triple bond; and each R₁₆₆, R₁₆₈, and R₁₆₉ that is attached to a nitrogen atom is selected independently from hydrogen and C₁-C₄ alkyl;
R₁₆₅ is substituted phenyl, substituted naphthyl, substituted pyridyl or substituted pyrimidyl, wherein each of the foregoing R₁₆₅ groups is substituted with from two to four substituents R₁₆₁₅, wherein from one to three of said substituents may be selected independently from chloro, C₁-C₆ alkyl, -O(C₁-C₆ alkyl) and -(C₁-C₆ alkylene)O(C₁-C₆ alkyl), and one of said substituents may be selected, independently, from bromo, iodo, formyl, cyano, trifluoromethyl, nitro, amino, -NH(C₁-C₄ alkyl), -N(C₁-C₂ alkyl)(C₁-C₆ alkyl), -C(=O)O(C₁-C₄ alkyl), -C(=O)(C₁-C₄ alkyl), -COOH, -SO₂NH(C₁-C₄ alkyl), -SO₂N(C₁-C₂ alkyl)(C₁-C₄ alkyl), -SO₂NH₂, -NHSO₂(C₁-C₄ alkyl), -S(C₁-C₆ alkyl) and -SO₂(C₁-C₆ alkyl), and wherein each of the C₁-C₄ alkyl moieties and C₁-C₆ alkyl moieties in the foregoing R₁₆₅ groups may optionally be substituted with one or two substituents independently selected from fluoro, hydroxy, amino, methylamino, dimethylamino and acetyl;
R₁₆₇ is hydrogen, methyl, halo (e.g. chloro, fluoro, iodo or bromo), hydroxy, methoxy, -C(=O)(C₁-C₂ alkyl), -C(=O)O(C₁-C₂ alkyl), trifluoromethoxy, hydroxymethyl, trifluoromethyl or formyl;
R₁₆₁₀ is hydrogen, hydroxy, methoxy or fluoro;
R₁₆₁₁ is hydrogen or C₁-C₄ alkyl;
R₁₆₁₂ is, hydrogen or methyl; and
Z₁₄ is NH, oxygen, sulfur, -N(C₁-C₄ alkyl) or -CR₁₆₁₃R₁₆₁₄, wherein R₁₆₁₃ and R₁₆₁₄ are independently selected from hydrogen and methyl with the exception that one of R₁₆₁₃ and R₁₆₁₄ may optionally be cyano, with the proviso that: (a) in 6 or 7 membered rings of structures in formula In, there cannot exist two double bonds adjacent to each other and (b) when D₅ is carbon and is double bonded to B₁₁, then B₁₁ is CR₁₆₁R₁₆₂,
or its pharmaceutically acceptable salt.

20. A pharmaceutical composition for prophylaxis or therapy of a postoperative stress as claimed in claim 1 or claim 2, wherein the compound having CRF antagonist activating effect is a compound represented by the formula Io 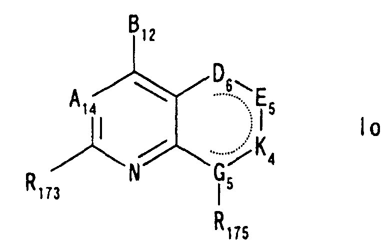 wherein the dashed lines represent optional double bonds;
A₁₄ is nitrogen or CR₁₇₇;
B₁₂ is -NR₁₇₁R₁₇₂, -CR₁₇₁R₁₇₂R₁₇₁₀, -C(=CR₁₇₂R₁₇₁₁)R₁₇₁, -NHCR₁₇₁R₁₇₂R₁₇₁₀, -OCR₁₇₁R₁₇₂R₁₇₁₀, -SCR₁₇₁R₁₇₂R₁₇₁₀, -CR₁₇₂R₁₇₁₀NHR₁₇₁, -CR₁₇₂R₁₇₁₀OR₁₇₁, -CR₁₇₂R₁₇₁₀SR₁₇₁ or -COR₁₇₂;
G₅ is nitrogen or CR₁₇₄ and is single bonded to all atoms to which it is attached, or G₅ is carbon and is double bonded to K₄;
K₄ is nitrogen or CR₁₇₆ when double bonded to G₅ or E₅, or K₄ is oxygen, sulfur, C=O, C=S, CR₁₇₆R₁₇₁₂ or NR₁₇₈ when single bonded to both adjacent ring atoms, or K₄ is a two atom spacer, wherein one of the atoms of the spacer is oxygen, nitrogen, sulfur, C=O, C=S, CR₁₇₆R₁₇₁₂, NR₁₇₆ or CR₁₇₆, and the other is CR₁₇₆R₁₇₁₂ or CR₁₇₉ ;
D₆ and E₅ are each independently C=O, C=S, sulfur, oxygen, CR₁₇₄R₁₇₆ or NR₁₇₈ when single bonded to both adjacent ring atoms, or nitrogen or CR₁₇₄ when it is double bonded to one adjacent ring atom;
6 or 7 membered ring that contains D₆, E₅, K₄ and G₅ may contain from one to three double bonds, from zero to two heteroatoms selected from oxygen, nitrogen and sulfur, and from zero to two C=O or C=S groups, wherein the carbon atoms of such groups are part of the ring and the oxygen and sulfur atoms are substituents on the ring;
R₁₇₁ is C₁-C₆ alkyl which may optionally be substituted with from one or two substituents independently selected from hydroxy, fluoro, chloro, bromo, iodo, C₁-C₄ alkoxy, CF₃, -C(=O)(C₁-C₄ alkyl), -C(=O)-O-(C₁-C₄ alkyl), -OC(=O)(C₁-C₄ alkyl), -OC(=O)N(C₁-C₄ alkyl)(C₁-C₂ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -S(C₁-C₄ alkyl), -CN, -NO₂, -SO(C₁-C₄ alkyl), SO₂NH(C₁-C₄ alkyl) and -SO₂N(C₁-C₄ alkyl)(C₁-C₂ alkyl), wherein each of the C₁-C₄ alkyl groups in the foregoing R₁₇₁ groups may optionally contain one or two double or triple bonds;
R₁₇₂ is C₁-C₁₂ alkyl which may optionally contain from one to three double or triple bonds, aryl or (C₁-C₄ alkylene)aryl, wherein said aryl and aryl moieties of said (C₁-C₄ alkylene)aryl is selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl, or C₃-C₈ cycloalkyl or (C₁-C₆ alkylene)(C₃-C₈ cycloalkyl), wherein one or two of the carbon atoms of said cycloalkyl and one or two of the carbon atoms of 5 to 8 membered cycloalkyl moieties of said (C₁-C₆ alkylene)(C₃-C₈ cycloalkyl) may optionally and independently be replaced by oxygen, sulfur or NZ₁₅ wherein Z₁₅ is hydrogen, C₁-C₄ alkyl or benzyl, and wherein each of the foregoing R₁₇₂ groups may optionally be substituted with from one to three substituents independently selected from chloro, fluoro and C₁-C₄ alkyl, or one substituent selected from C₁-C₆ alkoxy, -OC(=O)(C₁-C₆ alkyl), -OC(=O)N(C₁-C₄ alkyl)(C₁-C₂ alkyl), -S(C₁-C₆ alkyl), amino, -NH(C₁-C₂ alkyl), -N(C₁-C₂ alkyl)(C₁-C₄ alkyl), -N(C₁-C₄ alkyl)-CO-(C₁-C₄ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -SH, -CN, -NO₂, SO(C₁-C₄ alkyl), -SO₂(C₁-C₄ alkyl), SO₂NH(C₁-C₄ alkyl) and SO₂N(C₁-C₄ alkyl)(C₁-C₂ alkyl);
-NR₁₇₁R₁₇₂ or CR₁₇₁R₁₇₂R₁₇₁₀ may form a ring selected from saturated 3 to 8 membered rings, and 5 to 8 membered rings of which may optionally contain one or two double bonds, wherein one or two of the ring carbon atoms of such 5 to 8 membered rings may optionally and independently be replaced by oxygen, sulfur or NZ₁₇ wherein Z₁₇₃ is hydrogen, or C₁-C₄ alkyl;
R₁₇₃ is hydrogen, C₁-C₄ alkyl, -O(C₁-C₄ alkyl), fluoro, chloro, bromo, iodo, S(C₁-C₄ alkyl) or SO₂(C₁-C₄ alkyl);
each R₁₇₈, R₁₇₉ and R₁₇₁₂ is selected independently from hydrogen and C₁-C₂ alkyl;
R₁₇₄ and R₁₇₆ that is attached to a carbon atom is hydrogen, C₁-C₂ alkyl, hydroxy or fluoro;
each R₁₇₆, R₁₇₈ and R₁₇₉ that is attached to a carbon atom is selected independently from hydrogen, C₁-C₆ alkyl, fluoro, chloro, bromo, iodo, hydroxy, hydroxy(C₁-C₂ alkyl), trifluoromethyl, cyano, amino, nitro, -O(C₁-C₄ alkyl), -N(C₁-C₄ alkyl)(C₁-C₂ alkyl), CH₂SCH₃, -S(C₁-C₄ alkyl), CO(C₁-C₄ alkyl), -C(=O)H and C(=O)O(C₁-C₄ alkyl), wherein each of the C₁-C₂ alkyl moieties in the foregoing R₁₇₄ and R₁₇₆ groups may optionally contain one double or triple bond, and R₁₇₆ that is attached to a nitrogen atom is selected independently from hydrogen and C₁-C₄ alkyl;
R₁₇₅ is substituted phenyl, substituted naphthyl, substituted pyridyl or substituted pyrimidyl, wherein each of the foregoing R₁₇₅ groups is substituted with from two to four substituents R₁₇₁₃, wherein up to three of said substituents may be selected from chloro, C₁-C₆ alkyl, -O(C₁-C₆ alkyl) and -(C₁-C₆ alkylene)O(C₁-C₆ alkyl), and one of said substituents may be selected from bromo, iodo, formyl, cyano, trifluoromethyl, nitro, amino, -NH(C₁-C₄ alkyl), -N(C₁-C₂ alkyl)(C₁-C₆ alkyl), -C(=O)O(C₁-C₄ alkyl), -C(=O)(C₁-C₄ alkyl), -COOH, SO₂NH(C₁-C₆ alkyl), SO₂N(C₁-C₂ alkyl)(C₁-C₄ alkyl), SO₂NH₂, -NHSO₂(C₁-C₄ alkyl), -(C₀-C₁ alkylene)-S-(C₁-C₂ alkyl), -(C₀-C₁ alkylene)-SO-(C₁-C₂ alkyl), -(C₀-C₁ alkylene)-SO₂-(C₁-C₂ alkyl) and -(C₁-C₄ alkylene)-OH, and wherein each of the C₁-C₄ alkyl moieties and C₁-C₆ alkyl moieties in the foregoing R₁₇₅ groups may optionally be substituted with one or two substituents independently selected from fluoro, hydroxy, amino, methylamino, dimethylamino and acetyl;
R₁₇₇ is hydrogen, methyl, halo (e.g., chloro, fluoro, iodo or bromo), hydroxy, methoxy, -C(=O)(C₁-C₂ alkyl), -C(=O)O(C₁-C₂ alkyl), hydroxymethyl, trifluoromethyl or formyl;
R₁₇₁₀ is hydrogen, hydroxy, methoxy or fluoro;
R₁₇₁₁ is hydrogen or C₁-C₄ alkyl; and
R₁₇₁₂ is hydrogen or methyl; with the proviso that in the ring containing D₆, E₅, K₄ and G₅ of formula Io, there cannot exist two double bonds adjacent to each other,
or its pharmaceutically acceptable salt.

21. A pharmaceutical composition for prophylaxis or therapy of a postoperative stress as claimed in claim 1 or claim 2, wherein the compound having CRF antagonist activating effect is a compound represented by the formula Ip 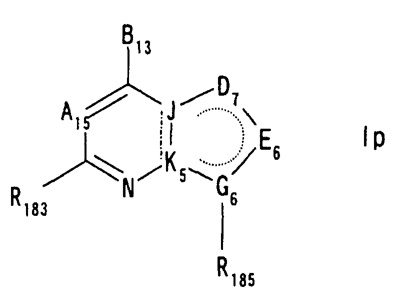 wherein the dashed lines represent optional double bonds;
A₁₅ is nitrogen or CR₁₈₇;
B₁₃ is -NR₁₈₁R₁₈₂, -CR₁₈₁R₁₈₂R₁₈₁₀, -C(=CR₁₈₂R₁₈₁₁)R₁₈₁, -NHCR₁₈₁R₁₈₂R₁₈₁₀, -OCR₁₈₁R₁₈₂R₁₈₁₀, -SCR₁₈₁R₁₈₂R₁₈₁₀, -CR₁₈₂R₁₈₁₀NHR₁₈₁, -CR₁₈₂R₁₈₁₀OR₁₈₁, -CR₁₈₂R₁₈₁₀SR₁₈₁ or -COR₁₈₂;
J and K₅ are each independently nitrogen or carbon and both J and K₅ are not nitrogens;
D₇ and E₆ are each selected independently from nitrogen, CR₁₈₄, C=O, C=S, sulfur, oxygen, CR₁₈₄R₁₈₆ and NR₁₈₈;
G₆ is nitrogen or carbon;
the ring containing D₇, E₆, G₆, K₅ and J in formula Ip may be a saturated or unsaturated 5 membered ring, and may optionally contain one or two double bonds and may optionally contain from one to three heteroatoms in the ring and may optionally have one or two C=O or C=S groups;
R₁₈₁ is C₁-C₆ alkyl which may optionally be substituted with one or two substituents independently selected from hydroxy, fluoro, chloro, bromo, iodo, C₁-C₄ alkyl, CF₃, -C(=O)-O-(C₁-C₄ alkyl), -OC(=O)(C₁-C₄ alkyl), -OC(=O)N(C₁-C₄ alkyl)(C₁-C₂ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -S(C₁-C₄ alkyl), -CN, -NO₂, -SO(C₁-C₄ alkyl), SO₂NH(C₁-C₄ alkyl) and SO₂N(C₁-C₄ alkyl)(C₁-C₂ alkyl), wherein each of the C₁-C₄ alkyl groups in the foregoing R₁₈₁ groups may optionally contain one or two double or triple bonds;
R₁₈₂ is C₁-C₁₂ alkyl which may optionally contain from one to three double or triple bonds, aryl or (C₁-C₄ alkylene)aryl, wherein said aryl and aryl moieties of said (C₁-C₄ alkylene)aryl is selected from the group consisting of phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl,, pyrrolyl, indolyl, pyrrolopyridyl, oxazolyl and benzoxazolyl, or C₃-C₈ cycloalkyl or (C₁-C₆ alkylene) (C₃-C₆ cycloalkyl), wherein one or two of the carbon atoms of said cycloalkyl and one or two of the carbon atoms of 5 to 8 membered cycloalkyl moieties of said (C₁-C₆ alkylene)(C₃-C₈ cycloalkyl) may optionally and independently be replaced by oxygen, sulfur or NZ₁₈₂ wherein Z₁₈₂ is selected from hydrogen, C₁-C₄ alkyl, benzyl and C₁-C₄ alkanoyl, and wherein each of the foregoing R₁₈₂ groups may optionally be substituted with from one to three substituents independently selected from chloro, fluoro and C₁-C₄ alkyl, or one substituent selected from bromo, iodo, C₁-C₆ alkoxy, -OC(=O)(C₁-C₆ alkyl), -OC(=O)N(C₁-C₄ alkyl)(C₁-C₂ alkyl), -S(C₁-C₆ alkyl), amino, -NH(C₁-C₂ alkyl), -N(C₁-C₂ alkyl)(C₁-C₄ alkyl), -N(C₁-C₄ alkyl)-CO-(C₁-C₄ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -SH, -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl);
-NR₁₈₁R₁₈₂ or CR₁₈₁R₁₈₂R₁₈₁₀ may form a saturated 3 to 8 membered carbocyclic ring which may optionally contain from one to three double bonds and wherein one or two of the ring carbon atoms of such 5 to 8 membered rings may optionally and independently be replaced by oxygen, sulfur atom or NZ₁₈₃ wherein Z₁₈₃ is hydrogen, C₁-C₄ alkyl, benzyl or C₁-C₄ alkanoyl;
R₁₈₃ is hydrogen, C₁-C₄ alkyl, -O(C₁-₄ alkyl), chloro, fluoro, bromo, iodo, (C₁-C₂ alkylene)-O-(C₁-C₂ alkyl), (C₁-C₂ alkylene)-OH, or -S(C₁-C₄ alkyl);
each R₁₈₄ is independently hydrogen, (C₁-C₆ alkyl), fluoro, chloro, bromo, iodo, hydroxy, cyano, amino, (C₁-C₂ alkylene)-OH, CF₃, CH₂SCH₃, nitro, -O(C₁-C₄ alkyl), -N(C₁-C₄ alkyl)(C₁-C₂ alkyl), -S(C₁-C₄ alkyl), -CO(C₁-C₄ alkyl), -C(=O)H or -C(=O)O(C₁-C₄ alkyl);
R₁₈₆ is hydrogen, methyl or ethyl;
R₁₈₈ is hydrogen or C₁-C₄ alkyl;
R₁₈₅ is phenyl, pyridyl, pyrazinyl, pyrimidyl, pyridazinyl, and wherein each of the foregoing R₁₈₅ groups is substituted with from one to four substituents R₁₈₁₃ wherein one to three of said substituents may be selected independently from fluoro, chloro, C₁-C₆ alkyl and -O(C₁-C₆ alkyl) and one of said substituents may be selected from bromo, iodo, formyl, OH, (C₁-C₄ alkylene)-OH, (C₁-C₄ alkylene)-O-(C₁-C₂ alkyl), cyano, trifluoromethyl, nitro, amino, -NH(C₁₋C₄ alkyl), -N(C₁₋C₂ alkyl)(C₁-C₆ alkyl), -OCO(C₁-C₄ alkyl), -(C₁-C₄ alkylene)-O-(C₁-C₄ alkyl), -S-(C₁-C₆ alkyl), -(C₁-C₄ alkylene)-S-(C₁-C₄ alkyl), -C(=O)O(C₁-C₄ alkyl), -C(=O)(C₁-C₄ alkyl), -COOH, -SO₂NH(C₁-C₆ alkyl), -SO₂N(C₁-C₂ alkyl)(C₁-C₄ alkyl), -SO₂NH₂, -NHSO₂(C₁-C₄ alkyl), -S(C₁-C₆ alkyl) and -SO₂(C₁-C₆ alkyl), and wherein each of the C₁-C₄ alkyl moieties and C₁-C₆ alkyl moieties in the foregoing R₁₈₅ groups may optionally have one or two double bonds;
R₁₈₇ is hydrogen, C₁-C₄ alkyl, halo, (e.g. chloro, fluoro, iodo or bromo), hydroxy, -O(C₁-C₄ alkyl), -C(=O)(C₁-C₄ alkyl), -C(=O)O(C₁-C₄ alkyl), -OCF₃, trifluoromethyl, hydroxymethyl or -CH₂O(C₁-C₂ alkyl);
R₁₈₁₀ is hydrogen, hydroxy, methoxy or fluoro;
R₁₈₁₁ is hydrogen or C₁-C₄ alkyl, with the proviso that in the formula IP (a) when both J and K₅ are carbons and D₇ is CR₁₈₄ and E₆ is nitrogen, then G₆ can not be nitrogen (b) when both J and K₅ are carbons and D₇ and G₆ are nitrogens, then E₆ can not be CR₁₈₄, C=O or C=S (c) when both J and K₅ are carbons and D₇ and E₆ are carbons, then G₆ can not be nitrogen (d) when G₆ is carbon, it must be double bonded to E₆ and (e) in the ring containing J, K₅, D₇, E₆ and G₆, there can not be two double bonds adjacent to each other,
or its pharmaceutically acceptable salt.

22. A pharmaceutical composition for prophylaxis or therapy of a postoperative stress as claimed in claim 1, wherein the compound having CRF antagonist activating effect is a compound represented by the formula (A) 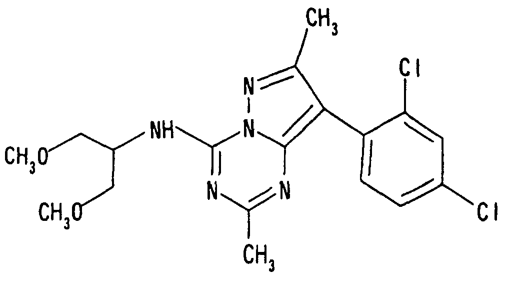 , the formula (B) 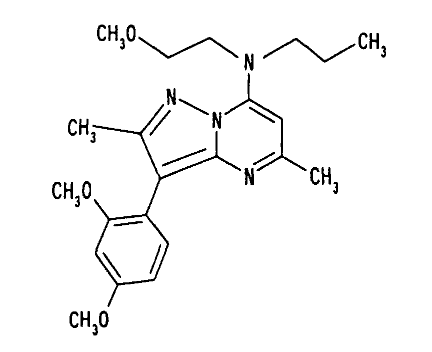 , the formula (C) 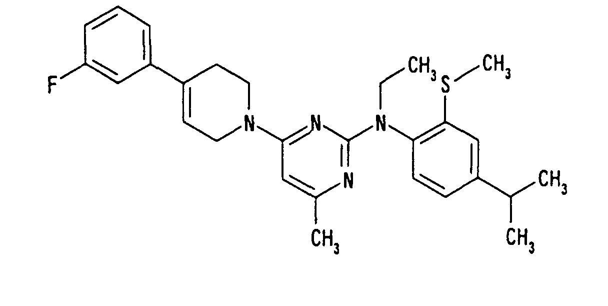 , the formula (D) 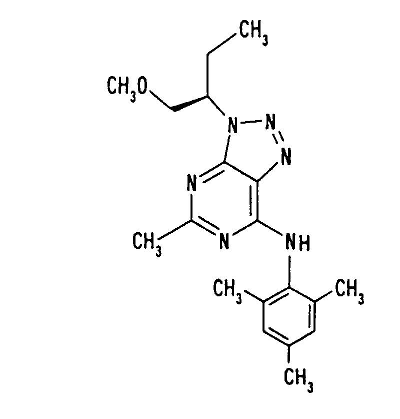 , the formula (E) 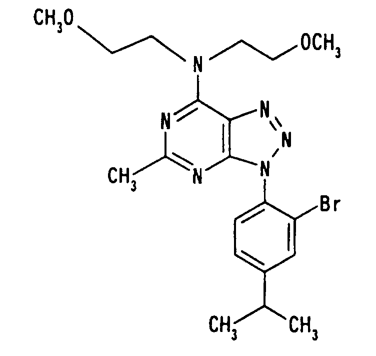 or
the formula (F)

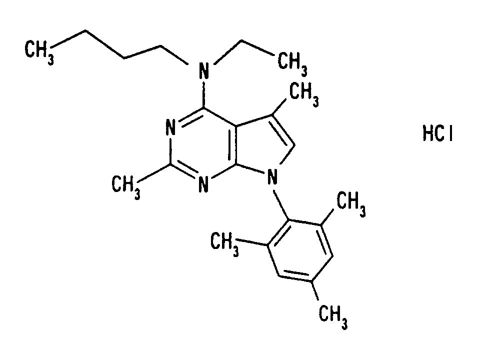

23. A pharmaceutical composition for prophylaxis or therapy of a postoperative stress as claimed in claim 1, wherein the compound having CRF antagonist activating effect is N-butyl-N-(2,5-dimethyl-7-(2,4,6-trimethylphenyl)-7H-pyrrolo(2,3-d)pyrimidine-4-yl)-N-ethylamine acid salt.

24. A compound having CRF antagonist activating effect is a compound represented by the formula Iq 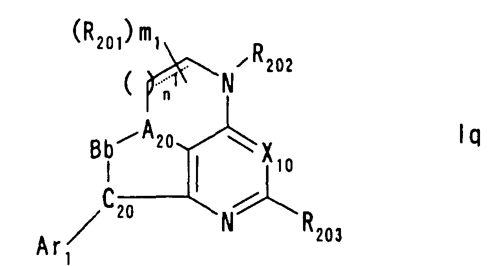 wherein n₁ is 1 or 2;
A₂₀ and C₂₀ are each independently nitrogen, carbon or CH;
Bb is nitrogen or CR₂₀₄;
with the proviso that at least one of A₂₀, Bb and C₂₀ is nitrogen, A₂₀ Bb and C₂₀ are not all nitrogen, and either A₂₀-Bb or Bb-C₂₀ is a double bond;
X₁₀ is nitrogen or CH;
Ar₁ is aryl, substituted aryl, heteroaryl or substituted heteroaryl;
R₂₀₁ is an optional substituent, which at each occurrence independently selected from alkyl, alkyliden, arylalkyl and heteroarylalkyl, and m₁ is 0, 1, 2 or 3 and represents the number of R₂₀₁ substituents;
R₂₀₂ is -C(H)_{0,1}(R₂₀₅)(R₂₀₆) or -SO₂R₂₀₆;
R₂₀₃ is hydrogen or alkyl;
R₂₀₄ is hydrogen, alkyl or haloalkyl;
R₂₀₅ is hydrogen, keto, alkyl, alkyliden or halo; and
R₂₀₆ is a radical of the formula -Y₂₀-Z₂₀-R₂₀₇, with the proviso that Y₂₀ is an alkanediyl, substituted alkanediyl or a bond, Z₂₀ is NH, -N(R₂₀₈), oxygen, sulfur, SO₂, C(=O), C(=O)O, OC(=O), NHC(=O), C(=O)NH, NH(SO₂), (SO₂)NH, NR₂₀₉C(=O)O or a bond;
R₂₀₇ is hydrogen, alkyl, substituted alkyl, aryl, substituted aryl, aralkyl, substituted aralkyl, heterocycle, substituted heterocycle, heterocyclealkyl, or substituted heterocyclealkyl; or
R₂₀₈ and R₂₀₉ are the same or different and are independently alkyl, substituted alkyl, aryl, substituted aryl, aralkyl, substituted aralkyl, heterocycle, substituted heterocycle, heterocyclealkyl or substituted heterocyclealkyl; or
R₂₀₇ and R₂₀₈ taken together with the nitrogen atom to which they are attached form a heterocycle ring or substituted heterocycle ring; or
R₂₀₅ and R₂₀₆ taken together form cycloalkyl, substituted cycloalkyl, cycloalkylcycloalkyl, substituted cycloalkylcycloalkyl, cycloalkylaryl, substituted cycloalkylaryl, cycloalkylheterocycle or substituted cycloalkylheterocycle, and the symbol ----- represents single bonds or double bonds,
its stereoisomer, pro-drugs or its pharmaceutically acceptable salt.

25. A pharmaceutical composition for prophylaxis or therapy of a postoperative stress as claimed in claim 1, wherein the compound having CRF antagonist activating effect is a compound represented by the formula Ir 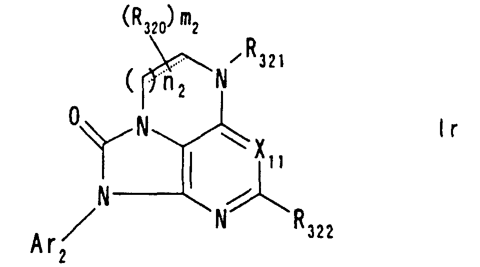 wherein n₂ is 1 or 2;
m₂ is 0, 1, 2 or 3;
X₁₁ is N or CR';
R₃₂₀ is an optional substituent, which at each occurrence may be independently selected from C₁-C₆ alkyl, C₃-C₆ alkenyl, C₁-C₆ alkylidenyl or C₁-C₆ alkylAr₂;
R₃₂₁ represents -C(H)_{0.1}(R₃₂₃)(R₃₂₄);
R₃₂₂ is hydrogen or C₁-C₆ alkyl;
R₃₂₃ is selected from hydrogen, keto, C₁-C₆ alkyl, mono or di(C₃-C₆ cycloalkyl)methyl, C₃-C₆ cycloalkyl, C₃-C₆ alkenyl, hydroxy C₁-C₆ alkyl, C₁-C₆ alkylcarbonyloxy C₁-C₆ alkyl or C₁-C₆ alkyloxy C₁-C₆ alkyl; and
R₃₂₄ is selected from hydrogen, Ar₂₁, C₁-C₆ alkyl Ar₂₁, OAr₂₁, C₁-C₈ alkyl, C₁-C₆ alkyloxy, C₃-C₆ cycloalkyl, mono or di(C₃-C₆ cycloalkyl)methyl, C₃-C₆ alkenyl, C₃-C₆ alkynyl, C₁-C₆ alkyloxy C₁-C₆ alkyl, C₁-C₆ alkoxyAr₂₁, hydroxyl C₁-C₆ alkyl, thienyl C₁-C₆ alkyl, furanyl C₁-C₆ alkyl, C₁-C₆ alkylthio C₁-C₆ alkyl, morpholinyl, mono or di(C₁-C₆ alkyl)amino C₁-C₆ alkyl, amino, (C₁-C₆ alkyl)amino, di(C₁-C₆ alkyl)amino, (C₁-C₆ alkylAr₂₁)amino, (C₁-C₆ alkyl)(Ar₂₁)amino, C₁-C₆ alkylcarbonyl C₁-C₆ alkyl, C₁-C₆ alkylcarbonyloxy C₁-C₆ alkyl, sulfonyl(C₁-C₈ alkyl), C(=O) C₁-C₆ alkyl, C₁-C₈ alkyl substituted with phthalimido, Ar₂₁, OAr₂₁, NHAr₂₁, C(=O)Ar₂₁, C(=O)NHAr₂₁ or -C(=O)NH₂, or a radical represented by the formula -(C₁₋₆alkanediyl)-Y₂₁-(CO)_{0.1}-Ar₂₁ wherein Y₂₁ is O, NH or chemical bond, or
R₃₂₃ and R₃₂₄ taken together with carbon atom to which they are attached form C₅-C₈ cycloalkyl, C₅-C₈ cycloalkenyl, C₃-C₁₂ heterocycle ring, phenyl, naphthyl or C₅-C₈ cycloalkyl condensed to Ar₂₁, which may optionally be substituted with not less than one substituents independently selected from C₁-C₆ alkyl;
Ar₂ is phenyl, naphthyl or aromatic C₃-C₁₂ heterocycle, each of which may optionally be substituted with one, two or three substituents independently selected from halo, C₁-C₆ alkyl, triflouromethyl, O(triflouromethyl), hydroxy, cyano, C₁-C₆ alkyloxy, phenyloxy, benzoxy, C₁-C₆ alkylthio, nitro, amino, mono or di(C₁-C₆ alkyl)amino, (C₁-C₆ alkyl)(C₁-C₆ alkanoyl)amino or piperidinyl, two of the above substituents optionally combining to form C₁-C₆ alkylidinyl or C₁-C₆ alkylidenyl, whose one, two or three carbons is/are replaced by hetero atom or hetero atoms selected independently from oxygen, nitrogen or sulfur, and
Ar₂₁ is phenyl, naphthyl or aromatic C₃-C₁₂ heterocycle ring optionally substituted with one, two or three substituents which is/are independently selected from a group consisting of halo, C₁-C₆ alkyl, C₁-C₆ alkyloxy, di(C₁-C₆ alkyl)amino, di(C₁-C₆ alkyl)amino C₁-C₆ alkyl, triflouromethyl, sulfonyl(C₁-C₆ alkyl) and C₁-C₆ alkyl substituted with morpholinyl, and the symbol ------ represents single bond or double bond,
its stereoisomer, pro-drugs or its pharmaceutically acceptable salt.

26. A pharmaceutical composition for prophylaxis or therapy of a postoperative stress as claimed in claim 1, wherein the compound having CRF antagonist activating effect is a compound represented by the formula Is 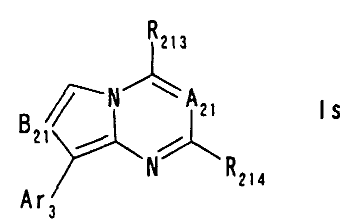 wherein A₂₁ and B₂₁ are selected from CR₂₁₅ wherein R₂₁₅ shows the group selected from hydrogen and C₁-C₆ alkyl, and nitrogen;
R₂₁₃ is NR₂₁₆R₂₁₇;
R₂₁₄ is C₁-C₆ alkyl;
R₂₁₆ is selected from the group consisting of hydrogen, C₁-C₆ alkyl, mono- or di(C₃-C₆ cycloalkyl)methyl, C₃-C₆ cycloalkyl, C₃-C₆ alkenyl, hydroxy C₁-C₄ alkyl, C₁-C₆ alkylcarbonyloxy C₁-C₆ alkyl and C₁-C₆ alkyloxy C₁-C₆ alkyl;
R₂₁₇ is selected from C₁-C₈ alkyl, mono or di(C₃-C₆ cycloalkyl)methyl, Ar₂₂CH₂, C₃-C₆ alkenyl, C₁-C₆ alkyloxy C₁-C₆ alkyl, hydroxyl C₁-C₆ alkyl, thienylmethyl, furanylmethyl, C₁-C₆ alkylthio C₁-C₆ alkyl, morpholinyl, mono or di(C₁-C₆alkyl)amino C₁-C₆ alkyl, di(C₁-C₆ alkyl)amino, C₁-C₆ alkylcarbonyl C₁-C₆ alkyl, C₁-C₆ alkyl substituted with imidazolyl, or a radical of the formula (C₁-C₆ alkanediyl)-O-CO-Ar₂₂;
or R₂₁₆ and R₂₁₇ taken together with nitrogen to which they are attached form a pyrrolidinyl, piperidinyl, homopiperidinyl or morpholinyl, which is optionally substituted with C₁-C₆ alkyl or C₁-C₆ alkyloxy;
Ar₃ is selected from phenyl substituted with one, two or three substituents independently selected from halo, C₁-C₆ alkyl, triflouromethyl, cyano, C₁-C₆ alkyloxy, benzyloxy, C₁-C₆ alkylthio, nitro, amino and mono or di(C₁-C₆ alkyl)amino, and pyridinyl substituted with one, two or three substituents independently selected from halo, C₁-C₆ alkyl, triflouromethyl, hydroxy, cyano, C₁-C₆ alkyloxy, benzyloxy, C₁-C₆ alkylthio, nitro, amino, mono or di(C₁-C₆ alkyl)amino and piperidinyl, and
Ar₂₂ is selected from phenyl, pyridinyl and phenyl substituted with one, two or three substituents independently selected from halo, C₁-C₆ alkyl, C₁-C₆ alkyloxy, di(C₁-C₆ alkyl)amino C₁-C₆ alkyl, triflouromethyl and C₁-C₆ alkyl substituted with morpholinyl,
its stereoisomer, pro-drugs or its pharmaceutically acceptable salt.

27. A pharmaceutical composition for prophylaxis or therapy of a postoperative stress as claimed in claim 1, wherein the compound having CRF antagonist activating effect is a compound represented by the formula It 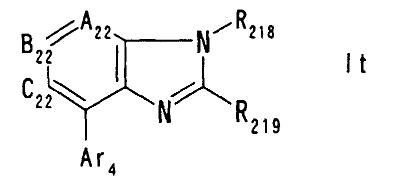 wherein A₂₂, B₂₂ and C₂₂ are selected from CR₂₂₀ and nitrogen; with the proviso that when B₂₂ is nitrogen, then A₂₂ and C₂₂ are CR₂₂₀;
R₂₂₀ is selected from hydrogen and C₁-C₆ alkyl;
R₂₁₈ is selected from NR₂₂₁R₂₂₂ and R₂₂₃;
R₂₁₉ is C₁-C₆ alkyl;
R₂₂₁ is selected from hydrogen, C₁-C₆ alkyl, mono- or di(C₃-C₆ cycloalkyl)methyl, C₃-C₆ cycloalkyl, C₃-C₆ alkenyl, hydroxy C₁-C₆ alkyl, C₁-C₆ alkylcarbonyloxy C₁-C₆ alkyl and C₁-C₆ alkyloxy C₁-C₆ alkyl;
R₂₂₂ and R₂₂₃ are independently selected from C₁-C₈ alkyl, mono or di(C₃-C₆ cycloalkyl)methyl, Ar₂₃CH₂, C₃-C₆ alkenyl, C₁-C₆ alkyloxy C₁-C₆ alkyl, hydroxy C₁-C₆ alkyl, thienylmethyl, furanylmethyl, C₁-C₆ alkylthio C₁-C₆ alkyl, morpholinyl, mono or di(C₁-C₆alkyl)amino C₁-C₆ alkyl, di(C₁-C₆ alkyl)amino, C₁-C₆ alkylcarbonyl C₁-C₆ alkyl, C₁-C₆ alkyl substituted with imidazolyl, or a radical of the formula (C₁-C₆ alkanediyl)-O-CO-Ar₂₃;
or R₂₂₁ and R₂₂₂ taken together with nitrogen to which they are attached form a pyrrolidinyl, piperidinyl, homopiperidinyl or morpholinyl, wherein optionally substituted with C₁-C₆ alkyl or C₁-C₆ alkyloxy;
Ar₄ is selected from phenyl substituted with one, two or three substituents independently selected from halo, C₁-C₆ alkyl, triflouromethyl, cyano, C₁-C₆ alkyloxy, benzyloxy, C₁-C₆ alkylthio, nitro, amino and mono or di(C₁-C₆ alkyl)amino, and pyridinyl substituted with one, two or three substituents independently selected from halo, C₁-C₆ alkyl, triflouromethyl, hydroxy, cyano, C₁-C₆ alkyloxy, benzyloxy, C₁-C₆ alkylthio, nitro, amino, mono or di(C₁-C₆ alkyl)amino and piperidinyl, and
Ar₂₃ is selected from phenyl, pyridinyl and phenyl substituted with one, two or three substituents independently selected from halo, C₁-C₆ alkyl, C₁-C₆ alkyloxy, di(C₁-C₆alkyl)amino C₁-C₆ alkyl, triflouromethyl and C₁-C₆ alkyl substituted with morpholinyl,
its stereoisomer, pro-drugs or its pharmaceutically acceptable salt.

28. A pharmaceutical composition for prophylaxis or therapy of a postoperative stress as claimed in claim 1, wherein the compound having CRF antagonist activating effect is a compound represented by the formula Iu 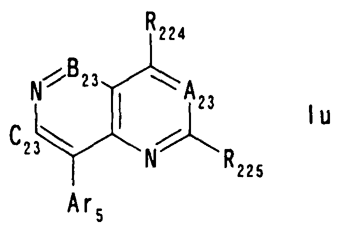 wherein A₂₃, B₂₃ and C₂₃ are selected from CR₂₂₆ and nitrogen, with the proviso that B₂₃ and C₂₃ are not both nitrogen;
R₂₂₆ is selected from hydrogen and C₁-C₆ alkyl;
R₂₂₄ is NR₂₂₇R₂₂₈;
R₂₂₅ is C₁-C₆ alkyl;
R₂₂₇ is selected from hydrogen, C₁-C₆ alkyl, mono or di(C₃-C₆ cycloalkyl)methyl, C₃-C₆ cycloalkyl, C₃-C₆ alkenyl, hydroxy C₁-C₆ alkyl, C₁-C₆ alkylcarbonyloxy C₁-C₆ alkyl and C₁-C₆ alkyloxy C₁-C₆ alkyl;
R₂₂₈ is independently selected from C₁-C₈ alkyl, mono or di(C₃-C₆ cycloalkyl)methyl, Ar₂₄CH₂, C₃-C₆ alkenyl, C₁-C₆ alkyloxy C₁-C₆ alkyl, hydroxy C₁-C₆ alkyl, thienylmethyl, furanylmethyl, C₁-C₆ alkylthio C₁-C₆ alkyl, morpholinyl, mono or di(C₁-C₆ alkyl)amino C₁-C₆ alkyl, di(C₁-C₆ alkyl)amino, C₁-C₆ alkylcarbonyl C₁-C₆ alkyl, C₁-C₆ alkyl substituted with imidazolyl, and a radical of the formula (C₁-C₆ alkanediyl)-O-CO-Ar₂₄;
or R₂₂₇ and R₂₂₈ taken together with nitrogen to which they are attached form a pyrrolidinyl, piperidinyl, homopiperidinyl or morpholinyl, which is optionally substituted with C₁-C₆ alkyl or C₁-C₆ alkyloxy;
Ar₅ is selected from phenyl substituted with one, two or three substituents independently selected from halo, C₁-C₆ alkyl, triflouromethyl, cyano, C₁-C₆ alkyloxy, benzyloxy, C₁-C₆ alkylthio, nitro, amino and mono or di(C₁-C₆ alkyl)amino, and pyridinyl substituted with one, two or three substituents independently selected from halo, C₁-C₆ alkyl, triflouromethyl, hydroxy, cyano, C₁-C₆ alkyloxy, benzyloxy, C₁-C₆ alkylthio, nitro, amino, mono or di(C₁₋₆ alkyl)amino and piperidinyl; and
Ar₂₄ is selected from phenyl, pyridinyl, and phenyl substituted with one, two or three substituents independently selected from halo, C₁-C₆ alkyl, C₁-C₆ alkyloxy, di(C₁-C₆ alkyl)amino C₁-C₆ alkyl, triflouromethyl and C₁-C₆ alkyl substituted with morpholinyl,
its stereoisomer, pro-drugs or its pharmaceutically acceptable salt.

29. A pharmaceutical composition for prophylaxis or therapy of a postoperative stress as claimed in claim 1, wherein the compound having CRF antagonist activating effect is a compound represented by the formula Iv 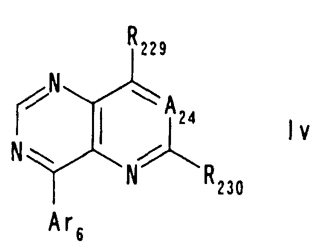 wherein A₂₄ is selected from CR₂₃₁ and nitrogen;
R₂₃₁ is selected from hydrogen and C₁-C₆ alkyl;
R₂₂₉ is NR₂₃₂R₂₃₃;
R₂₃₀ is C₁-C₆ alkyl;
R₂₃₂ is selected from hydrogen, C₁-C₆ alkyl, mono or di(C₃-C₆ cycloalkyl)methyl, C₃-C₆ cycloalkyl, C₃-C₆ alkenyl, hydroxy C₁-C₆ alkyl, C₁-C₆ alkylcarbonyloxy C₁-C₆ alkyl and C₁-C₆ alkyloxy C₁-C₆ alkyl;
R₂₃₃ is independently selected from C₁-C₈ alkyl, mono or di(C₃-C₆ cycloalkyl)methyl, Ar₂₅CH₂, C₃-C₆ alkenyl, C₁-C₆ alkyloxy C₁-C₆ alkyl, hydroxy C₁-C₆ alkyl, thienylmethyl, furanylmethyl, C₁-C₆ alkylthio C₁-C₆ alkyl, morpholinyl, mono or di(C₁-C₆ alkyl)amino C₁-C₆ alkyl, di(C₁-C₆ alkyl)amino, C₁-C₆ alkylcarbonyl C₁-C₆ alkyl, C₁-C₆ alkyl substituted with imidazolyl, and a radical of the formula-(C₁-C₆ alkanediyl)-O-CO-Ar₂₅;
or R₂₃₂ and R₂₃₃ taken together with nitrogen to which they are attached form a pyrrolidinyl, piperidinyl, homopiperidinyl or morpholinyl, which is optionally substituted with C₁-C₆ alkyl or C₁-C₆ alkyloxy;
Ar₆ is selected from phenyl substituted with one, two or three substituents independently selected from halo, C₁-C₆ alkyl, triflouromethyl, cyano, C₁-C₆ alkyloxy, benzyloxy, C₁-C₆ alkylthio, nitro, amino and mono or di(C₁-C₆ alkyl)amino, and pyridinyl substituted with one, two or three substituents independently selected from halo, C₁-C₆ alkyl, triflouromethyl, hydroxy, cyano, C₁-C₆ alkyloxy, benzyloxy, C₁-C₆ alkylthio, nitro, amino, mono or di(C₁-C₆ alkyl)amino and piperidinyl; and
Ar₂₅ is selected from phenyl, pyridinyl, and phenyl substituted with one, two or three substituents independently selected from halo, C₁-C₆ alkyl, C₁-C₆ alkyloxy, di(C₁-C₆ alkyl)amino C₁-C₆ alkyl, triflouromethyl and C₁-C₆ alkyl substituted with morpholinyl,
its stereoisomer, pro-drugs or its pharmaceutically acceptable salt.

30. A pharmaceutical composition for prophylaxis or therapy of a postoperative stress as claimed in claim 1, wherein the compound having CRF antagonist activating effect is a compound represented by the formula Iw 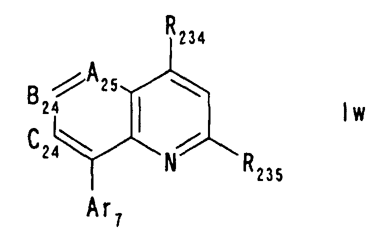 wherein A₂₅, B₂₄ and C₂₄ are selected from CR₂₃₆ and nitrogen, with the proviso that B₂₄ and C₂₄ are not both nitrogen;
R₂₃₆ is selected from hydrogen and C₁-C₆ alkyl;
R₂₃₄ is NR₂₃₇R₂₃₈;
R₂₃₅ is C₁-C₆ alkyl;
R₂₃₇ is selected from hydrogen, C₁-C₆ alkyl, mono or di(C₃-C₆ cycloalkyl)methyl, C₃-C₆ cycloalkyl, C₃-C₆ alkenyl, hydroxy C₁-C₆ alkyl, C₁-C₆ alkylcarbonyloxy C₁-C₆ alkyl and C₁-C₆ alkyloxy C₁-C₆ alkyl;
R₂₃₈ is independently selected from C₁-C₈ alkyl, mono or di(C₃-C₆ cycloalkyl)methyl, Ar₂₆CH₂, C₃-C₆ alkenyl, C₁-C₆ alkyloxy C₁-C₆ alkyl, hydroxy C₁-C₆ alkyl, thienylmethyl, furanylmethyl, C₁-C₆ alkylthio C₁-C₆ alkyl, morpholinyl, mono or di(C₁-C₆ alkyl)amino C₁-C₆ alkyl, di(C₁-C₆ alkyl)amino, C₁-C₆ alkylcarbonyl C₁-C₆ alkyl, C₁-C₆ alkyl substituted with imidazolyl, and a radical of the formula -( C₁-C₆ alkanediyl)-O-CO-Ar₂₆;
or R₂₃₇ and R₂₃₈ taken together with nitrogen to which they are attached form a pyrrolidinyl, piperidinyl, homopiperidinyl or morpholinyl, which is optionally substituted with C₁-C₆ alkyl or C₁-C₆ alkyloxy;
Ar₇ is selected from phenyl substituted with one, two or three substituents independently selected from halo, C₁-C₆ alkyl, triflouromethyl, cyano, C₁-C₆ alkyloxy, benzyloxy, C₁-C₆ alkylthio, nitro, amino and mono or di(C₁-C₆ alkyl)amino, and pyridinyl substituted with 1, 2 or 3 substituents independently selected from halo, C₁-C₆ alkyl, triflouromethyl, hydroxy, cyano, C₁-C₆ alkyloxy, benzyloxy, C₁-C₆ alkylthio, nitro, amino, mono or di(C₁-C₆ alkyl)amino and piperidinyl; and
Ar₂₆ is selected from phenyl, pyridinyl, and phenyl substituted with one, two or three substituents independently selected from halo, C₁-C₆ alkyl, C₁-C₆ alkyloxy, di(C₁-C₆ alkyl)amino C₁-C₆ alkyl, triflouromethyl and C₁-C₆ alkyl substituted with morpholinyl,
its stereoisomer, pro-drugs or its pharmaceutically acceptable salt.

31. A pharmaceutical composition for prophylaxis or therapy of a postoperative stress as claimed in claim 1, wherein the compound having CRF antagonist activating effect is a compound represented by the formula Ix 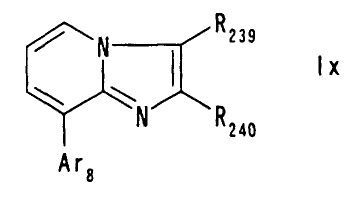 wherein R₂₃₉ is selected from NR₂₄₁R₂₄₂ and R₂₄₃;
R₂₄₀ is C₁-C₆ alkyl;
R₂₄₁ is selected from hydrogen, C₁-C₆ alkyl, mono or di(C₃-C₆ cycloalkyl)methyl, C₃-C₆ cycloalkyl, C₃-C₆ alkenyl, hydroxy C₁-C₆ alkyl, C₁-C₆ alkylcarbonyloxy C₁-C₆ alkyl and C₁-C₆ alkyloxy C₁-C₆ alkyl;
R₂₄₂ and R₂₄₃ are independently selected from C₁-C₈ alkyl, mono or di(C₃-C₆ cycloalkyl)methyl, Ar₂₇CH₂, C₃-C₆ alkenyl, C₁-C₆ alkyloxy C₁-C₆ alkyl, hydroxy C₁-C₆ alkyl, thienylmethyl, furanylmethyl, C₁-C₆ alkylthio C₁-C₆ alkyl, morpholinyl, mono or di(C₁-C₆ alkyl)amino C₁-C₆ alkyl, di(C₁-C₆ alkyl)amino, C₁-C₆ alkylcarbonyl C₁-C₆ alkyl, C₁-C₆ alkyl substituted with imidazolyl, and a radical of the formula-(C₁-C₆ alkanediyl)-O-CO-Ar₂₇;
or R₂₄₁ and R₂₄₂ taken together with nitrogen to which they are attached form a pyrrolidinyl, piperidinyl, homopiperidinyl or morpholinyl, which is optionally substituted with C₁-C₆ alkyl or C₁-C₆ alkyloxy;
Ar₈ is selected from phenyl substituted with one, two or three substituents independently selected from halo, C₁-C₆ alkyl, triflouromethyl, cyano, C₁-C₆ alkyloxy, benzyloxy, C₁-C₆ alkylthio, nitro, amino and mono or di(C₁-C₆ alkyl)amino, and pyridinyl substituted with one, two or three substituents independently selected from halo, C₁-C₆ alkyl, triflouromethyl, hydroxy, cyano, C₁-C₆ alkyloxy, benzyloxy, C₁-C₆ alkylthio, nitro, amino, mono or di(C₁-C₆ alkyl)amino and piperidinyl; and
Ar₂₇ is selected from phenyl, pyridinyl, and phenyl substituted with one, two or three substituents independently selected from halo, C₁-C₆ alkyl, C₁-C₆ alkyloxy, di(C₁-C₆ alkyl)amino C₁-C₆ alkyl, triflouromethyl and C₁-C₆ alkyl substituted with morpholinyl,
its stereoisomer, pro-drugs or its pharmaceutically acceptable salt.

32. A pharmaceutical composition for prophylaxis or therapy of a postoperative stress as claimed in claim 1, wherein the compound having CRF antagonist activating effect is a compound represented by the formula Iy and Iz 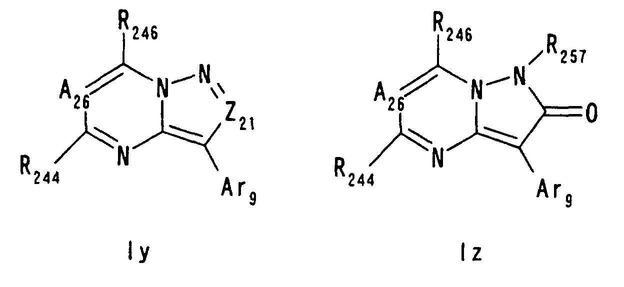 wherein A₂₆ is nitrogen or CR₂₄₇;
Z₂₁ is nitrogen or CR₂₄₅;
Ar₉ is selected from phenyl, naphthyl, pyridyl, pyrimidyl, triazinyl, furanyl, thienyl, benzothienyl, benzofuranyl, 2,3-dihydrobenzofuranyl, 2,3-dihydrobenzothienyl, indanyl, 1,2-benzopyranyl, 3,4-dihydro-1,2-benzopyranyl, tetralinyl, each Ar₉ is optionally substituted with 1 to 5 R₂₄₈ groups and each Ar₉ is attached to an unsaturated carbon atom;
R₂₄₇ is, at each occurrence, independently selected from hydrogen, C₁-C₄ alkyl, C₂-C₄ alkenyl, C₂-C₄ alkynyl, C₃-C₆ cycloalkyl, C₄-C₇ cycloalkylalkyl, halo, cyano, C₁-C₄ haloalkyl;
R₂₄₄ is, at each occurrence, independently selected from hydrogen, C₁-C₄ alkyl, C₂-C₄ alkenyl, C₂-C₄ alkynyl, halo, cyano, C₁-C₄ haloalkyl, C₁-C₁₂ hydroxyalkyl, C₂-C₁₂ alkoxyalkyl, C₂-C₁₀ cyanoalkyl, C₃-C₆ cycloalkyl, C₄-C₁₀ cycloalkylalkyl, NR₂₅₂R₂₅₃, C₁-C₄ alkyl-NR₂₅₂R₂₅₃, NR₂₅₂COR₂₅₃, OR₂₅₄, SH and S(O)n₃R₂₅₅;
R₂₄₅ is selected from hydrogen, C₁-C₄ alkyl, C₂-C₄ alkenyl, C₂-C₄ alkynyl, C₃-C₆ cycloalkyl, C₄-C₁₀ cycloalkylalkyl, C₁-C₄ hydroxyalkyl, halo, cyano, -NR₂₄₉R₂₅₀, NR₂₅₂COR₂₅₃, -NR₂₄₉S(O)n₃R₂₅₀, S(O)n₃R₂₄₉R₂₅₀, C₁-C₄ haloalkyl, -OR₂₅₀, SH and -S(O)n₃R₂₅₅;
R₂₄₆ is selected from hydrogen, OR₂₅₀, SH, S(O)n₃R₂₅₆, COR₂₅₀, CO₂R₂₅₀, OC(O)R₂₅₆, NR₂₅₁COR₂₅₀, N(COR₂₅₀)₂, NR₂₅₁CONR₂₄₉R₂₅₀, NR₂₅₁CO₂R₂₅₆, NR₂₄₉R₂₅₀, NR₂₄₉ₐR₂₅₀ₐ, N(OR₂₅₀)R₂₄₉, CONR₂₄₉R₂₅₀, aryl heteroaryl and heterocyclyl, or is selected from C₁-C₁₀ alkyl, C₂-C₁₀ alkenyl, C₂-C₁₀ alkynyl, C₃-C₈ cycloalkyl, C₅-C₈ cycloalkenyl, C₄-C₁₂ cycloalkylalkyl or C₆-C₁₀ cycloalkenylalkyl, each of which may be optionally substituted with one to three substituents independently selected from the group consisting of C₁-C₆ alkyl, C₃-C₆ cycloalkyl, halo, C₁-C₄ haloalkyl, cyano, OR₂₅₈, SH, S(O)n₃R₂₅₆, COR₂₅₈, CO₂R₂₅₈, OC(O)R₂₅₆, NR₂₅₁COR₂₅₈, N(COR₂₅₈)₂, NR₂₅₁CONR₂₅₉R₂₅₈, NR₂₅₁CO₂R₂₅₆, NR₂₅₉R₂₅₈, CONR₂₅₉R₂₅₈, aryl, heteroaryl and heterocyclyl;
R₂₄₈ is, at each occurrence, independently selected at each occurrence from C₁-C₁₀ alkyl, C₂-C₁₀ alkenyl, C₂-C₁₀ alkynyl, C₃-C₆ cycloalkyl, C₄-C₁₂ cycloalkylalkyl, NO₂, halo, cyano, C₁-C₄ haloalkyl, NR₂₄₉R₂₅₀, NR₂₅₁COR₂₅₀, NR₂₅₁CO₂R₂₅₀, COR₂₅₀, OR₂₅₀, CONR₂₄₉R₂₅₀, CO(NOR₂₅₂)R₂₅₀, CO₂R₂₅₀ and S(O)n₃R₂₅₀, wherein each of such C₁-C₁₀ alkyl, C₂-C₁₀ alkenyl, C₂-C₁₀ alkynyl, C₃-C₆ cycloalkyl and C₄-C₁₂ cycloalkylalkyl of the above groups are, at each occurrence, optionally substituted with one to three substituents independently selected from C₁-C₄ alkyl, NO₂, halo, cyano, NR₂₄₉R₂₅₀, NR₂₅₁COR₂₅₀, NR₂₅₁CO₂R₂₅₀, COR₂₅₀, OR₂₅₀, CONR₂₄₉R₂₅₀, CO₂R₂₅₀, CO(NOR₂₅₂)R₂₅₀ and S(O)n₃R₂₅₀;
R₂₄₉ and R₂₅₀, R₂₄₉ₐ and R₂₅₀ₐ are, at each occurrence, independently selected from hydrogen, C₁-C₁₀ alkyl, C₃-C₁₀ alkenyl, C₃-C₁₀ alkynyl, C₁-C₁₀ haloalkyl with 1 to 10 halogens, C₂-C₈ alkoxyalkyl, C₃-C₆ cycloalkyl, C₄-C₁₂ cycloalkylalkyl, C₅-C₁₀ cycloalkenyl, or C₆-C₁₄ cycloalkenylalkyl, wherein each of the above groups, at each occurrence, may optionally be substituted with one to three substituents independently selected from C₁-C₆ alkyl, C₃-C₆ cycloalkyl, halo, C₁-C₄ haloalkyl, cyano, OR₂₅₈, SH, S(O)n₃R₂₅₆, COR₂₅₈, CO₂R₂₅₈, OC(O)R₂₅₆, NR₂₅₁COR₂₅₈, N(COR₂₅₈)₂, NR₂₅₁CONR₂₅₉R₂₅₈, NR₂₅₁CO₂R₂₅₆, NR₂₅₉R₂₅₈, CONR₂₅₉R₂₅₈, aryl, heteroaryl and heterocyclyl, or R₂₄₉ and R₂₅₀, R₂₄₉ₐ and R₂₅₀ₐ are, at each occurrence, independently selected from aryl, aryl(C₁-C₄ alkyl), heteroaryl, heteroaryl(C₁-C₄ alkyl), heterocyclyl and heterocyclyl(C₁-C₄ alkyl);
alternatively, NR₂₄₉R₂₅₀ and NR₂₄₉ₐR₂₅₀ₐ are independently piperidine, pyrrolidine, piperazine, N-methylpiperidine, morpholine or thiomorpholine, each optionally substituted with one to three of C₁-C₄ alkyl;
R₂₅₁ is, at each occurrence, independently selected from hydrogen or C₁-C₄ alkyl;
R₂₅₂ and R₂₅₃ are, at each occurrence, each independently selected from hydrogen, C₁-C₄ alkyl and C₃-C₆ cycloalkyl;
R₂₅₄ is selected from hydrogen, C₁-C₄ alkyl, C₁-C₄ haloalkyl and C₃-C₆ cycloalkyl;
R₂₅₅ is C₁-C₄ alkyl or C₁-C₄ haloalkyl;
R₂₅₆ is selected from C₁-C₄ alkyl, C₁-C₄ haloalkyl, C₂-C₈ alkoxyalkyl, C₃-C₆ cycloalkyl, C₄-C₁₂ cycloalkylalkyl, aryl, aryl(C₁-C₄ alkyl)-, heteroaryl or heteroaryl(C₁-C₄ alkyl)-;
R₂₅₇ is, at each occurrence, selected from C₁-C₁₀ alkyl, C₃-C₁₀ alkenyl, C₃-C₁₀ alkynyl, C₃-C₈ cycloalkyl, or C₄-C₁₂ cycloalkylalkyl, each of which is optionally substituted with one to three substituents independently selected from C₁-C₆ alkyl, C₃-C₆ cycloalkyl, halo, C₁-C₄ haloalkyl, cyano, OR₂₅₈, SH, S(O)n₃R₂₅₈, COR₂₅₈, CO₂R₂₅₈, OC(O)R₂₅₈, NR₂₅₁COR₂₅₈, N(COR₂₅₈)₂, NR₂₅₁CONR₂₅₉R₂₅₈, NR₂₅₁CO₂R₂₅₈, NR₂₅₉R₂₅₈, CONR₂₅₉R₂₅₈, and C₁-C₆ alkylthio, C₁-C₆ alkylsulfinyl and C₁-C₆ alkylsulfonyl;
R₂₅₈ and R₂₅₉ are, at each occurrence, independently selected from hydrogen, C₁-C₆ alkyl, C₃-C₁₀ cycloalkyl and C₄-C₁₆ cycloalkylalkyl, with the proviso that R₂₅₈ of S(O)ₙ₃R₂₅₈ can not be hydrogen;
aryl is, at each occurrence, phenyl or naphthyl, each optionally substituted with one to five substituents independently selected from C₁-C₆ alkyl, C₃-C₆ cycloalkyl, halo, C₁-C₄ haloalkyl, cyano, OR₂₅₈, SH, S(O)n₃R₂₅₈, COR₂₅₈, CO₂R₂₅₈, OC(O)R₂₅₈, NR₂₅₁COR₂₅₈, N(COR₂₅₈)₂, NR₂₅₁CONR₂₅₉R₂₅₈, NR₂₅₁CO₂R₂₅₈, NR₂₅₉R₂₅₈ and CONR₂₅₉R₂₅₈;
heteroaryl is, at each occurrence, pyridyl, pyrimidyl, triazinyl, furanyl, pyranyl, quinolinyl, isoquinolinyl, thienyl, imidazolyl, thiazolyl, indolyl, pyrrolyl, oxazolyl, benzofuranyl, benzothienyl, benzothiazolyl, isoxazolyl, pyrazolyl, 2,3-dihydrobenzothienyl or 2,3-dihydrobenzofuranyl, each being optionally substituted with one to five substituents independently selected from C₁-C₆ alkyl, C₃-C₆ cycloalkyl, halo, C₁-C₄ haloalkyl, cyano, OR₂₅₈, SH, S(O)n₃R₂₅₈, -COR₂₅₈, CO₂R₂₅₈, OC(O)R₂₅₈, NR₂₅₁COR₂₅₈, N(COR₂₅₈)₂, NR₂₅₁CONR₂₅₉R₂₅₈, NR₂₅₁CO₂R₂₅₈, NR₂₅₉R₂₅₈ and CONR₂₅₉R₂₅₈;
heterocyclyl is, at each occurrence, a saturated or partially saturated heteroaryl optionally substituted with one to five substituents independently selected from C₁-C₆ alkyl, C₃-C₆ cycloalkyl, halo, C₁-C₄ haloalkyl, cyano, OR₂₅₈, SH, S(O)n₃R₂₅₈, -COR₂₅₈, CO₂R₂₅₈, OC(O)R₂₅₈, NR₂₅₁COR₂₅₈, N(COR₂₅₈)₂, NR₂₅₁CONR₂₅₉R₂₅₈, NR₂₅₁CO₂R₂₅₈, NR₂₅₉R₂₅₈ and CONR₂₅₉R₂₅₈;
n3 is, at each occurrence, independently 0, 1 or 2; its isomer, its stereoisomer forms or mixture of its stereoisomer, or its pharmaceutically acceptable salt or its pro-drugs form compound.

33. A pharmaceutical composition for prophylaxis or therapy of a postoperative stress as claimed in claim 1, wherein the compound having CRF antagonist activating effect is a compound represented by the formula Iaa 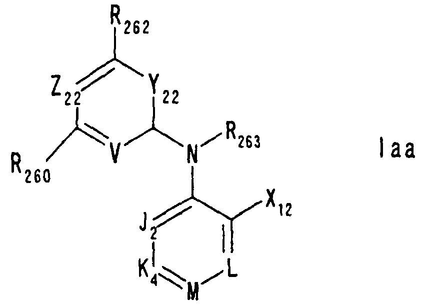 wherein
Y₂₂ is CR₂₆₂ₐ, N, or CR₂₈₈;
when Y₂₂ is CR₂₆₂ₐ or N:
R₂₆₀ is independently, at each occurrence, selected from the group consisting of C₁-C₄ alkyl, C₂-C₄ alkenyl, C₂-C₄ alkynyl, halogen, C₁-C₂ haloalkyl, NR₂₆₅R₂₆₆, OR₂₆₇, and S(O)ₙₙR₂₆₇;
R₂₆₂ is C₁-C₄ alkyl, aryl, C₃-C₆ cycloalkyl, C₁-C₂ haloalkyl, halogen, nitro, NR₂₆₅R₂₆₆, OR₂₆₇, S(O)ₙₙR₂₆₇, C(=O)R₂₆₈, C (=O)NR₂₆₅R₂₆₆, C(=S)NR₂₆₅R₂₆₆, -(CHR₂₇₅)ₖₙNR₂₆₅R₂₆₆, (CH₂)ₖₙOR₂₆₇, C(=O)NR₂₆₉CH(R₂₇₀)CO₂R₂₇₁, -C(OH)(R₂₈₄)(R₂₈₄ₐ), - (CH₂)ₚₙS(O)ₙₙ-alkyl, -(CHR₂₇₅)R₂₈₄, -C(CN)(R₂₈₄)(R₂₇₅) when R₂₈₄ is not -NH- containing ring groups, -C(=O)R₂₈₄, -CH(CO₂R₂₇₅)₂, NR₂₆₉C(=O)CH(R₂₇₀)NR₂₆₉R₂₇₁, NR₂₆₉CH(R₂₇₀)CO₂R₂₇₁; substituted C₁-C₄ alkyl, substituted C₂-C₄ alkenyl, substituted C₂-C₄ alkynyl, substituted C₁-C₄ alkoxy, aryl-(substituted C₁-C₄) alkyl, aryl-(substituted C₁-C₄)alkoxy, substituted C₃-C₆ cycloalkyl, amino-(substituted C₁-C₄)alkyl, substituted C₁-C₄ alkylamino, wherein any substituent containing carbon can be substituted with R₂₈₆;
2-pyridinyl, imidazolyl, 3-pyridinyl, 4-pyridinyl, 2-methyl-3-pyridinyl, 4-methyl-3-pyridinyl, furanyl, 5-methyl-2-furanyl, 2,5-dimethyl-3-furanyl, 2-thienyl, 3-thienyl, 5-methyl-2-thienyl, 2-pheno-thiazinyl, 4-pyrazinyl, azetidinyl, phenyl, 1H-indazolyl, 2-pyrrolidonyl, 2H,6H-1,5,2-dithiazinyl, 2H-pyrrolyl, 3H-indolyl, 4-piperidonyl, 4aH-carbazolyl, 4H-quinolizinyl, 6H-1,2,5-thiadiazinyl, acridinyl, azocinyl, azepinyl, benzofuranyl, benzothiophenyl, carbazolyl, chromanyl, chromenyl, cinnolinyl, decahydroquinolinyl, furazanyl, imidazolidinyl, indolinyl, indolizinyl, indolyl, isobenzofuranyl, isochromanyl, isoindolinyl, isoindolyl, isoquinolinyl, benzimidazolyl, isothiazolyl, isoxazolyl, morpholinyl, naphthyridinyl, octahydroisoquinolinyl, oxazolidinyl, oxazolyl, phenanthridinyl, phenanthrolinyl, phenazinyl, phenoxathiinyl, phenoxazinyl, phthalazinyl, piperazinyl, piperidinyl, pteridinyl, purinyl, pyranyl, pyrazolidinyl, pyrazolinyl, pyrazolyl, pyridazinyl, pyrimidinyl, pyrrolidinyl, pyrrolinyl, pyrrolyl, quinazolinyl, quinolinyl, quinoxalinyl, quinuclidinyl, β-carbolinyl, tetrahydrofuranyl, tetrahydroisoquinolinyl, tetrahydroquinolinyl, tetrazolyl, thianthrenyl, thiazolyl, thiophenyl, triazinyl, xanthenyl; or 1-tetrahydroquinolinyl or 2-tetrahydroisoquinolinyl either of which can be substituted with 0 to 3 groups chosen from keto and C₁-C₄ alkyl;
J₂, K₄ and L are, at each occurrence, independently selected from the group consisting of N, CH, and CX₁₂₁;
M is CR₂₆₄ or N;
V is CR₂₆₀ₐ or N;
Z₂₂ is CR₂₆₁ or N;
R₂₆₀, R₂₆₁, and R₂₆₂ₐ are, at each occurrence, independently selected from the group consisting of hydrogen, halo, halomethyl, C₁-C₃ alkyl, and cyano;
R₂₆₃ is (CH₂)ₘₙOR₂₇₅, C₁-C₄ alkyl, allyl, propargyl, (CH₂)ₘₙR₂₇₂, or -(CH₂)ₘₙOC(O)R₂₇₅;
X₁₂ is halogen, aryl, heteroaryl, S(O)_{ZN}R₂₆₇, SR₂₆₇, halomethyl, - (CH₂)ₚₙOR₂₆₇, cyano, -(CHR₂₇₅)ₚₙNR₂₇₃R₂₇₄, -C(=O)R₂₆₇, C₁-C₆ alkyl, C₄-C₁₀ cycloalkylalkyl, C₁-C₁₀ alkenyl, C₂-C₁₀ alkynyl, C₁-C₁₀ alkoxy, aryl-(C₂-C₁₀)-alkyl, C₃-C₆ cycloalkyl, aryl-(C₁-C₁₀)-alkoxy, nitro, thio-(C₁-C₁₀)-alkyl, -C(=NOR₂₇₅)- C₁-C₄-alkyl, -C(=NOR₂₇₅)H, or -C(=O)NR₂₇₃R₂₇₄, wherein any carbon containing substituents can be substituted with R₂₇₇;
X₁₂₁ is, at each occurrence, independently selected from the group consisting of hydrogen, halogen, aryl, heteroaryl, S(O)ₙₙR₂₆₇, halomethyl, (CHR₂₇₅)ₚₙOR₂₆₇, cyano, -(CHR₂₇₅)ₚₙNR₂₇₃R₂₇₄, C(=O)R₂₆₇, C₁-C₆ alkyl, C₂-C₁₀ alkenyl, C₂-C₁₀ alkynyl, C₁-C₁₀ alkoxy, aryl-(C₁-C₁₀)-alkyl, C₃-C₆ cycloalkyl, aryl-(C₁-C₁₀)-alkoxy, nitro, thio-(C₁-C₁₀)-alkyl, -C(=NOR₂₇₅)-C₁-C₄-alkyl, -C(=NOR ₂₇₅)H, and -C(=O)NR₂₇₃R₂₇₄,
wherein any carbon containing substituents can be substituted by R₂₇₇;
R₂₆₄ is halo, -C(=NOR₂₇₅)-C₁-C₄-alkyl, C₁-C₆ alkyl, C₁-C₃ haloalkyl, -(CHR₂₇₅)ₚₙOR₂₆₇, -(CHR₂₇₅)ₚₙS(O)ₙₙR₂₆₇, -(CHR₂₇₅)ₚₙNR₂₇₃R₂₇₄, C₃-C₆ cycloalkyl, C₂-C₁₀ alkenyl, C₂-C₁₀ alkynyl, aryl-(C₂-C₁₀)-alkyl, aryl-(C₁-C₁₀)-alkoxy, cyano, C₃-C₆ cycloalkoxy, nitro, amino- (C₂-C₁₀)-alkyl, thio-(C₂-C₁₀)-alkyl, SOₙₙ(R₂₆₇), C(=O)R₂₆₇, -C(=NOR₂₇₅)H, or C(=O)NR₂₇₃R₂₇₄, wherein any carbon containing substituents can be substituted by R₂₇₇;
R₂₆₅ and R₂₆₆ are, at each occurrence, independently selected from the group consisting of hydrogen, C₁-C₆ alkyl, C₃-C₁₀ cycloalkyl, C₁-C₆ alkoxy, (C₄-C₁₂)-cycloalkylalkyl, - (CH₂)ₖₙR₂₇₂, (CHR₂₇₅)ₚₙOR₂₆₇, -(C₁-C₆ alkyl)-aryl, heteroaryl, -S(O)_{zn}-aryl or -(C₁-C₆ alkyl)-heteroaryl and aryl, wherein said aryl or heteroaryl groups are optionally substituted with 1-3 groups selected from the group consisting of hydrogen, halogen, C₁-C₆ alkyl, C₁-C₆ alkoxy, amino, NHC(=O)(C₁-C₆ alkyl), NH(C₁-C₆ alkyl), N(C₁-C₆ alkyl)₂, nitro, carboxy, CO₂(C₁-C₆ alkyl), cyano, and S(O)_{zn}-(C₁-C₆-alkyl); or can be taken together to form -(CH₂)_{qn}A₂₇(CH₂)ᵣₙ- which is optionally substituted with 0 to 3 R₂₇₆; or, when considered with the commonly attached nitrogen, can be taken together to form a heterocycle, said heterocycle being substituted on carbon with 1 to 3 groups consisting of hydrogen, C₁-C₆ alkyl, hydroxy, or C₁-C₆ alkoxy;
A₂₇ is CH₂, O, NR₂₈₄, C(=O),S(O)ₙₙ, N(C(=O)R₂₇₆), N(R₂₇₈),C(H)(NR₂₇₃R₂₇₄), C(H)(OR₂₇₉), C(H) (C(=O)R₂₈₀), or N(S(O)ₙₙR₂₈₀);
R₂₆₇ is, at each occurrence, independently selected from the group consisting of hydrogen; C₁-C₆ alkyl, -(C₄-C₁₂) cycloalkylalkyl, (CH₂)ₜₙR₂₈₁, C₃-C₁₀ cycloalkyl, -NR₂₆₅R₂₆₆, aryl, heteroaryl, -NR₂₇₅(CH₂)ₙₙNR₂₆₅R₂₆₆, -(CH₂)ₖₙR₂₈₄, and (CH₂)ₜₙheteroaryl or (CH₂)ₜₙaryl, either of which can optionally be substituted with 1 to 3 groups selected from the group consisting of hydrogen, halogen, C₁-C₆ alkyl, C₁-C₆ alkoxy, amino, NHC(=O)(C₁-C₆ alkyl), NH(C₁-C₆ alkyl), N(C₁-C₆ alkyl)₂, nitro, carboxy, CO₂(C₁-C₆ alkyl), cyano, or S(O)_{zn}(C₁-C₆ -alkyl;
R₂₆₈ is, at each occurrence, independently selected from R₂₆₉, hydroxy, C₁-C₄ alkoxy, C₃-C₆ cycloalkyl, C₂-C₄ alkenyl, aryl substituted with 0 to 3 R₂₇₇ and -(C₁-C₆ alkyl)-aryl substituted with 0 to 3 R₂₇₇;
R₂₆₉, R₂₇₅, R₂₈₂ and R₂₈₃ are, at each occurrence, independently selected from hydrogen or C₁-C₄ alkyl;
R₂₇₀ is C₁-C₄ alkyl substituted with 0 to 3 groups chosen from the group consisting of keto, amino, sulfhydryl, hydroxy, guanidinyl, p-hydroxyphenyl, imidazolyl, phenyl, indolyl, and indolinyl, or (CH₂)ₜₙ when taken together with an adjacent R₂₆₉;
R₂₇₁ is hydrogen or an appropriate amine protecting group for nitrogen or an appropriate carboxylic acid protecting group for carboxyl;
R₂₇₂ is, at each occurrence, independently selected from the group consisting of CN, OR₂₇₈, SR₂₇₈, and C₃-C₆ cycloalkyl;
R₂₇₃ and R₂₇₄ are, at each occurrence, independently selected from the group consisting of hydrogen, C₄-C₁₀ cycloalkyl-alkyl, and R₂₇₈;
R₂₇₆ is, at each occurrence, independently selected from the group consisting of R₂₆₉, C₁-C₄ alkoxy, halo, OR₂₈₂, SR₂₈₂, NR₂₈₂R₂₈₃, and (C₁-C₆) alkyl (C₁-C₄)alkoxy,
R₂₇₇ is, at each occurrence, independently selected from the group consisting of R₂₆₉, hydroxy, halogen, C₁-C₂ haloalkyl, C₁-C₄ alkoxy, C(=O)R₂₈₃, and cyano;
R₂₇₈ is, at each occurrence, independently selected from the group consisting of C₁-C₆ alkyl, C₃-C₆ cycloalkyl, (CH₂)_{wn}R₂₈₁, and aryl substituted with 0 to 3 R₂₇₇;
R₂₇₉ is, at each occurrence, independently selected from the group consisting of R₂₆₉, C(=O)R₂₉₀, and C₂-C₄ alkenyl;
R₂₈₀ is, at each occurrence, independently selected from the group consisting of R₂₆₉, C₁-C₄ alkoxy, NR₂₈₂R₂₈₃, and hydroxyl;
R₂₈₁ is, at each occurrence, independently selected from the group consisting of cyano, OR₂₈₃, SR₂₈₃, SR₂₈₂R₂₈₃, C₁-C₆ alkyl, C₃-C₆ cycloalkyl, -S(O)ₙₙR₂₉₀, and -C(=O)R₂₈₄;
R₂₈₄, which can be optionally substituted with 0 to 3 R₂₇₆, is independently selected, at each occurrence, from the group consisting of phenyl, pyrazolyl, imidazolyl, 2-methyl-3-pyridinyl, 4-methyl-3-pyridinyl, furanyl, 5-methyl-2-furanyl, 2,5-dimethyl-3-furanyl, 2-thienyl, 3-thienyl, 5-methyl-2-thienyl, 2-pheno-thiazinyl, 4-pyrazinyl, azetidinyl, 1H-indazolyl, 2-pyrrolidonyl, 2H,6H-1,5,2-dithiazinyl, 2H-pyrrolyl, 3H-indolyl, 4-piperidonyl, 4aH-carbazolyl, 4H-quinolizinyl, 6H-1,2,5-thiadiazinyl, acridinyl, azocinyl, azepinyl, benzofuranyl, benzothiophenyl, carbazolyl, chromanyl, chromenyl, cinnolinyl, decahydroquinolinyl, furazanyl, indolinyl, indolizinyl, indolyl, isobenzofuranyl, isochromanyl, isoindolinyl, isoindolyl, isoquinolinyl, benzimidazolyl, isothiazolyl, isoxazolyl, morpholinyl, naphthyridinyl, octahydroisoquinolinyl, oxazolidinyl, oxazolyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazolidinyl, pyridazinyl, pyridyl, pyrimidinyl, pyrrolidinyl, pyrrolinyl, pyrrolyl, quinazolinyl, quinolinyl, quinoxalinyl, quinuclidinyl, β-carbolinyl, tetrahydrofuranyl, tetrazolyl, thianthrenyl, thiazolyl, thiophenyl, triazinyl, xanthenyl; and 2 -tetrahydroquinolinyl or 2-tetrahydroisoquinolinyl either of which can be substituted with 0 to 3 groups chosen from keto and C₁-C₄ alkyl;
R₂₈₄ₐ, which can be optionally substituted with 0 to 3 R₂₇₆, is independently selected, at each occurrence, from the group consisting of H and R₂₈₄,
R₂₈₆ is, at each occurrence, independently selected from the group consisting of C₁-C₃ alkyl, C₂-C₄ alkenyl, C₂-C₄ alkynyl, C₂-C₄ alkoxy, aryl, nitro, cyano, halogen, aryloxy, and heterocycle optionally linked through oxygen;
R₂₉₀ is, at each occurrence, independently selected from the group consisting of C₁-C₄ alkyl, C₃-C₇ cycloalkyl, C₄-C₁₀ cycloalkylalkyl, and aryl-(C₁-C₄)alkyl;
kn, mn, and rn are, at each occurrence, independently selected from 1 to 4;
nn is, at each occurrence, independently selected from 0 to 2;
pn, qn, and zn are, at each occurrence, independently selected from 0 to 3;
tn and wn are, at each occurrence, independently selected from 1 to 6, with the proviso that when J₂ is CX₁₂₁ and K₄ and L are both CH, and M is CR₂₆₄, then
(A) when V and Y₂₂ are N, Z₂₂ is CH and R₂₆₀ and R₂₆₂ are methyl,
(1) and when R₂₆₃ is methyl, then
(a) R₂₆₄ can not be methyl when X₁₂ is OH and X₁₂₁ is H;
(b) R₂₆₄ can not be -NHCH₃-, or -N(CH₃)₂ when X₁₂ and X₁₂₁ are -OCH₃; and
(c) R₂₆₄ can not be -N(CH₃)₂, when X₁₂ and X₁₂₁ are -OCH₂CH₃;
(2) and when R₂₆₃ is ethyl, then
(a) then R₂₆₄ can not be methylamine when X₁₂ and X₁₂₁ are -OCH₃;
(b) R₂₆₄ can not be OH when X₁₂ is Br and X₁₂₁ is OH; and
(c) R₂₆₄ can not be -CH₂OH or -CH₂N(CH₃)₂, when X₁₂ is -SCH₃ and X₁₂₁ is H;
(B) when V and Y₂₂ are N, Z₂₂ is CH, R₂₆₃ is ethyl, R₂₆₄ is isopropyl, X₁₂ is Br, X₁₂₁ is H, and
(1) when R₂₆₀ is CH₃, then
(a) R₂₆₂ can not be OH, piperazin-1-yl, -CH₂-piperidin-1-yl, -CH₂-(N-4-methylpiperazin-1-yl), -C(O)NH-phenyl, -CO₂H, -CH₂O-(4-pyridyl), -C(O)NH₂, 2-indolyl, -CH₂O-(4-carboxyphenyl), -N(CH₂CH₃)(2-bromo-4-isopropylphenyl);
(2) when R₂₆₀ is -CH₂CH₂CH₃, then R₂₆₂ can not be -CH₂CH₂CH₃;
(C) when V, Y₂₂ and Z₂₂ are N, R₂₆₃ is ethyl, and
(1) R₂₆₄ is isopropyl, X₁₂ is bromo, and X₁₂₁ is H, and
(a) R₂₆₂ can not be OH or -OCH₂CN when R₂₆₀ is CH₃; and
(b) R₂₆₂ can not be -N((H₃)₂ when R₂₆₀ is -N(CH₃)₂;
(2) R₂₆₄ is -OCH₃, X₁₂ is -OCH₃, and X₁₂₁ is H, then R₂₆₂ and R₂₆₀ can not both be chloro;
further provided that when J₂, K₄, and L are all CH and M is CR₂₆₄, then
(D) at least one of V, Y₂₂, and Z₂₂ must be N;
(E) when V is CR₂₆₀ₐ, Z₂₂ and Y₂₂ can not both be N;
(F) when Y₂₂ is CR₂₆₂ₐ, Z₂₂ and V can not both be N;
(G) when Z₂₂ is CR₂₆₁, V and Y₂₂ must both be N;
(H) Z₂₂ can be N only when both V and Y₂₂ are N or when V is CR₂₆₀ₐ and Y₂₂ is CR₂₆₂ₐ,
(I) when V and Y₂₂ are N, Z₂₂ is CR₂₆₁, and R₂₆₁ is H or C₁-C₃ alkyl, and R₂₆₃ is C₁-C₃ alkyl, then R₂₆₂ can not be 2-pyridinyl, indolyl, indolinyl, imidazolyl, 3-pyridinyl, 4-pyridinyl, 2-methyl-3-pyridinyl, 4-methyl-3-pyridinyl, furanyl, 5-methyl-2-furanyl, 2,5-dimethyl-3-furanyl, 2-thienyl, 3-thienyl, 5-methl-2-thienyl, 2-phenothiazinyl, or 4-pyrazinyl;
(J) when V and Y₂₂ are N, Z₂₂ is CR₂₆₁, R₂₆₁ is H or C₁-C₃ alkyl, R₂₆₃ is C₁-C₄ alkyl, R₂₆₄, X₁₂ and/or X₁₂₁ are OH, halo, CF₃, C₁-C₄ alkyl, C₁-C₄ alkoxy, C₁-C₄ alkylthio, cyano, amino, carbarmoyl, or C₁-C₄ alkanoyl, and when R₂₆₀ is C₁-C₄ alkyl, then R₂₆₂ can not be -NH(substituted phenyl) or -N(C₁-C₄ alkyl)(substituted phenyl);
and in the formula Iaa, when Y₂₂ is CR₂₈₈:
J₂, K₄, L, M, Z₂₂, A₂₇, kn, mn, nn, pn, qn, rn, tn, wn, R₂₆₂, R₂₆₉, R₂₇₀, R₂₇₁, R₂₇₂, R₂₇₅, R₂₇₇, R₂₇₈, R₂₈₀, R₂₈₂, R₂₈₃, R₂₈₄, and R₂₈₆ are as defined above, then R₂₈₄ₐ, in addition to being as defined above, can also be C₁-C₄ alkyl, with the proviso that
V is N;
R₂₆₀ is C₁-C₂ alkyl, C₂-C₄ alkenyl, C₂-C₄ alkynyl, C₂-C₄ alkoxy, halogen, amino, methylamino, dimethylamino, aminomethyl, or N-methylaminomethyl;
R₂₆₁ is, at each occurrence, independently selected from the group consisting of hydrogen, halo, C₁-C₃ alkyl, nitro, amino, and -CO₂R₂₆₉;
R₂₆₃ is taken together with R₂₈₈ to form a 5-membered ring and is -C(R₂₈₇) = or -N= when R₂₈₈ is -C(R₂₈₉) or -N=, or -CH(R₂₈₇) when R₂₈₈ is -CH(R₂₈₉);
X₁₂ is Cl, Br, I, S(O)ₙₙR₂₆₇, OR₂₆₇, halomethyl, - (CHR₂₇₅)ₚₙOR₂₆₇, cyano, - (CHR₂₇₅)ₚₙNR₂₇₃R₂₇₄, C(=O)R₂₆₇, C₁-C₆ alkyl, C₂-C₁₀ alkenyl, C₂-C₁₀ alkynyl, C₁-C₁₀ alkoxy, aryl-(C₁-C₁₀)-alkyl, C₃-C₆ cycloalkyl, aryl-(C₁-C₁₀)-alkoxy, nitro, thio-(C₁-C₁₀)-alkyl, -C(=NOR₂₇₅)- C₁-C₄ -alkyl, -(=NOR₂₇₅)H, or C(=O)NR₂₇₃R₂₇₄, where substitution by R₂₇₇ can occur on any carbon containing substituents;
X₁₂₁ is hydrogen, Cl, Br, I, S(O)ₙₙR₂₆₇, -(CHR₂₇₅)ₚₙOR₂₆₇, halomethyl, cyano, - (CHR₂₇₅)ₚₙNR₂₇₃R₂₇₄, C(=O)R₂₆₇, C₁-C₆ alkyl, C₂-C₁₀ alkenyl, C₂-C₁₀ alkynyl, C₁-C₁₀ alkoxy, aryl-(C₁-C₁₀)-alkyl, C₃-C₆ cycloalkyl, aryl-(C₂-C₁₀)-alkoxy, nitro, thio-(C₂-C₁₀)-alkyl, -C(=NOR ₂₇₅)-C₁-C₄-alkyl, -C(=NOR₂₇₅)H, or C(=O)NR₂₆₇R₂₇₄, where substitution by R₂₇₇ can occur on any carbon containing substituents;
R₂₆₄ is halo, -C(=NOR₂₇₅)-C₁-C₄ alkyl, C₁-C₆ alkyl, C₁-C₃ haloalkyl, C₁-C₆ alkoxy, (CHR₂₇₅)ₚₙOR₂₆₇, (CHR₂₇₅)ₚₙS(O)ₙₙR₂₆₇, (CHR₂₇₅)ₚₙNR₂₇₃R₂₇₄, C₃-C₆ cycloalkyl, C₂-C₁₀ alkenyl, C₂-C₁₀ alkynyl, aryl-(C₂-C₁₀)-alkyl, aryl-(C₁-C₁₀)-alkoxy, cyano, C₃-C₆ cycloalkoxy, nitro, amino-(C₁-C₁₀)-alkyl, thio-(C₁-C₁₀)-alkyl, SOₙₙ(R₂₆₇), C(=O)R₂₆₇, -C(=NOR₂₇₅)H, or C(=O)NR₂₆₇R₂₇₄, wherein substitution by R₂₇₇ can occur on any carbon containing substituents;
R₂₆₅ and R₂₆₆ are, at each occurrence, independently selected from the group consisting of hydrogen, C₁-C₆ alkyl, C₃-C₁₀ cycloalkyl, -(CH₂)ₖₙR₂₇₂, (C₄-C₁₂)-cycloalkylalkyl, C₁-C₆ alkoxy, -(C₁-C₆ alkyl)-aryl, heteroaryl, aryl, -S(O)_{zn}-aryl or -(C₁-C₆ alkyl)-heteroaryl and aryl, wherein the aryl or heteroaryl groups are optionally substituted with 1 to 3 substituents selected from hydrogen, halogen, C₁-C₆ alkyl, C₁-C₆ alkoxy, amino, NHC(=O)(C₁-C₆ alkyl), NH(C₁-C₆ alkyl), N(C₁-C₆ alkyl)₂, nitro, carboxy, CO₂(C₁-C₆ alkyl), and cyano; or can be taken together to form -(CH₂)_{qn}A₂₇(CH₂)ᵣₙ-, optionally substituted with 0 to 3 R₂₇₆; or, when considered with the commonly attached nitrogen, can be taken together to form a heterocycle, said heterocycle being substituted on carbon with 1 to 3 groups consisting of hydrogen, C₁-C₆ alkyl, hydroxy, or C₁-C₆ alkoxy;
R₂₆₇ is, at each occurrence, independently selected from the group consisting of hydrogen, C₁-C₆ alkyl, -(C₄-C₁₂) cycloalkylalkyl, (CH₂)ₜₙR₂₈₁, C₃-C₁₀ cycloalkyl, -(C₁-C₆ alkyl)-aryl, heteroaryl, -NR₂₇₅, -N(CH₂)ₙₙNR₂₆₅R₂₆₆, -(CH₂)ₖₙR₂₈₄, (C₁-C₆ alkyl)-heteroaryl or aryl optionally substituted with 1-3 groups selected from hydrogen, halogen, C₁-C₆ alkyl, C₁-C₆ alkoxy, amino, NHC(=O)(C₁-C₆ alkyl), NH(C₁-C₆ alkyl), N(C₁-C₆ alkyl)₂, nitro, carboxy, CO₂(C₁-C₆ alkyl), and cyano;
R₂₆₈ is, at each occurrence, independently selected from R₂₆₉, hydroxy, C₁-C₄ alkoxy, C₃-C₆ cycloalkyl, C₂-C₄ alkenyl, and aryl substituted with 0 to 3 R₂₇₇;
R₂₇₃ and R₂₇₄ are, at each occurrence, independently selected from the group consisting of hydrogen, C₁-C₆ alkyl, C₃-C₆ cycloalkyl, (CH₂)ₜₙR₂₈₁, and aryl substituted with 0 to 3 R₂₇₇;
R₂₇₆ is, at each occurrence, independently selected at each occurrence from the group consisting of R₂₆₉, C₁-C₄ alkoxy, halo, OR₂₆₂, SR ₂₈₂, and NR₂₈₂R₂₈₃;
R₂₇₉ is, at each occurrence, independently selected from the group consisting of R₂₆₉ and C(=O)R₂₉₀;
R₂₈₁ is, at each occurrence, independently selected from the group consisting of OR₂₈₃, SR₂₈₃, NR₂₈₂R₂₈₃, C₃-C₆ cycloalkyl, -S(O)ₙₙR₂₉₀, and -C(=O)R₂₈₄;
R₂₈₅ is hydrogen or halogen;
R₂₈₇ is C₁-C₂ alkyl, C₂-C₄ alkenyl, C₂-C₄ alkynyl, hydrogen, C₁-C₂ alkoxy, halogen, or C₂-C₄ alkylamino;
R₂₈₈ is taken together with R₂₆₃ to form a five membered ring and is:
- CH(R₂₈₉)- when R₂₆₃ is -CH(R₂₈₇)-,
- C(R₂₈₉)- when R₂₆₃ is -C(R₂₈₇)= or -N=;
R₂₆₉ is hydrogen, cyano, C₁-C₂ alkyl, C₁-C₂ alkoxy, halogen, C₁-C₂ alkenyl, nitro, amido, carboxy, or amino;
R₂₉₀ is C₁-C₄ alkyl, C₃-C₇ cycloalkyl, or aryl-(C₁-C₄) alkyl, with the proviso that when J₂, K₄, and L are all CH, M is CR₂₆₄, Z₂₂ is CH, R₂₆₂ is CH₃,
R₂₈₇ is H, R₂₆₄ is isopropyl, X₁₂ is Br, X₁₂₁ is H, and R₂₆₀ is CH₃ then R₂₈₉ can not be H, -CO₂H, or -CH₂NH₂;
and further provided that when J₂, K₄ and L are all CH; M is CR₂₆₄; Z₂₂ is N; and
(A) R₂₈₈ is -C(R₂₈₉), then one of R₂₈₇ or R₂₈₉ is hydrogen;
(B) R₂₈₈ is N, then R₂₆₂ is not halo, NH₂, NO₂, CF₃, CO₂H, CO₂-alkyl, alkyl, acyl, alkoxy, OH, or -(CH₂)ₘₙO alkyl;
(C) R₂₈₈ is N, X₁₂ or X₁₂₁ are bromo or methyl, and R₂₆₄ is nitro, then R₂₈₇ is not methyl; or
(D) R₂₈₈ is N, R₂₆₀ is CH₃ and R₂₆₂ is amino, then R₂₆₄ is not halogen or methyl,
its pharmaceutically acceptable salt or pro-drugs.

34. A pharmaceutical composition for prophylaxis or therapy of a postoperative stress as claimed in claim 1, wherein the compound having CRF antagonist activating effect is a compound represented by the formula Iab or Iac 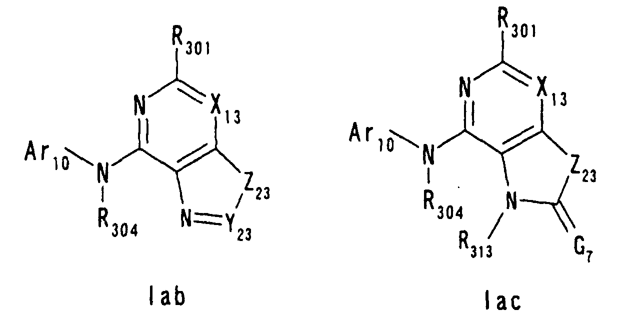 wherein
X₁₃ is N or CR₃₀₁;
Y₂₃ is N or CR₃₀₂;
Z₂₃ is NR₃₀₃, O, or S(O)n₄;
G₇ is O or S;
Ar₁₀ is phenyl, naphthyl, pyridyl, pyrimidinyl, triazinyl, furanyl, quinolinyl, isoquinolinyl, thienyl, imidazolyl, thiazolyl, indolyl, pyrrolyl, oxazolyl, benzofuranyl, benzothienyl, benzthiazolyl, isoxazolyl or pyrazolyl, each optionally substituted with 1 to 5 R₃₀₅ groups;
R₃₀₁ is, at each occurrence, independently H, C₁-C₄ alkyl, C₂-C₄ alkenyl, C₂-C₄ alkynyl, halo, CN, C₁-C₄ haloalkyl, -NR₃₀₉R₃₁₀, NR₃₀₉COR₃₁₀, -OR₃₁₁, SH or -S(O)n₄R₃₁₂;
R₃₀₂ is H, C₁-C₄ alkyl, C₁-C₆ cycloalkyl, halo, CN, -NR₃₀₆R₃₀₇, NR₃₀₉COR₃₁₀, C₁-C₄ haloalkyl, -OR₃₀₇, SH or -S(O) ₙ₄R₃₁₂;
R₃₀₃ is H, C₁-C₁₀ alkyl, C₂-C₁₀ alkenyl, C₂-C₁₀ alkynyl, C₃-C₈ cycloalkyl or C₄-C₁₂ cycloalkylalkyl, each optionally substituted with 1 to 3 substituents independently selected at each occurrence from C₁-C₆ alkyl, C₃-C₆ cycloalkyl, halo, C₁-C₄ haloalkyl, cyano, -OR₃₀₇, SH, -S(O)ₙ₄R₃₁₃, -COR₃₀₇, -CO₂R₃₀₇, -OC(O)R₃₁₃, -NR₃₀₈COR₃₀₇, -N(COR₃₀₇)₂, -NR₃₀₈CONR₃₀₆R₃₀₇, -NR₃₀₈CO₂R₃₁₃, -NR₃₀₆R₃₀₇, -CONR₃₀₆R₃₀₇, aryl², heteroaryl² and heterocyclyl², where said aryl², heteroaryl² or heterocyclyl² is optionally substituted with 1 to 3 substituents independently selected at each occurrence from a group consisting of C₁-C₆ alkyl, C₃-C₆ cycloalkyl, halo, C₁-C₄ haloalkyl, cyano, -OR₃₀₇, SH, -S(O)ₙ₄R₃₁₃, -COR₃₀₇, -CO₂R₃₀₇, -OC(O)R₃₁₃, -NR₃₀₈COR₃₀₇, -N(COR₃₀₇)₂, -NR₃₀₈CONR₃₀₆R₃₀₇, -NR₃₀₈CO₂R₃₁₃, -NR₃₀₆R₃₀₇ and -CONR₃₀₆R₃₀₇;
R₃₀₄ is H, C₁-C₄ alkyl, allyl, or propargyl, wherein C₁-C₄ alkyl, allyl, or propargyl is optionally substituted with C₃-C₆ cycloalkyl and, wherein C₁-C₄ alkyl is optionally substituted with -OR₃₀₇, -S(O)ₙ₄R₃₁₂ or -CO₂R₃₀₇;
R₃₀₅ is, at each occurrence, independently C₁-C₁₀ alkyl, C₂-C₁₀ alkenyl, C₂-C₁₀ alkynyl, C₃-C₈ cycloalkyl, C₄-C₁₂ cycloalkylalkyl, -NO₂, halo, -CN, C₁-C₄ haloalkyl, -NR₃₀₆R₃₀₇, -NR₃₀₈COR₃₀₇, -NR₃₀₈CO₂R₃₀₇, -COR₃₀₇, -OR₃₀₇, -CONR₃₀₆R₃₀₇, -CO(NOR₃₀₉)R₃₀₇, CO₂R₃₀₇, or -S(O)ₙ₄R₃₀₇, where C₁-C₁₀ alkyl, C₂-C₁₀ alkenyl, C₂-C₁₀ alkynyl, C₃-C₆ cycloalkyl and C₄-C₁₂ cycloalkylalkyl are optionally substituted with 1 to 3 substituents optionally and independently selected from C₁-C₄ alkyl, nitro, halo, -CN, -NR₃₀₆R₃₀₇, NR₃₀₈COR₃₀₇, NR₃₀₈CO₂R₃₀₇, -COR₃₀₇, -OR₃₀₇, -CONR₃₀₆R₃₀₇, CO₂R₃₀₇, -CO(NOR₃₀₉)R₃₀₇, or -S(O)ₙ₄R₃₀₇;
R₃₀₆ and R₃₀₇ are, at each occurrence, independently H, C₁-C₄ alkyl, C₁-C₄ haloalkyl, C₂-C₈ alkoxyalkyl, C₃-C₆ cycloalkyl, C₄-C₁₂ cycloalkylalkyl, aryl², aryl² (C₁-C₄ alkyl)-, heteroaryl² or heteroaryl²(C₁-C₄ alkyl)-; or NR₃₀₆R₃₀₇ is piperidine, pyrrolidine, piperazine, N-methylpiperazine, morpholine or thiomorpholine;
R₃₀₈ is, at each occurrence, independently H or C₁-C₄ alkyl;
R₃₀₉ and R₃₁₀ are, at each occurrence, independently selected from H, C₁-C₄ alkyl, or C₃-C₆ cycloalkyl;
R₃₁₁ is H, C₁-C₄ alkyl, C₁-C₄ haloalkyl, or C₃-C₆ cycloalkyl;
R₃₁₂ is C₁-C₄ alkyl or C₁-C₄ haloalkyl;
R₃₁₃ is C₁-C₄ alkyl, C₁-C₄ haloalkyl, C₂-C₈ alkoxyalkyl, C₃-C₆ cycloalkyl, C₄-C₁₂ cycloalkylalkyl, aryl², aryl²(C₁-C₄ alkyl)-, heteroaryl² or heteroaryl²(C₁-C₄ alkyl)-;
Aryl² is phenyl or naphthyl, each optionally substituted with 1 to 3 substituents optionally and independently selected from C₁-C₆ alkyl, C₃-C₆ cycloalkyl, halo, C₁-C₄ haloalkyl, cyano, -OR₃₀₇, SH, -S(O)ₙ₄R₃₁₃, -COR₃₀₇, -CO₂R₃₀₇, -OC(O)R₃₁₃, -NR₃₀₈COR₃₀₇, -N(COR₃₀₇)₂, -NR₃₀₈CONR₃₀₆R₃₀₇, -NR₃₀₈CO₂R₃₁₃, -NR₃₀₆R₃₀₇, and -CONR₃₀₆R₃₀₇;
heteroaryl² is pyridyl, pyrimidinyl, triazinyl, furanyl, quinolinyl, isoquinolinyl, thienyl, imidazolyl, thiazolyl, indolyl, pyrrolyl, oxazolyl, benzofuranyl, benzothienyl, benzthiazolyl, isoxazolyl, pyrazolyl, triazolyl, tetrazolyl, or indazolyl, each optionally substituted with 1 to 3 substituents optionally and independently selected from C₁-C₆ alkyl, C₃-C₆ cycloalkyl, halo, C₁-C₄ haloalkyl, cyano, -OR₃₀₇, SH, -S(O)ₙ₄R₃₁₃, -COR₃₀₇, -CO₂R₃₀₇, -OC(O)R₃₁₃, -NR₃₀₈COR₃₀₇, -N(COR₃₀₇)₂, -NR₃₀₈CONR₃₀₆R₃₀₇, -NR₃₀₈CO₂R₃₁₃, -NR₃₀₆R₃₀₇, and -CONR₃₀₆R₃₀₇;
heterocyclyl² is saturated or partially saturated heteroaryl, optionally substituted with 1 to 3 substituents optionally and independently selected from C₁-C₆ alkyl, C₃-C₆ cycloalkyl, halo, C₁-C₄ haloalkyl, cyano, -OR₃₀₇, SH, -S(O)ₙ₄R₃₁₃, -COR₃₀₇, -CO₂R₃₀₇, -OC(O)R₃₁₃, -NR₃₀₈COR₃₀₇, -N(COR₃₀₇)₂, -NR₃₀₈CONR₃₀₆R₃₀₇, -NR₃₀₈CO₂R₃₁₃, -NR₃₀₆R₃₀₇, and -CONR₃₀₆R₃₀₇;
n4 is optionally and independently 0, 1 or 2;
provided that R₃₀₄ in formula Iab is not H;
(a) when X₁₃ is N, Y₂₃ is N, and Z₂₃ is NR₃₀₃, then R₃₀₁ is H, R₃₀₃ is H or benzyl, and Ar₁₀ is p-methylphenyl;
(b) when X₁₃ is N, Y₂₃ is N, and Z₂₃ is NR₃₀₃, then R₃₀₁ is butyl, R₃₀₃ is benzyl, and Ar₁₀ is phenyl;
(c) when X₁₃ is N, Y₂₃ is CH, and Z₂₃ is NR₃₀₃, then R₃₀₃ is methyl, R₃₀₁ is H, and Ar₁₀ is phenyl or 2-fluorophenyl;
(d) when X₁₃ is N, Y₂₃ is CH, and Z₂₃ is NR₃₀₃, then R₃₀₃ is methyl, R₃₀₁ is Cl and Ar₁₀ is phenyl;
(e) when X₁₃ is N, Y₂₃ is CH, and Z₂₃ is NR₃₀₃, then R₃₀₁ is Cl, R₃₀₃ is benzyl, and Ar₁₀ is phenyl or substituted phenyl;
(f) when X₁₃ is N, Y₂₃ is CH, and Z₂₃ is NR₃₀₃, then R₃₀₃ is p-methylbenzyl, and Ar₁₀ is phenyl;
(g) when X₁₃ is N, Y₂₃ is CR₃₀₂, and Z₂₃ is NR₃₀₃, then R₃₀₂ is CH₃, R₃₀₃ is H, and Ar₁₀ is phenyl or phenyl substituted with methyl, ethyl, isopropyl, fluoro or chloro;
(h) when X₁₃ is N, Y₂₃ is N, and Z₂₃ is NR₃₀₃, then R₃₀₃ is cyclopropylmethyl, R₃₀₁ is H, and Ar₁₀ is 2-bromo-4-isopropylphenyl, or
(i) when X₁₃ is N, Y₂₃ is N, and Z₂₃ is S, then R₃₀₁ is H, and Ar₁₀ is 2-bromo-4-isopropylphenyl, its pharmaceutically acceptable salt or pro-drugs.

35. A pharmaceutical composition for prophylaxis or therapy of a postoperative stress as claimed in claim 1, wherein the compound having CRF antagonist activating effect is a compound represented by the formula Iad 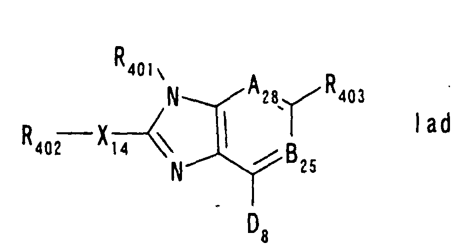 wherein
A₂₈ is N or C-R₄₀₇;
B₂₅ is N or C-R₄₀₈;
with the proviso that at least one of the groups A₂₈ and B₂₅ is N;
D₈ is an aryl³ or heteroaryl³ group attached to an unsaturated carbon atom;
X₁₄ is selected from the group consisting of CH-R₄₀₉, N-R₄₁₀, O, S(O)n₅ and a bond;
N₅ is 0, 1 or 2;
R₄₀₁ is selected from the group consisting of C₁-C₁₀ alkyl, C₂-C₁₀ alkenyl, C₂-C₁₀ alkynyl, C₃-C₈ cycloalkyl, C₃-C₆ cycloalkyl- C₁-C₆ alkyl, C₁-C₄ alkoxy- C₁-C₄ alkyl, -SO₂- C₁-C₁₀ alkyl, -SO₂-R₄₀₁ₐ, and -SO₂-R_{401b};
R₄₀₁ is substituted with 0 to 1 substituent selected from the group consisting of -CN, -S(O)n₅R_{414b}, -COR₄₁₃ₐ, -CO₂R₄₁₃ₐ, -NR₄₁₅ₐCOR₄₁₃ₐ, -N(COR₄₁₃ₐ)₂, -NR₄₁₅ₐCONR₄₁₃ₐR₄₁₆ₐ, -NR₄₁₅ₐCO₂R_{414b}, -CONR₄₁₃ₐR₄₁₆ₐ, 1-morpholinyl, 1-piperidinyl, 1-piperazinyl, and C₃-C₈ cycloalkyl, wherein 0 to 1 carbon atom in said C₄-C₈ cycloalkyl is replaced by 0 to 1 group selected from the group consisting of -O, -S(O)n₅, -NR₄₁₃ₐ, -NCO₂R_{414b}, -NCOR_{414b} and -NSO₂R_{414b}, and wherein N₄ in said 1-piperazinyl is substituted with 0 to 1 substituent selected from the group consisting of R₄₁₃ₐ, CO₂R_{414b}, COR_{414b} and SO₂R_{414b};
Also R₄₀₁ is optionally substituted with 0 to 3 substituents independently selected from the group consisting of R₄₀₁ₐ, R_{401b}, R_{401c}, C₁-C₆ alkyl, C₂-C₈ alkenyl, C₂-C₈ alkynyl, Br, Cl, F, I, C₁-C₄ haloalkyl, -OR₄₁₃ₐ, -NR₄₁₃ₐR₄₁₆ₐ, C₁-C₄ alkoxy- C₁-C₄ alkyl, and C₃-C₈ cycloalkyl which is substituted with 0 to 1 R₄₀₉ and in which 0 to 1 carbons of C₄-C₈ cycloalkyl is replaced by -O-;
with the proviso that R₄₀₁ is other than
(a) cyclohexyl-(CH₂)₂- group;
(b) 3-cyclopropyl-3-methoxypropyl group;
(c) unsubstituted-(alkoxy)methyl group; and,
(d) 1-hydroxyalkyl group;
also with the proviso that when R₄₀₁ is alkyl substituted with OH, then the carbon adjacent to N in the ring is other than CH₂;
R₄₀₁ₐ is aryl and is selected from the group consisting of phenyl, naphthyl, indanyl and indenyl, each R₄₀₁ₐ being substituted with 0 to 1 -OR₄₁₇ and 0 to 5 substituents optionally and independently selected from the group consisting of C₁-C₆ alkyl, C₃-C₆ cycloalkyl, Br, Cl, F, I, C₁-C₄ haloalkyl, -CN, nitro, SH, -S(O)n₅R₄₁₈, -COR₄₁₇, -OC(O)R₄₁₈, -NR₄₁₅ₐCOR₄₁₇, -N(COR₄₁₇)₂, -NR₄₁₅ₐCONR₄₁₇ₐR₄₁₉ₐ, -NR₄₁₅ₐCO₂R₄₁₈, -NR₄₁₇ₐR₄₁₉ₐ, and -CONR₄₁₇ₐR₄₁₉ₐ;
R_{401b} is heteroaryl and is selected from the group consisting of pyridyl, pyrimidinyl, triazinyl, furanyl, quinolinyl, isoquinolinyl, thienyl, imidazolyl, thiazolyl, indolyl, pyrrolyl, oxazolyl, benzofuranyl, benzothienyl, benzothiazolyl, benzoxazolyl, isoxazolyl, pyrazolyl, triazolyl, tetrazolyl, indazolyl, 2,3-dihydrobenzofuranyl, 2,3-dihydrobenzothienyl, 2,3-dihydrobenzothienyl-S-oxide, 2,3-dihydrobenzothienyl-S-dioxide, indolinyl, benzoxazolin-2-onyl, benzodioxolanyl and benzodioxane, each heteroaryl being substituted on 0 to 4 carbon atoms with a substituent optionally and independently selected from the group consisting of C₁-C₆ alkyl, C₃-C₆ cycloalkyl, Br, Cl, F, I, C₁-C₄ haloalkyl, -CN, nitro, -OR₄₁₇, SH, -S(O)ₘ₃R₄₁₈, -COR₄₁₇, -OC(O)R₄₁₈, -NR₄₁₅ₐCOR₄₁₇, -N(COR₄₁₇)₂, -NR₄₁₅ₐCONR₄₁₇ₐR₄₁₉ₐ, -NR₄₁₅ₐCO₂R₄₁₈, -NR₄₁₇ₐR₄₁₉ₐ, and -CONR₄₁₇ₐR₄₁₉ₐ and each heteroaryl being substituted on any nitrogen atom with 0 to 1 substituent selected from the group consisting of R₄₁₅ₐ, CO₂R_{414b}, COR_{414b}, and SO₂R_{414b};
R_{401c} is heterocyclyl and is a saturated or partially saturated heteroaryl³, each heterocyclyl being substituted on 0 to 4 carbon atoms with a substituent optionally and independently selected from the group consisting of C₁-C₆ alkyl, C₃-C₆ cycloalkyl, Br, Cl, F, I, C₁-C₄ haloalkyl, -CN, nitro, -OR₄₁₃ₐ, SH, -S(O)n₅R_{414b}, -COR₄₁₃ₐ, -OC(O)R_{414b}, -NR₄₁₅ₐCOR₄₁₃ₐ, -N(COR₄₁₃ₐ)₂, -NR₄₁₅ₐCONR₄₁₃ₐR₄₁₆ₐ, -NR₄₁₅ₐCO₂R_{414b}, -NR₄₁₃ₐR₄₁₆ₐ, and -CONR₄₁₃ₐR₄₁₆ₐ and each heterocyclyl being substituted on any nitrogen atom with 0 to 1 substituent selected from the group consisting of R₄₁₃ₐ,CO₂R_{414b}, COR_{414b} and SO₂R_{414b} and wherein any sulfur atom is optionally monooxidized or dioxidized;
with the proviso that R₄₀₁ is other than a -(CH₂)₁₋₄-aryl³, -(CH₂)₁₋₄-heteroaryl³, or -(CH₂)₁₋₄ heterocyclyl, wherein the aryl³, heteroaryl³, or heterocyclyl group is substituted or unsubstituted;
R₄₀₂ is selected from the group consisting of C₁-C₄ alkyl, C₃-C₈ cycloalkyl, C₂-C₄ alkenyl, and C₂-C₄ alkynyl and is substituted with 0 to 3 substituents selected from the group -CN, hydroxy, halo and C₁-C₄ alkoxy;
alternatively, when X₁₄ is a bond, then R₄₀₂ is selected from the group consisting of -CN, CF₃ and C₂F₅;
R₄₀₃, R₄₀₇ and R₄₀₈ are, at each occurrence, independently selected from the group H, Br, Cl, F, I, -CN, C₁-C₄ alkyl, C₃-C₈ cycloalkyl, C₁-C₄ alkoxy, C₁-C₄ alkylthio, C₁-C₄ alkylsulfinyl, C₁-C₄ alkylsulfonyl, amino, C₁-C₄ alkylamino, (C₁-C₄ alkyl)₂ amino and phenyl, wherein each phenyl is substituted with 0 to 3 groups selected from the group consisting of C₁-C₇ alkyl, C₃-C₈ cycloalkyl, Br, Cl, F, I, C₁-C₄ haloalkyl, nitro, C₁-C₄ alkoxy, C₁-C₄ haloalkoxy, C₁-C₄ alkylthio, C₁-C₄ alkyl sulfinyl, C₁-C₄ alkylsulfonyl, C₁-C₆ alkylamino and (C₁-C₄ alkyl)2amino;
with the proviso that when R₄₀₁ is unsubstituted C₁-C₁₀ alkyl, then R₄₀₃ is other than substituted or unsubstituted phenyl;
R₄₀₉ and R₄₁₀ are, at each occurrence, independently selected from the group consisting of H, C₁-C₄ alkyl, C₃-C₆ cycloalkyl- C₁-C₄ alkyl and C₃-C₈ cycloalkyl;
R₄₁₃ is selected from the group consisting of H, C₁-C₄ alkyl, C₁-C₄ haloalkyl, C₁-C₄ alkoxy- C₁-C₄ alkyl, C₃-C₆ cycloalkyl, C₃-C₆ cycloalkyl- C₁-C₆ alkyl, aryl³, aryl³(C₁-C₄ alkyl)-, heteroaryl³ and heteroaryl³(C₁-C₄ alkyl)-;
R₄₁₃ₐ and R₄₁₆ₐ are, at each occurrence, independently selected from the group consisting of H, C₁-C₄ alkyl, C₁-C₄ haloalkyl, C₁-C₄ alkoxy- C₁-C₄ alkyl, C₃-C₆ cycloalkyl, and C₃-C₆ cycloalkyl C₁-C₆ alkyl;
R₄₁₄ is selected from the group consisting of C₁-C₄ alkyl, C₁-C₄ haloalkyl, C₁-C₄ alkoxy- C₁-C₄ alkyl, C₃-C₆ cycloalkyl, C₃-C₆ cycloalkyl- C₁-C₆ alkyl, aryl³, aryl³(C₁-C₄ alkyl)-, heteroaryl³ and heteroaryl³(C₁-C₄ alkyl)- and benzyl, each benzyl being substituted on the aryl moiety with 0 to 1 substituent selected from the group consisting of C₁-C₄ alkyl, Br, Cl, F, I, C₁-C₄ haloalkyl, nitro, C₁-C₄ alkoxy C₁-C₄ haloalkoxy, and dimethylamino;
R₄₁₄ₐ is selected from the group consisting of C₁-C₄ alkyl, C₁-C₄ haloalkyl, C₁-C₄ alkoxy- C₁-C₄ alkyl, C₃-C₆ cycloalkyl, C₃-C₆ cycloalkyl- C₁-C₆ alkyl and benzyl, each benzyl being substituted on the aryl moiety with 0 to 1 substituents selected from the group consisting of C₁-C₄ alkyl, Br, Cl, F, I, C₁-C₄ haloalkyl, nitro, C₁-C₄ alkoxy, C₁-C₄ haloalkoxy, and dimethylamino;
R_{414b} is selected from the group consisting of C₁-C₄ alkyl, C₁-C₄ haloalkyl, C₁-C₄ alkoxy- C₁-C₄ alkyl, C3-C6 cycloalkyl, and C₃-C₆ cycloalkyl- C₁-C₆ alkyl;
R₄₁₅ is, at each occurrence, independently selected from the group consisting of H, C₁-C₄ alkyl, C₃-C₇ cycloalkyl, C₃-C₆ cycloalkyl- C₁-C₆ alkyl, phenyl and benzyl, each phenyl or benzyl being substituted on the aryl moiety with 0-3 groups chosen from the group consisting of C₁-C₄ alkyl, Br, Cl, F, I, C₁-C₄ haloalkyl, nitro, C₁-C₄ alkoxy, C₁-C₄ haloalkoxy, and dimethylamino;
R₄₁₅ₐ is, at each occurrence, independently selected from the group consisting of H, C₁-C₄ alkyl, C₃-C₇ cycloalkyl, and C₃-C₆ cycloalkyl- C₁-C₆ alkyl;
R₄₁₇ is, at each occurrence, selected from the group consisting of H, C₁-C₆ alkyl, C₃-C₁₀ cycloalkyl, C₃-C₆ cycloalkyl-C₁-C₆ alkyl, C₁-C₂ alkoxy -C₁-C₂ alkyl, C₁-C₄ haloalkyl, R₄₁₄S(O)n₅- C₁-C₄ alkyl, and R_{417b}R_{419b}N- C₂-C₄ alkyl;
R₄₁₈ and R₄₁₉ are, at each occurrence, independently selected from the group consisting of H, C₁-C₆ alkyl, C₃-C₁₀ cycloalkyl, C₃-C₆ cycloalkyl- C₁-C₆ alkyl, C₁-C₂ alkoxy- C₁-C₂ alkyl, and C₁-C₄ haloalkyl;
alternatively, in NR₄₁₇R₄₁₉ moiety, R₄₁₇ and R₄₁₉ taken together form 1-pyrrolidinyl, 1-morpholinyl, 1-piperidinyl or 1-piperazinyl, wherein N₄ in 1-piperazinyl is substituted with 0 to 1 substituent selected from the group consisting of R₄₁₃, CO₂R₄₁₄, COR₄₁₄ and SO₂R₄₁₄;
alternatively, in NR_{417b}R_{419b} moiety, R_{417b} and R_{419b} taken together form 1-pyrrolidinyl, 1-morpholinyl, 1-piperidinyl or 1-piperazinyl, wherein N₄ in 1-piperazinyl is substituted with 0 to 1 substituent selected from the group consisting of R₄₁₃, CO₂R₄₁₄, COR₄₁₄ and SO₂R₄₁₄;
R₄₁₇ₐ and R₄₁₉ₐ are, at each occurrence, independently selected from the group consisting of H, C₁-C₆ alkyl, C₃-C₁₀ cycloalkyl, C₃-C₆ cycloalkyl- C₁-C₆ alkyl and C₁-C₄ haloalkyl;
aryl³ is, at each occurrence, independently selected from the group consisting of phenyl, naphthyl, indanyl and indenyl, each aryl³ being substituted with 0 to 5 substituents optionally and independently selected from the group consisting of C₁-C₆ alkyl, C₃-C₆ cycloalkyl, methylenedioxy, C₁-C₄ alkoxy- C₁-C₄ alkoxy, -OR₄₁₇, Br, Cl, F, I, C₁-C₄ haloalkyl, -CN, -NO₂, SH, -S(O)n₅R₄₁₈, -COR₄₁₇, -CO₂R₄₁₇, -OC(O)R₄₁₈, -NR₄₁₅COR₄₁₇, -N (COR₄₁₇)₂, -NR₄₁₅CONR₄₁₇R₄₁₉, -NR₄₁₅CO₂R₄₁₈, -NR₄₁₇R₄₁₉, and -CONR₄₁₇R₄₁₉ and up to 1 phenyl, each phenyl substituent being substituted with 0 to 4 substituents selected from the group consisting of C₁-C₃ alkyl, C₁-C₃ alkoxy, Br, Cl, F, I, -CN, dimethylamino, CF₃, C₂F₅, OCF₃, SO₂Me and acetyl;
heteroaryl³ is, at each occurrence, independently selected from the group consisting of pyridyl, pyrimidinyl, triazinyl, furanyl, quinolinyl, isoquinolinyl, thienyl, imidazolyl, thiazolyl, indolyl, pyrrolyl, oxazolyl, benzofuranyl, benzothienyl, benzothiazolyl, benzoxazolyl, isoxazolyl, triazolyl, tetrazolyl, indazolyl, 2,3-dihydrobenzofuranyl, 2,3-dihydrobenzothienyl, 2,3-dihydrobenzothienyl-S-oxide, 2,3-dihydrobenzothienyl-S-dioxide, indolinyl, benzoxazolin-2-on-yl, benzodioxolanyl and benzodioxane, each heteroaryl³ being substituted on 0 to 4 carbon atoms with a substituent optionally and independently selected from the group consisting of C₁-C₆ alkyl, C₃-C₆ cycloalkyl, Br, Cl, F, I, C₁-C₄ haloalkyl, -CN, nitro, -OR₄₁₇, SH, -S(O)ₘ₃R₄₁₈, -COR₄₁₇, -CO₂R₄₁₇, -OC(O)R₄₁₈, -NR₄₁₅COR₄₁₇, -N(COR₄₁₇)₂, -NR₄₁₅CONR₄₁₇R₄₁₉, -NR₄₁₅CO₂R₄₁₈, -NR₄₁₇R₄₁₉, and -CONR₄₁₇R₄₁₉ and each heteroaryl being substituted on any nitrogen atom with 0 to 1 substituent selected from the group consisting of R₄₁₅, CO₂R₄₁₄ₐ, COR₄₁₄ₐ and SO₂R₄₁₄ₐ;
with the proviso that when D₈ is imidazole or triazole, then R₄₀₁ is other than unsubstituted linear or branched C₁-C₆ alkyl or C₃-C₆ cycloalkyl,
its stereoisomer or its pharmaceutically acceptable salt.

36. A pharmaceutical composition for prophylaxis or therapy of a postoperative stress as claimed in claim 1, wherein the compound having CRF antagonist activating effect is a compound represented by the formula Iae 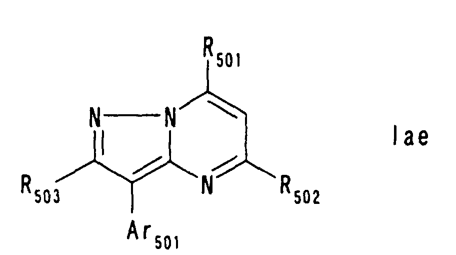 wherein
R₅₀₁ is NR₅₀₄R₅₀₅ or OR₅₀₅;
R₅₀₂ is C₁-C₆ alkyl, C₁-C₆ alkoxy or C₁-C₆ alkylthio;
R₅₀₃ is hydrogen, C₁-C₆ alkyl, C₁-C₆ alkylsulfonyl, C₁-C₆ alkylsulfoxy or C₁-C₆ alkylthio;
R₅₀₄ is hydrogen, C₁-C₆ alkyl, mono or di(C₃-C₆ cycloalkyl)methyl, C₃-C₆ cycloalkyl, C₃-C₆ alkenyl, hydroxy C₁-C₆ alkyl, C₁-C₆ alkylcarbonyloxy C₁-C₆ alkyl or C₁-C₆ alkyloxy C₁-C₆ alkyl;
R₅₀₅ is C₁-C₈ alkyl, mono or di(C₃-C₆ cycloalkyl)methyl, Ar₅₀₂CH₂, C₃-C₆ alkenyl, C₁-C₆ alkyloxy C₁-C₆ alkyl, hydroxy C₁-C₆ alkyl, thienylmethyl, furanylmethyl, C₁-C₆ alkylthio C₁-C₆ alkyl, morpholinyl, mono or di(C₁-C₆ alkyl)amino C₁-C₆ alkyl, di(C₁-C₆ alkyl)amino, C₁-C₆ alkylcarbonyl C₁-C₆ alkyl, C₁-C₆ alkyl substituted with imidazolyl; or a radical represented by the formula -Alk-O-CO-Ar₅₀₂;
or R₅₀₄ and R₅₀₅ taken together with the nitrogen to which they are attached may form a pyrrolidinyl, piperidinyl, homopiperidinyl or morpholinyl, optionally substituted with C₁-C₆ alkyl or C₁-C₆ alkyloxy C₁-C₆ alkyl; and
Ar₅₀₁ is phenyl, phenyl substituted with one, two or three substituents independently selected from halo C₁-C₆ alkyl, trifluoromethyl, hydroxy, cyano, C₁-C₆ alkyloxy, benzyloxy, C₁-C₆ alkylthio, nitro, amino and mono or di(C₁-C₆ alkyl)amino, pyridinyl, pyridinyl substituted with one, two or three substituents independently selected from halo, C₁-C₆ alkyl, trifluoromethyl, hydroxy, cyano, C₁-C₆ alkyloxy, benzyloxy, C₁-C₆ alkylthio, nitro, amino and mono or di(C₁-C₆ alkyl)amino and piperidinyl, and additionally wherein said substituted phenyl may optionally be substituted with one or more halogens;
Ar₅₀₂ is phenyl, phenyl substituted with one, two or three substituents each independently selected from halo, C₁-C₆ alkyl, C₁-C₆ alkyloxy, di(C₁-C₆ alkyl)amino C₁-C₆ alkyl, trifluoromethyl and C₁-C₆ alkyl substituted with morpholinyl, or pyridinyl; and
Alk is C₁-C₆ alkanediyl; with the proviso that 5-methyl-3-phenyl-7-(phenylmethoxy)-pyrazolo(1,5-a)-pyrimidine and 2,5-dimethyl-7-(methylamino)-3-phenyl-pyrazolo(1,5-a)-pyrim idine are not included,
its stereoisomer or its pharmaceutically acceptable acid addition salt.

37. A pharmaceutical composition for prophylaxis or therapy of a postoperative stress as claimed in claim 1, wherein the compound having CRF antagonist activating effect is a compound represented by the formula Iaf 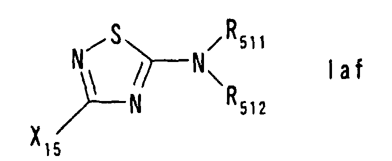 wherein R₅₁₁ and R₅₁₂ are substituents independently selected from a group consisting of C₁-C₆ alkyl, C₃-C₆ alkenyl, C₂-C₆ alkylether, C₂-C₆ alkylthioether, C₄-C₉ cycloalkylalkyl, C₇-C₂₀ dicycloalkylalkyl, C₇-C₂₀ cycloalkylarylalkyl, C₇-C₂₀ arylalkyl, C₇-C₂₀ diarylalkyl, C₃-C₁₄ heteroaryl and C₃-C₁₄ heteroarylalkyl, and substituted derivatives thereof; and
X₁₅ is a substituent selected from substituted C₃-C₁₄ monocyclic or fused homoaryl or heteroaryl groups, its stereoisomer or pharmaceutically acceptable salt.

38. A pharmaceutical composition for prophylaxis or therapy of a postoperative stress as claimed in claim 1, wherein the compound having CRF antagonist activating effect is a compound represented by the formula Iag or Iah 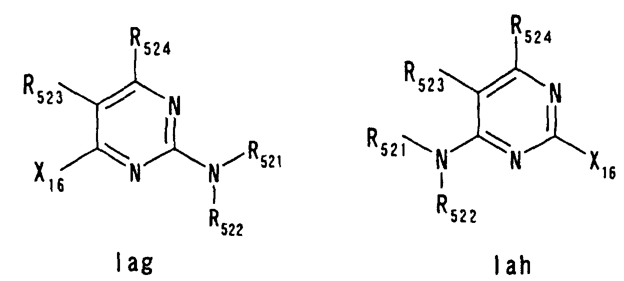 wherein R₅₂₁ and R₅₂₂ are independently selected from a group consisting of C₁-C₆ alkyl, C₃-C₆ alkenyl, C₂-C₆ alkylether, C₂-C₆ alkylthioether, C₄-C₉ cycloalkylalkyl, C₇-C₂₀ dicycloalkylalkyl, C₇-C₂₀ cycloalkylarylalkyl, C₇-C₂₀ arylalkyl, C₇-C₂₀ diarylalkyl, C₃-C₁₄ heteroaryl and C₃-C₁₄ heteroarylalkyl, and substituted derivatives thereof;
R₅₂₃ and R₅₂₄ are independently selected from a group consisting of hydrogen, amino, C₁-C₆ substituted amino, halogen, C₁-C₂ alkyl, C₁-C₆ carbonyl-containing groups and C₁-C₆ sulfur containing groups; and
X₁₆ is selected from substituted C₃-C₁₄ monocyclic or fused homoaryl or heteroaryl,
its stereoisomer or its pharmaceutically acceptable salt.

39. A pharmaceutical composition for prophylaxis or therapy of a postoperative stress as claimed in claim 1, wherein the compound having CRF antagonist activating effect is a compound represented by the formula Iai 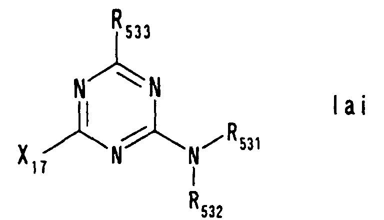 wherein R₅₃₁ and R₅₃₂ are independently selected from C₁-C₆ alkyl, C₃-C₆ alkenyl, C₂-C₆ alkylether, C₂-C₆ alkylthioether, C₄-C₉ cycloalkylalkyl, C₇-C₂₀ dicycloalkylalkyl, C₇-C₂₀ cycloalkylarylalkyl, C₇-C₂₀ arylalkyl, C₇-C₂₀ diarylalkyl, C₃-C₁₄ heteroaryl and C₃-C₁₄ heteroarylalkyl, and substituted derivatives thereof;
R₅₃₃ is selected from hydrogen, amino, C₁-C₆ alkyl and C₁-C₆ alkoxy; and
X₁₇ is selected from substituted C₃-C₁₄ monocyclic or fused homoaryl or heteroaryl,
its stereoisomer or its pharmaceutically acceptable salt.

40. A pharmaceutical composition for prophylaxis or therapy of a postoperative stress as claimed in claim 1, wherein the compound having CRF antagonist activating effect is a compound represented by the formula Iaj or Iak 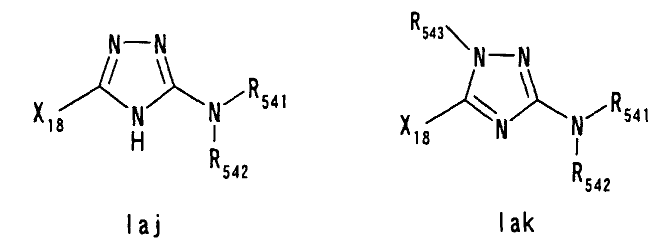 wherein R₅₄₁ and R₅₄₂ are independently selected from C₁-C₆ alkyl, C₃-C₆ alkenyl, C₂-C₆ alkylether, C₂-C₆ alkylthioether, C₄-C₉ cycloalkylalkyl, C₇-C₂₀ dicycloalkylalkyl, C₇-C₂₀ cycloalkylarylalkyl, C₇-C₂₀ arylalkyl, C₇-C₂₀ diarylalkyl, C₃-C₁₄ heteroaryl and C₃-C₁₄ heteroarylalkyl, and substituted derivatives thereof;
R₅₄₃ is a radical selected from hydrogen or methyl; and
X₁₈ is selected from substituted C₃-C₁₄ monocyclic or fused homoaryl or heteroaryl,
its stereoisomer or its pharmaceutically acceptable salt.

41. A pharmaceutical composition for the prophylaxis or therapy of a postoperative stress, wherein the compound having CRF antagonist activating effect is a compound represented by any one of the formula Ial 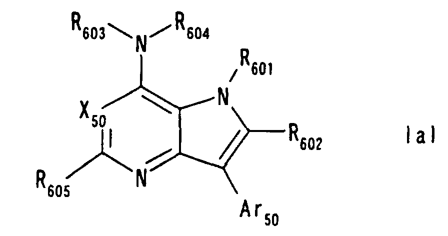 wherein Ar₅₀ is phenyl, 2-, 3- or 4- pyridyl, 2- or 3-thienyl,
4- or 5-pyrimidyl, each of which is mono-, di- or trisubstituted with halogen, hydroxy and lower alkyl, or lower alkoxy, with the proviso that at least one of the ortho positions of the Ar₅₀ substituent is substituted;
X₅₀ is CH or nitrogen;
R₆₀₁ is lower alkyl;
R₆₀₂ is hydrogen, halogen, lower alkyl, lower alkoxy or thioalkoxy; or
R₆₀₁ and R₆₀₂ taken together represent -(CH₂)n₅-A10-(CH₂)m₅-, wherein n5 is 2, 3 or 4, A10 is methylene, oxygen, sulfur or NR_{606,} wherein R₆₀₆ is lower alkyl, and m5 is 0, 1 or 2;
R₆₀₃ and R₆₀₄ are the same or different and represent hydrogen or lower alkyl;
phenyl, 2-, 3- or 4-pyridyl, 2- or 3-thienyl or 2-, 4-or 5-pyrimidyl, each of which is mono- or disubstituted with halogen, hydroxy, lower alkyl or lower alkoxy; phenyl lower alkyl, 2-, 3- or 4-pyridyl lower alkyl, 2-or 3-thienyl lower alkyl or 2-, 4- or 5-pyrimidyl lower alkyl;
cycloalkyl having 3-8 carbon atoms or cycloalkyl lower alkyl where the cycloalkyl portion has 3-8 carbon atoms; 2-hydroxyethyl or 3-hydroxypropyl optionally mono or di substituted with lower alkyl with the proviso that not both R₆₀₃ and R₆₀₄ are hydrogen; or
R₆₀₃ and R₆₀₄ taken together represent -(CH₂)ₙ₆-A11-(CH₂)m₆- wherein n6 is 2 or 3,
A11 is methylene, 1, 2-phenylene, oxygen, sulfur or NR₆₀₆ wherein R₆₀₇ is lower alkyl, phenyl, 2-, 3- or 4-pyridyl, 2- or 3-thienyl or 2-, 4- or 5-pyrimidyl, phenyl lower alkyl, 2-, 3- or 4-pyridyl lower alkyl, 2- or 3-thienyl lower alkyl, or 2-, 4- or 5-pyrimidyl lower alkyl, and m6 is 1, 2 or 3 and
R₆₀₅ is hydrogen, halogen, lower alkyl, lower alkoxy or thioalkoxy,
the formula Iam 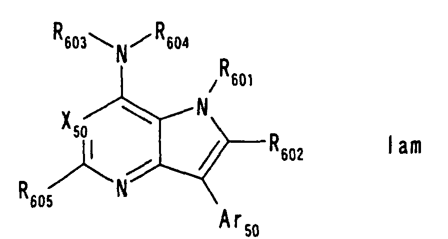 wherein Ar₅₀ is phenyl, 2-, 3- or 4-pyridyl, 2- or 3-thienyl, 4- or 5-pyrimidyl, each of which is mono-, di-, or trisubstituted with halogen, hydroxy, lower alkyl, or lower alkoxy, with the proviso that at least one of the ortho positions of the Ar₅₀ substituent is substituted;
X₅₀ is CH or nitrogen;
R₆₀₁ is lower alkyl;
R₆₀₂ is hydrogen, halogen, lower alkyl, lower alkoxy, or thioalkoxy; or
R₆₀₃ and R₆₀₄ are the same or different represent hydrogen or lower alkyl;
phenyl, 2-, 3- or 4-pyridyl, 2- or 3-thienyl or 2-, 4-or 5-pyrimidyl, each of which is mono- or disubstituted with halogen, hydroxy, lower alkyl or lower alkoxy;
phenyl lower alkyl, 2-, 3- or 4-pyridyl lower alkyl, 2-or 3-thienyl lower alkyl or 2-, 4- or 5-pyrimidyl lower alkyl;
cycloalkyl having 3 to 8 carbon atoms, or cycloalkyl lower alkyl where the cycloalkyl portion has 3 to 8 carbon atoms;
2-hydroxyethyl or 3-hydroxypropyl optionally mono or disubstituted with lower alkyl with the proviso that not both R₆₀₃ and R₆₀₄ are hydrogen; or
R₆₀₃ and R₆₀₄ taken together represent -(CH₂)ₙ₆-A12-(CH₂)ₘ₆- where n6 is 2 or 3,
A12 is methylene, 1, 2-phenylene, oxygen, sulfur or NR_{606,} wherein R₆₀₆ is lower alkyl, and
m6 is 1, 2 or 3, and
R₆₀₅ is hydrogen, halogen, lower alkyl, lower alkoxy or thioalkoxy,
the formula Ian 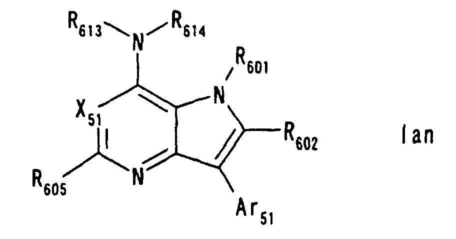 wherein Ar₅₁ is a phenyl mono-, di-, or trisubstituted with halogen, hydroxy, lower alkyl or lower alkoxy, with the proviso that at least one of the ortho positions of the Ar₅₁ substituent is substituted;
X₅₁ is nitrogen;
R₆₀₁ is lower alkyl;
R₆₀₂ is hydrogen, halogen, lower alkyl, lower alkoxy or thioalkoxy; or
R₆₁₃ and R₆₁₄ are the same or different and represent hydrogen or lower alkyl;
cycloalkyl having 3 to 8 carbon atoms, or cycloalkyl lower alkyl where the cycloalkyl portion has 3 to 8 carbon atoms; 2-hydroxyethyl or 3-hydroxypropyl optionally mono or disubstituted with lower alkyl with the proviso that not both R₆₁₃ and R₆₁₄ are hydrogen; or
R₆₁₃ and R₆₁₄ taken together represent -(CH₂)ₙ₆-A12-(CH₂)ₘ₆- where n5 is 2 or 3,
A12 is methylene, 1,2phenylene, oxygen, sulfur or NR₆₀₆, wherein R₆₀₆ is lower alkyl, and
m6 is 1, 2 or 3;
R₆₀₅ is hydrogen, halogen, lower alkyl, lower alkoxy or thioalkoxy, and
the formula Iao 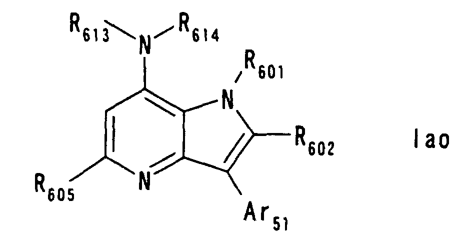 wherein Ar₅₁ is phenyl mono-, di-, or trisbstituted with halogen, hydroxy, lower alkyl or lower alkoxy, with the proviso that at least one of the ortho positions of the Ar₅₁ substituent is substituted;
R₆₀₁ is lower alkyl;
R₆₀₂ is hydrogen, halogen, lower alkyl, lower alkoxy or thioalkoxy; or
R₆₁₃ and R₆₁₄ are the same or different and represent hydrogen or lower alkyl;
cycloalkyl having 3 to 8 carbon atoms or cycloalkyl lower alkyl where cycloalkyl portion has 3 to 8 carbon atoms;
2-hydroxyethyl or 3-hydroxypropyl optionally mono or disubstituted with lower alkyl with the proviso that not both R₆₁₃ and R₆₁₄ are hydrogen; or
R₆₁₃ and R₆₁₄ taken together represent -(CH₂)ₙ₆-A12-(CH₂)ₘ₆- wherein n6 is 2 or 3,
A12 is methylene, 1, 2-phenylene, oxygen, sulfur or NR₆₀₆
wherein R₆₀₆ is lower alkyl, and
m6 is 1, 2 or 3, and
R₆₀₅ is hydrogen, halogen, lower alkyl, lower alkoxy or thioalkoxy,
or its pharmaceutically acceptable salts.

42. A pharmaceutical composition for prophylaxis or therapy of a postoperative stress as claimed in claim 1, wherein the compound having CRF antagonist activating effect is a compound represented by the formula Iap 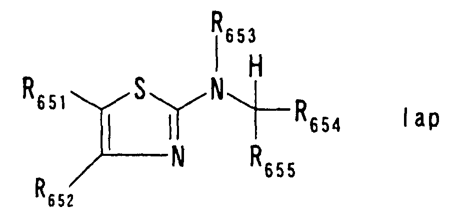 wherein R₆₅₁ is hydrogen or C₁-C₅ alkyl;
R₆₅₂ is a radical represented by the formula (A20): 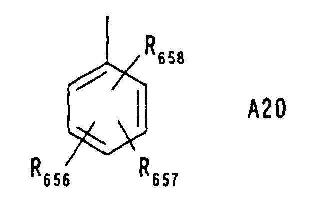 wherein R₆₅₆ is C₁-C₅ hydroxyalkyl, and R₆₅₇ and R₆₅₈, which may be the same or different, each represents hydrogen, halogen or C₁-C₅ hydroxyalkyl, trifluoromethyl, C₁-C₅ alkoxy or C₁-C₅ alkyl, a radical represented by the formula (B20): 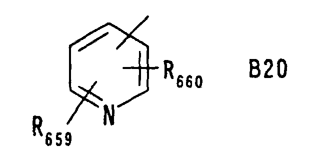 wherein R₆₅₉ and R₆₆₀, which may be the same or different, each represents hydrogen, halogen, C₁-C₅ alkyl or C₁-C₅ alkoxy, 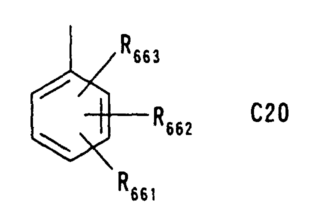 wherein R₆₆₁, R₆₆₂ and R₆₆₃, which may be the same or different, each represents hydrogen, halogen, trifluoromethyl, C₁-C₅ alkoxy or a C₁-C₅ alkyl,
R₆₅₃ is C₁-C₅ alkyl, C₁-C₅ hydroxyalkyl, 2-tetrahydropyranyloxyalkyl wherein the alkyl moiety comprises one to five carbon atoms, C₂-C₁₀ alkoxyalkyl or C₃-C₁₁ acyloxyalkyl;
R₆₅₄ is C₃-C₆ cycloalkyl, C₁-C₅ hydroxyalkyl, C₂-C₁₀ alkoxyalkyl, C₄-C₁₁ cycloalkyloxyalkyl, C₂-C₁₀ hydroxyalkyloxyalkyl, C₃-C₁₂ alkoxyalkyloxyalkyl, C₃-C₁₁ acyloxyalkyl or C₂-C₁₀ alkylthioalkyl; and
R₆₅₅ is C₃-C₆ cycloalkyl, phenyl, thienyl or pyridyl, which may be optionally substituted with one or more halogens, C₁-C₅ alkoxy, C₁-C₅ alkyl or trifluoromethyl, or a radical represented by the formula (D20): 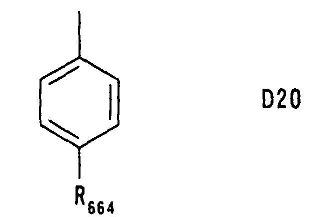 wherein R₆₆₄ is carboxyl, C₂-C₆ carboxyalkyl, C₂-C₆ alkoxycarbonyl, C₃-C₁₁ acyloxyalkyl, C₂-C₁₀ alkoxyalkyl, C₈-C₁₆ aralkoxyalkyl, wherein optionally substituted on the aromatic ring with one or more halogens, C₁-C₃ alkoxy or trifluoromethyl, monohalogenated C₁-C₅ alkyl, linear or branched C₁-C₅ hydroxyalkyl, a radical represented by the formula (E20): 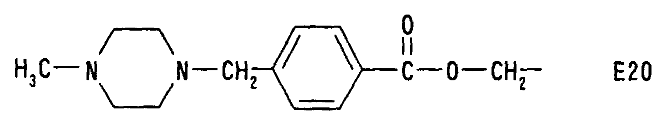 or C₁-C₅ sulfoxyalkyl,
3-hydroxyalkyl-6-pyridyl or 2-hydroxyalkyl-5-pyridyl, wherein the alkyl contain one to five carbon atoms, with the proviso that when R₆₅₃ is C₁-C₅ alkyl, R₆₅₄ is cycloalkyl and R₆₅₅ is either cycloalkyl or phenyl, thienyl or pyridyl, which may be optionally substituted with one or more halogens, C₁-C₅ alkoxy, C₁-C₅ alkyl or trifluoromethyl, then R₆₅₂ is not a radical represented by the formula (C20),
its stereoisomer or its additional salt.

43. A pharmaceutical composition for prophylaxis or therapy of a postoperative stress as claimed in claim 1, wherein the compound having CRF antagonist activating effect is a compound represented by Iaq 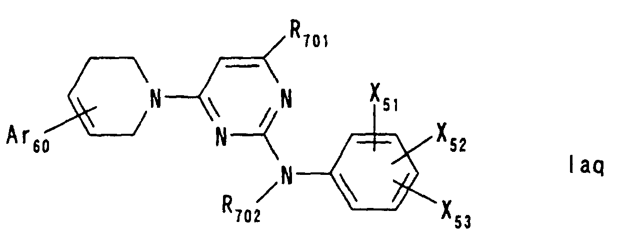 wherein Ar₆₀ represents phenyl substituted with one to three substituents selected from halogen, C₁-C₅ alkyl, C₁-C₅ alkoxy and trifluoromethyl, phenyl, thienyl or furanyl; R₇₀₁ represents hydrogen, C₁-C₅ alkyl, amino or amino substituted with one or two C₁-C₅ alkyl groups; R₇₀₂ represents C₁-C₅ alkyl, C₄-C₇ cycloalkylalkyl, C₂-C₅ alkenyl or C₂-C₅ alkynyl; and X₅₁, X₅₂ and X₅₃, which may be the same or different, each represent hydrogen, halogen, C₁-C₅ alkyl, C₁-C₅ alkoxy, C₁-C₅ alkylthio, amino or amino substituted with one or two C₁-C₅ alkyl groups, or its pharmaceutically acceptable salt.

44. A pharmaceutical composition for prophylaxis or therapy of a postoperative stress as claimed in claim 1, wherein the compound having CRF antagonist activating effect is a compound represented by the formula Iar, 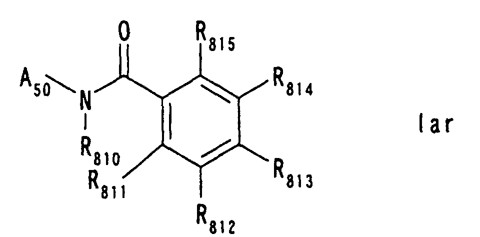 wherein A₅₀ is a group represented by the formula Ias; 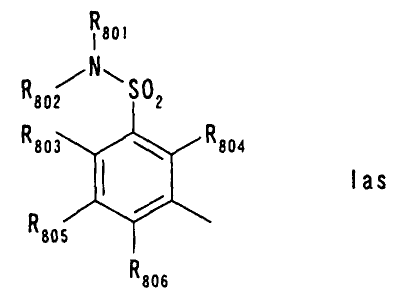 (wherein R₈₀₁ and R₈₀₂ may be the same or different and represent
hydrogen;
C₁-C₄ lower alkyl which may be substituted with substituent selected from hydroxy, C₁-C₄ lower alkoxy, C₃-C₇ cycloalkyl and aryl;
halogenated C₁-C₄ lower alkyl;
C₃-C₇ cycloalkyl or
aryl, or
R₈₀₁ and R₈₀₂ taken together may form a saturated 5 to 6 membered heterocycle ring, which may be substituted by one to four C₁-C₄ lower alkyls and which optionally may contain nitrogen, oxygen or sulfur in addition to nitrogen adjacent to R₈₀₁ and R₈₀₂,
R₈₀₃ and R ₈₀₄ may be the same or different and represent
hydrogen;
halogen;
C₁-C₄ lower alkyl;
C₁-C₄ lower alkoxy or
amino which may be substituted with substituent selected from C₁-C₄ lower alkyl and C₁-C₄ lower alkylsulfonyl,
R₈₀₅ is hydrogen;
halogen;
C₁-C₄ lower alkyl which may be substituted with substituent selected from hydroxy and C₁-C₄ lower alkoxy;
halogenated C₁-C₄ lower alkyl;
C₁-C₄ lower alkoxy which may be substituted with substituent selected from halogen and C₃-C₇ cycloalkyl;
C₂-C₅ lower alkanoyl;
nitro;
cyano;
carbamoyl, which may be substituted with C₁-C₄ lower alkyl;
C₁-C₄ lower alkylsulfonyl;
carboxy;
C₂-C₅ lower alkoxycarbonyl;
amino, which may be substituted with substituent selected from a group consisting of C₁-C₄ lower alkyl which may be substituted with substituent selected from halogen atom and C₃-C₇ cycloalkyl, C₂-C₅ lower alkanoyl, and C₃-C₇ cycloalkyl;
a saturated 5 to 6 membered heterocycle ring having nitrogen;
C₃-C₇ cycloalkyl or
C₃-C₇ cycloalkoxy,
R₈₀₆ is hydrogen;
halogen atom;
C₁-C₄ lower alkyl;
amino, which may be substituted with C₁-C₄ lower alkyl;
cyano;
carboxy;
C₂-C₅ lower alkoxycarbonyl;
C₂-C₅ lower alkanoyl;
carbamoyl;
C₁-C₄ lower alkylthio;
C₁-C₄ lower alkyl sulfinyl;
C₁-C₄ lower alkylsulfonyl;
C₃-C₇ cycloalkyl;
a saturated 5 to 6 membered heterocycle ring having one to two heteroatoms selected from nitrogen, oxygen and sulfur or
-OR₈₁₆, wherein R₈₁₆ is hydrogen;
C₁-C₆ alkyl, which may be substituted with substituent selected from hydroxy, C₁-C₄ lower alkoxy, triflouromethyl, cyano, amino which may be substituted with C₁-C₄ lower alkyl, carboxy and C₂-C₅ lower alkoxycarbonyl;
C₂-C₄ lower alkenyl;
C₂-C₄ lower alkynyl;
carbamoyl which may be substituted with C₁-C₄ lower alkenyl;
C₁-C₄ lower alkylsulfonyl;
phosphono;
C₃-C₇ cycloalkyl;
aryl or benzyl, or
R₈₀₅ and R₈₁₆ may be taken together to form a saturated 5 to 6 membered heterocyclic ring which may be substituted with C₁-C₄ lower alkyl or oxo),
or the formula Iat 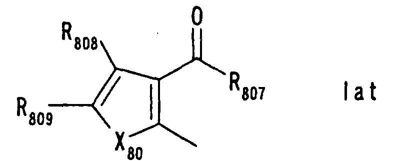 (wherein R₈₀₇ is
hydroxy;
C₁-C₄ lower alkoxy or -NR₈₁₇R₈₁₈ wherein
R₈₁₇ and R₈₁₈ may be the same or different and represent hydrogen;
C₁-C₄ lower alkyl;
C₁-C₄ lower alkoxy or benzyl, or
R₈₁₇ and R₈₁₈ may be taken together to form a saturated 5 to 6 membered heterocyclic ring, which at each occurrence may have nitrogen, oxygen and sulfur in addition to nitrogen adjacent to R₈₁₇ and R₈₁₈,
R₈₀₈ is C₁-C₄ lower alkyl, which may be substituted with a substituent selected from C₁-C₄ lower alkoxy and amino which may be substituted with C₁-C₄ lower alkyl,
R₈₀₉ is aminosulfonyl, which may be substituted with C₁-C₄ lower
alkyl or
-COR₈₁₉ wherein R₈₁₉ is hydroxy;
C₁-C₄ lower alkyl;
C₁-C₄ lower alkoxy;
C₃-C₇ cycloalkyl;
aryl or -NR₈₂₀R₈₂₁
wherein R₈₂₀ and R₈₂₁ may be the same or different and represent hydrogen;
C₁-C₄ lower alkyl;
C₃-C₇ cycloalkyl;
aryl or
benzyl, or
R₈₂₀ and R₈₂₁ may be taken together to form a saturated 5 to 6 membered heterocyclic ring, which may have nitrogen, oxygen or sulfur in addition to nitrogen adjacent to R₈₂₀ and R₈₂₁,
X₈₀ is -S-;
-O- or
-NH-),
R₈₁₀ is hydrogen;
C₁-C₄ lower alkyl, which may be substituted with substituent selected from carboxy and C₂-C₅ lower alkoxycarbonyl,
R₈₁₁ is hydrogen;
halogen atom or hydroxy,
R₈₁₂ is hydrogen;
halogen atom or nitro,
R₈₁₃ represents
-OR₈₂₂ wherein R₈₂₂ is
hydrogen;
alkali metal or phosphono,
-YR₈₂₃ wherein Y represents -O- or -NH-, and R₈₂₃ is C₁-C₄ lower alkyl which may be substituted with substituent selected from aryl, carboxy and C₂-C₅ lower alkoxycarbonyl;
aryl which may be substituted with carboxy or carboxy,
R₈₁₄ is hydrogen;
halogen atom;
nitro;
triflouromethyl;
amino which may be substituted with C₁-C₄ lower alkyl or C₁-C₄ lower alkylsulfonyl;
aminosulfonyl;
C₁-C₄ lower alkylsulfonyl;
C₂-C₅ lower alkanoyl;
carboxy;
C₂-C₅ lower alkoxycarbonyl;
carbamoyl which may be substituted with hydroxy or cyano,
R₈₁₅ is hydrogen;
halogen atom or
hydroxy,
its pharmaceutical acceptable salt, hydrate or solvate.

45. A pharmaceutical composition for prophylaxis or therapy of a postoperative stress as claimed in claim 1, wherein the compound having CRF antagonist activating effect is a compound represented by each of the following formula 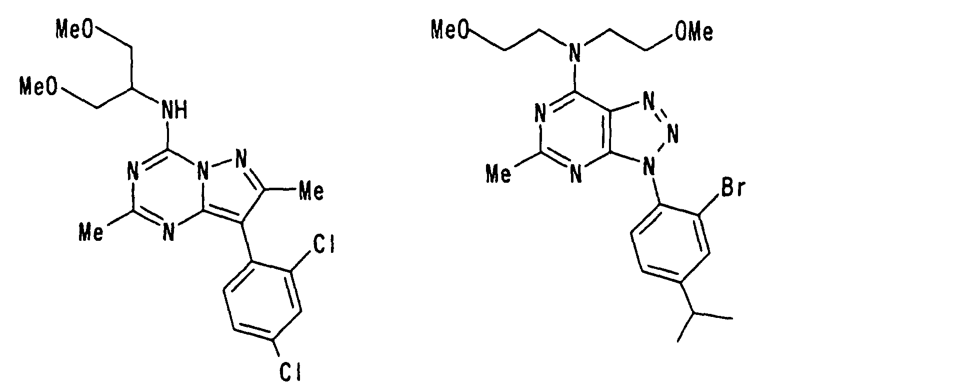 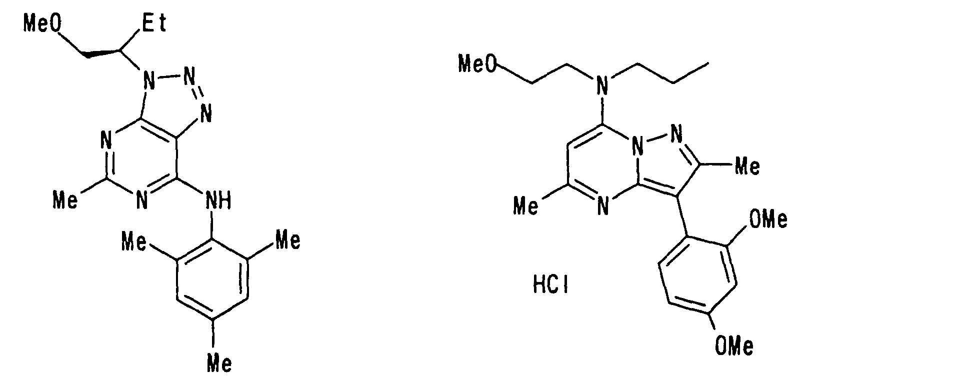 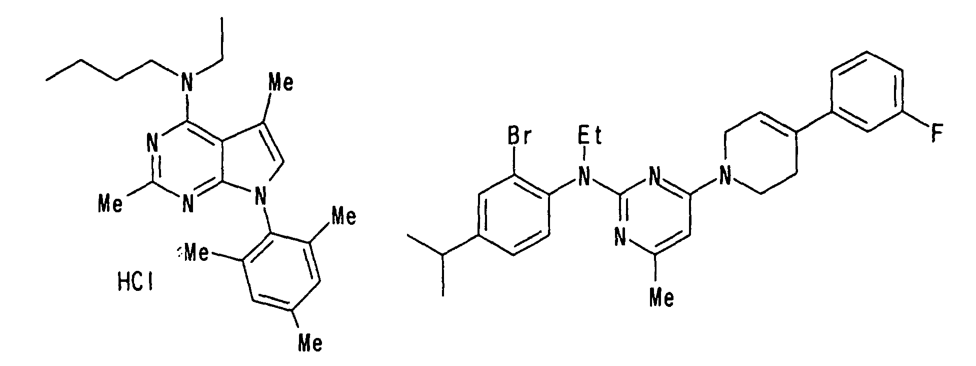 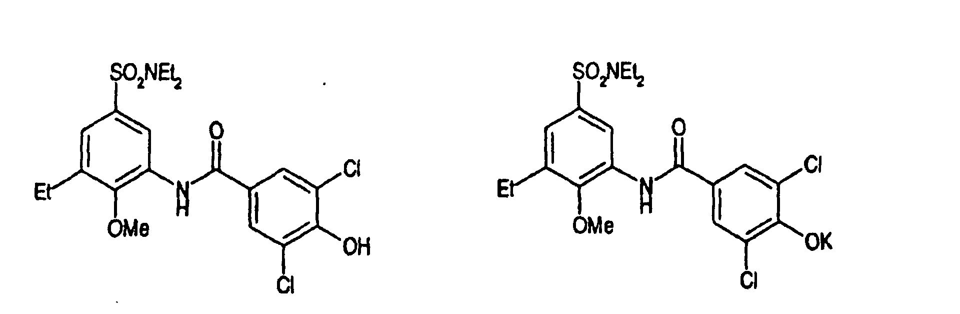 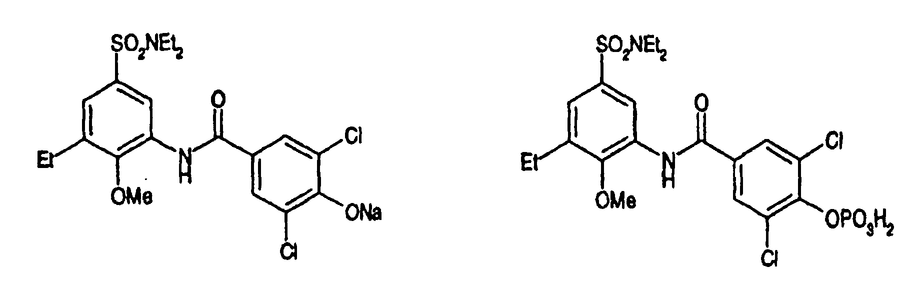 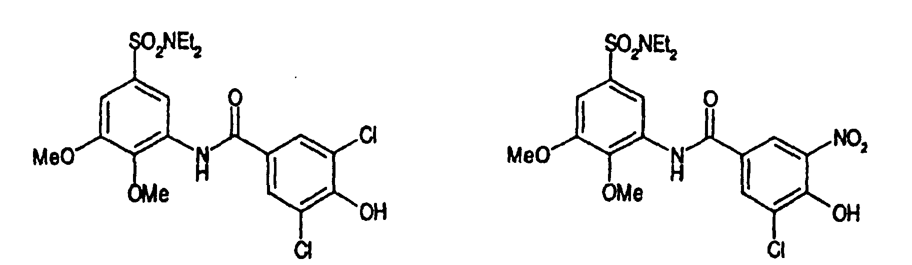 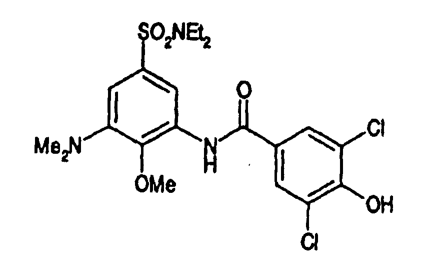 wherein Me and Et are each methyl and ethyl, or its pharmaceutically acceptable salt.

46. A pharmaceutical composition for prophylaxis or therapy of a postoperative stress as claimed in claim 1, wherein the compound having CRF antagonist activating effect is a compound represented by one of the following development numbers: DMP-695; DPC-696; SG-058; SK-390; SP904; XQ-041; IL-488; NBI-27728; NBI-29356; NBI-30525; NBI-30775; NBI-34041; NBI-35965; NBI-37582; NGD-98-1 and CRA-0165, or its pharmaceutically acceptable salt.
